# EUROPÄISCHE PATENTSCHRIFT

(11) **EP 2 816 896 B1**
(45) Veröffentlichungstag und Bekanntmachung des Hinweises auf die Patenterteilung: **07.09.2016**
(21) Anmeldenummer: 13704465.7
(22) Anmeldetag: 18.02.2013
(51) Int. Cl.: A01N 41/02, A01N 43/56, A01N 43/80, C07C 313/06, C07D 231/20, C07D 261/08, A01P 13/00

(54) **HERBIZID WIRKSAME SULFINIMIDOYL- UND SULFONIMIDOYLBENZOYLDERIVATE**
HERBICIDALLY ACTIVE SULFINIMIDOYL- AND SULFONIMIDOYLBENZOYL DERIVATIVES
DÉRIVÉS HERBICIDES PORTANT UN GROUPEMENT SULFINIMIDOYL- OU SULFONIMIDOYLBENZOYL

(30) Priorität: 21.02.2012 EP 12156310
(43) Veröffentlichungstag der Anmeldung: 31.12.2014
(73) Patentinhaber: Bayer Intellectual Property GmbH, 40789 Monheim (DE)
(72) Erfinder: AHRENS, Hartmut, 63329 Egelsbach (DE); DÖRNER-RIEPING, Simon, 61267 Neu-Anspach (DE); LEHR, Stefan, 69006 Lyon (FR); DIETRICH, Hansjörg, 65835 Liederbach am Taunus (DE); GATZWEILER, Elmar, 61231 Bad Nauheim (DE); ROSINGER, Christopher Hugh, 65719 Hofheim am Taunus (DE); SCHMUTZLER, Dirk, 65795 Hattersheim (DE)
(74) Vertreter: BIP Patents
(86) Internationale Anmeldenummer: PCT/EP2013/053151
(87) Internationale Veröffentlichungsnummer: WO 2013/124230

(56) Entgegenhaltungen:
- WO-A1-03/014071
- WO-A1-2004/052849
- WO-A1-2008/125214
- WO-A1-2011/012247
- WO-A2-2009/149806

## Beschreibung

Die Erfindung betrifft das technische Gebiet der Herbizide, insbesondere das der Herbizide zur selektiven Bekämpfung von Unkräutern und Ungräsern in Nutzpflanzenkulturen.

Aus WO 03/014071 A1 und WO 2011/012247 A1 sind herbizid wirksame Benzoylcyclohexandione bekannt, die in 3-Position des Phenylrings eine Alkylsulfenyl-, Alkylsulfinyl- oder Alkylsulfonyl-Gruppe tragen. Aus WO 2008/125214 A1 und WO 2009/149806 A2 sind herbizid wirksame Benzoylpyrazole bekannt, die in 3-Position des Phenylrings eine Alkylsulfenyl-, Alkylsulfinyl- oder Alkylsulfonyl-Gruppe tragen. Aus US 2011/0144345 A1 und WO 2004/052849 A1 sind jeweils Benzoylderivate bekannt, die in 3-Position des Phenylrings eine Sulfoxyimino-Gruppe tragen. Die herbizide Wirksamkeit und/oder die Kulturpflanzenverträglichkeit der in diesen Schriften genannten Verbindungen ist jedoch nicht immer ausreichend.

Aufgabe der vorliegenden Erfindung war die Bereitstellung von weiteren herbizid wirksamen Verbindungen.

Es wurde nun gefunden, dass Benzoylderivate, die in 3-Position des Phenylrings eine Sulfin- oder Sulfonimidoyl-Gruppe tragen, als Herbizide besonders gut geeignet sind.

Ein Gegenstand der vorliegenden Erfindung sind somit Sulfinimidoyl- und Sulfonimidoylbenzoylderivate der Formel (I) oder deren Salze 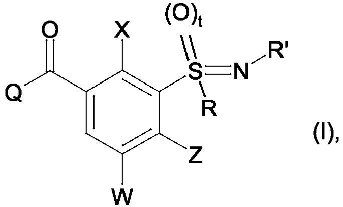 worin die Symbole und Indizes folgende Bedeutungen haben:
Q bedeutet einen Rest Q1, Q2, Q3, Q4 oder Q5, 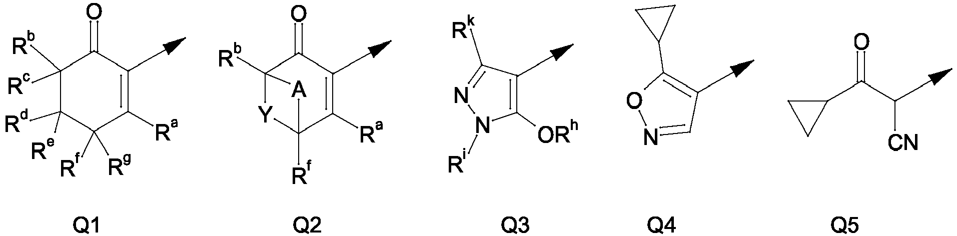
R^{a} bedeutet Hydroxy, R⁶S, R⁷(R⁸)N,
R^{b}, R^{c}, R^{f} und R^{g} bedeuten unabhängig voneinander jeweils Wasserstoff oder (C₁-C₄)-Alkyl,
R^{d}, R^{e} bedeuten unabhängig voneinander jeweils Wasserstoff oder (C₁-C₄)-Alkyl oder bilden gemeinsam mit dem Kohlenstoffatom, an das sie gebunden sind, eine Carbonylgruppe,
R^{h} bedeutet Wasserstoff, (C₁-C₆)-Alkylsulfonyl, (C₁-C₄)-Alkoxy-(C₁-C₆)-alkylsulfonyl, Phenylsulfonyl, Thiophenyl-2-sulfonyl, Benzoyl, Benzoyl-(C₁-C₆)-alkyl, Benzyl, wobei die fünf letztgenannten Reste gegebenenfalls einfach oder mehrfach durch Halogen, (C₁-C₄)-Alkyl oder (C₁-C₄)-Alkoxy substituiert sein können,
Rⁱ bedeutet (C₁-C₄)-Alkyl,
R^{k} bedeutet Wasserstoff, (C₁-C₄)-Alkyl, (C₃-C₇)-Cycloalkyl,
A und Y bedeuten unabhängig voneinander jeweils Sauerstoff, S(O)ₙ, N(R³), Carbonyl oder durch n Reste R⁹ substituiertes (C₁-C₄)-Alkylen, das durch n Elemente aus der Gruppe bestehend aus Sauerstoff, S(O)ₙ, N(R³) und Carbonyl unterbrochen ist,
X bedeutet Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkenyl, Halogen-(C₃-C₆)-cycloalkenyl, (C₃-C₆)-Cycloalkyl-(C₁-C₆)-alkyl, Halogen-(C₃-C₆)-cycloalkyl-(C₁-C₆)-alkyl, (C₃-C₆)-Cycloalkenyl-(C₁-C₆)-alkyl, Halogen-(C₃-C₆)-cycloalkenyl-(C₁-C₆)-alkyl, R¹(O)C, R¹(R¹ON=)C, R¹O(O)C, (R¹)₂N(O)C, R¹(R¹O)N(O)C, (R¹)₂N(R¹)N(O)C, R¹(O)C(R¹)N(O)C, R²O(O)C(R¹)N(O)C, (R¹)₂N(O)C(R¹)N(O)C, R²(O)₂S(R¹)N(O)C, R¹O(O)₂S(R¹)N(O)C, (R¹)₂N(O)₂S(R¹)N(O)C, R¹O, R¹(O)CO, R²(O)₂SO, R²O(O)CO, (R¹)₂N(O)CO, (R¹)₂N, R¹(O)C(R¹)N, R²(O)₂S(R¹)N, R²O(O)C(R¹)N, (R¹)₂N(O)C(R¹)N, R¹O(O)₂S(R¹)N, (R¹)₂N(O)₂S(R¹)N, R²(O)ₙS, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S, (R⁵O)₂(O)P, R¹(O)C-(C₁-C₆)-Alkyl, R¹O(O)C-(C₁-C₆)-Alkyl, (R¹)₂N(O)C-(C₁-C₆)-Alkyl, (R¹O)(R¹)N(O)C-(C₁-C₆)-Alkyl, (R¹)₂N(R¹)N(O)C-(C₁-C₆)-Alkyl, R¹(O)C(R¹)N(O)C-(C₁-C₆)-Alkyl, R²O(O)C(R¹)N(O)C-(C₁-C₆)-Alkyl, (R¹)₂N(O)C(R¹)N(O)C-(C₁-C₆)-Alkyl, R²(O)₂S(R¹)N(O)C-(C₁-C₆)-Alkyl, R¹O(O)₂S(R¹)N(O)C-(C₁-C₆)-Alkyl, (R¹)₂N(O)₂S(R¹)N(O)C-(C₁-C₆)-Alkyl, NC-(C₁-C₆)-Alkyl, R¹O-(C₁-C₆)-Alkyl, R¹(O)CO-(C₁-C₆)-Alkyl, R²(O)₂SO-(C₁-C₆)-Alkyl, R²O(O)CO-(C₁-C₆)-Alkyl, (R¹)₂N(O)CO-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R²O(O)C(R¹)N-(C₁-C₆)-Alkyl, (R¹)₂N(O)C(R¹)N-(C₁-C₆)-Alkyl, R¹O(O)₂S(R¹)N-(C₁-C₆)-Alkyl, (R¹)₂N(O)₂S(R¹)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, R¹(O)C(R¹)N(O)₂S-(C₁-C₆)-Alkyl, R²O(O)C(R¹)N(O)₂S-(C₁-C₆)-Alkyl, (R¹)₂N(O)C(R¹)N(O)₂S-(C₁-C₆)-Alkyl, (R⁵O)₂(O)P-(C₁-C₆)-Alkyl, Phenyl, Heteroaryl, Heterocyclyl, Phenyl-(C₁-C₆)-alkyl, Heteroaryl-(C₁-C₆)-alkyl, Heterocyclyl-(C₁-C₆)-alkyl, wobei die sechs letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl substituiert sind, und wobei Heterocyclyl n Oxogruppen trägt,
Z bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkenyl, Halogen-(C₃-C₆)-cycloalkenyl, (C₃-C₆)-Cycloalkyl-(C₁-C₆)-alkyl, Halogen-(C₃-C₆)-cycloalkyl-(C₁-C₆)-alkyl, (C₃-C₆)-Cycloalkenyl-(C₁-C₆)-alkyl, Halogen-(C₃-C₆)-cycloalkenyl-(C₁-C₆)-alkyl, R¹(O)C, R¹(R¹ON=)C, R¹O(O)C, (R¹)₂N(O)C, R¹(R¹O)N(O)C, (R¹)₂N(R¹)N(O)C, R¹(O)C(R¹)N(O)C, R²O(O)C(R¹)N(O)C, (R¹)₂N(O)C(R¹)N(O)C, R²(O)₂S(R¹)N(O)C, R¹O(O)₂S(R¹)N(O)C, (R¹)₂N(O)₂S(R¹)N(O)C, R¹O, R¹(O)CO, R²(O)₂SO, R²O(O)CO, (R¹)₂N(O)CO, (R¹)₂N, R¹(O)C(R¹)N, R²(O)₂S(R¹)N, R²O(O)C(R¹)N, (R¹)₂N(O)C(R¹)N, R¹O(O)₂S(R¹)N, (R¹)₂N(O)₂S(R¹)N, R²(O)ₙS, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S, (R⁵O)₂(O)P, R¹(O)C-(C₁-C₆)-Alkyl, R¹O(O)C-(C₁-C₆)-Alkyl, (R¹)₂N(O)C-(C₁-C₆)-Alkyl, (R¹O)(R¹)N(O)C-(C₁-C₆)-Alkyl, (R¹)₂N(R¹)N(O)C-(C₁-C₆)-Alkyl, R¹(O)C(R¹)N(O)C-(C₁-C₆)-Alkyl, R²O(O)C(R¹)N(O)C-(C₁-C₆)-Alkyl, (R¹)₂N(O)C(R¹)N(O)C-(C₁-C₆)-Alkyl, R²(O)₂S(R¹)N(O)C-(C₁-C₆)-Alkyl, R¹O(O)₂S(R¹)N(O)C-(C₁-C₆)-Alkyl, (R¹)₂N(O)₂S(R¹)N(O)C-(C₁-C₆)-Alkyl, NC-(C₁-C₆)-Alkyl, R¹O-(C₁-C₆)-Alkyl, R¹(O)CO-(C₁-C₆)-Alkyl, R²(O)₂SO-(C₁-C₆)-Alkyl, R²O(O)CO-(C₁-C₆)-Alkyl, (R¹)₂N(O)CO-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R²O(O)C(R¹)N-(C₁-C₆)-Alkyl, (R¹)₂N(O)C(R¹)N-(C₁-C₆)-Alkyl, R¹O(O)₂S(R¹)N-(C₁-C₆)-Alkyl, (R¹)₂N(O)₂S(R¹)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, R¹(O)C(R¹)N(O)₂S-(C₁-C₆)-Alkyl, R²O(O)C(R¹)N(O)₂S-(C₁-C₆)-Alkyl, (R¹)₂N(O)C(R¹)N(O)₂S-(C₁-C₆)-Alkyl, (R⁵O)₂(O)P-(C₁-C₆)-Alkyl, Phenyl, Heteroaryl, Heterocyclyl, Phenyl-(C₁-C₆)-alkyl, Heteroaryl-(C₁-C₆)-alkyl, Heterocyclyl-(C₁-C₆)-alkyl, wobei die sechs letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl substituiert sind, und wobei Heterocyclyl n Oxogruppen trägt,
W bedeutet Wasserstoff, Halogen, Nitro, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₇)-Cycloalkyl, Halogen-(C₃-C₇)-cycloalkyl, (C₁-C₆)-Alkoxy, Halogen-(C₁-C₆)-alkoxy, (C₁-C₆)-Alkyl-(O),S-, (C₁-C₆)-Halogenalkyl-(O)ₙS-, (C₁-C₆)-Alkoxy-(C₁-C₄)-alkyl, (C₁-C₆)-Alkoxy-(C₁-C₄)-halogenalkyl, R¹(O)C, R¹(R¹ON=)C, R¹O(O)C, (R¹)₂N, R¹(O)C(R¹)N oder R²(O)₂S(R¹)N,
R bedeutet jeweils durch s Reste aus der Gruppe bestehend aus Nitro, Halogen, Cyano, Rhodano, (C₃-C₆)-Cycloalkyl, R¹(O)C, R¹(R¹ON=)C, R¹O(O)C, (R¹)₂N(O)C, R¹(R¹O)N(O)C, R²(O)₂S(R¹)N(O)C, R¹O(O)₂S(R¹)N(O)C, (R¹)₂N(O)₂S(R¹)N(O)C, R¹S(O)C, R¹O, R¹(O)CO, R²(O)₂SO, R²O(O)CO, (R¹)₂N(O)CO, (R¹)₂N, R¹O(R¹)N, R¹(O)C(R¹)N, R²(O)₂S(R¹)N, R²O(O)C(R¹)N, (R¹)₂N(O)C(R¹)N, R¹O(O)₂S(R¹)N, (R¹)₂N(O)₂S(R¹)N, R²(O)ₙS, R¹C(O)S, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S und (R⁵O)₂(O)P substituiertes (C₁-C₆)-Alkyl, (C₂-C₆)-Alkenyl oder (C₂-C₆)-Alkinyl, oder
   jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹(O)C, R¹(R¹ON=)C, R¹O(O)C, (R¹)₂N(O)C, R¹(R¹O)N(O)C, R²(O)₂S(R¹)N(O)C, R¹O(O)₂S(R¹)N(O)C, (R¹)₂N(O)₂S(R¹)N(O)C, R¹S(O)C, R¹O, R¹(O)CO, R²(O)₂SO, R²O(O)CO, (R¹)₂N(O)CO, (R¹)₂N, R¹O(R¹)N, R¹(O)C(R¹)N, R²(O)₂S(R¹)N, R²O(O)C(R¹)N, (R¹)₂N(O)C(R¹)N, R¹O(O)₂S(R¹)N, (R¹)₂N(O)₂S(R¹)N, R²(O)ₙS, R¹C(O)S, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S, (R⁵O)₂(O)P und R¹O-(C₁-C₆)-Alkyl im cyclischen Teil substituiertes (C₃-C₆)-Cycloalkyl, (C₃-C₆)-Cycloalkenyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, Phenyl-N(R¹)-(C₁-C₆)-alkyl, Heteroaryl- N(R¹)-(C₁-C₆)-alkyl, Heterocyclyl- N(R¹)-(C₁-C₆)-alkyl, Phenyl-S(O)ₙ-(C₁-C₆)-alkyl, Heteroaryl-S(O)ₙ-(C₁-C₆)-alkyl oder Heterocyclyl-S(O)ₙ-(C₁-C₆)-alkyl, und wobei Heterocyclyl n Oxogruppen trägt,
R' bedeutet Wasserstoff, Nitro, Halogen, Cyano, (C₁-C₆)-Alkyl, Halogen-(C₁-C₆)-alkyl, (C₃-C₆)-Alkenyl, Halogen-(C₃-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C, R²O(O)C, (R¹)₂N(O)C, R²S(O)C, (R¹)₂N(S)C, R¹(R¹O)N(O)C, R²(O)₂S(R¹)N(O)C, (R¹)₂N(O)₂S(R¹)N(O)C, R¹O, (R¹)₂N, R²(O)ₙS, (R²)₃Si-(C₁-C₆)-Alkyl-(O)ₙS, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S, R²(O)₂S(R¹)N(O)₂S, (R⁵O)₂(O)P, (R²)₃Si, R¹(O)C-(C₁-C₆)-Alkyl, R¹O(O)C-(C₁-C₆)-Alkyl, (R¹)₂N(O)C-(C₁-C₆)-Alkyl, (R¹O)(R¹)N(O)C-(C₁-C₆)-Alkyl, R²(O)₂S(R¹)N(O)C-(C₁-C₆)-Alkyl, R¹O(O)₂S(R¹)N(O)C-(C₁-C₆)-Alkyl, (R¹)₂N(O)₂S(R¹)N(O)C-(C₁-C₆)-Alkyl, R¹O-(C₁-C₆)-Alkyl, R¹(O)CO-(C₁-C₆)-Alkyl, R²(O)₂SO-(C₁-C₆)-Alkyl, R²O(O)CO-(C₁-C₆)-Alkyl, (R¹)₂N(O)CO-(C₁-C₆)-Alkyl, (R¹)2N-(C₁-C₆)-Alkyl, R¹(O)C(R¹)N-(C₁-C₆)-Alkyl, R²(O)₂S(R¹)N-(C₁-C₆)-Alkyl, R²O(O)C(R1)N-(C₁-C₆)-Alkyl, (R¹)₂N(O)C(R¹)N-(C₁-C₆)-Alkyl, R¹O(O)₂S(R¹)N-(C₁-C₆)-Alkyl, (R¹)₂N(O)₂S(R¹)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, R¹(O)C(R¹)N(O)₂S-(C₁-C₆)-Alkyl, R²O(O)C(R¹)N(O)₂S-(C₁-C₆)-Alkyl, (R¹)₂N(O)C(R¹)N(O)₂S-(C₁-C₆)-Alkyl, (R⁵O)₂(O)P-(C₁-C₆)-Alkyl, (R²)₃Si-(C₁-C₆)-Alkyl, Phenyl, Heteroaryl, Heterocyclyl, Phenyl-(C₁-C₆)-alkyl, Heteroaryl-(C₁-C₆)-alkyl, Heterocyclyl-(C₁-C₆)-alkyl, wobei die sechs vorstehend genannten Phenyl-, Heteroaryl- und Heterocyclylreste im cyclischen Teil jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl substituiert sind, und wobei Heterocyclyl n Oxogruppen trägt,
R¹ bedeutet Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, Phenyl-N(R³)-(C₁-C₆)-alkyl, Heteroaryl-N(R³)-(C₁-C₆)-alkyl, Heterocyclyl-N(R³)-(C₁-C₆)-alkyl, Phenyl-S(O)ₙ-(C₁-C₆)-alkyl, Heteroaryl-S(O)ₙ-(C₁-C₆)-alkyl, Heterocyclyl-S(O)ₙ-(C₁-C₆)-alkyl, wobei die fünfzehn letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R³O(O)C, (R³)₂N(O)C, R³O, (R³)₂N, R⁴(O)ₙS, R³O(O)₂S, (R³)₂N(O)₂S und R³O-(C₁-C₆)-Alkyl substituiert sind, und wobei Heterocyclyl n Oxogruppen trägt,
R² bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, Phenyl-N(R³)-(C₁-C₆)-alkyl, Heteroaryl-N(R³)-(C₁-C₆)-alkyl, Heterocyclyl-N(R³)-(C₁-C₆)-alkyl, Phenyl-S(O)ₙ-(C₁-C₆)-alkyl, Heteroaryl-S(O)ₙ-(C₁-C₆)-alkyl, Heterocyclyl-S(O)ₙ-(C₁-C₆)-alkyl, wobei die fünfzehn letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R³O(O)C, (R³)₂N(O)C, R³O, (R³)₂N, R⁴(O)ₙS, R³O(O)₂S, (R³)₂N(O)₂S und R³O-(C₁-C₆)-Alkyl substituiert sind, und wobei Heterocyclyl n Oxogruppen trägt,
R³ bedeutet Wasserstoff, (C₁-C₆)-Alkyl, Halogen-(C₁-C₆)-alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl,
R⁴ bedeutet (C₁-C₆)-Alkyl, Halogen-(C₁-C₆)-alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl,
R⁵ bedeutet Wasserstoff oder (C₁-C₄)-Alkyl,
R⁶ bedeutet (C₁-C₄)-Alkyl oder durch m Reste aus der Gruppe bestehend aus Halogen, Nitro, Cyano, (C₁-C₄)-Alkyl, (C₁-C₄)-Halogenalkyl, (C₁-C₄)-Alkoxy und (C₁-C₄)-Halogenalkoxy substituiertes Phenyl,
R⁷ bedeutet Wasserstoff, (C₁-C₄)-Alkyl oder (C₁-C₄)-Alkoxy,
R⁸ bedeutet Wasserstoff oder (C₁-C₄)-Alkyl,
oder
R⁷ und R⁸ bilden gemeinsam mit dem Stickstoffatom, an das sie gebunden sind, einen 5- oder 6-gliedrigen gesättigten, partiell gesättigten oder ungesättigten Ring, der zusätzlich n Heteroatome aus der Gruppe bestehend aus Stickstoff, Sauerstoff und Schwefel enthält und der durch m Reste aus der Gruppe bestehend aus Halogen, Cyano, (C₁-C₄)-Alkyl, (C₁-C₄)-Halogenalkyl, (C₁-C₄)-Alkoxy und (C₁-C₄)-Halogenalkoxy substituiert ist,
R⁹ bedeutet Halogen, (C₁-C₄)-Alkyl, (C₁-C₄)-Halogenalkyl, (C₁-C₄)-Alkoxy, (C₁-C₄)-Halogenalkoxy oder (C₁-C₄)-Alkoxy-(C₁-C₄)-alkyl,
m bedeutet 0, 1, 2, 3, 4 oder 5,
n bedeutet 0, 1 oder 2,
s bedeutet 0, 1, 2 oder 3,
t bedeutet 0 oder 1.

In der Formel (I) und allen nachfolgenden Formeln können Alkylreste mit mehr als zwei Kohlenstoffatomen geradkettig oder verzweigt sein. Alkylreste bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl. Analog bedeutet Alkenyl z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl. Alkinyl bedeutet z.B. Propargyl, But-2-in-1-yl, But-3-in-1-yl, 1-Methyl-but-3-in-1-yl. Die Mehrfachbindung kann sich jeweils in beliebiger Position des ungesättigten Rests befinden. Cycloalkyl bedeutet ein carbocyclisches, gesättigtes Ringsystem mit drei bis sechs C-Atomen, z.B. Cyclopropyl, Cyclobutyl, Cyclopentyl oder Cyclohexyl. Analog bedeutet Cycloalkenyl eine monocyclische Alkenylgruppe mit drei bis sechs Kohlenstoffringgliedern, z.B. Cyclopropenyl, Cyclobutenyl, Cyclopentenyl und Cyclohexenyl, wobei sich die Doppelbindung an beliebiger Position befinden kann.

Halogen steht für Fluor, Chlor, Brom oder Iod.

Heterocyclyl bedeutet einen gesättigten, teilgesättigten oder vollständig ungesättigten cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heterocyclyl für Piperidinyl, Pyrrolidinyl, Tetrahydrofuranyl, Dihydrofuranyl und Oxetanyl,

Heteroaryl bedeutet einen aromatischen cyclischen Rest, der 3 bis 6 Ringatome enthält, von denen 1 bis 4 aus der Gruppe Sauerstoff, Stickstoff und Schwefel stammen, und der zusätzlich durch einen Benzoring annelliert sein kann. Beispielsweise steht Heteroaryl für Benzimidazol-2-yl, Furanyl, Imidazolyl, Isoxazolyl, Isothiazolyl, Oxazolyl, Pyrazinyl, Pyrimidinyl, Pyridazinyl, Pyridinyl, Benzisoxazolyl, Thiazolyl, Pyrrolyl, Pyrazolyl, Thiophenyl, 1,2,3-Oxadiazolyl, 1,2,4-Oxadiazolyl, 1,2,5-Oxadiazolyl, 1,3,4-Oxadiazolyl, 1,2,4-Triazolyl, 1,2,3-Triazolyl, 1,2,5-Triazolyl, 1,3,4-Triazolyl, 1,2,4-Triazolyl, 1,2,4-Thiadiazolyl, 1,3,4-Thiadiazolyl, 1,2,3-Thiadiazolyl, 1,2,5-Thiadiazolyl, 2H-1,2,3,4-Tetrazolyl, 1H-1,2,3,4-Tetrazolyl, 1,2,3,4-Oxatriazolyl, 1,2,3,5-Oxatriazolyl, 1,2,3,4-Thiatriazolyl und 1,2,3,5-Thiatriazolyl.

Ist eine Gruppe mehrfach durch Reste substituiert, so ist darunter zu verstehen, daß diese Gruppe durch ein oder mehrere gleiche oder verschiedene der genannten Reste substituiert ist. Analoges gilt für den Aufbau von Ringsystemen durch verschiedene Atome und Elemente. Dabei sollen solche Verbindungen vom Anspruchsbegehren ausgenommen sein, von denen der Fachmann weiß, dass sie unter Normalbedingungen chemisch instabil sind.

Die Verbindungen der allgemeinen Formel (I) können je nach Art und Verknüpfung der Substituenten als Stereoisomere vorliegen. Sind beispielsweise ein oder mehrere asymmetrisch substituierte Kohlenstoffatome vorhanden, so können Enantiomere und Diastereomere auftreten. Ebenso treten Stereoisomere auf, wenn in der Gruppierung S(O)ₙ n für 1 steht (Sulfoxide). Außerdem liegt das Schwefelatom in der Sulfoximinogruppe bzw. in der Sulfiliminogruppe als chirales Zentrum vor. Stereoisomere lassen sich aus den bei der Herstellung anfallenden Gemischen nach üblichen Trennmethoden, beispielsweise durch chromatographische Trennverfahren, erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft auch alle Stereoisomeren und deren Gemische, die von der allgemeinen Formel (I) umfasst, jedoch nicht spezifisch definiert sind.

Die Verbindungen der Formel (I) können Salze bilden. Salzbildung kann durch Einwirkung einer Base auf solche Verbindungen der Formel (I) erfolgen, die ein acides Wasserstoffatom tragen, z.B. in dem Fall, dass R^{a} für eine Hydroxygruppe oder R^{h} für Wasserstoff stehen. Geeignete Basen sind beispielsweise organische Amine , wie Trialkylamine, Morpholin, Piperidin oder Pyridin sowie Ammonium-, Alkali- oder Erdalkalimetallhydroxide, -carbonate und -hydrogencarbonate, insbesondere Natrium- und Kaliumhydroxid, Natrium- und Kaliumcarbonat und Natrium- und Kaliumhydrogencarbonat. Diese Salze sind Verbindungen, in denen der acide Wasserstoff durch ein für die Landwirtschaft geeignetes Kation ersetzt wird, beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze, zum Beispiel mit Kationen der Formel [NRR*R**R***]⁺, worin R, R*, R** und R***unabhängig voneinander jeweils einen organischen Rest, insbesondere Alkyl, Aryl, Aralkyl oder Alkylaryl darstellen. Infrage kommen auch Alkylsulfonium- und Alkylsulfoxoniumsalze, wie (C₁-C₄)-Trialkylsulfonium- und (C₁-C₄)-Trialkylsulfoxoniumsalze.

Die Verbindungen der Formel (I) können durch Anlagerung einer geeigneten anorganischen oder organischen Säure, wie beispielsweise Mineralsäuren, wie beispielsweise HCl, HBr, H₂SO₄, H₃PO₄ oder HNO₃, oder organische Säuren, z. B. Carbonsäuren, wie Ameisensäure, Essigsäure, Propionsäure, Oxalsäure, Milchsäure oder Salicylsäure oder Sulfonsäuren, wie zum Beispiel p-Toluolsulfonsäure, an eine basische Gruppe, wie z.B. Amino, Alkylamino, Dialkylamino, Piperidino, Morpholino oder Pyridino, Salze bilden. Diese Salze enthalten dann die konjugierte Base der Säure als Anion.

Bevorzugt sind Verbindungen der allgemeinen Formel (I), worin
Q bedeutet einen Rest Q1, Q2, Q3, Q4 oder Q5, 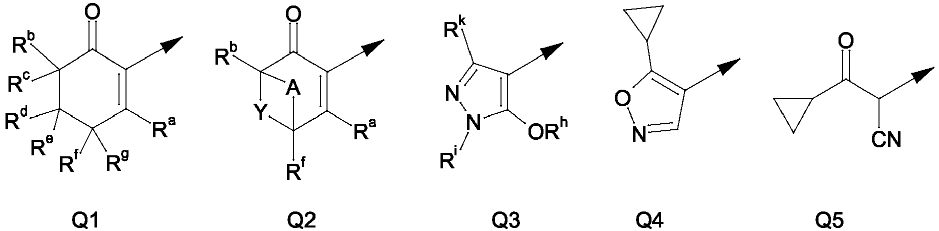
R^{a} bedeutet Hydroxy,
R^{b}, R^{c}, R^{f} und R^{g} bedeuten unabhängig voneinander jeweils Wasserstoff oder (C₁-C₄)-Alkyl,
R^{d}, R^{e} bedeuten unabhängig voneinander jeweils Wasserstoff oder (C₁-C₄)-Alkyl oder bilden gemeinsam mit dem Kohlenstoffatom, an das sie gebunden sind, eine Carbonylgruppe,
R^{h} bedeutet Wasserstoff,
Rⁱ bedeutet (C₁-C₄)-Alkyl,
R^{k} bedeutet Wasserstoff, (C₁-C₄)-Alkyl, (C₃-C₇)-Cycloalkyl,
A und Y bedeuten unabhängig voneinander jeweils Sauerstoff oder durch n Reste R⁹ substituiertes (C₁-C₄)-Alkylen,
X bedeutet Nitro, Halogen, Cyano, (C₁-C₆)-Alkyl, Halogen-(C₁-C₆)-alkyl, (C₂-C₆)-Alkenyl, (C₂-C₆)-Alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C, R¹(R¹ON=)C, R¹O(O)C, (R¹)₂N(O)C, R¹O, (R¹)₂N, R¹(O)C(R¹)N, R²(O)₂S(R¹)N, R²O(O)C(R¹)N, (R¹)₂N(O)C(R¹)N, R²(O)ₙS, R¹O(O)₂S, (R¹)₂N(O)₂S, (R⁵O)₂(O)P, R¹(O)C-(C₁-C₆)-Alkyl, R¹O(O)C-(C₁-C₆)-Alkyl, (R¹)₂N(O)C-(C₁-C₆)-Alkyl, NC-(C₁-C₆)-Alkyl, R¹O-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R²O(O)C(R¹)N-(C₁-C₆)-Alkyl, (R¹)₂N(O)C(R¹)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, (R⁵O)₂(O)P-(C₁-C₆)-Alkyl, Phenyl, Heteroaryl, Heterocyclyl, Phenyl-(C₁-C₆)-alkyl, Heteroaryl-(C₁-C₆)-alkyl, Heterocyclyl-(C₁-C₆)-alkyl, wobei die sechs letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, R¹O, (R¹)₂N, R²(O)ₙS, R¹O(O)₂S, (R¹)₂N(O)₂S und R¹O-(C₁-C₆)-Alkyl substituiert sind und wobei Heterocyclyl n Oxogruppen trägt,
Z bedeutet Wasserstoff, Nitro, Halogen, Cyano, (C₁-C₆)-Alkyl, Halogen-(C₁-C₆)-alkyl, (C₂-C₆)-Alkenyl, (C₂-C₆)-Alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C, R¹(R¹ON=)C, R¹O(O)C, (R¹)₂N(O)C, R¹O, (R¹)₂N, R¹(O)C(R¹)N, R²(O)₂S(R¹)N, R²O(O)C(R¹)N, (R¹)₂N(O)C(R¹)N, R²(O)ₙS, R¹O(O)₂S, (R¹)₂N(O)₂S, (R⁵O)₂(O)P, R¹(O)C-(C₁-C₆)-Alkyl, R'0(0)C-(C₁-C₆)-Alkyl, (R¹)₂N(O)C-(C₁-C₆)-Alkyl, NC-(C₁-C₆)-Alkyl, R¹O-(C₁-C₆)-Alkyl, (R¹)₂N-(C₁-C₆)-Alkyl, R¹(O)C(R¹)N-(C₁-C₆)-Alkyl, R²(O)₂S(R1)N-(C₁-C₆)-Alkyl, R²O(O)C(R¹)N-(C₁-C₆)-Alkyl, (R¹)₂N(O)C(R1)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, (R⁵O)₂(O)P-(C₁-C₆)-Alkyl, Phenyl, Heteroaryl, Heterocyclyl, Phenyl-(C₁-C₆)-alkyl, Heteroaryl-(C₁-C₆)-alkyl, Heterocyclyl-(C₁-C₆)-alkyl, wobei die sechs letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, R¹O, (R¹)₂N, R²(O)ₙS, R¹O(O)₂S, (R¹)₂N(O)₂S und R¹O-(C₁-C₆)-Alkyl substituiert sind und wobei Heterocyclyl n Oxogruppen trägt,
W bedeutet Wasserstoff, Halogen, Nitro, Cyano, (C₁-C₆)-Alkyl, Halogen-(C₁-C₆)-alkyl, (C₃-C₇)-Cycloalkyl, (C₁-C₆)-Alkoxy, (C₁-C₆)-Alkyl-(O)ₙS-, R¹O(O)C, (R¹)₂N, R¹(O)C(R¹)N oder R²(O)₂S(R¹)N,
R bedeutet jeweils durch s Reste aus der Gruppe bestehend aus Halogen, Cyano, (C₃-C₆)-Cycloalkyl, R¹(O)C, R¹(R¹ON=)C, R¹O(O)C, (R¹)₂N(O)C, R²(O)₂S(R¹)N(O)C, R¹O, (R¹)₂N, R¹(O)C(R¹)N, R²(O)₂S(R¹)N, R²O(O)C(R¹)N, (R¹)₂N(O)C(R¹)N, R²(O)ₙS, R¹O(O)₂S, (R¹)₂N(O)₂S, R¹(O)C(R¹)N(O)₂S, R²O(O)C(R¹)N(O)₂S und (R¹)₂N(O)C(R¹)N(O)₂S substituiertes (C₁-C₆)-Alkyl oder
   jeweils durch s Reste aus der Gruppe bestehend aus Halogen, (C₁-C₆)-Alkyl, Halogen-(C₁-C₆)-alkyl, (C₃-C₆)-Cycloalkyl, R¹O(O)C und (R¹)₂N(O)C substituiertes (C₃-C₆)-Cycloalkyl,
R' bedeutet Wasserstoff, Nitro, Cyano, (C₁-C₆)-Alkyl, Halogen-(C₁-C₆)-alkyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C, R²O(O)C, (R¹)₂N(O)C, R²(O)₂S, R¹(O)C-(C₁-C₆)-Alkyl, R¹O(O)C-(C₁-C₆)-Alkyl, (R¹)₂N(O)C-(C₁-C₆)-Alkyl, R¹O-(C₁-C₆)-Alkyl, (R¹)₂N-(C₁-C₆)-Alkyl, R²(O)ₙS-(C₁-C₆)-Alkyl,
R¹ bedeutet Wasserstoff, (C₁-C₆)-Alkyl, Halogen-(C₁-C₆)-alkyl, (C₃-C₆)-Cycloalkyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, wobei die neun letztgenannten Reste jeweils durch s Reste aus der Gruppe bestehend aus Nitro, Halogen, (C₁-C₆)-Alkyl, Halogen-(C₁-C₆)-alkyl, R³O(O)C, (R³)₂N(O)C, R³O, (R³)₂N, R⁴(O)ₙS und R³O-(C₁-C₆)-Alkyl substituiert sind, und wobei Heterocyclyl n Oxogruppen trägt,
R² bedeutet (C₁-C₆)-Alkyl, Halogen-(C₁-C₆)-alkyl, (C₃-C₆)-Cycloalkyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, wobei die neun letztgenannten Reste jeweils durch s Reste aus der Gruppe bestehend aus Nitro, Halogen, (C₁-C₆)-Alkyl, Halogen-(C₁-C₆)-alkyl, R³O(O)C, (R³)₂N(O)C, R³O, (R³)₂N, R⁴(O)ₙS und R³O-(C₁-C₆)-Alkyl substituiert sind, und wobei Heterocyclyl n Oxogruppen trägt,
R³ bedeutet Wasserstoff oder (C₁-C₆)-Alkyl,
R⁴ bedeutet (C₁-C₆)-Alkyl,
R⁵ bedeutet Wasserstoff oder (C₁-C₄)-Alkyl,
R⁹ bedeutet Halogen, (C₁-C₄)-Alkyl, (C₁-C₄)-Halogenalkyl, (C₁-C₄)-Alkoxy, (C₁-C₄)-Halogenalkoxy oder (C₁-C₄)-Alkoxy-(C₁-C₄)-alkyl,
n bedeutet 0, 1 oder 2,
s bedeutet 0, 1, 2 oder 3,
t bedeutet 0 oder 1.

Besonders bevorzugt sind Verbindungen der allgemeinen Formel (I), worin
Q bedeutet einen Rest Q1, Q2, Q3, Q4 oder Q5, 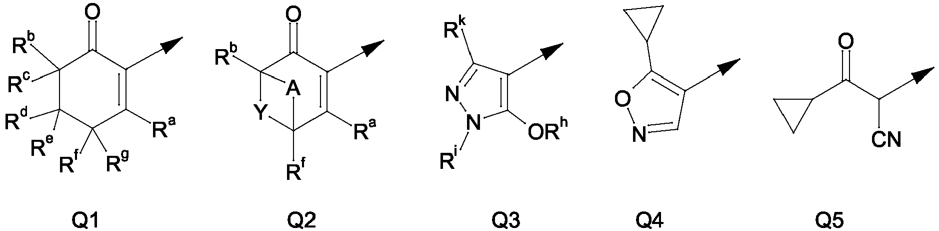
R^{a} bedeutet Hydroxy,
R^{b}, R^{c}, R^{f} und R^{g} bedeuten unabhängig voneinander jeweils Wasserstoff oder Methyl,
R^{d}, R^{e} bedeuten Wasserstoff oder bilden gemeinsam mit dem Kohlenstoffatom, an das sie gebunden sind, eine Carbonylgruppe,
R^{h} bedeutet Wasserstoff,
Rⁱ bedeutet Methyl oder Ethyl,
R^{k} bedeutet Wasserstoff, Methyl oder Cyclopropyl,
A und Y bedeuten unabhängig voneinander jeweils CH₂ oder CH₂CH₂,
X bedeutet Nitro, Halogen, Methyl, Ethyl, n-Propyl, iso-Propyl, Trifluormethyl, Difluormethyl, Chlordifluormethyl, Dichlorfluormethyl, Trichlormethyl, Pentafluorethyl, Heptafluorisopropyl, Cyclopropyl, Methoxy, Ethoxy, Methylsulfanyl, Methylsulfinyl, Methylsulfonyl, Methoxymethyl, Ethoxymethyl, Methoxyethyl, Methoxyethoxymethyl, Methylthiomethyl, Methylsulfinylmethyl oder Methylsulfonylmethyl,
Z bedeutet Wasserstoff, Nitro, Cyano, Halogen, Methyl, Ethyl, n-Propyl, iso-Propyl, Trifluormethyl, Difluormethyl, Chlordifluormethyl, Dichlorfluormethyl, Trichlormethyl, Pentafluorethyl, Heptafluorisopropyl, Cyclopropyl, Methoxy, Ethoxy, Methylsulfanyl, Methylsulfinyl oder Methylsulfonyl,
W bedeutet Wasserstoff, Chlor oder Methyl,
R bedeutet Methyl, Ethyl oder n-Propyl,
R' bedeutet Wasserstoff oder Cyano,
t bedeutet 0 oder 1.

Die erfindungsgemäßen Verbindungen der Formel (I) können beispielsweise aus den entsprechenden Thioethern der Formel (I-Thioether) hergestellt werden (Schema 1). Dazu wird der Thioether beispielsweise mit Cyanamid und einem Oxidationsmittel (Iodosobenzoldiacetat, Natriumhypochlorit oder N-Bromsuccinimid) in das entsprechende Sulfilimin überführt, welches weiter zum Sulfoximin oxidiert werden kann. Für die Oxidation zum Sulfoximin sind Oxidationsmittel wie beispielsweise meta-Chlorperbenzoesäure, Natriumpermanagant oder ein Gemisch aus Natriumperiodat und Rutheniumtrichlorid geeignet. NH-Sulfoximine sind beispielsweise aus Sulfoxiden mit Natriumazid und Schwefelsäure zugänglich und können am Stickstoffatom mit Reagenzien wie beispielswesie Bromcyan, Säurechloriden oder Säureanhydriden, Chlorameisensäureester, Salpetersäure oder anderen Verbindungen funktionalisiert werden. Die Oxidation von N-sulfonierten Sulfiliminen zu den entsprechenden Sulfoximinen gelingt beispielsweise mit Wasserstoffperoxid. Alternativ können Sulfoxide zu N-acylierten oder N-sulfonierten Sulfoximinen umgesetzt werden. Das Carboxamid bzw. Sulfonamid kann anschließend zum NH-Sulfoximin gespalten werden. Solche Synthesemethoden für die Generierung von Sulfiliminen und Sulfoximinen aus Thioethern oder für die Generierung von Sulfoximinen aus Sulfoxiden bzw. für die Derivatisierung von Sulfiliminen und Sulfoximinen, unter anderem auch von NH-Sulfoximinen, sind beispielsweise in Bolm, C. Org. Lett. 2004, 6, 1305; Bolm, C. Org. Lett. 2007, 9, 3809; Bolm, C. Synthesis 2010, 17, 2922; Bolm, C. Adv. Synth. Catal. 2010, 352, 309, WO 2007/095229 A1, WO 2008/141843 A1, WO 2008/097235 A1, US 2008/0207910 A1, US 2008/0194634 A1 und
US 2010/0056534 A1 beschrieben.

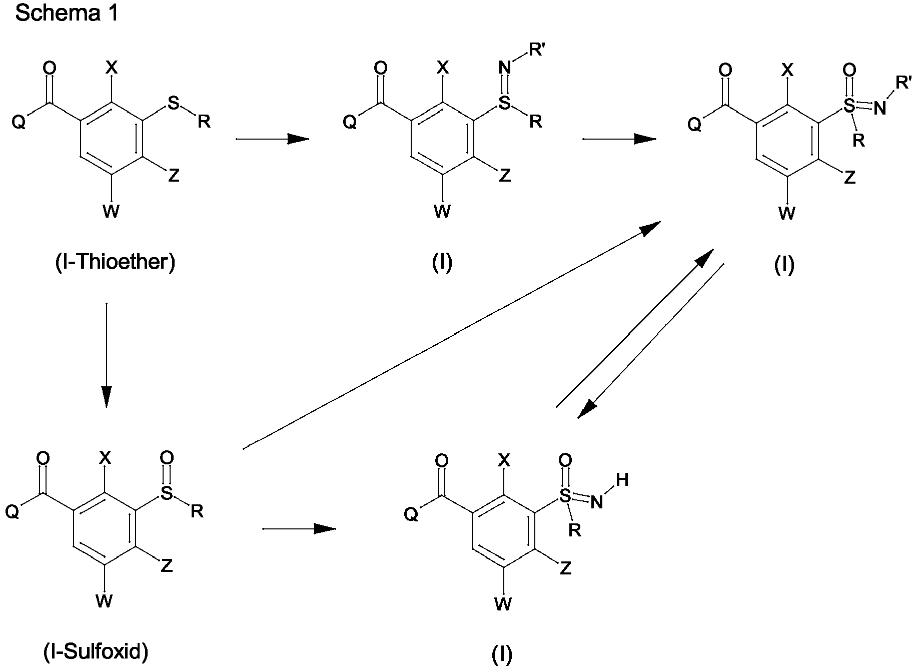

Gegebenenfalls müssen für solche Synthesesequenzen Schutzgruppen eingesetzt werden, um eine hinreichende Selektivität zu erreichen. Insbesondere die Funktionalisierung am NH-Sulfoximin steht prinzipiell in Konkurrenz zur Funktionalisierung an anderen Stellen des Moleküls. Die optimale Vorgehensweise hängt vom jeweiligen Substitutionsmuster ab.

Verbindungen der Formel (I-Thioether) und der Formel (I-Sulfoxid) sind bekannt und beispielsweise in WO 2003/014071 A1, WO 2008/125214 A1, WO 2009/149806 A1, WO 2011012247 A1, WO 2011012247 A1, EP 0 609 798 A1 sowie EP 0 625 508 A1 beschrieben.

Es kann zweckmäßig sein, Reaktionsschritte in ihrer Reihenfolge zu ändern. Sulfoximine und insbesondere Sulfilimine sind unter manchen Bedingungen nicht hinreichend stabil (Bolm, C. Adv. Synth. Catal. 2010, 352, 309), so dass es von Vorteil sein kann, wie in Schema 1 gezeigt, zuerst auf Thioether-Stufe das Benzoylderivat zu synthetisieren und erst am Ende der Synthesesequenz das Sulfilimin bzw. das Sulfoximin aus dem Thioether zu generieren. Bei hinreichender Stabilität kann es aber je nach Substitutionsmuster auch sinnvoll sein, zuerst auf der Benzoesäure-Stufe (oder in einem noch früheren Schritt) das Sulfilimin bzw. das Sulfoximin aus dem Thioether zu generieren und erst danach die Benzoesäure in ihr Benzoylderivat zu überführen (Schema 2). Die Überführung von Benzoesäuren in ihre Benzoylderivate ist für zahlreiche Strukturklassen, die keine Sulfiliminogruppe oder Sulfoximinogruppe enthalten, bekannt und beispielsweise in WO 2003/014071 A1, WO 2008/125214 A1, WO 2009/149806 A1, WO 2011012247 A1, WO 2011012247 A1, EP 0 609 798 A1 sowie EP 0 625 508 A1 beschrieben. Je nach Substitutionsmuster kann dieser Weg zu den erfindungsgemäßen Verbindungen der Formel (I) zweckmäßig sein.

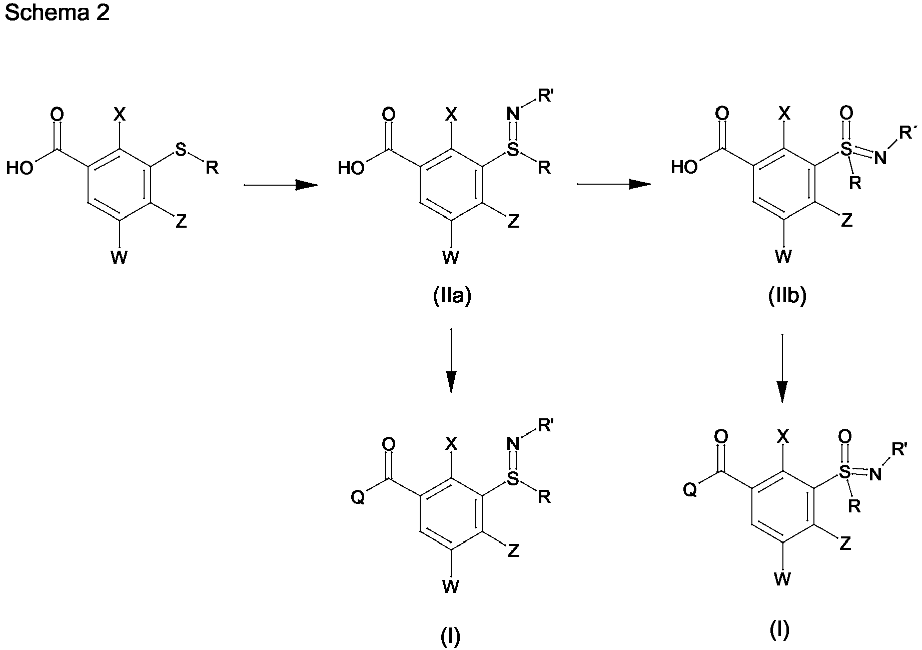

Gegebenenfalls ist es von Vorteil, für die Umsetzungen nicht die freie Benzoesäure sondern Derivate hiervon zu verwenden. Manchmal ist es für die Stabilität einer funktionellen Gruppe schon ausreichend, sich nur im sauren oder nur im basischen Medium zu bewegen, das heißt, nur mit der freien Benzoesäure oder nur mit ihrem Salz zu arbeiten. In vielen Fällen sind Ester wie Methyl- oder Ethylester geeignet. Tert.-Butylester schirmen die Carboxylgruppe oftmals sterisch wirksam gegen nucleophile Reagentien ab und sind im sauren Medium leicht spaltbar (T. W. Greene, P. G. M. Wuts, Protective Groups in Organic Synthesis, 2nd Edition, John Wiley & Sons, Inc. 1991, S. 227 ff.). Ferner sind Reste geeignet, die wesentlich stabiler als Carboxylgruppen sind, jedoch einfach aus Carbonsäuren zugänglich sind und sich auch einfach wieder in die freien Carbonsäuren überführen lassen. Hierzu zählen beispielsweise Oxazoline (T. W. Greene, P. G. M. Wuts, Protective Groups in Organic Synthesis, 2nd Edition, John Wiley & Sons, Inc. 1991, S. 265 ff.; Z. Hell et al, Tetrahedron Letters 43 (2002), 3985 - 3987).

Die vorstehend genannten Benzoesäuren der Formeln (IIa) und (IIb) sowie deren Ethylester, Methylester und Benzoesäurechloride sind neu und werden durch die Formel (II) dargestellt.

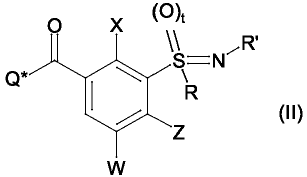

In Formel (II) steht Q* für Hydroxy, Ethoxy, Methoxy oder Chlor. R, R', X, W, Z und t haben die unter Formel (I) angegebenen Bedeutungen. Die Verbindungen der Formel (II) eignen sich insbesondere zur Herstellung der erfindungsgemäßen Verbindungen der Formel (I). Die Verbindungen der Formel (II) sind ebenfalls ein Gegenstand vorliegender Erfindung.

Die Aufarbeitung der jeweiligen Reaktionsmischungen erfolgt in der Regel nach bekannten Verfahren, beispielsweise durch Kristallisation, wässrig-extraktive Aufarbeitung, durch chromatographische Methoden oder durch Kombination dieser Methoden.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, lowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmar, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasenunterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß der hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfaßt.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüberhinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen in wirtschaftlich bedeutenden transgenen Kulturen von Nutz- und Zierpflanzen, z. B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt, siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY, oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet. Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227, Wolter et al., Proc. Natl. Acad. Sci. USA 85 (1988), 846-850, Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.
Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986, Wade van Valkenburg, "Pesticide Formulations", Marcel Dekker, N.Y., 1973, K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry", 2nd Ed., J. Wiley & Sons, N.Y., C. Marsden, "Solvents Guide", 2nd Ed., Interscience, N.Y. 1963, McCutcheon's "Detergents and Emulsifiers Annual", MC Publ. Corp., Ridgewood N.J., Sisley and Wood, "Encyclopedia of Surface Active Agents", Chem. Publ. Co. Inc., N.Y. 1964, Schönfeldt, "Grenzflächenaktive Äthylenoxidaddukte", Wiss. Verlagsgesell., Stuttgart 1976, Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix. Geeignete Safener sind beispielsweise Mefenpyr-diethyl, Cyprosulfamid, Isoxadifen-ethyl, Cloquintocet-mexyl und Dichlormid.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London, J.E. Browning, "Agglomeration", Chemical and Engineering 1967, Seiten 147 ff, "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen. In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 14th edition, The British Crop Protection Council and the Royal Soc. of Chemistry, 2003 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere.

Zur Anwendung werden die in handelsüblicher Form vorliegenden Formulierungen gegebenenfalls in üblicher Weise verdünnt z.B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (I). Sie kann innerhalb weiter Grenzen schwanken, z.B. zwischen 0,001 und 1,0 kg/ha oder mehr Aktivsubstanz, vorzugsweise liegt sie jedoch zwischen 0,005 und 750 g/ha.

Die nachstehenden Beispiele erläutern die Erfindung.

### A. Chemische Beispiele

### Synthese von 5-Hydroxy-1,3-dimethyl-4-[3-(N-Cyan-S-methylsulfonimidoyl)-2-methoxy-4-(trifluormethyl)benzoyl]pyrazol (Tabellenbeispiel Nr. 4-160)

### Schritt 1: Synthese von 3-(N-Cyan-S-methylsulfonimidoyl)-2-methoxy-4-(trifluormethyl)benzoesäure (Tabellenbeispiel Nr. 15-28)

3.71 g (33.1 mmol) Kalium-tert.-butylat wurden zu einer Lösung von 4.00 g (15.0 mmol) 2-Methoxy-3-(methylsulfanyl)-4-(trifluormethyl)benzoesäure in 250 ml Methanol gegeben. Das Gemisch wurde 10 Minuten gerührt und dann nacheinander mit 1.07 g (25.5 mmol) Cyanamid und 4.81 g (27.0 mmol) N-Bromsuccinimid versetzt. Der Inhalt wurde anschließend 2 h bei (Raumtemperatur) RT gerührt. Danach wurde das Gemisch vom Lösungsmittel befreit und der Rückstand wurde in einer Mischung aus je 120 ml Acetonitril und Wasser aufgenommen. Das Gemisch wurde mit 7.21 g (45.1 mmol) Natriumpermanganat-monohydrat versetzt und eine Woche bei RT gerührt. Innerhalb dieser Woche wurden sowohl nach einem Tag als auch nach einem weiteren Tag jeweils 3.6 g (22.5 mmol) Natriumpermanganat-monohydrat zugegeben. Zur Aufarbeitung wurde eine wässrige 10 Gew.-proz. Lösung von Natriumhydrogensulfit zugegeben. Dann wurde bei einer Temperatur von maximal 30 °C das Lösungsmittel weitgehend abgetrennt. Der Rückstand wurde im Eisbad abgekühlt und dann mit 1 M Salzsäure angesäuert. Das Gemisch wurde mit eiskaltem Dichlormethan dreimal extrahiert. Die vereinigten organischen Phasen wurden vom Lösungsmittel befreit und der Rückstand wurde chromatographisch gereinigt, wobei 1.30 g Produkt mit einer Reinheit von 80 Gew.-% gewonnen wurden.

### Schritt 2: Synthese von 5-Hydroxy-1,3-dimethyl-4-[3-(N-Cyan-S-methylsulfonimidoyl)-2-methoxy-4-(trifluormethyl)benzoyl]pyrazol

165 mg (75 Gew.-%; 0.384 mmol) 3-(N-Cyan-S-methylsulfonimidoyl)-2-methoxy-4-(trifluormethyl)benzoesäure und 68.9 mg (0.614 mmol) 5-Hydroxy-1,3-dimethylpyrazol wurden in 20 ml Dichlormethan vorgelegt und mit 128 mg (0.666 mmol) 1-(3-Dimethylaminopropyl)-3-ethylcarbodiimid Hydrochlorid versetzt. Das Gemisch wurde bei RT 16 h gerührt und zur Aufarbeitung mit 1 M Salzsäure gewaschen. Nach der Phasentrennung wurde die organische Phase getrocknet, filtriert und am Rotationsverdampfer vom Lösungsmittel befreit. Der Rückstand wurde chromatographisch gereinigt und das gewonnene Zwischenprodukt wurde anschließend in 15 ml Acetonitril gelöst. Danach wurden 104 mg (1.02 mmol) Triethylamin, acht Tropfen Trimethylcyanid sowie eine Spatelspitze Kaliumcyanid zugegeben. Das Gemisch wurde 16 h bei RT gerührt und zur Aufarbeitung vom Lösungsmittel befreit. Der Rückstand wurde in Dichlormethan aufgenommen und mit 3 ml 1 M Salzsäure gewaschen. Nach der Phasentrennung wurde die organische Phase vom Lösungsmittel befreit und der Rückstand wurde chromatographisch gereinigt, wobei 31.3 mg 5-Hydroxy-1,3-dimethyl-4-[3-(N-Cyan-S-methylsulfonimidoyl)-2-methoxy-4-(trifluormethyl)benzoyl]pyrazol in einer Reinheit von 85 Gew.-% gewonnen wurden.

Die in den nachfolgenden Tabellen aufgeführten Beispiele wurden analog oben genannten Methoden hergestellt beziehungsweise sind analog oben genannten Methoden erhältlich. Die in den nachfolgenden Tabellen aufgeführten Verbindungen sind ganz besonders bevorzugt.

Die verwendeten Abkürzungen bedeuten:

| | | | |
|---|---|---|---|
| Et = Ethyl | Me = Methyl | n-Pr = n-Propyl | i-Pr = Isopropyl |
| c-Pr = Cyclopropyl | Ph = Phenyl | | |

**Tabelle 1: Erfindungsgemäße Verbindungen der allgemeinen Formel (I), worin Q für Q1, R^{a} für eine Hydroxylgruppe, die Reste R^{b}, R^{c}, R^{d}, R^{e}, R^{f}, R^{g} und W jeweils für Wasserstoff stehen, t = 1 ist und die anderen Reste die in der Tabelle angegebenen Bedeutungen haben**

| | | | | | |
|---|---|---|---|---|---|
| 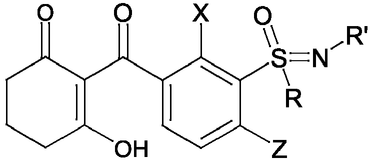 | | | | | |

| Nr. | X | Z | R | R' | Physikalische Daten (¹H-NMR) |
|---|---|---|---|---|---|
| 1-1 | Me | Me | Me | H | |
| 1-2 | Me | F | Me | H | |
| 1-3 | Me | Cl | Me | H | |
| 1-4 | Me | Br | Me | H | |
| 1-5 | Me | I | Me | H | |
| 1-6 | Me | CF₃ | Me | H | |
| 1-7 | Me | CHF₂ | Me | H | |
| 1-8 | Me | CF₂Cl | Me | H | |
| 1-9 | Me | OMe | Me | H | |
| 1-10 | Me | NO₂ | Me | H | |
| 1-11 | Me | SO₂Me | Me | H | |
| 1-12 | Cl | Me | Me | H | |
| 1-13 | Cl | F | Me | H | |
| 1-14 | Cl | Cl | Me | H | |
| 1-15 | Cl | Br | Me | H | |
| 1-16 | Cl | I | Me | H | |
| 1-17 | Cl | CF₃ | Me | H | |
| 1-18 | Cl | CHF₂ | Me | H | |
| 1-19 | Cl | CF₂Cl | Me | H | |
| 1-20 | Cl | OMe | Me | H | |
| 1-21 | Cl | NO₂ | Me | H | |
| 1-22 | Cl | SO₂Me | Me | H | |
| 1-23 | OMe | Me | Me | H | |
| 1-24 | OMe | F | Me | H | |
| 1-25 | OMe | Cl | Me | H | |
| 1-26 | OMe | Br | Me | H | |
| 1-27 | OMe | I | Me | H | |
| 1-28 | OMe | CF₃ | Me | H | |
| 1-29 | OMe | CHF₂ | Me | H | |
| 1-30 | OMe | CF₂Cl | Me | H | |
| 1-31 | OMe | OMe | Me | H | |
| 1-32 | OMe | NO₂ | Me | H | |
| 1-33 | OMe | SO₂Me | Me | H | |
| 1-34 | SO₂Me | Me | Me | H | |
| 1-35 | SO₂Me | F | Me | H | |
| 1-36 | SO₂Me | Cl | Me | H | |
| 1-37 | SO₂Me | Br | Me | H | |
| 1-38 | SO₂Me | I | Me | H | |
| 1-39 | SO₂Me | CF₃ | Me | H | |
| 1-40 | SO₂Me | CHF₂ | Me | H | |
| 1-41 | SO₂Me | CF₂Cl | Me | H | |
| 1-42 | SO₂Me | OMe | Me | H | |
| 1-43 | SO₂Me | NO₂ | Me | H | |
| 1-44 | SO₂Me | SO₂Me | Me | H | |
| 1-45 | Me | Me | Et | H | |
| 1-46 | Me | F | Et | H | |
| 1-47 | Me | Cl | Et | H | |
| 1-48 | Me | Br | Et | H | |
| 1-49 | Me | I | Et | H | |
| 1-50 | Me | CF₃ | Et | H | |
| 1-51 | Me | CHF₂ | Et | H | |
| 1-52 | Me | CF₂Cl | Et | H | |
| 1-53 | Me | OMe | Et | H | |
| 1-54 | Me | NO₂ | Et | H | |
| 1-55 | Me | SO₂Me | Et | H | |
| 1-56 | Cl | Me | Et | H | |
| 1-57 | Cl | F | Et | H | |
| 1-58 | Cl | Cl | Et | H | |
| 1-59 | Cl | Br | Et | H | |
| 1-60 | Cl | I | Et | H | |
| 1-61 | Cl | CF₃ | Et | H | |
| 1-62 | Cl | CHF₂ | Et | H | |
| 1-63 | Cl | CF₂Cl | Et | H | |
| 1-64 | Cl | OMe | Et | H | |
| 1-65 | Cl | NO₂ | Et | H | |
| 1-66 | Cl | SO₂Me | Et | H | |
| 1-67 | OMe | Me | Et | H | |
| 1-68 | OMe | F | Et | H | |
| 1-69 | OMe | Cl | Et | H | |
| 1-70 | OMe | Br | Et | H | |
| 1-71 | OMe | I | Et | H | |
| 1-72 | OMe | CF₃ | Et | H | |
| 1-73 | OMe | CHF₂ | Et | H | |
| 1-74 | OMe | CF₂Cl | Et | H | |
| 1-75 | OMe | OMe | Et | H | |
| 1-76 | OMe | NO₂ | Et | H | |
| 1-77 | OMe | SO₂Me | Et | H | |
| 1-78 | SO₂Me | Me | Et | H | |
| 1-79 | SO₂Me | F | Et | H | |
| 1-80 | SO₂Me | Cl | Et | H | |
| 1-81 | SO₂Me | Br | Et | H | |
| 1-82 | SO₂Me | I | Et | H | |
| 1-83 | SO₂Me | CF₃ | Et | H | |
| 1-84 | SO₂Me | CHF₂ | Et | H | |
| 1-85 | SO₂Me | CF₂Cl | Et | H | |
| 1-86 | SO₂Me | OMe | Et | H | |
| 1-87 | SO₂Me | NO₂ | Et | H | |
| 1-88 | SO₂Me | SO₂Me | Et | H | |
| 1-89 | Me | Me | CH₂CH₂OMe | H | |
| 1-90 | Me | F | CH₂CH₂OMe | H | |
| 1-91 | Me | Cl | CH₂CH₂OMe | H | |
| 1-92 | Me | Br | CH₂CH₂OMe | H | |
| 1-93 | Me | I | CH₂CH₂OMe | H | |
| 1-94 | Me | CF₃ | CH₂CH₂OMe | H | |
| 1-95 | Me | CHF₂ | CH₂CH₂OMe | H | |
| 1-96 | Me | CF₂Cl | CH₂CH₂OMe | H | |
| 1-97 | Me | OMe | CH₂CH₂OMe | H | |
| 1-98 | Me | NO₂ | CH₂CH₂OMe | H | |
| 1-99 | Me | SO₂Me | CH₂CH₂OMe | H | |
| 1-100 | Cl | Me | CH₂CH₂OMe | H | |
| 1-101 | Cl | F | CH₂CH₂OMe | H | |
| 1-102 | Cl | Cl | CH₂CH₂OMe | H | |
| 1-103 | Cl | Br | CH₂CH₂OMe | H | |
| 1-104 | Cl | I | CH₂CH₂OMe | H | |
| 1-105 | Cl | CF₃ | CH₂CH₂OMe | H | |
| 1-106 | Cl | CHF₂ | CH₂CH₂OMe | H | |
| 1-107 | Cl | CF₂Cl | CH₂CH₂OMe | H | |
| 1-108 | Cl | OMe | CH₂CH₂OMe | H | |
| 1-109 | Cl | NO₂ | CH₂CH₂OMe | H | |
| 1-110 | Cl | SO₂Me | CH₂CH₂OMe | H | |
| 1-111 | OMe | Me | CH₂CH₂OMe | H | |
| 1-112 | OMe | F | CH₂CH₂OMe | H | |
| 1-113 | OMe | Cl | CH₂CH₂OMe | H | |
| 1-114 | OMe | Br | CH₂CH₂OMe | H | |
| 1-115 | OMe | I | CH₂CH₂OMe | H | |
| 1-116 | OMe | CF₃ | CH₂CH₂OMe | H | |
| 1-117 | OMe | CHF₂ | CH₂CH₂OMe | H | |
| 1-118 | OMe | CF₂Cl | CH₂CH₂OMe | H | |
| 1-119 | OMe | OMe | CH₂CH₂OMe | H | |
| 1-120 | OMe | NO₂ | CH₂CH₂OMe | H | |
| 1-121 | OMe | SO₂Me | CH₂CH₂OMe | H | |
| 1-122 | SO₂Me | Me | CH₂CH₂OMe | H | |
| 1-123 | SO₂Me | F | CH₂CH₂OMe | H | |
| 1-124 | SO₂Me | Cl | CH₂CH₂OMe | H | |
| 1-125 | SO₂Me | Br | CH₂CH₂OMe | H | |
| 1-126 | SO₂Me | I | CH₂CH₂OMe | H | |
| 1-127 | SO₂Me | CF₃ | CH₂CH₂OMe | H | |
| 1-128 | SO₂Me | CHF₂ | CH₂CH₂0Me | H | |
| 1-129 | SO₂Me | CF₂Cl | CH₂CH₂OMe | H | |
| 1-130 | SO₂Me | OMe | CH₂CH₂OMe | H | |
| 1-131 | SO₂Me | NO₂ | CH₂CH₂OMe | H | |
| 1-132 | SO₂Me | SO₂Me | CH₂CH₂OMe | H | |
| 1-133 | Me | Me | Me | CN | |
| 1-134 | Me | F | Me | CN | |
| 1-135 | Me | Cl | Me | CN | |
| 1-136 | Me | Br | Me | CN | |
| 1-137 | Me | I | Me | CN | |
| 1-138 | Me | CF₃ | Me | CN | |
| 1-139 | Me | CHF₂ | Me | CN | |
| 1-140 | Me | CF₂Cl | Me | CN | |
| 1-141 | Me | OMe | Me | CN | |
| 1-142 | Me | NO₂ | Me | CN | |
| 1-143 | Me | SO₂Me | Me | CN | |
| 1-144 | Cl | Me | Me | CN | |
| 1-145 | Cl | F | Me | CN | |
| 1-146 | Cl | Cl | Me | CN | |
| 1-147 | Cl | Br | Me | CN | |
| 1-148 | Cl | I | Me | CN | |
| 1-149 | CI | CF₃ | Me | CN | |
| 1-150 | Cl | CHF₂ | Me | CN | |
| 1-151 | Cl | CF₂Cl | Me | CN | |
| 1-152 | Cl | OMe | Me | CN | |
| 1-153 | Cl | NO₂ | Me | CN | |
| 1-154 | Cl | SO₂Me | Me | CN | |
| 1-155 | OMe | Me | Me | CN | |
| 1-156 | OMe | F | Me | CN | |
| 1-157 | OMe | Cl | Me | CN | |
| 1-158 | OMe | Br | Me | CN | |
| 1-159 | OMe | l | Me | CN | |
| 1-160 | OMe | CF₃ | Me | CN | |
| 1-161 | OMe | CHF₂ | Me | CN | |
| 1-162 | OMe | CF₂Cl | Me | CN | |
| 1-163 | OMe | OMe | Me | CN | |
| 1-164 | OMe | NO₂ | Me | CN | |
| 1-165 | OMe | SO₂Me | Me | CN | |
| 1-166 | SO₂Me | Me | Me | CN | |
| 1-167 | SO₂Me | F | Me | CN | |
| 1-168 | SO₂Me | Cl | Me | CN | |
| 1-169 | SO₂Me | Br | Me | CN | |
| 1-170 | SO₂Me | I | Me | CN | |
| 1-171 | SO₂Me | CF₃ | Me | CN | |
| 1-172 | SO₂Me | CHF₂ | Me | CN | |
| 1-173 | SO₂Me | CF₂Cl | Me | CN | |
| 1-174 | SO₂Me | OMe | Me | CN | |
| 1-175 | SO₂Me | NO₂ | Me | CN | |
| 1-176 | SO₂Me | SO₂Me | Me | CN | |
| 1-177 | Me | Me | Et | CN | |
| 1-178 | Me | F | Et | CN | |
| 1-179 | Me | Cl | Et | CN | |
| 1-180 | Me | Br | Et | CN | |
| 1-181 | Me | I | Et | CN | |
| 1-182 | Me | CF₃ | Et | CN | |
| 1-183 | Me | CHF₂ | Et | CN | |
| 1-184 | Me | CF₂Cl | Et | CN | |
| 1-185 | Me | OMe | Et | CN | |
| 1-186 | Me | NO₂ | Et | CN | |
| 1-187 | Me | SO₂Me | Et | CN | |
| 1-188 | Cl | Me | Et | CN | |
| 1-189 | Cl | F | Et | CN | |
| 1-190 | Cl | Cl | Et | CN | |
| 1-191 | Cl | Br | Et | CN | |
| 1-192 | Cl | I | Et | CN | |
| 1-193 | Cl | CF₃ | Et | CN | |
| 1-194 | Cl | CHF₂ | Et | CN | |
| 1-195 | Cl | CF₂Cl | Et | CN | |
| 1-196 | Cl | OMe | Et | CN | |
| 1-197 | Cl | NO₂ | Et | CN | |
| 1-198 | Cl | SO₂Me | Et | CN | |
| 1-199 | OMe | Me | Et | CN | |
| 1-200 | OMe | F | Et | CN | |
| 1-201 | OMe | Cl | Et | CN | |
| 1-202 | OMe | Br | Et | CN | |
| 1-203 | OMe | I | Et | CN | |
| 1-204 | OMe | CF₃ | Et | CN | |
| 1-205 | OMe | CHF₂ | Et | CN | |
| 1-206 | OMe | CF₂Cl | Et | CN | |
| 1-207 | OMe | OMe | Et | CN | |
| 1-208 | OMe | NO₂ | Et | CN | |
| 1-209 | OMe | SO₂Me | Et | CN | |
| 1-210 | SO₂Me | Me | Et | CN | |
| 1-211 | SO₂Me | F | Et | CN | |
| 1-212 | SO₂Me | Cl | Et | CN | |
| 1-213 | SO₂Me | Br | Et | CN | |
| 1-214 | SO₂Me | I | Et | CN | |
| 1-215 | SO₂Me | CF₃ | Et | CN | |
| 1-216 | SO₂Me | CHF₂ | Et | CN | |
| 1-217 | SO₂Me | CF₂Cl | Et | CN | |
| 1-218 | SO₂Me | OMe | Et | CN | |
| 1-219 | SO₂Me | NO₂ | Et | CN | |
| 1-220 | SO₂Me | SO₂Me | Et | CN | |
| 1-221 | Me | Me | CH₂CH₂OMe | CN | |
| 1-222 | Me | F | CH₂CH₂OMe | CN | |
| 1-223 | Me | Cl | CH₂CH₂OMe | CN | |
| 1-224 | Me | Br | CH₂CH₂OMe | CN | |
| 1-225 | Me | I | CH₂CH₂OMe | CN | |
| 1-226 | Me | CF₃ | CH₂CH₂OMe | CN | |
| 1-227 | Me | CHF₂ | CH₂CH₂OMe | CN | |
| 1-228 | Me | CF₂Cl | CH₂CH₂OMe | CN | |
| 1-229 | Me | OMe | CH₂CH₂OMe | CN | |
| 1-230 | Me | NO₂ | CH₂CH₂OMe | CN | |
| 1-231 | Me | SO₂Me | CH₂CH₂OMe | CN | |
| 1-232 | Cl | Me | CH₂CH₂OMe | CN | |
| 1-233 | Cl | F | CH₂CH₂OMe | CN | |
| 1-234 | Cl | Cl | CH₂CH₂OMe | CN | |
| 1-235 | Cl | Br | CH₂CH₂OMe | CN | |
| 1-236 | Cl | I | CH₂CH₂OMe | CN | |
| 1-237 | Cl | CF₃ | CH₂CH₂OMe | CN | |
| 1-238 | Cl | CHF₂ | CH₂CH₂OMe | CN | |
| 1-239 | Cl | CF₂Cl | CH₂CH₂OMe | CN | |
| 1-240 | Cl | OMe | CH₂CH₂OMe | CN | |
| 1-241 | Cl | NO₂ | CH₂CH₂OMe | CN | |
| 1-242 | Cl | SO₂Me | CH₂CH₂OMe | CN | |
| 1-243 | OMe | Me | CH₂CH₂OMe | CN | |
| 1-244 | OMe | F | CH₂CH₂OMe | CN | |
| 1-245 | OMe | Cl | CH₂CH₂OMe | CN | |
| 1-246 | OMe | Br | CH₂CH₂OMe | CN | |
| 1-247 | OMe | I | CH₂CH₂OMe | CN | |
| 1-248 | OMe | CF₃ | CH₂CH₂OMe | CN | |
| 1-249 | OMe | CHF₂ | CH₂CH₂OMe | CN | |
| 1-250 | OMe | CF₂Cl | CH₂CH₂OMe | CN | |
| 1-251 | OMe | OMe | CH₂CH₂OMe | CN | |
| 1-252 | OMe | NO₂ | CH₂CH₂OMe | CN | |
| 1-253 | OMe | SO₂Me | CH₂CH₂OMe | CN | |
| 1-254 | SO₂Me | Me | CH₂CH₂OMe | CN | |
| 1-255 | SO₂Me | F | CH₂CH₂OMe | CN | |
| 1-256 | SO₂Me | Cl | CH₂CH₂OMe | CN | |
| 1-257 | SO₂Me | Br | CH₂CH₂OMe | CN | |
| 1-258 | SO₂Me | I | CH₂CH₂OMe | CN | |
| 1-259 | SO₂Me | CF₃ | CH₂CH₂OMe | CN | |
| 1-260 | SO₂Me | CHF₂ | CH₂CH₂OMe | CN | |
| 1-261 | SO₂Me | CF₂Cl | CH₂CH₂OMe | CN | |
| 1-262 | SO₂Me | OMe | CH₂CH₂OMe | CN | |
| 1-263 | SO₂Me | NO₂ | CH₂CH₂OMe | CN | |
| 1-264 | SO₂Me | SO₂Me | CH₂CH₂OMe | CN | |

**Tabelle 2: Erfindungsgemäße Verbindungen der allgemeinen Formel (I), worin Q für Q2, R^{a} für eine Hydroxylgruppe, R^{b}, R^{f} und W jeweils für Wasserstoff, A für CH₂CH₂, Y für CH₂ stehen, t = 1 ist und die anderen Reste die in der Tabelle angegebenen Bedeutungen haben**

| | | | | | |
|---|---|---|---|---|---|
| 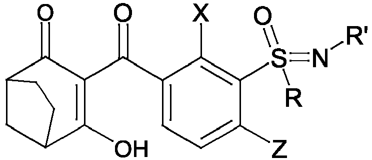 | | | | | |

| Nr. | X | Z | R | R' | Physikalische Daten (¹H-NMR) |
|---|---|---|---|---|---|
| 2-1 | Me | Me | Me | H | |
| 2-2 | Me | F | Me | H | |
| 2-3 | Me | Cl | Me | H | |
| 2-4 | Me | Br | Me | H | |
| 2-5 | Me | I | Me | H | |
| 2-6 | Me | CF₃ | Me | H | |
| 2-7 | Me | CHF₂ | Me | H | |
| 2-8 | Me | CF₂Cl | Me | H | |
| 2-9 | Me | OMe | Me | H | |
| 2-10 | Me | NO₂ | Me | H | |
| 2-11 | Me | SO₂Me | Me | H | |
| 2-12 | Cl | Me | Me | H | |
| 2-13 | Cl | F | Me | H | |
| 2-14 | Cl | Cl | Me | H | |
| 2-15 | Cl | Br | Me | H | |
| 2-16 | Cl | I | Me | H | |
| 2-17 | Cl | CF₃ | Me | H | |
| 2-18 | Cl | CHF₂ | Me | H | |
| 2-19 | Cl | CF₂Cl | Me | H | |
| 2-20 | Cl | OMe | Me | H | |
| 2-21 | Cl | NO₂ | Me | H | |
| 2-22 | Cl | SO₂Me | Me | H | |
| 2-23 | OMe | Me | Me | H | |
| 2-24 | OMe | F | Me | H | |
| 2-25 | OMe | Cl | Me | H | |
| 2-26 | OMe | Br | Me | H | |
| 2-27 | OMe | I | Me | H | |
| 2-28 | OMe | CF₃ | Me | H | |
| 2-29 | OMe | CHF₂ | Me | H | |
| 2-30 | OMe | CF₂Cl | Me | H | |
| 2-31 | OMe | OMe | Me | H | |
| 2-32 | OMe | NO₂ | Me | H | |
| 2-33 | OMe | SO₂Me | Me | H | |
| 2-34 | SO₂Me | Me | Me | H | |
| 2-35 | SO₂Me | F | Me | H | |
| 2-36 | SO₂Me | Cl | Me | H | |
| 2-37 | SO₂Me | Br | Me | H | |
| 2-38 | SO₂Me | I | Me | H | |
| 2-39 | S0₂Me | CF₃ | Me | H | |
| 2-40 | SO₂Me | CHF₂ | Me | H | |
| 2-41 | SO₂Me | CF₂Cl | Me | H | |
| 2-42 | SO₂Me | OMe | Me | H | |
| 2-43 | SO₂Me | NO₂ | Me | H | |
| 2-44 | SO₂Me | SO₂Me | Me | H | |
| 2-45 | Me | Me | Et | H | |
| 2-46 | Me | F | Et | H | |
| 2-47 | Me | Cl | Et | H | |
| 2-48 | Me | Br | Et | H | |
| 2-49 | Me | I | Et | H | |
| 2-50 | Me | CF₃ | Et | H | |
| 2-51 | Me | CHF₂ | Et | H | |
| 2-52 | Me | CF₂Cl | Et | H | |
| 2-53 | Me | OMe | Et | H | |
| 2-54 | Me | NO₂ | Et | H | |
| 2-55 | Me | SO₂Me | Et | H | |
| 2-56 | Cl | Me | Et | H | |
| 2-57 | Cl | F | Et | H | |
| 2-58 | Cl | Cl | Et | H | |
| 2-59 | Cl | Br | Et | H | |
| 2-60 | Cl | I | Et | H | |
| 2-61 | Cl | CF₃ | Et | H | |
| 2-62 | Cl | CHF₂ | Et | H | |
| 2-63 | Cl | CF₂Cl | Et | H | |
| 2-64 | Cl | OMe | Et | H | |
| 2-65 | Cl | NO₂ | Et | H | |
| 2-66 | Cl | SO₂Me | Et | H | |
| 2-67 | OMe | Me | Et | H | |
| 2-68 | OMe | F | Et | H | |
| 2-69 | OMe | Cl | Et | H | |
| 2-70 | OMe | Br | Et | H | |
| 2-71 | OMe | I | Et | H | |
| 2-72 | OMe | CF₃ | Et | H | |
| 2-73 | OMe | CHF₂ | Et | H | |
| 2-74 | OMe | CF₂Cl | Et | H | |
| 2-75 | OMe | OMe | Et | H | |
| 2-76 | OMe | NO₂ | Et | H | |
| 2-77 | OMe | SO₂Me | Et | H | |
| 2-78 | SO₂Me | Me | Et | H | |
| 2-79 | SO₂Me | F | Et | H | |
| 2-80 | SO₂Me | Cl | Et | H | |
| 2-81 | SO₂Me | Br | Et | H | |
| 2-82 | SO₂Me | I | Et | H | |
| 2-83 | SO₂Me | CF₃ | Et | H | |
| 2-84 | SO₂Me | CHF₂ | Et | H | |
| 2-85 | SO₂Me | CF₂Cl | Et | H | |
| 2-86 | SO₂Me | OMe | Et | H | |
| 2-87 | SO₂Me | NO₂ | Et | H | |
| 2-88 | SO₂Me | SO₂Me | Et | H | |
| 2-89 | Me | Me | CH₂CH₂OMe | H | |
| 2-90 | Me | F | CH₂CH₂OMe | H | |
| 2-91 | Me | Cl | CH₂CH₂OMe | H | |
| 2-92 | Me | Br | CH₂CH₂OMe | H | |
| 2-93 | Me | I | CH₂CH₂OMe | H | |
| 2-94 | Me | CF₃ | CH₂CH₂OMe | H | |
| 2-95 | Me | CHF₂ | CH₂CH₂OMe | H | |
| 2-96 | Me | CF₂Cl | CH₂CH₂OMe | H | |
| 2-97 | Me | OMe | CH₂CH₂OMe | H | |
| 2-98 | Me | NO₂ | CH₂CH₂OMe | H | |
| 2-99 | Me | SO₂Me | CH₂CH₂OMe | H | |
| 2-100 | Cl | Me | CH₂CH₂OMe | H | |
| 2-101 | Cl | F | CH₂CH₂OMe | H | |
| 2-102 | Cl | Cl | CH₂CH₂OMe | H | |
| 2-103 | Cl | Br | CH₂CH₂OMe | H | |
| 2-104 | Cl | I | CH₂CH₂OMe | H | |
| 2-105 | Cl | CF₃ | CH₂CH₂OMe | H | |
| 2-106 | Cl | CHF₂ | CH₂CH₂OMe | H | |
| 2-107 | Cl | CF₂Cl | CH₂CH₂OMe | H | |
| 2-108 | Cl | OMe | CH₂CH₂OMe | H | |
| 2-109 | Cl | NO₂ | CH₂CH₂OMe | H | |
| 2-110 | Cl | SO₂Me | CH₂CH₂OMe | H | |
| 2-111 | OMe | Me | CH₂CH₂OMe | H | |
| 2-112 | OMe | F | CH₂CH₂OMe | H | |
| 2-113 | OMe | Cl | CH₂CH₂OMe | H | |
| 2-114 | OMe | Br | CH₂CH₂OMe | H | |
| 2-115 | OMe | I | CH₂CH₂OMe | H | |
| 2-116 | OMe | CF₃ | CH₂CH₂OMe | H | |
| 2-117 | OMe | CHF₂ | CH₂CH₂OMe | H | |
| 2-118 | OMe | CF₂Cl | CH₂CH₂OMe | H | |
| 2-119 | OMe | OMe | CH₂CH₂OMe | H | |
| 2-120 | OMe | NO₂ | CH₂CH₂OMe | H | |
| 2-121 | OMe | SO₂Me | CH₂CH₂OMe | H | |
| 2-122 | SO₂Me | Me | CH₂CH₂OMe | H | |
| 2-123 | SO₂Me | F | CH₂CH₂0me | H | |
| 2-124 | SO₂Me | Cl | CH₂CH₂OMe | H | |
| 2-125 | SO₂Me | Br | CH₂CH₂OMe | H | |
| 2-126 | SO₂Me | I | CH₂CH₂OMe | H | |
| 2-127 | SO₂Me | CF₃ | CH₂CH₂OMe | H | |
| 2-128 | SO₂Me | CHF₂ | CH₂CH₂OMe | H | |
| 2-129 | SO₂Me | CF₂Cl | CH₂CH₂OMe | H | |
| 2-130 | SO₂Me | OMe | CH₂CH₂OMe | H | |
| 2-131 | SO₂Me | NO₂ | CH₂CH₂OMe | H | |
| 2-132 | SO₂Me | SO₂Me | CH₂CH₂OMe | H | |
| 2-133 | Me | Me | Me | CN | |
| 2-134 | Me | F | Me | CN | |
| 2-135 | Me | Cl | Me | CN | |
| 2-136 | Me | Br | Me | CN | |
| 2-137 | Me | I | Me | CN | |
| 2-138 | Me | CF₃ | Me | CN | |
| 2-139 | Me | CHF₂ | Me | CN | |
| 2-140 | Me | CF₂Cl | Me | CN | |
| 2-141 | Me | OMe | Me | CN | |
| 2-142 | Me | NO₂ | Me | CN | |
| 2-143 | Me | SO₂Me | Me | CN | |
| 2-144 | Cl | Me | Me | CN | |
| 2-145 | Cl | F | Me | CN | |
| 2-146 | Cl | Cl | Me | CN | |
| 2-147 | Cl | Br | Me | CN | |
| 2-148 | Cl | I | Me | CN | |
| 2-149 | Cl | CF₃ | Me | CN | |
| 2-150 | Cl | CHF₂ | Me | CN | |
| 2-151 | Cl | CF₂Cl | Me | CN | |
| 2-152 | Cl | OMe | Me | CN | |
| 2-153 | Cl | NO₂ | Me | CN | |
| 2-154 | Cl | SO₂Me | Me | CN | |
| 2-155 | OMe | Me | Me | CN | |
| 2-156 | OMe | F | Me | CN | |
| 2-157 | OMe | Cl | Me | CN | |
| 2-158 | OMe | Br | Me | CN | |
| 2-159 | OMe | I | Me | CN | |
| 2-160 | OMe | CF₃ | Me | CN | (400 MHz, CDCI₃ δ, ppm) 7.81 (m,1H), 7.65 (d,1 H), 3.91 + 3.88 (s + s,3H), 3.65 + 3.62 (s + s,3H), 3.21 (m,1 H), 2.91 (m,1H) |
| 2-161 | OMe | CHF₂ | Me | CN | |
| 2-162 | OMe | CF₂Cl | Me | CN | |
| 2-163 | OMe | OMe | Me | CN | |
| 2-164 | OMe | NO₂ | Me | CN | |
| 2-165 | OMe | SO₂Me | Me | CN | |
| 2-166 | SO₂Me | Me | Me | CN | |
| 2-167 | SO₂Me | F | Me | CN | |
| 2-168 | SO₂Me | Cl | Me | CN | |
| 2-169 | SO₂Me | Br | Me | CN | |
| 2-170 | SO₂Me | I | Me | CN | |
| 2-171 | SO₂Me | CF₃ | Me | CN | |
| 2-172 | SO₂Me | CHF₂ | Me | CN | |
| 2-173 | SO₂Me | CF₂Cl | Me | CN | |
| 2-174 | SO₂Me | OMe | Me | CN | |
| 2-175 | SO₂Me | NO₂ | Me | CN | |
| 2-176 | SO₂Me | SO₂Me | Me | CN | |
| 2-177 | Me | Me | Et | CN | |
| 2-178 | Me | F | Et | CN | |
| 2-179 | Me | Cl | Et | CN | |
| 2-180 | Me | Br | Et | CN | |
| 2-181 | Me | I | Et | CN | |
| 2-182 | Me | CF₃ | Et | CN | |
| 2-183 | Me | CHF₂ | Et | CN | |
| 2-184 | Me | CF₂Cl | Et | CN | |
| 2-185 | Me | OMe | Et | CN | |
| 2-186 | Me | NO₂ | Et | CN | |
| 2-187 | Me | SO₂Me | Et | CN | |
| 2-188 | Cl | Me | Et | CN | |
| 2-189 | Cl | F | Et | CN | |
| 2-190 | Cl | Cl | Et | CN | |
| 2-191 | Cl | Br | Et | CN | |
| 2-192 | Cl | I | Et | CN | |
| 2-193 | Cl | CF₃ | Et | CN | |
| 2-194 | Cl | CHF₂ | Et | CN | |
| 2-195 | Cl | CF₂Cl | Et | CN | |
| 2-196 | Cl | OMe | Et | CN | |
| 2-197 | Cl | NO₂ | Et | CN | |
| 2-198 | Cl | SO₂Me | Et | CN | |
| 2-199 | OMe | Me | Et | CN | |
| 2-200 | OMe | F | Et | CN | |
| 2-201 | OMe | Cl | Et | CN | |
| 2-202 | OMe | Br | Et | CN | |
| 2-203 | OMe | I | Et | CN | |
| 2-204 | OMe | CF₃ | Et | CN | |
| 2-205 | OMe | CHF₂ | Et | CN | |
| 2-206 | OMe | CF₂Cl | Et | CN | |
| 2-207 | OMe | OMe | Et | CN | |
| 2-208 | OMe | NO₂ | Et | CN | |
| 2-209 | OMe | SO₂Me | Et | CN | |
| 2-210 | SO₂Me | Me | Et | CN | |
| 2-211 | SO₂Me | F | Et | CN | |
| 2-212 | SO₂Me | Cl | Et | CN | |
| 2-213 | SO₂Me | Br | Et | CN | |
| 2-214 | SO₂Me | I | Et | CN | |
| 2-215 | SO₂Me | CF₃ | Et | CN | |
| 2-216 | SO₂Me | CHF₂ | Et | CN | |
| 2-217 | SO₂Me | CF₂Cl | Et | CN | |
| 2-218 | SO₂Me | OMe | Et | CN | |
| 2-219 | SO₂Me | NO₂ | Et | CN | |
| 2-220 | SO₂Me | SO₂Me | Et | CN | |
| 2-221 | Me | Me | CH₂CH₂OMe | CN | |
| 2-222 | Me | F | CH₂CH₂OMe | CN | |
| 2-223 | Me | Cl | CH₂CH₂OMe | CN | |
| 2-224 | Me | Br | CH₂CH₂OMe | CN | |
| 2-225 | Me | I | CH₂CH₂OMe | CN | |
| 2-226 | Me | CF₃ | CH₂CH₂OMe | CN | |
| 2-227 | Me | CHF₂ | CH₂CH₂OMe | CN | |
| 2-228 | Me | CF₂Cl | CH₂CH₂OMe | CN | |
| 2-229 | Me | OMe | CH₂CH₂OMe | CN | |
| 2-230 | Me | NO₂ | CH₂CH₂OMe | CN | |
| 2-231 | Me | SO₂Me | CH₂CH₂OMe | CN | |
| 2-232 | Cl | Me | CH₂CH₂OMe | CN | |
| 2-233 | Cl | F | CH₂CH₂OMe | CN | |
| 2-234 | Cl | Cl | CH₂CH₂OMe | CN | |
| 2-235 | Cl | Br | CH₂CH₂OMe | CN | |
| 2-236 | Cl | I | CH₂CH₂OMe | CN | |
| 2-237 | Cl | CF₃ | CH₂CH₂OMe | CN | |
| 2-238 | Cl | CHF₂ | CH₂CH₂OMe | CN | |
| 2-239 | Cl | CF₂Cl | CH₂CH₂OMe | CN | |
| 2-240 | Cl | OMe | CH₂CH₂OMe | CN | |
| 2-241 | Cl | NO₂ | CH₂CH₂OMe | CN | |
| 2-242 | Cl | SO₂Me | CH₂CH₂OMe | CN | |
| 2-243 | OMe | Me | CH₂CH₂OMe | CN | |
| 2-244 | OMe | F | CH₂CH₂OMe | CN | |
| 2-245 | OMe | Cl | CH₂CH₂OMe | CN | |
| 2-246 | OMe | Br | CH₂CH₂OMe | CN | |
| 2-247 | OMe | I | CH₂CH₂OMe | CN | |
| 2-248 | OMe | CF₃ | CH₂CH₂OMe | CN | |
| 2-249 | OMe | CHF₂ | CH₂CH₂OMe | CN | |
| 2-250 | OMe | CF₂Cl | CH₂CH₂OMe | CN | |
| 2-251 | OMe | OMe | CH₂CH₂OMe | CN | |
| 2-252 | OMe | NO₂ | CH₂CH₂OMe | CN | |
| 2-253 | OMe | SO₂Me | CH₂CH₂OMe | CN | |
| 2-254 | SO₂Me | Me | CH₂CH₂OMe | CN | |
| 2-255 | SO₂Me | F | CH₂CH₂OMe | CN | |
| 2-256 | SO₂Me | Cl | CH₂CH₂OMe | CN | |
| 2-257 | SO₂Me | Br | CH₂CH₂OMe | CN | |
| 2-258 | SO₂Me | I | CH₂CH₂OMe | CN | |
| 2-259 | SO₂Me | CF₃ | CH₂CH₂OMe | CN | |
| 2-260 | SO₂Me | CHF₂ | CH₂CH₂OMe | CN | |
| 2-261 | SO₂Me | CF₂Cl | CH₂CH₂OMe | CN | |
| 2-262 | SO₂Me | OMe | CH₂CH₂OMe | CN | |
| 2-263 | SO₂Me | NO₂ | CH₂CH₂OMe | CN | |
| 2-264 | SO₂Me | SO₂Me | CH₂CH₂OMe | CN | |

**Tabelle 3: Erfindungsgemäße Verbindungen der allgemeinen Formel (I), worin Q für Q3, Rⁱ für Methyl und R^{h}, R^{k} sowie W jeweils für Wasserstoff stehen, t = 1 ist und die anderen Reste die in der Tabelle angegebenen Bedeutungen haben**

| | | | | | |
|---|---|---|---|---|---|
| 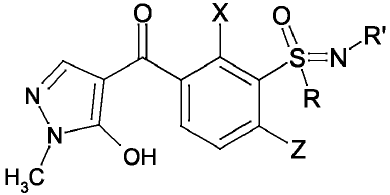 | | | | | |

| Nr. | X | Z | R | R' | Physikalische Daten (¹H-NMR) |
|---|---|---|---|---|---|
| 3-1 | Me | Me | Me | H | |
| 3-2 | Me | F | Me | H | |
| 3-3 | Me | Cl | Me | H | |
| 3-4 | Me | Br | Me | H | |
| 3-5 | Me | I | Me | H | |
| 3-6 | Me | CF₃ | Me | H | |
| 3-7 | Me | CHF₂ | Me | H | |
| 3-8 | Me | CF₂Cl | Me | H | |
| 3-9 | Me | OMe | Me | H | |
| 3-10 | Me | NO₂ | Me | H | |
| 3-11 | Me | SO₂Me | Me | H | |
| 3-12 | Cl | Me | Me | H | |
| 3-13 | Cl | F | Me | H | |
| 3-14 | Cl | Cl | Me | H | |
| 3-15 | Cl | Br | Me | H | |
| 3-16 | Cl | I | Me | H | |
| 3-17 | Cl | CF₃ | Me | H | |
| 3-18 | Cl | CHF₂ | Me | H | |
| 3-19 | Cl | CF₂Cl | Me | H | |
| 3-20 | Cl | OMe | Me | H | |
| 3-21 | Cl | NO₂ | Me | H | |
| 3-22 | Cl | SO₂Me | Me | H | |
| 3-23 | OMe | Me | Me | H | |
| 3-24 | OMe | F | Me | H | |
| 3-25 | OMe | Cl | Me | H | |
| 3-26 | OMe | Br | Me | H | |
| 3-27 | OMe | I | Me | H | |
| 3-28 | OMe | CF₃ | Me | H | |
| 3-29 | OMe | CHF₂ | Me | H | |
| 3-30 | OMe | CF₂Cl | Me | H | |
| 3-31 | OMe | OMe | Me | H | |
| 3-32 | OMe | NO₂ | Me | H | |
| 3-33 | OMe | SO₂Me | Me | H | |
| 3-34 | SO₂Me | Me | Me | H | |
| 3-35 | SO₂Me | F | Me | H | |
| 3-36 | SO₂Me | Cl | Me | H | |
| 3-37 | SO₂Me | Br | Me | H | |
| 3-38 | SO₂Me | I | Me | H | |
| 3-39 | S0₂Me | CF₃ | Me | H | |
| 3-40 | SO₂Me | CHF₂ | Me | H | |
| 3-41 | SO₂Me | CF₂Cl | Me | H | |
| 3-42 | SO₂Me | OMe | Me | H | |
| 3-43 | SO₂Me | NO₂ | Me | H | |
| 3-44 | SO₂Me | SO₂Me | Me | H | |
| 3-45 | Me | Me | Et | H | |
| 3-46 | Me | F | Et | H | |
| 3-47 | Me | Cl | Et | H | |
| 3-48 | Me | Br | Et | H | |
| 3-49 | Me | I | Et | H | |
| 3-50 | Me | CF₃ | Et | H | |
| 3-51 | Me | CHF₂ | Et | H | |
| 3-52 | Me | CF₂Cl | Et | H | |
| 3-53 | Me | OMe | Et | H | |
| 3-54 | Me | NO₂ | Et | H | |
| 3-55 | Me | SO₂Me | Et | H | |
| 3-56 | Cl | Me | Et | H | |
| 3-57 | Cl | F | Et | H | |
| 3-58 | Cl | Cl | Et | H | |
| 3-59 | Cl | Br | Et | H | |
| 3-60 | Cl | I | Et | H | |
| 3-61 | Cl | CF₃ | Et | H | |
| 3-62 | Cl | CHF₂ | Et | H | |
| 3-63 | Cl | CF₂Cl | Et | H | |
| 3-64 | Cl | OMe | Et | H | |
| 3-65 | Cl | NO₂ | Et | H | |
| 3-66 | Cl | SO₂Me | Et | H | |
| 3-67 | OMe | Me | Et | H | |
| 3-68 | OMe | F | Et | H | |
| 3-69 | OMe | Cl | Et | H | |
| 3-70 | OMe | Br | Et | H | |
| 3-71 | OMe | I | Et | H | |
| 3-72 | OMe | CF₃ | Et | H | |
| 3-73 | OMe | CHF₂ | Et | H | |
| 3-74 | OMe | CF₂Cl | Et | H | |
| 3-75 | OMe | OMe | Et | H | |
| 3-76 | OMe | NO₂ | Et | H | |
| 3-77 | OMe | SO₂Me | Et | H | |
| 3-78 | SO₂Me | Me | Et | H | |
| 3-79 | SO₂Me | F | Et | H | |
| 3-80 | SO₂Me | Cl | Et | H | |
| 3-81 | SO₂Me | Br | Et | H | |
| 3-82 | SO₂Me | I | Et | H | |
| 3-83 | SO₂Me | CF₃ | Et | H | |
| 3-84 | SO₂Me | CHF₂ | Et | H | |
| 3-85 | SO₂Me | CF₂Cl | Et | H | |
| 3-86 | SO₂Me | OMe | Et | H | |
| 3-87 | SO₂Me | NO₂ | Et | H | |
| 3-88 | SO₂Me | SO₂Me | Et | H | |
| 3-89 | Me | Me | CH₂CH₂OMe | H | |
| 3-90 | Me | F | CH₂CH₂OMe | H | |
| 3-91 | Me | Cl | CH₂CH₂OMe | H | |
| 3-92 | Me | Br | CH₂CH₂OMe | H | |
| 3-93 | Me | I | CH₂CH₂OMe | H | |
| 3-94 | Me | CF₃ | CH₂CH₂OMe | H | |
| 3-95 | Me | CHF₂ | CH₂CH₂OMe | H | |
| 3-96 | Me | CF₂Cl | CH₂CH₂OMe | H | |
| 3-97 | Me | OMe | CH₂CH₂OMe | H | |
| 3-98 | Me | NO₂ | CH₂CH₂OMe | H | |
| 3-99 | Me | SO₂Me | CH₂CH₂OMe | H | |
| 3-100 | Cl | Me | CH₂CH₂OMe | H | |
| 3-101 | Cl | F | CH₂CH₂OMe | H | |
| 3-102 | Cl | Cl | CH₂CH₂OMe | H | |
| 3-103 | Cl | Br | CH₂CH₂OMe | H | |
| 3-104 | Cl | I | CH₂CH₂OMe | H | |
| 3-105 | Cl | CF₃ | CH₂CH₂OMe | H | |
| 3-106 | Cl | CHF₂ | CH₂CH₂OMe | H | |
| 3-107 | Cl | CF₂Cl | CH₂CH₂OMe | H | |
| 3-108 | Cl | OMe | CH₂CH₂OMe | H | |
| 3-109 | Cl | NO₂ | CH₂CH₂OMe | H | |
| 3-110 | Cl | SO₂Me | CH₂CH₂OMe | H | |
| 3-111 | OMe | Me | CH₂CH₂OMe | H | |
| 3-112 | OMe | F | CH₂CH₂OMe | H | |
| 3-113 | OMe | Cl | CH₂CH₂OMe | H | |
| 3-114 | OMe | Br | CH₂CH₂OMe | H | |
| 3-115 | OMe | I | CH₂CH₂OMe | H | |
| 3-116 | OMe | CF₃ | CH₂CH₂OMe | H | |
| 3-117 | OMe | CHF₂ | CH₂CH₂OMe | H | |
| 3-118 | OMe | CF₂Cl | CH₂CH₂OMe | H | |
| 3-119 | OMe | OMe | CH₂CH₂OMe | H | |
| 3-120 | OMe | NO₂ | CH₂CH₂OMe | H | |
| 3-121 | OMe | SO₂Me | CH₂CH₂OMe | H | |
| 3-122 | SO₂Me | Me | CH₂CH₂OMe | H | |
| 3-123 | SO₂Me | F | CH₂CH₂OMe | H | |
| 3-124 | SO₂Me | Cl | CH₂CH₂OMe | H | |
| 3-125 | SO₂Me | Br | CH₂CH₂OMe | H | |
| 3-126 | SO₂Me | I | CH₂CH₂OMe | H | |
| 3-127 | SO₂Me | CF₃ | CH₂CH₂OMe | H | |
| 3-128 | SO₂Me | CHF₂ | CH₂CH₂OMe | H | |
| 3-129 | SO₂Me | CF₂Cl | CH₂CH₂OMe | H | |
| 3-130 | SO₂Me | OMe | CH₂CH₂OMe | H | |
| 3-131 | SO₂Me | NO₂ | CH₂CH₂OMe | H | |
| 3-132 | SO₂Me | SO₂Me | CH₂CH₂OMe | H | |
| 3-133 | Me | Me | Me | CN | |
| 3-134 | Me | F | Me | CN | |
| 3-135 | Me | Cl | Me | CN | |
| 3-136 | Me | Br | Me | CN | |
| 3-137 | Me | I | Me | CN | |
| 3-138 | Me | CF₃ | Me | CN | |
| 3-139 | Me | CHF₂ | Me | CN | |
| 3-140 | Me | CF₂Cl | Me | CN | |
| 3-141 | Me | OMe | Me | CN | |
| 3-142 | Me | NO₂ | Me | CN | |
| 3-143 | Me | SO₂Me | Me | CN | |
| 3-144 | Cl | Me | Me | CN | |
| 3-145 | Cl | F | Me | CN | |
| 3-146 | Cl | Cl | Me | CN | |
| 3-147 | Cl | Br | Me | CN | |
| 3-148 | Cl | I | Me | CN | |
| 3-149 | Cl | CF₃ | Me | CN | |
| 3-150 | Cl | CHF₂ | Me | CN | |
| 3-151 | Cl | CF₂Cl | Me | CN | |
| 3-152 | Cl | OMe | Me | CN | |
| 3-153 | Cl | NO₂ | Me | CN | |
| 3-154 | Cl | SO₂Me | Me | CN | |
| 3-155 | OMe | Me | Me | CN | |
| 3-156 | OMe | F | Me | CN | |
| 3-157 | OMe | Cl | Me | CN | |
| 3-158 | OMe | Br | Me | CN | |
| 3-159 | OMe | I | Me | CN | |
| 3-160 | OMe | CF₃ | Me | CN | (400 MHz, CDCl₃ δ, ppm) 7.94 (d,1H), 7.86 (d,1H), 7.52 (s,1H), 3.99 (s,3H), 3.75 (s,3H), 3.69 (s,3H) |
| 3-161 | OMe | CHF₂ | Me | CN | |
| 3-162 | OMe | CF₂Cl | Me | CN | |
| 3-163 | OMe | OMe | Me | CN | |
| 3-164 | OMe | NO₂ | Me | CN | |
| 3-165 | OMe | SO₂Me | Me | CN | |
| 3-166 | SO₂Me | Me | Me | CN | |
| 3-167 | SO₂Me | F | Me | CN | |
| 3-168 | SO₂Me | Cl | Me | CN | |
| 3-169 | SO₂Me | Br | Me | CN | |
| 3-170 | SO₂Me | I | Me | CN | |
| 3-171 | SO₂Me | CF₃ | Me | CN | |
| 3-172 | SO₂Me | CHF₂ | Me | CN | |
| 3-173 | SO₂Me | CF₂Cl | Me | CN | |
| 3-174 | SO₂Me | OMe | Me | CN | |
| 3-175 | SO₂Me | NO₂ | Me | CN | |
| 3-176 | SO₂Me | SO₂Me | Me | CN | |
| 3-177 | Me | Me | Et | CN | |
| 3-178 | Me | F | Et | CN | |
| 3-179 | Me | Cl | Et | CN | |
| 3-180 | Me | Br | Et | CN | |
| 3-181 | Me | I | Et | CN | |
| 3-182 | Me | CF₃ | Et | CN | |
| 3-183 | Me | CHF₂ | Et | CN | |
| 3-184 | Me | CF₂Cl | Et | CN | |
| 3-185 | Me | OMe | Et | CN | |
| 3-186 | Me | NO₂ | Et | CN | |
| 3-187 | Me | SO₂Me | Et | CN | |
| 3-188 | Cl | Me | Et | CN | |
| 3-189 | Cl | F | Et | CN | |
| 3-190 | Cl | Cl | Et | CN | |
| 3-191 | Cl | Br | Et | CN | |
| 3-192 | Cl | I | Et | CN | |
| 3-193 | Cl | CF₃ | Et | CN | |
| 3-194 | Cl | CHF₂ | Et | CN | |
| 3-195 | Cl | CF₂Cl | Et | CN | |
| 3-196 | Cl | OMe | Et | CN | |
| 3-197 | Cl | NO₂ | Et | CN | |
| 3-198 | Cl | SO₂Me | Et | CN | |
| 3-199 | OMe | Me | Et | CN | |
| 3-200 | OMe | F | Et | CN | |
| 3-201 | OMe | Cl | Et | CN | |
| 3-202 | OMe | Br | Et | CN | |
| 3-203 | OMe | I | Et | CN | |
| 3-204 | OMe | CF₃ | Et | CN | |
| 3-205 | OMe | CHF₂ | Et | CN | |
| 3-206 | OMe | CF₂Cl | Et | CN | |
| 3-207 | OMe | OMe | Et | CN | |
| 3-208 | OMe | NO₂ | Et | CN | |
| 3-209 | OMe | SO₂Me | Et | CN | |
| 3-210 | SO₂Me | Me | Et | CN | |
| 3-211 | SO₂Me | F | Et | CN | |
| 3-212 | SO₂Me | Cl | Et | CN | |
| 3-213 | SO₂Me | Br | Et | CN | |
| 3-214 | SO₂Me | I | Et | CN | |
| 3-215 | SO₂Me | CF₃ | Et | CN | |
| 3-216 | SO₂Me | CHF₂ | Et | CN | |
| 3-217 | SO₂Me | CF₂Cl | Et | CN | |
| 3-218 | SO₂Me | OMe | Et | CN | |
| 3-219 | SO₂Me | NO₂ | Et | CN | |
| 3-220 | SO₂Me | SO₂Me | Et | CN | |
| 3-221 | Me | Me | CH₂CH₂OMe | CN | |
| 3-222 | Me | F | CH₂CH₂OMe | CN | |
| 3-223 | Me | Cl | CH₂CH₂OMe | CN | |
| 3-224 | Me | Br | CH₂CH₂OMe | CN | |
| 3-225 | Me | I | CH₂CH₂OMe | CN | |
| 3-226 | Me | CF₃ | CH₂CH₂OMe | CN | |
| 3-227 | Me | CHF₂ | CH₂CH₂OMe | CN | |
| 3-228 | Me | CF₂Cl | CH₂CH₂OMe | CN | |
| 3-229 | Me | OMe | CH₂CH₂OMe | CN | |
| 3-230 | Me | NO₂ | CH₂CH₂OMe | CN | |
| 3-231 | Me | SO₂Me | CH₂CH₂OMe | CN | |
| 3-232 | Cl | Me | CH₂CH₂OMe | CN | |
| 3-233 | Cl | F | CH₂CH₂OMe | CN | |
| 3-234 | Cl | Cl | CH₂CH₂OMe | CN | |
| 3-235 | Cl | Br | CH₂CH₂OMe | CN | |
| 3-236 | Cl | I | CH₂CH₂OMe | CN | |
| 3-237 | Cl | CF₃ | CH₂CH₂OMe | CN | |
| 3-238 | Cl | CHF₂ | CH₂CH₂OMe | CN | |
| 3-239 | Cl | CF₂Cl | CH₂CH₂OMe | CN | |
| 3-240 | Cl | OMe | CH₂CH₂OMe | CN | |
| 3-241 | Cl | NO₂ | CH₂CH₂OMe | CN | |
| 3-242 | Cl | SO₂Me | CH₂CH₂OMe | CN | |
| 3-243 | OMe | Me | CH₂CH₂OMe | CN | |
| 3-244 | OMe | F | CH₂CH₂OMe | CN | |
| 3-245 | OMe | Cl | CH₂CH₂OMe | CN | |
| 3-246 | OMe | Br | CH₂CH₂OMe | CN | |
| 3-247 | OMe | I | CH₂CH₂OMe | CN | |
| 3-248 | OMe | CF₃ | CH₂CH₂OMe | CN | |
| 3-249 | OMe | CHF₂ | CH₂CH₂OMe | CN | |
| 3-250 | OMe | CF₂Cl | CH₂CH₂OMe | CN | |
| 3-251 | OMe | OMe | CH₂CH₂OMe | CN | |
| 3-252 | OMe | NO₂ | CH₂CH₂OMe | CN | |
| 3-253 | OMe | SO₂Me | CH₂CH₂OMe | CN | |
| 3-254 | SO₂Me | Me | CH₂CH₂OMe | CN | |
| 3-255 | SO₂Me | F | CH₂CH₂OMe | CN | |
| 3-256 | SO₂Me | Cl | CH₂CH₂OMe | CN | |
| 3-257 | SO₂Me | Br | CH₂CH₂OMe | CN | |
| 3-258 | SO₂Me | I | CH₂CH₂OMe | CN | |
| 3-259 | SO₂Me | CF₃ | CH₂CH₂OMe | CN | |
| 3-260 | SO₂Me | CHF₂ | CH₂CH₂OMe | CN | |
| 3-261 | SO₂Me | CF₂Cl | CH₂CH₂OMe | CN | |
| 3-262 | SO₂Me | OMe | CH₂CH₂OMe | CN | |
| 3-263 | SO₂Me | NO₂ | CH₂CH₂OMe | CN | |
| 3-264 | SO₂Me | SO₂Me | CH₂CH₂OMe | CN | |

**Tabelle 3a: Erfindungsgemäße Verbindungen der allgemeinen Formel (I), worin Q für Q3, Rⁱ für Ethyl und R^{h}, R^{k} sowie W jeweils für Wasserstoff stehen, t = 1 ist und die anderen Reste die in der Tabelle angegebenen Bedeutungen haben**

| | | | | | |
|---|---|---|---|---|---|
| 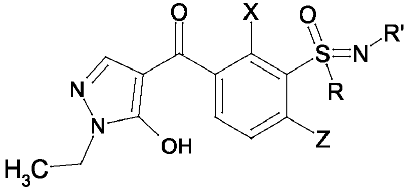 | | | | | |

| Nr. | X | Z | R | R' | Physikalische Daten (¹H-NMR) |
|---|---|---|---|---|---|
| 3a-1 | Me | Me | Me | H | |
| 3a-2 | Me | F | Me | H | |
| 3a-3 | Me | Cl | Me | H | |
| 3a-4 | Me | Br | Me | H | |
| 3a-5 | Me | I | Me | H | |
| 3a-6 | Me | CF₃ | Me | H | |
| 3a-7 | Me | CHF₂ | Me | H | |
| 3a-8 | Me | CF₂Cl | Me | H | |
| 3a-9 | Me | OMe | Me | H | |
| 3a-10 | Me | NO₂ | Me | H | |
| 3a-11 | Me | SO₂Me | Me | H | |
| 3a-12 | Cl | Me | Me | H | |
| 3a-13 | Cl | F | Me | H | |
| 3a-14 | Cl | Cl | Me | H | |
| 3a-15 | Cl | Br | Me | H | |
| 3a-16 | Cl | I | Me | H | |
| 3a-17 | Cl | CF₃ | Me | H | |
| 3a-18 | Cl | CHF₂ | Me | H | |
| 3a-19 | Cl | CF₂Cl | Me | H | |
| 3a-20 | Cl | OMe | Me | H | |
| 3a-21 | Cl | NO₂ | Me | H | |
| 3a-22 | Cl | SO₂Me | Me | H | |
| 3a-23 | OMe | Me | Me | H | |
| 3a-24 | OMe | F | Me | H | |
| 3a-25 | OMe | Cl | Me | H | |
| 3a-26 | OMe | Br | Me | H | |
| 3a-27 | OMe | I | Me | H | |
| 3a-28 | OMe | CF₃ | Me | H | |
| 3a-29 | OMe | CHF₂ | Me | H | |
| 3a-30 | OMe | CF₂Cl | Me | H | |
| 3a-31 | OMe | OMe | Me | H | |
| 3a-32 | OMe | NO₂ | Me | H | |
| 3a-33 | OMe | SO₂Me | Me | H | |
| 3a-34 | SO₂Me | Me | Me | H | |
| 3a-35 | SO₂Me | F | Me | H | |
| 3a-36 | SO₂Me | Cl | Me | H | |
| 3a-37 | SO₂Me | Br | Me | H | |
| 3a-38 | SO₂Me | I | Me | H | |
| 3a-39 | SO₂Me | CF₃ | Me | H | |
| 3a-40 | SO₂Me | CHF₂ | Me | H | |
| 3a-41 | SO₂Me | CF₂Cl | Me | H | |
| 3a-42 | SO₂Me | OMe | Me | H | |
| 3a-43 | SO₂Me | NO₂ | Me | H | |
| 3a-44 | SO₂Me | SO₂Me | Me | H | |
| 3a-45 | Me | Me | Et | H | |
| 3a-46 | Me | F | Et | H | |
| 3a-47 | Me | Cl | Et | H | |
| 3a-48 | Me | Br | Et | H | |
| 3a-49 | Me | I | Et | H | |
| 3a-50 | Me | CF₃ | Et | H | |
| 3a-51 | Me | CHF₂ | Et | H | |
| 3a-52 | Me | CF₂Cl | Et | H | |
| 3a-53 | Me | OMe | Et | H | |
| 3a-54 | Me | NO₂ | Et | H | |
| 3a-55 | Me | SO₂Me | Et | H | |
| 3a-56 | Cl | Me | Et | H | |
| 3a-57 | Cl | F | Et | H | |
| 3a-58 | Cl | Cl | Et | H | |
| 3a-59 | Cl | Br | Et | H | |
| 3a-60 | Cl | I | Et | H | |
| 3a-61 | Cl | CF₃ | Et | H | |
| 3a-62 | Cl | CHF₂ | Et | H | |
| 3a-63 | Cl | CF₂Cl | Et | H | |
| 3a-64 | Cl | OMe | Et | H | |
| 3a-65 | Cl | NO₂ | Et | H | |
| 3a-66 | Cl | SO₂Me | Et | H | |
| 3a-67 | OMe | Me | Et | H | |
| 3a-68 | OMe | F | Et | H | |
| 3a-69 | OMe | Cl | Et | H | |
| 3a-70 | OMe | Br | Et | H | |
| 3a-71 | OMe | I | Et | H | |
| 3a-72 | OMe | CF₃ | Et | H | |
| 3a-73 | OMe | CHF₂ | Et | H | |
| 3a-74 | OMe | CF₂Cl | Et | H | |
| 3a-75 | OMe | OMe | Et | H | |
| 3a-76 | OMe | NO₂ | Et | H | |
| 3a-77 | OMe | SO₂Me | Et | H | |
| 3a-78 | SO₂Me | Me | Et | H | |
| 3a-79 | SO₂Me | F | Et | H | |
| 3a-80 | SO₂Me | Cl | Et | H | |
| 3a-81 | SO₂Me | Br | Et | H | |
| 3a-82 | SO₂Me | I | Et | H | |
| 3a-83 | SO₂Me | CF₃ | Et | H | |
| 3a-84 | SO₂Me | CHF₂ | Et | H | |
| 3a-85 | SO₂Me | CF₂Cl | Et | H | |
| 3a-86 | SO₂Me | OMe | Et | H | |
| 3a-87 | SO₂Me | NO₂ | Et | H | |
| 3a-88 | SO₂Me | SO₂Me | Et | H | |
| 3a-89 | Me | Me | CH₂CH₂OMe | H | |
| 3a-90 | Me | F | CH₂CH₂OMe | H | |
| 3a-91 | Me | Cl | CH₂CH₂OMe | H | |
| 3a-92 | Me | Br | CH₂CH₂OMe | H | |
| 3a-93 | Me | I | CH₂CH₂OMe | H | |
| 3a-94 | Me | CF₃ | CH₂CH₂OMe | H | |
| 3a-95 | Me | CHF₂ | CH₂CH₂OMe | H | |
| 3a-96 | Me | CF₂Cl | CH₂CH₂OMe | H | |
| 3a-97 | Me | OMe | CH₂CH₂OMe | H | |
| 3a-98 | Me | NO₂ | CH₂CH₂OMe | H | |
| 3a-99 | Me | SO₂Me | CH₂CH₂OMe | H | |
| 3a-100 | Cl | Me | CH₂CH₂OMe | H | |
| 3a-101 | Cl | F | CH₂CH₂OMe | H | |
| 3a-102 | Cl | Cl | CH₂CH₂OMe | H | |
| 3a-103 | Cl | Br | CH₂CH₂OMe | H | |
| 3a-104 | Cl | I | CH₂CH₂OMe | H | |
| 3a-105 | Cl | CF₃ | CH₂CH₂OMe | H | |
| 3a-106 | Cl | CHF₂ | CH₂CH₂OMe | H | |
| 3a-107 | Cl | CF₂Cl | CH₂CH₂OMe | H | |
| 3a-108 | Cl | OMe | CH₂CH₂OMe | H | |
| 3a-109 | Cl | NO₂ | CH₂CH₂OMe | H | |
| 3a-110 | Cl | SO₂Me | CH₂CH₂OMe | H | |
| 3a-111 | OMe | Me | CH₂CH₂OMe | H | |
| 3a-112 | OMe | F | CH₂CH₂OMe | H | |
| 3a-113 | OMe | Cl | CH₂CH₂OMe | H | |
| 3a-114 | OMe | Br | CH₂CH₂OMe | H | |
| 3a-115 | OMe | I | CH₂CH₂OMe | H | |
| 3a-116 | OMe | CF₃ | CH₂CH₂OMe | H | |
| 3a-117 | OMe | CHF₂ | CH₂CH₂OMe | H | |
| 3a-118 | OMe | CF₂Cl | CH₂CH₂OMe | H | |
| 3a-119 | OMe | OMe | CH₂CH₂OMe | H | |
| 3a-120 | OMe | NO₂ | CH₂CH₂OMe | H | |
| 3a-121 | OMe | SO₂Me | CH₂CH₂OMe | H | |
| 3a-122 | SO₂Me | Me | CH₂CH₂OMe | H | |
| 3a-123 | SO₂Me | F | CH₂CH₂OMe | H | |
| 3a-124 | SO₂Me | Cl | CH₂CH₂OMe | H | |
| 3a-125 | SO₂Me | Br | CH₂CH₂OMe | H | |
| 3a-126 | SO₂Me | I | CH₂CH₂OMe | H | |
| 3a-127 | SO₂Me | CF₃ | CH₂CH₂OMe | H | |
| 3a-128 | SO₂Me | CHF₂ | CH₂CH₂OMe | H | |
| 3a-129 | SO₂Me | CF₂Cl | CH₂CH₂OMe | H | |
| 3a-130 | SO₂Me | OMe | CH₂CH₂OMe | H | |
| 3a-131 | SO₂Me | NO₂ | CH₂CH₂OMe | H | |
| 3a-132 | SO₂Me | SO₂Me | CH₂CH₂OMe | H | |
| 3a-133 | Me | Me | Me | CN | |
| 3a-134 | Me | F | Me | CN | |
| 3a-135 | Me | Cl | Me | CN | |
| 3a-136 | Me | Br | Me | CN | |
| 3a-137 | Me | I | Me | CN | |
| 3a-138 | Me | CF₃ | Me | CN | |
| 3a-139 | Me | CHF₂ | Me | CN | |
| 3a-140 | Me | CF₂Cl | Me | CN | |
| 3a-141 | Me | OMe | Me | CN | |
| 3a-142 | Me | NO₂ | Me | CN | |
| 3a-143 | Me | SO₂Me | Me | CN | |
| 3a-144 | Cl | Me | Me | CN | |
| 3a-145 | Cl | F | Me | CN | |
| 3a-146 | Cl | Cl | Me | CN | |
| 3a-147 | Cl | Br | Me | CN | |
| 3a-148 | Cl | I | Me | CN | |
| 3a-149 | Cl | CF₃ | Me | CN | |
| 3a-150 | Cl | CHF₂ | Me | CN | |
| 3a-151 | Cl | CF₂Cl | Me | CN | |
| 3a-152 | Cl | OMe | Me | CN | |
| 3a-153 | Cl | NO₂ | Me | CN | |
| 3a-154 | Cl | SO₂Me | Me | CN | |
| 3a-155 | OMe | Me | Me | CN | |
| 3a-156 | OMe | F | Me | CN | |
| 3a-157 | OMe | Cl | Me | CN | |
| 3a-158 | OMe | Br | Me | CN | |
| 3a-159 | OMe | I | Me | CN | |
| 3a-160 | OMe | CF₃ | Me | CN | |
| 3a-161 | OMe | CHF₂ | Me | CN | |
| 3a-162 | OMe | CF₂Cl | Me | CN | |
| 3a-163 | OMe | OMe | Me | CN | |
| 3a-164 | OMe | NO₂ | Me | CN | |
| 3a-165 | OMe | SO₂Me | Me | CN | |
| 3a-166 | SO₂Me | Me | Me | CN | |
| 3a-167 | SO₂Me | F | Me | CN | |
| 3a-168 | SO₂Me | Cl | Me | CN | |
| 3a-169 | SO₂Me | Br | Me | CN | |
| 3a-170 | SO₂Me | I | Me | CN | |
| 3a-171 | SO₂Me | CF₃ | Me | CN | |
| 3a-172 | SO₂Me | CHF₂ | Me | CN | |
| 3a-173 | SO₂Me | CF₂Cl | Me | CN | |
| 3a-174 | SO₂Me | OMe | Me | CN | |
| 3a-175 | SO₂Me | NO₂ | Me | CN | |
| 3a-176 | SO₂Me | SO₂Me | Me | CN | |
| 3a-177 | Me | Me | Et | CN | |
| 3a-178 | Me | F | Et | CN | |
| 3a-179 | Me | Cl | Et | CN | |
| 3a-180 | Me | Br | Et | CN | |
| 3a-181 | Me | I | Et | CN | |
| 3a-182 | Me | CF₃ | Et | CN | |
| 3a-183 | Me | CHF₂ | Et | CN | |
| 3a-184 | Me | CF₂Cl | Et | CN | |
| 3a-185 | Me | OMe | Et | CN | |
| 3a-186 | Me | NO₂ | Et | CN | |
| 3a-187 | Me | SO₂Me | Et | CN | |
| 3a-188 | Cl | Me | Et | CN | |
| 3a-189 | Cl | F | Et | CN | |
| 3a-190 | Cl | Cl | Et | CN | |
| 3a-191 | Cl | Br | Et | CN | |
| 3a-192 | Cl | I | Et | CN | |
| 3a-193 | Cl | CF₃ | Et | CN | |
| 3a-194 | Cl | CHF₂ | Et | CN | |
| 3a-195 | Cl | CF₂Cl | Et | CN | |
| 3a-196 | Cl | OMe | Et | CN | |
| 3a-197 | Cl | NO₂ | Et | CN | |
| 3a-198 | Cl | SO₂Me | Et | CN | |
| 3a-199 | OMe | Me | Et | CN | |
| 3a-200 | OMe | F | Et | CN | |
| 3a-201 | OMe | Cl | Et | CN | |
| 3a-202 | OMe | Br | Et | CN | |
| 3a-203 | OMe | I | Et | CN | |
| 3a-204 | OMe | CF₃ | Et | CN | |
| 3a-205 | OMe | CHF₂ | Et | CN | |
| 3a-206 | OMe | CF₂Cl | Et | CN | |
| 3a-207 | OMe | OMe | Et | CN | |
| 3a-208 | OMe | NO₂ | Et | CN | |
| 3a-209 | OMe | SO₂Me | Et | CN | |
| 3a-210 | SO₂Me | Me | Et | CN | |
| 3a-211 | SO₂Me | F | Et | CN | |
| 3a-212 | SO₂Me | Cl | Et | CN | |
| 3a-213 | SO₂Me | Br | Et | CN | |
| 3a-214 | SO₂Me | I | Et | CN | |
| 3a-215 | SO₂Me | CF₃ | Et | CN | |
| 3a-216 | SO₂Me | CHF₂ | Et | CN | |
| 3a-217 | SO₂Me | CF₂Cl | Et | CN | |
| 3a-218 | SO₂Me | OMe | Et | CN | |
| 3a-219 | SO₂Me | NO₂ | Et | CN | |
| 3a-220 | SO₂Me | SO₂Me | Et | CN | |
| 3a-221 | Me | Me | CH₂CH₂OMe | CN | |
| 3a-222 | Me | F | CH₂CH₂OMe | CN | |
| 3a-223 | Me | Cl | CH₂CH₂OMe | CN | |
| 3a-224 | Me | Br | CH₂CH₂OMe | CN | |
| 3a-225 | Me | I | CH₂CH₂OMe | CN | |
| 3a-226 | Me | CF₃ | CH₂CH₂OMe | CN | |
| 3a-227 | Me | CHF₂ | CH₂CH₂OMe | CN | |
| 3a-228 | Me | CF₂Cl | CH₂CH₂OMe | CN | |
| 3a-229 | Me | OMe | CH₂CH₂OMe | CN | |
| 3a-230 | Me | NO₂ | CH₂CH₂OMe | CN | |
| 3a-231 | Me | SO₂Me | CH₂CH₂OMe | CN | |
| 3a-232 | Cl | Me | CH₂CH₂OMe | CN | |
| 3a-233 | Cl | F | CH₂CH₂OMe | CN | |
| 3a-234 | Cl | Cl | CH₂CH₂OMe | CN | |
| 3a-235 | Cl | Br | CH₂CH₂OMe | CN | |
| 3a-236 | Cl | I | CH₂CH₂OMe | CN | |
| 3a-237 | Cl | CF₃ | CH₂CH₂OMe | CN | |
| 3a-238 | Cl | CHF₂ | CH₂CH₂OMe | CN | |
| 3a-239 | Cl | CF₂Cl | CH₂CH₂OMe | CN | |
| 3a-240 | Cl | OMe | CH₂CH₂OMe | CN | |
| 3a-241 | Cl | NO₂ | CH₂CH₂OMe | CN | |
| 3a-242 | Cl | SO₂Me | CH₂CH₂OMe | CN | |
| 3a-243 | OMe | Me | CH₂CH₂OMe | CN | |
| 3a-244 | OMe | F | CH₂CH₂OMe | CN | |
| 3a-245 | OMe | Cl | CH₂CH₂OMe | CN | |
| 3a-246 | OMe | Br | CH₂CH₂OMe | CN | |
| 3a-247 | OMe | I | CH₂CH₂OMe | CN | |
| 3a-248 | OMe | CF₃ | CH₂CH₂OMe | CN | |
| 3a-249 | OMe | CHF₂ | CH₂CH₂OMe | CN | |
| 3a-250 | OMe | CF₂Cl | CH₂CH₂OMe | CN | |
| 3a-251 | OMe | OMe | CH₂CH₂OMe | CN | |
| 3a-252 | OMe | NO₂ | CH₂CH₂OMe | CN | |
| 3a-253 | OMe | SO₂Me | CH₂CH₂OMe | CN | |
| 3a-254 | SO₂Me | Me | CH₂CH₂OMe | CN | |
| 3a-255 | SO₂Me | F | CH₂CH₂OMe | CN | |
| 3a-256 | SO₂Me | Cl | CH₂CH₂OMe | CN | |
| 3a-257 | SO₂Me | Br | CH₂CH₂OMe | CN | |
| 3a-258 | SO₂Me | I | CH₂CH₂OMe | CN | |
| 3a-259 | SO₂Me | CF₃ | CH₂CH₂OMe | CN | |
| 3a-260 | SO₂Me | CHF₂ | CH₂CH₂OMe | CN | |
| 3a-261 | SO₂Me | CF₂Cl | CH₂CH₂OMe | CN | |
| 3a-262 | SO₂Me | OMe | CH₂CH₂OMe | CN | |
| 3a-263 | SO₂Me | NO₂ | CH₂CH₂OMe | CN | |
| 3a-264 | SO₂Me | SO₂Me | CH₂CH₂OMe | CN | |

**Tabelle 4: Erfindungsgemäße Verbindungen der allgemeinen Formel (I), worin Q für Q3, Rⁱ und R^{k} jeweils für Methyl, R^{h} sowie W jeweils für Wasserstoff stehen, t = 1 ist und die anderen Reste die in der Tabelle angegebenen Bedeutungen haben**

| | | | | | |
|---|---|---|---|---|---|
| 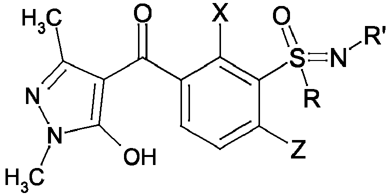 | | | | | |

| Nr. | X | Z | R | R' | Physikalische Daten (1H-NMR) |
|---|---|---|---|---|---|
| 4-1 | Me | Me | Me | H | |
| 4-2 | Me | F | Me | H | |
| 4-3 | Me | Cl | Me | H | |
| 4-4 | Me | Br | Me | H | |
| 4-5 | Me | I | Me | H | |
| 4-6 | Me | CF₃ | Me | H | |
| 4-7 | Me | CHF₂ | Me | H | |
| 4-8 | Me | CF₂Cl | Me | H | |
| 4-9 | Me | OMe | Me | H | |
| 4-10 | Me | NO₂ | Me | H | |
| 4-11 | Me | SO₂Me | Me | H | |
| 4-12 | Cl | Me | Me | H | |
| 4-13 | Cl | F | Me | H | |
| 4-14 | Cl | Cl | Me | H | |
| 4-15 | Cl | Br | Me | H | |
| 4-16 | Cl | I | Me | H | |
| 4-17 | Cl | CF₃ | Me | H | |
| 4-18 | Cl | CHF₂ | Me | H | |
| 4-19 | Cl | CF₂Cl | Me | H | |
| 4-20 | Cl | OMe | Me | H | |
| 4-21 | Cl | NO₂ | Me | H | |
| 4-22 | Cl | SO₂Me | Me | H | |
| 4-23 | OMe | Me | Me | H | |
| 4-24 | OMe | F | Me | H | |
| 4-25 | OMe | Cl | Me | H | |
| 4-26 | OMe | Br | Me | H | |
| 4-27 | OMe | I | Me | H | |
| 4-28 | OMe | CF₃ | Me | H | |
| 4-29 | OMe | CHF₂ | Me | H | |
| 4-30 | OMe | CF₂Cl | Me | H | |
| 4-31 | OMe | OMe | Me | H | |
| 4-32 | OMe | NO₂ | Me | H | |
| 4-33 | OMe | SO₂Me | Me | H | |
| 4-34 | SO₂Me | Me | Me | H | |
| 4-35 | SO₂Me | F | Me | H | |
| 4-36 | SO₂Me | Cl | Me | H | |
| 4-37 | SO₂Me | Br | Me | H | |
| 4-38 | SO₂Me | I | Me | H | |
| 4-39 | SO₂Me | CF₃ | Me | H | |
| 4-40 | SO₂Me | CHF₂ | Me | H | |
| 4-41 | SO₂Me | CF₂Cl | Me | H | |
| 4-42 | SO₂Me | OMe | Me | H | |
| 4-43 | SO₂Me | NO₂ | Me | H | |
| 4-44 | SO₂Me | SO₂Me | Me | H | |
| 4-45 | Me | Me | Et | H | |
| 4-46 | Me | F | Et | H | |
| 4-47 | Me | Cl | Et | H | |
| 4-48 | Me | Br | Et | H | |
| 4-49 | Me | I | Et | H | |
| 4-50 | Me | CF₃ | Et | H | |
| 4-51 | Me | CHF₂ | Et | H | |
| 4-52 | Me | CF₂Cl | Et | H | |
| 4-53 | Me | OMe | Et | H | |
| 4-54 | Me | NO₂ | Et | H | |
| 4-55 | Me | SO₂Me | Et | H | |
| 4-56 | Cl | Me | Et | H | |
| 4-57 | Cl | F | Et | H | |
| 4-58 | Cl | Cl | Et | H | |
| 4-59 | Cl | Br | Et | H | |
| 4-60 | Cl | I | Et | H | |
| 4-61 | Cl | CF₃ | Et | H | |
| 4-62 | Cl | CHF₂ | Et | H | |
| 4-63 | Cl | CF₂Cl | Et | H | |
| 4-64 | Cl | OMe | Et | H | |
| 4-65 | Cl | NO₂ | Et | H | |
| 4-66 | Cl | SO₂Me | Et | H | |
| 4-67 | OMe | Me | Et | H | |
| 4-68 | OMe | F | Et | H | |
| 4-69 | OMe | Cl | Et | H | |
| 4-70 | OMe | Br | Et | H | |
| 4-71 | OMe | I | Et | H | |
| 4-72 | OMe | CF₃ | Et | H | |
| 4-73 | OMe | CHF₂ | Et | H | |
| 4-74 | OMe | CF₂Cl | Et | H | |
| 4-75 | OMe | OMe | Et | H | |
| 4-76 | OMe | NO₂ | Et | H | |
| 4-77 | OMe | SO₂Me | Et | H | |
| 4-78 | SO₂Me | Me | Et | H | |
| 4-79 | SO₂Me | F | Et | H | |
| 4-80 | SO₂Me | Cl | Et | H | |
| 4-81 | SO₂Me | Br | Et | H | |
| 4-82 | SO₂Me | I | Et | H | |
| 4-83 | SO₂Me | CF₃ | Et | H | |
| 4-84 | SO₂Me | CHF₂ | Et | H | |
| 4-85 | SO₂Me | CF₂Cl | Et | H | |
| 4-86 | SO₂Me | OMe | Et | H | |
| 4-87 | SO₂Me | NO₂ | Et | H | |
| 4-88 | SO₂Me | SO₂Me | Et | H | |
| 4-89 | Me | Me | CH₂CH₂OMe | H | |
| 4-90 | Me | F | CH₂CH₂OMe | H | |
| 4-91 | Me | Cl | CH₂CH₂OMe | H | |
| 4-92 | Me | Br | CH₂CH₂OMe | H | |
| 4-93 | Me | I | CH₂CH₂OMe | H | |
| 4-94 | Me | CF₃ | CH₂CH₂OMe | H | |
| 4-95 | Me | CHF₂ | CH₂CH₂OMe | H | |
| 4-96 | Me | CF₂Cl | CH₂CH₂OMe | H | |
| 4-97 | Me | OMe | CH₂CH₂OMe | H | |
| 4-98 | Me | NO₂ | CH₂CH₂OMe | H | |
| 4-99 | Me | SO₂Me | CH₂CH₂OMe | H | |
| 4-100 | Cl | Me | CH₂CH₂OMe | H | |
| 4-101 | Cl | F | CH₂CH₂OMe | H | |
| 4-102 | Cl | Cl | CH₂CH₂OMe | H | |
| 4-103 | Cl | Br | CH₂CH₂OMe | H | |
| 4-104 | Cl | I | CH₂CH₂OMe | H | |
| 4-105 | Cl | CF₃ | CH₂CH₂OMe | H | |
| 4-106 | Cl | CHF₂ | CH₂CH₂OMe | H | |
| 4-107 | Cl | CF₂C1 | CH₂CH₂OMe | H | |
| 4-108 | Cl | OMe | CH₂CH₂OMe | H | |
| 4-109 | Cl | NO₂ | CH₂CH₂OMe | H | |
| 4-110 | Cl | SO₂Me | CH₂CH₂OMe | H | |
| 4-111 | OMe | Me | CH₂CH₂OMe | H | |
| 4-112 | OMe | F | CH₂CH₂OMe | H | |
| 4-113 | OMe | Cl | CH₂CH₂OMe | H | |
| 4-114 | OMe | Br | CH₂CH₂OMe | H | |
| 4-115 | OMe | I | CH₂CH₂OMe | H | |
| 4-116 | OMe | CF₃ | CH₂CH₂OMe | H | |
| 4-117 | OMe | CHF₂ | CH₂CH₂OMe | H | |
| 4-118 | OMe | CF₂Cl | CH₂CH₂OMe | H | |
| 4-119 | OMe | OMe | CH₂CH₂OMe | H | |
| 4-120 | OMe | NO₂ | CH₂CH₂OMe | H | |
| 4-121 | OMe | SO₂Me | CH₂CH₂OMe | H | |
| 4-122 | SO₂Me | Me | CH₂CH₂OMe | H | |
| 4-123 | SO₂Me | F | CH₂CH₂OMe | H | |
| 4-124 | SO₂Me | Cl | CH₂CH₂OMe | H | |
| 4-125 | SO₂Me | Br | CH₂CH₂OMe | H | |
| 4-126 | SO₂Me | I | CH₂CH₂OMe | H | |
| 4-127 | SO₂Me | CF₃ | CH₂CH₂OMe | H | |
| 4-128 | SO₂Me | CHF₂ | CH₂CH₂OMe | H | |
| 4-129 | SO₂Me | CF₂Cl | CH₂CH₂OMe | H | |
| 4-130 | SO₂Me | OMe | CH₂CH₂OMe | H | |
| 4-131 | SO₂Me | NO₂ | CH₂CH₂OMe | H | |
| 4-132 | SO₂Me | SO₂Me | CH₂CH₂OMe | H | |
| 4-133 | Me | Me | Me | CN | |
| 4-134 | Me | F | Me | CN | |
| 4-135 | Me | Cl | Me | CN | |
| 4-136 | Me | Br | Me | CN | |
| 4-137 | Me | I | Me | CN | |
| 4-138 | Me | CF₃ | Me | CN | |
| 4-139 | Me | CHF₂ | Me | CN | |
| 4-140 | Me | CF₂Cl | Me | CN | |
| 4-141 | Me | OMe | Me | CN | |
| 4-142 | Me | NO₂ | Me | CN | |
| 4-143 | Me | SO₂Me | Me | CN | |
| 4-144 | Cl | Me | Me | CN | |
| 4-145 | Cl | F | Me | CN | |
| 4-146 | Cl | Cl | Me | CN | |
| 4-147 | Cl | Br | Me | CN | |
| 4-148 | Cl | I | Me | CN | |
| 4-149 | Cl | CF₃ | Me | CN | |
| 4-150 | Cl | CHF₂ | Me | CN | |
| 4-151 | Cl | CF₂Cl | Me | CN | |
| 4-152 | Cl | OMe | Me | CN | |
| 4-153 | Cl | NO₂ | Me | CN | |
| 4-154 | Cl | SO₂Me | Me | CN | |
| 4-155 | OMe | Me | Me | CN | |
| 4-156 | OMe | F | Me | CN | |
| 4-157 | OMe | Cl | Me | CN | |
| 4-158 | OMe | Br | Me | CN | |
| 4-159 | OMe | I | Me | CN | |
| 4-160 | OMe | CF₃ | Me | CN | (400 MHz, CDCl₃ δ, ppm) 7.85 (d,1H), 7.75 (d,1H), 4.03 (s,3H), 3.68 (s,3H), 3.65 (s,3H), 1.90 (s,3H) |
| 4-161 | OMe | CHF₂ | Me | CN | |
| 4-162 | OMe | CF₂Cl | Me | CN | |
| 4-163 | OMe | OMe | Me | CN | |
| 4-164 | OMe | NO₂ | Me | CN | |
| 4-165 | OMe | SO₂Me | Me | CN | |
| 4-166 | SO₂Me | Me | Me | CN | |
| 4-167 | SO₂Me | F | Me | CN | |
| 4-168 | SO₂Me | Cl | Me | CN | |
| 4-169 | SO₂Me | Br | Me | CN | |
| 4-170 | SO₂Me | I | Me | CN | |
| 4-171 | SO₂Me | CF₃ | Me | CN | |
| 4-172 | SO₂Me | CHF₂ | Me | CN | |
| 4-173 | SO₂Me | CF₂Cl | Me | CN | |
| 4-174 | SO₂Me | OMe | Me | CN | |
| 4-175 | SO₂Me | NO₂ | Me | CN | |
| 4-176 | SO₂Me | SO₂Me | Me | CN | |
| 4-177 | Me | Me | Et | CN | |
| 4-178 | Me | F | Et | CN | |
| 4-179 | Me | Cl | Et | CN | |
| 4-180 | Me | Br | Et | CN | |
| 4-181 | Me | I | Et | CN | |
| 4-182 | Me | CF₃ | Et | CN | |
| 4-183 | Me | CHF₂ | Et | CN | |
| 4-184 | Me | CF₂Cl | Et | CN | |
| 4-185 | Me | OMe | Et | CN | |
| 4-186 | Me | NO₂ | Et | CN | |
| 4-187 | Me | SO₂Me | Et | CN | |
| 4-188 | Cl | Me | Et | CN | |
| 4-189 | Cl | F | Et | CN | |
| 4-190 | Cl | Cl | Et | CN | |
| 4-191 | Cl | Br | Et | CN | |
| 4-192 | Cl | I | Et | CN | |
| 4-193 | Cl | CF₃ | Et | CN | |
| 4-194 | Cl | CHF₂ | Et | CN | |
| 4-195 | Cl | CF₂Cl | Et | CN | |
| 4-196 | Cl | OMe | Et | CN | |
| 4-197 | Cl | NO₂ | Et | CN | |
| 4-198 | Cl | SO₂Me | Et | CN | |
| 4-199 | OMe | Me | Et | CN | |
| 4-200 | OMe | F | Et | CN | |
| 4-201 | OMe | Cl | Et | CN | |
| 4-202 | OMe | Br | Et | CN | |
| 4-203 | OMe | I | Et | CN | |
| 4-204 | OMe | CF₃ | Et | CN | |
| 4-205 | OMe | CHF₂ | Et | CN | |
| 4-206 | OMe | CF₂Cl | Et | CN | |
| 4-207 | OMe | OMe | Et | CN | |
| 4-208 | OMe | NO₂ | Et | CN | |
| 4-209 | OMe | SO₂Me | Et | CN | |
| 4-210 | SO₂Me | Me | Et | CN | |
| 4-211 | SO₂Me | F | Et | CN | |
| 4-212 | SO₂Me | Cl | Et | CN | |
| 4-213 | SO₂Me | Br | Et | CN | |
| 4-214 | SO₂Me | I | Et | CN | |
| 4-215 | SO₂Me | CF₃ | Et | CN | |
| 4-216 | SO₂Me | CHF₂ | Et | CN | |
| 4-217 | SO₂Me | CF₂Cl | Et | CN | |
| 4-218 | SO₂Me | OMe | Et | CN | |
| 4-219 | SO₂Me | NO₂ | Et | CN | |
| 4-220 | SO₂Me | SO₂Me | Et | CN | |
| 4-221 | Me | Me | CH₂CH₂OMe | CN | |
| 4-222 | Me | F | CH₂CH₂OMe | CN | |
| 4-223 | Me | Cl | CH₂CH₂OMe | CN | |
| 4-224 | Me | Br | CH₂CH₂OMe | CN | |
| 4-225 | Me | I | CH₂CH₂OMe | CN | |
| 4-226 | Me | CF₃ | CH₂CH₂OMe | CN | |
| 4-227 | Me | CHF₂ | CH₂CH₂OMe | CN | |
| 4-228 | Me | CF₂Cl | CH₂CH₂OMe | CN | |
| 4-229 | Me | OMe | CH₂CH₂OMe | CN | |
| 4-230 | Me | NO₂ | CH₂CH₂OMe | CN | |
| 4-231 | Me | SO₂Me | CH₂CH₂OMe | CN | |
| 4-232 | Cl | Me | CH₂CH₂OMe | CN | |
| 4-233 | Cl | F | CH₂CH₂OMe | CN | |
| 4-234 | Cl | Cl | CH₂CH₂OMe | CN | |
| 4-235 | Cl | Br | CH₂CH₂OMe | CN | |
| 4-236 | Cl | I | CH₂CH₂OMe | CN | |
| 4-237 | Cl | CF₃ | CH₂CH₂OMe | CN | |
| 4-238 | Cl | CHF₂ | CH₂CH₂OMe | CN | |
| 4-239 | Cl | CF₂Cl | CH₂CH₂OMe | CN | |
| 4-240 | Cl | OMe | CH₂CH₂OMe | CN | |
| 4-241 | Cl | NO₂ | CH₂CH₂OMe | CN | |
| 4-242 | Cl | SO₂Me | CH₂CH₂OMe | CN | |
| 4-243 | OMe | Me | CH₂CH₂OMe | CN | |
| 4-244 | OMe | F | CH₂CH₂OMe | CN | |
| 4-245 | OMe | Cl | CH₂CH₂OMe | CN | |
| 4-246 | OMe | Br | CH₂CH₂OMe | CN | |
| 4-247 | OMe | I | CH₂CH₂OMe | CN | |
| 4-248 | OMe | CF₃ | CH₂CH₂OMe | CN | |
| 4-249 | OMe | CHF₂ | CH₂CH₂OMe | CN | |
| 4-250 | OMe | CF₂Cl | CH₂CH₂OMe | CN | |
| 4-251 | OMe | OMe | CH₂CH₂OMe | CN | |
| 4-252 | OMe | NO₂ | CH₂CH₂OMe | CN | |
| 4-253 | OMe | SO₂Me | CH₂CH₂OMe | CN | |
| 4-254 | SO₂Me | Me | CH₂CH₂OMe | CN | |
| 4-255 | SO₂Me | F | CH₂CH₂OMe | CN | |
| 4-256 | SO₂Me | Cl | CH₂CH₂OMe | CN | |
| 4-257 | SO₂Me | Br | CH₂CH₂OMe | CN | |
| 4-258 | SO₂Me | I | CH₂CH₂OMe | CN | |
| 4-259 | SO₂Me | CF₃ | CH₂CH₂OMe | CN | |
| 4-260 | SO₂Me | CHF₂ | CH₂CH₂OMe | CN | |
| 4-261 | SO₂Me | CF₂Cl | CH₂CH₂OMe | CN | |
| 4-262 | SO₂Me | OMe | CH₂CH₂OMe | CN | |
| 4-263 | SO₂Me | NO₂ | CH₂CH₂OMe | CN | |
| 4-264 | SO₂Me | SO₂Me | CH₂CH₂OMe | CN | |

**Tabelle 5: Erfindungsgemäße Verbindungen der allgemeinen Formel (I), worin Q für Q4 steht, W Wasserstoff bedeutet, t = 1 ist und die anderen Reste die in der Tabelle angegebenen Bedeutungen haben**

| | | | | | |
|---|---|---|---|---|---|
| 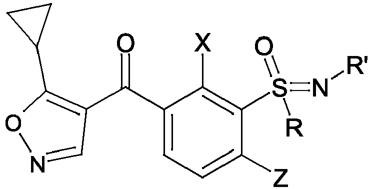 | | | | | |

| Nr. | X | Z | R | R' | Physikalische Daten (1H-NMR) |
|---|---|---|---|---|---|
| 5-1 | Me | Me | Me | H | |
| 5-2 | Me | F | Me | H | |
| 5-3 | Me | Cl | Me | H | |
| 5-4 | Me | Br | Me | H | |
| 5-5 | Me | I | Me | H | |
| 5-6 | Me | CF₃ | Me | H | |
| 5-7 | Me | CHF₂ | Me | H | |
| 5-8 | Me | CF₂Cl | Me | H | |
| 5-9 | Me | OMe | Me | H | |
| 5-10 | Me | NO₂ | Me | H | |
| 5-11 | Me | SO₂Me | Me | H | |
| 5-12 | Cl | Me | Me | H | |
| 5-13 | Cl | F | Me | H | |
| 5-14 | Cl | Cl | Me | H | |
| 5-15 | Cl | Br | Me | H | |
| 5-16 | Cl | I | Me | H | |
| 5-17 | Cl | CF₃ | Me | H | |
| 5-18 | Cl | CHF₂ | Me | H | |
| 5-19 | Cl | CF₂Cl | Me | H | |
| 5-20 | Cl | OMe | Me | H | |
| 5-21 | Cl | NO₂ | Me | H | |
| 5-22 | Cl | SO₂Me | Me | H | |
| 5-23 | OMe | Me | Me | H | |
| 5-24 | OMe | F | Me | H | |
| 5-25 | OMe | Cl | Me | H | |
| 5-26 | OMe | Br | Me | H | |
| 5-27 | OMe | I | Me | H | |
| 5-28 | OMe | CF₃ | Me | H | |
| 5-29 | OMe | CHF₂ | Me | H | |
| 5-30 | OMe | CF₂Cl | Me | H | |
| 5-31 | OMe | OMe | Me | H | |
| 5-32 | OMe | NO₂ | Me | H | |
| 5-33 | OMe | SO₂Me | Me | H | |
| 5-34 | SO₂Me | Me | Me | H | |
| 5-35 | SO₂Me | F | Me | H | |
| 5-36 | SO₂Me | Cl | Me | H | |
| 5-37 | SO₂Me | Br | Me | H | |
| 5-38 | SO₂Me | I | Me | H | |
| 5-39 | SO₂Me | CF₃ | Me | H | |
| 5-40 | SO₂Me | CHF₂ | Me | H | |
| 5-41 | SO₂Me | CF₂Cl | Me | H | |
| 5-42 | SO₂Me | OMe | Me | H | |
| 5-43 | SO₂Me | NO₂ | Me | H | |
| 5-44 | SO₂Me | SO₂Me | Me | H | |
| 5-45 | Me | Me | Et | H | |
| 5-46 | Me | F | Et | H | |
| 5-47 | Me | Cl | Et | H | |
| 5-48 | Me | Br | Et | H | |
| 5-49 | Me | I | Et | H | |
| 5-50 | Me | CF₃ | Et | H | |
| 5-51 | Me | CHF₂ | Et | H | |
| 5-52 | Me | CF₂Cl | Et | H | |
| 5-53 | Me | OMe | Et | H | |
| 5-54 | Me | NO₂ | Et | H | |
| 5-55 | Me | SO₂Me | Et | H | |
| 5-56 | Cl | Me | Et | H | |
| 5-57 | Cl | F | Et | H | |
| 5-58 | Cl | Cl | Et | H | |
| 5-59 | Cl | Br | Et | H | |
| 5-60 | Cl | I | Et | H | |
| 5-61 | Cl | CF₃ | Et | H | |
| 5-62 | Cl | CHF₂ | Et | H | |
| 5-63 | Cl | CF₂Cl | Et | H | |
| 5-64 | Cl | OMe | Et | H | |
| 5-65 | Cl | NO₂ | Et | H | |
| 5-66 | Cl | SO₂Me | Et | H | |
| 5-67 | OMe | Me | Et | H | |
| 5-68 | OMe | F | Et | H | |
| 5-69 | OMe | Cl | Et | H | |
| 5-70 | OMe | Br | Et | H | |
| 5-71 | OMe | I | Et | H | |
| 5-72 | OMe | CF₃ | Et | H | |
| 5-73 | OMe | CHF₂ | Et | H | |
| 5-74 | OMe | CF₂Cl | Et | H | |
| 5-75 | OMe | OMe | Et | H | |
| 5-76 | OMe | NO₂ | Et | H | |
| 5-77 | OMe | SO₂Me | Et | H | |
| 5-78 | SO₂Me | Me | Et | H | |
| 5-79 | SO₂Me | F | Et | H | |
| 5-80 | SO₂Me | Cl | Et | H | |
| 5-81 | SO₂Me | Br | Et | H | |
| 5-82 | SO₂Me | I | Et | H | |
| 5-83 | SO₂Me | CF₃ | Et | H | |
| 5-84 | SO₂Me | CHF₂ | Et | H | |
| 5-85 | SO₂Me | CF₂Cl | Et | H | |
| 5-86 | SO₂Me | OMe | Et | H | |
| 5-87 | SO₂Me | NO₂ | Et | H | |
| 5-88 | SO₂Me | SO₂Me | Et | H | |
| 5-89 | Me | Me | CH₂CH₂OMe | H | |
| 5-90 | Me | F | CH₂CH₂OMe | H | |
| 5-91 | Me | Cl | CH₂CH₂OMe | H | |
| 5-92 | Me | Br | CH₂CH₂OMe | H | |
| 5-93 | Me | I | CH₂CH₂OMe | H | |
| 5-94 | Me | CF₃ | CH₂CH₂OMe | H | |
| 5-95 | Me | CHF₂ | CH₂CH₂OMe | H | |
| 5-96 | Me | CF₂Cl | CH₂CH₂OMe | H | |
| 5-97 | Me | OMe | CH₂CH₂OMe | H | |
| 5-98 | Me | NO₂ | CH₂CH₂OMe | H | |
| 5-99 | Me | SO₂Me | CH₂CH₂OMe | H | |
| 5-100 | Cl | Me | CH₂CH₂OMe | H | |
| 5-101 | Cl | F | CH₂CH₂OMe | H | |
| 5-102 | Cl | Cl | CH₂CH₂OMe | H | |
| 5-103 | Cl | Br | CH₂CH₂OMe | H | |
| 5-104 | Cl | I | CH₂CH₂OMe | H | |
| 5-105 | Cl | CF₃ | CH₂CH₂OMe | H | |
| 5-106 | Cl | CHF₂ | CH₂CH₂OMe | H | |
| 5-107 | Cl | CF₂Cl | CH₂CH₂OMe | H | |
| 5-108 | Cl | OMe | CH₂CH₂OMe | H | |
| 5-109 | Cl | NO₂ | CH₂CH₂OMe | H | |
| 5-110 | Cl | SO₂Me | CH₂CH₂OMe | H | |
| 5-111 | OMe | Me | CH₂CH₂OMe | H | |
| 5-112 | OMe | F | CH₂CH₂OMe | H | |
| 5-113 | OMe | Cl | CH₂CH₂OMe | H | |
| 5-114 | OMe | Br | CH₂CH₂OMe | H | |
| 5-115 | OMe | I | CH₂CH₂OMe | H | |
| 5-116 | OMe | CF₃ | CH₂CH₂OMe | H | |
| 5-117 | OMe | CHF₂ | CH₂CH₂OMe | H | |
| 5-118 | OMe | CF₂Cl | CH₂CH₂OMe | H | |
| 5-119 | OMe | OMe | CH₂CH₂OMe | H | |
| 5-120 | OMe | NO₂ | CH₂CH₂OMe | H | |
| 5-121 | OMe | SO₂Me | CH₂CH₂OMe | H | |
| 5-122 | SO₂Me | Me | CH₂CH₂OMe | H | |
| 5-123 | SO₂Me | F | CH₂CH₂OMe | H | |
| 5-124 | SO₂Me | Cl | CH₂CH₂OMe | H | |
| 5-125 | SO₂Me | Br | CH₂CH₂OMe | H | |
| 5-126 | SO₂Me | I | CH₂CH₂OMe | H | |
| 5-127 | SO₂Me | CF₃ | CH₂CH₂OMe | H | |
| 5-128 | SO₂Me | CHF₂ | CH₂CH₂OMe | H | |
| 5-129 | SO₂Me | CF₂Cl | CH₂CH₂OMe | H | |
| 5-130 | SO₂Me | OMe | CH₂CH₂OMe | H | |
| 5-131 | SO₂Me | NO₂ | CH₂CH₂OMe | H | |
| 5-132 | SO₂Me | SO₂Me | CH₂CH₂OMe | H | |
| 5-133 | Me | Me | Me | CN | |
| 5-134 | Me | F | Me | CN | |
| 5-135 | Me | Cl | Me | CN | |
| 5-136 | Me | Br | Me | CN | |
| 5-137 | Me | I | Me | CN | |
| 5-138 | Me | CF₃ | Me | CN | |
| 5-139 | Me | CHF₂ | Me | CN | |
| 5-140 | Me | CF₂Cl | Me | CN | |
| 5-141 | Me | OMe | Me | CN | |
| 5-142 | Me | NO₂ | Me | CN | |
| 5-143 | Me | SO₂Me | Me | CN | |
| 5-144 | Cl | Me | Me | CN | |
| 5-145 | Cl | F | Me | CN | |
| 5-146 | Cl | Cl | Me | CN | |
| 5-147 | Cl | Br | Me | CN | |
| 5-148 | Cl | I | Me | CN | |
| 5-149 | Cl | CF₃ | Me | CN | |
| 5-150 | Cl | CHF₂ | Me | CN | |
| 5-151 | Cl | CF₂Cl | Me | CN | |
| 5-152 | Cl | OMe | Me | CN | |
| 5-153 | Cl | NO₂ | Me | CN | |
| 5-154 | Cl | SO₂Me | Me | CN | |
| 5-155 | OMe | Me | Me | CN | |
| 5-156 | OMe | F | Me | CN | |
| 5-157 | OMe | Cl | Me | CN | |
| 5-158 | OMe | Br | Me | CN | |
| 5-159 | OMe | I | Me | CN | |
| 5-160 | OMe | CF₃ | Me | CN | |
| 5-161 | OMe | CHF₂ | Me | CN | |
| 5-162 | OMe | CF₂Cl | Me | CN | |
| 5-163 | OMe | OMe | Me | CN | |
| 5-164 | OMe | NO₂ | Me | CN | |
| 5-165 | OMe | SO₂Me | Me | CN | |
| 5-166 | SO₂Me | Me | Me | CN | |
| 5-167 | SO₂Me | F | Me | CN | |
| 5-168 | SO₂Me | Cl | Me | CN | |
| 5-169 | SO₂Me | Br | Me | CN | |
| 5-170 | SO₂Me | I | Me | CN | |
| 5-171 | SO₂Me | CF₃ | Me | CN | |
| 5-172 | SO₂Me | CHF₂ | Me | CN | |
| 5-173 | SO₂Me | CF₂Cl | Me | CN | |
| 5-174 | SO₂Me | OMe | Me | CN | |
| 5-175 | SO₂Me | NO₂ | Me | CN | |
| 5-176 | SO₂Me | SO₂Me | Me | CN | |
| 5-177 | Me | Me | Et | CN | |
| 5-178 | Me | F | Et | CN | |
| 5-179 | Me | Cl | Et | CN | |
| 5-180 | Me | Br | Et | CN | |
| 5-181 | Me | I | Et | CN | |
| 5-182 | Me | CF₃ | Et | CN | |
| 5-183 | Me | CHF₂ | Et | CN | |
| 5-184 | Me | CF₂Cl | Et | CN | |
| 5-185 | Me | OMe | Et | CN | |
| 5-186 | Me | NO₂ | Et | CN | |
| 5-187 | Me | SO₂Me | Et | CN | |
| 5-188 | Cl | Me | Et | CN | |
| 5-189 | Cl | F | Et | CN | |
| 5-190 | Cl | Cl | Et | CN | |
| 5-191 | Cl | Br | Et | CN | |
| 5-192 | Cl | I | Et | CN | |
| 5-193 | Cl | CF₃ | Et | CN | |
| 5-194 | Cl | CHF₂ | Et | CN | |
| 5-195 | Cl | CF₂Cl | Et | CN | |
| 5-196 | Cl | OMe | Et | CN | |
| 5-197 | Cl | NO₂ | Et | CN | |
| 5-198 | Cl | SO₂Me | Et | CN | |
| 5-199 | OMe | Me | Et | CN | |
| 5-200 | OMe | F | Et | CN | |
| 5-201 | OMe | Cl | Et | CN | |
| 5-202 | OMe | Br | Et | CN | |
| 5-203 | OMe | I | Et | CN | |
| 5-204 | OMe | CF₃ | Et | CN | |
| 5-205 | OMe | CHF₂ | Et | CN | |
| 5-206 | OMe | CF₂Cl | Et | CN | |
| 5-207 | OMe | OMe | Et | CN | |
| 5-208 | OMe | NO₂ | Et | CN | |
| 5-209 | OMe | SO₂Me | Et | CN | |
| 5-210 | SO₂Me | Me | Et | CN | |
| 5-211 | SO₂Me | F | Et | CN | |
| 5-212 | SO₂Me | Cl | Et | CN | |
| 5-213 | SO₂Me | Br | Et | CN | |
| 5-214 | SO₂Me | I | Et | CN | |
| 5-215 | SO₂Me | CF₃ | Et | CN | |
| 5-216 | SO₂Me | CHF₂ | Et | CN | |
| 5-217 | SO₂Me | CF₂Cl | Et | CN | |
| 5-218 | SO₂Me | OMe | Et | CN | |
| 5-219 | SO₂Me | NO₂ | Et | CN | |
| 5-220 | SO₂Me | SO₂Me | Et | CN | |
| 5-221 | Me | Me | CH₂CH₂OMe | CN | |
| 5-222 | Me | F | CH₂CH₂OMe | CN | |
| 5-223 | Me | Cl | CH₂CH₂OMe | CN | |
| 5-224 | Me | Br | CH₂CH₂OMe | CN | |
| 5-225 | Me | I | CH₂CH₂OMe | CN | |
| 5-226 | Me | CF₃ | CH₂CH₂OMe | CN | |
| 5-227 | Me | CHF₂ | CH₂CH₂OMe | CN | |
| 5-228 | Me | CF₂Cl | CH₂CH₂OMe | CN | |
| 5-229 | Me | OMe | CH₂CH₂OMe | CN | |
| 5-230 | Me | NO₂ | CH₂CH₂OMe | CN | |
| 5-231 | Me | SO₂Me | CH₂CH₂OMe | CN | |
| 5-232 | Cl | Me | CH₂CH₂OMe | CN | |
| 5-233 | Cl | F | CH₂CH₂OMe | CN | |
| 5-234 | Cl | Cl | CH₂CH₂OMe | CN | |
| 5-235 | Cl | Br | CH₂CH₂OMe | CN | |
| 5-236 | Cl | I | CH₂CH₂OMe | CN | |
| 5-237 | Cl | CF₃ | CH₂CH₂OMe | CN | |
| 5-238 | Cl | CHF₂ | CH₂CH₂OMe | CN | |
| 5-239 | Cl | CF₂Cl | CH₂CH₂OMe | CN | |
| 5-240 | Cl | OMe | CH₂CH₂OMe | CN | |
| 5-241 | Cl | NO₂ | CH₂CH₂OMe | CN | |
| 5-242 | Cl | SO₂Me | CH₂CH₂OMe | CN | |
| 5-243 | OMe | Me | CH₂CH₂OMe | CN | |
| 5-244 | OMe | F | CH₂CH₂OMe | CN | |
| 5-245 | OMe | Cl | CH₂CH₂OMe | CN | |
| 5-246 | OMe | Br | CH₂CH₂OMe | CN | |
| 5-247 | OMe | I | CH₂CH₂OMe | CN | |
| 5-248 | OMe | CF₃ | CH₂CH₂OMe | CN | |
| 5-249 | OMe | CHF₂ | CH₂CH₂OMe | CN | |
| 5-250 | OMe | CF₂Cl | CH₂CH₂OMe | CN | |
| 5-251 | OMe | OMe | CH₂CH₂OMe | CN | |
| 5-252 | OMe | NO₂ | CH₂CH₂OMe | CN | |
| 5-253 | OMe | SO₂Me | CH₂CH₂OMe | CN | |
| 5-254 | SO₂Me | Me | CH₂CH₂OMe | CN | |
| 5-255 | SO₂Me | F | CH₂CH₂OMe | CN | |
| 5-256 | SO₂Me | Cl | CH₂CH₂OMe | CN | |
| 5-257 | SO₂Me | Br | CH₂CH₂OMe | CN | |
| 5-258 | SO₂Me | I | CH₂CH₂OMe | CN | |
| 5-259 | SO₂Me | CF₃ | CH₂CH₂OMe | CN | |
| 5-260 | SO₂Me | CHF₂ | CH₂CH₂OMe | CN | |
| 5-261 | SO₂Me | CF₂Cl | CH₂CH₂OMe | CN | |
| 5-262 | SO₂Me | OMe | CH₂CH₂OMe | CN | |
| 5-263 | SO₂Me | NO₂ | CH₂CH₂OMe | CN | |
| 5-264 | SO₂Me | SO₂Me | CH₂CH₂OMe | CN | |

**Tabelle 6: Erfindungsgemäße Verbindungen der allgemeinen Formel (I), worin Q für Q5 steht, W Wasserstoff bedeutet, t = 1 ist und die anderen Reste die in der Tabelle angegebenen Bedeutungen haben**

| | | | | | |
|---|---|---|---|---|---|
| 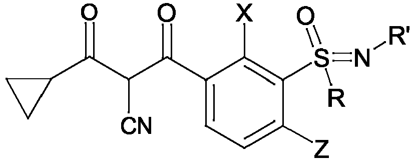 | | | | | |

| Nr. | X | Z | R | R' | Physikalische Daten (1H-NMR) |
|---|---|---|---|---|---|
| 6-1 | Me | Me | Me | H | |
| 6-2 | Me | F | Me | H | |
| 6-3 | Me | Cl | Me | H | |
| 6-4 | Me | Br | Me | H | |
| 6-5 | Me | I | Me | H | |
| 6-6 | Me | CF₃ | Me | H | |
| 6-7 | Me | CHF₂ | Me | H | |
| 6-8 | Me | CF₂Cl | Me | H | |
| 6-9 | Me | OMe | Me | H | |
| 6-10 | Me | NO₂ | Me | H | |
| 6-11 | Me | SO₂Me | Me | H | |
| 6-12 | Cl | Me | Me | H | |
| 6-13 | Cl | F | Me | H | |
| 6-14 | Cl | Cl | Me | H | |
| 6-15 | Cl | Br | Me | H | |
| 6-16 | Cl | I | Me | H | |
| 6-17 | Cl | CF₃ | Me | H | |
| 6-18 | Cl | CHF₂ | Me | H | |
| 6-19 | Cl | CF₂Cl | Me | H | |
| 6-20 | Cl | OMe | Me | H | |
| 6-21 | Cl | NO₂ | Me | H | |
| 6-22 | Cl | SO₂Me | Me | H | |
| 6-23 | OMe | Me | Me | H | |
| 6-24 | OMe | F | Me | H | |
| 6-25 | OMe | Cl | Me | H | |
| 6-26 | OMe | Br | Me | H | |
| 6-27 | OMe | I | Me | H | |
| 6-28 | OMe | CF₃ | Me | H | |
| 6-29 | OMe | CHF₂ | Me | H | |
| 6-30 | OMe | CF₂Cl | Me | H | |
| 6-31 | OMe | OMe | Me | H | |
| 6-32 | OMe | NO₂ | Me | H | |
| 6-33 | OMe | SO₂Me | Me | H | |
| 6-34 | SO₂Me | Me | Me | H | |
| 6-35 | SO₂Me | F | Me | H | |
| 6-36 | SO₂Me | Cl | Me | H | |
| 6-37 | SO₂Me | Br | Me | H | |
| 6-38 | SO₂Me | I | Me | H | |
| 6-39 | SO₂Me | CF₃ | Me | H | |
| 6-40 | SO₂Me | CHF₂ | Me | H | |
| 6-41 | SO₂Me | CF₂Cl | Me | H | |
| 6-42 | SO₂Me | OMe | Me | H | |
| 6-43 | SO₂Me | NO₂ | Me | H | |
| 6-44 | SO₂Me | SO₂Me | Me | H | |
| 6-45 | Me | Me | Et | H | |
| 6-46 | Me | F | Et | H | |
| 6-47 | Me | Cl | Et | H | |
| 6-48 | Me | Br | Et | H | |
| 6-49 | Me | I | Et | H | |
| 6-50 | Me | CF₃ | Et | H | |
| 6-51 | Me | CHF₂ | Et | H | |
| 6-52 | Me | CF₂Cl | Et | H | |
| 6-53 | Me | OMe | Et | H | |
| 6-54 | Me | NO₂ | Et | H | |
| 6-55 | Me | SO₂Me | Et | H | |
| 6-56 | Cl | Me | Et | H | |
| 6-57 | Cl | F | Et | H | |
| 6-58 | Cl | Cl | Et | H | |
| 6-59 | Cl | Br | Et | H | |
| 6-60 | Cl | I | Et | H | |
| 6-61 | Cl | CF₃ | Et | H | |
| 6-62 | Cl | CHF₂ | Et | H | |
| 6-63 | Cl | CF₂Cl | Et | H | |
| 6-64 | Cl | OMe | Et | H | |
| 6-65 | Cl | NO₂ | Et | H | |
| 6-66 | Cl | SO₂Me | Et | H | |
| 6-67 | OMe | Me | Et | H | |
| 6-68 | OMe | F | Et | H | |
| 6-69 | OMe | Cl | Et | H | |
| 6-70 | OMe | Br | Et | H | |
| 6-71 | OMe | I | Et | H | |
| 6-72 | OMe | CF₃ | Et | H | |
| 6-73 | OMe | CHF₂ | Et | H | |
| 6-74 | OMe | CF₂Cl | Et | H | |
| 6-75 | OMe | OMe | Et | H | |
| 6-76 | OMe | NO₂ | Et | H | |
| 6-77 | OMe | SO₂Me | Et | H | |
| 6-78 | SO₂Me | Me | Et | H | |
| 6-79 | SO₂Me | F | Et | H | |
| 6-80 | SO₂Me | Cl | Et | H | |
| 6-81 | SO₂Me | Br | Et | H | |
| 6-82 | SO₂Me | I | Et | H | |
| 6-83 | SO₂Me | CF₃ | Et | H | |
| 6-84 | SO₂Me | CHF₂ | Et | H | |
| 6-85 | SO₂Me | CF₂Cl | Et | H | |
| 6-86 | SO₂Me | OMe | Et | H | |
| 6-87 | SO₂Me | NO₂ | Et | H | |
| 6-88 | SO₂Me | SO₂Me | Et | H | |
| 6-89 | Me | Me | CH₂CH₂OMe | H | |
| 6-90 | Me | F | CH₂CH₂OMe | H | |
| 6-91 | Me | Cl | CH₂CH₂OMe | H | |
| 6-92 | Me | Br | CH₂CH₂OMe | H | |
| 6-93 | Me | I | CH₂CH₂OMe | H | |
| 6-94 | Me | CF₃ | CH₂CH₂OMe | H | |
| 6-95 | Me | CHF₂ | CH₂CH₂OMe | H | |
| 6-96 | Me | CF₂Cl | CH₂CH₂OMe | H | |
| 6-97 | Me | OMe | CH₂CH₂OMe | H | |
| 6-98 | Me | NO₂ | CH₂CH₂OMe | H | |
| 6-99 | Me | SO₂Me | CH₂CH₂OMe | H | |
| 6-100 | Cl | Me | CH₂CH₂OMe | H | |
| 6-101 | Cl | F | CH₂CH₂OMe | H | |
| 6-102 | Cl | Cl | CH₂CH₂OMe | H | |
| 6-103 | Cl | Br | CH₂CH₂OMe | H | |
| 6-104 | Cl | I | CH₂CH₂OMe | H | |
| 6-105 | Cl | CF₃ | CH₂CH₂OMe | H | |
| 6-106 | Cl | CHF₂ | CH₂CH₂OMe | H | |
| 6-107 | Cl | CF₂Cl | CH₂CH₂OMe | H | |
| 6-108 | Cl | OMe | CH₂CH₂OMe | H | |
| 6-109 | Cl | NO₂ | CH₂CH₂OMe | H | |
| 6-110 | Cl | SO₂Me | CH₂CH₂OMe | H | |
| 6-111 | OMe | Me | CH₂CH₂OMe | H | |
| 6-112 | OMe | F | CH₂CH₂OMe | H | |
| 6-113 | OMe | Cl | CH₂CH₂OMe | H | |
| 6-114 | OMe | Br | CH₂CH₂OMe | H | |
| 6-115 | OMe | I | CH₂CH₂OMe | H | |
| 6-116 | OMe | CF₃ | CH₂CH₂OMe | H | |
| 6-117 | OMe | CHF₂ | CH₂CH₂OMe | H | |
| 6-118 | OMe | CF₂Cl | CH₂CH₂OMe | H | |
| 6-119 | OMe | OMe | CH₂CH₂OMe | H | |
| 6-120 | OMe | NO₂ | CH₂CH₂OMe | H | |
| 6-121 | OMe | SO₂Me | CH₂CH₂OMe | H | |
| 6-122 | SO₂Me | Me | CH₂CH₂OMe | H | |
| 6-123 | SO₂Me | F | CH₂CH₂OMe | H | |
| 6-124 | SO₂Me | Cl | CH₂CH₂OMe | H | |
| 6-125 | SO₂Me | Br | CH₂CH₂OMe | H | |
| 6-126 | SO₂Me | I | CH₂CH₂OMe | H | |
| 6-127 | SO₂Me | CF₃ | CH₂CH₂OMe | H | |
| 6-128 | SO₂Me | CHF₂ | CH₂CH₂OMe | H | |
| 6-129 | SO₂Me | CF₂Cl | CH₂CH₂OMe | H | |
| 6-130 | SO₂Me | OMe | CH₂CH₂OMe | H | |
| 6-131 | SO₂Me | NO₂ | CH₂CH₂OMe | H | |
| 6-132 | SO₂Me | SO₂Me | CH₂CH₂OMe | H | |
| 6-133 | Me | Me | Me | CN | |
| 6-134 | Me | F | Me | CN | |
| 6-135 | Me | Cl | Me | CN | |
| 6-136 | Me | Br | Me | CN | |
| 6-137 | Me | I | Me | CN | |
| 6-138 | Me | CF₃ | Me | CN | |
| 6-139 | Me | CHF₂ | Me | CN | |
| 6-140 | Me | CF₂Cl | Me | CN | |
| 6-141 | Me | OMe | Me | CN | |
| 6-142 | Me | NO₂ | Me | CN | |
| 6-143 | Me | SO₂Me | Me | CN | |
| 6-144 | Cl | Me | Me | CN | |
| 6-145 | Cl | F | Me | CN | |
| 6-146 | Cl | Cl | Me | CN | |
| 6-147 | Cl | Br | Me | CN | |
| 6-148 | Cl | I | Me | CN | |
| 6-149 | Cl | CF₃ | Me | CN | |
| 6-150 | Cl | CHF₂ | Me | CN | |
| 6-151 | Cl | CF₂Cl | Me | CN | |
| 6-152 | Cl | OMe | Me | CN | |
| 6-153 | Cl | NO₂ | Me | CN | |
| 6-154 | Cl | SO₂Me | Me | CN | |
| 6-155 | OMe | Me | Me | CN | |
| 6-156 | OMe | F | Me | CN | |
| 6-157 | OMe | Cl | Me | CN | |
| 6-158 | OMe | Br | Me | CN | |
| 6-159 | OMe | I | Me | CN | |
| 6-160 | OMe | CF₃ | Me | CN | |
| 6-161 | OMe | CHF₂ | Me | CN | |
| 6-162 | OMe | CF₂Cl | Me | CN | |
| 6-163 | OMe | OMe | Me | CN | |
| 6-164 | OMe | NO₂ | Me | CN | |
| 6-165 | OMe | SO₂Me | Me | CN | |
| 6-166 | SO₂Me | Me | Me | CN | |
| 6-167 | SO₂Me | F | Me | CN | |
| 6-168 | SO₂Me | Cl | Me | CN | |
| 6-169 | SO₂Me | Br | Me | CN | |
| 6-170 | SO₂Me | I | Me | CN | |
| 6-171 | SO₂Me | CF₃ | Me | CN | |
| 6-172 | SO₂Me | CHF₂ | Me | CN | |
| 6-173 | SO₂Me | CF₂Cl | Me | CN | |
| 6-174 | SO₂Me | OMe | Me | CN | |
| 6-175 | SO₂Me | NO₂ | Me | CN | |
| 6-176 | SO₂Me | SO₂Me | Me | CN | |
| 6-177 | Me | Me | Et | CN | |
| 6-178 | Me | F | Et | CN | |
| 6-179 | Me | Cl | Et | CN | |
| 6-180 | Me | Br | Et | CN | |
| 6-181 | Me | I | Et | CN | |
| 6-182 | Me | CF₃ | Et | CN | |
| 6-183 | Me | CHF₂ | Et | CN | |
| 6-184 | Me | CF₂Cl | Et | CN | |
| 6-185 | Me | OMe | Et | CN | |
| 6-186 | Me | NO₂ | Et | CN | |
| 6-187 | Me | SO₂Me | Et | CN | |
| 6-188 | Cl | Me | Et | CN | |
| 6-189 | Cl | F | Et | CN | |
| 6-190 | Cl | Cl | Et | CN | |
| 6-191 | Cl | Br | Et | CN | |
| 6-192 | Cl | I | Et | CN | |
| 6-193 | Cl | CF₃ | Et | CN | |
| 6-194 | Cl | CHF₂ | Et | CN | |
| 6-195 | Cl | CF₂Cl | Et | CN | |
| 6-196 | Cl | OMe | Et | CN | |
| 6-197 | Cl | NO₂ | Et | CN | |
| 6-198 | Cl | SO₂Me | Et | CN | |
| 6-199 | OMe | Me | Et | CN | |
| 6-200 | OMe | F | Et | CN | |
| 6-201 | OMe | Cl | Et | CN | |
| 6-202 | OMe | Br | Et | CN | |
| 6-203 | OMe | I | Et | CN | |
| 6-204 | OMe | CF₃ | Et | CN | |
| 6-205 | OMe | CHF₂ | Et | CN | |
| 6-206 | OMe | CF₂Cl | Et | CN | |
| 6-207 | OMe | OMe | Et | CN | |
| 6-208 | OMe | NO₂ | Et | CN | |
| 6-209 | OMe | SO₂Me | Et | CN | |
| 6-210 | SO₂Me | Me | Et | CN | |
| 6-211 | SO₂Me | F | Et | CN | |
| 6-212 | SO₂Me | Cl | Et | CN | |
| 6-213 | SO₂Me | Br | Et | CN | |
| 6-214 | SO₂Me | I | Et | CN | |
| 6-215 | SO₂Me | CF₃ | Et | CN | |
| 6-216 | SO₂Me | CHF₂ | Et | CN | |
| 6-217 | SO₂Me | CF₂Cl | Et | CN | |
| 6-218 | SO₂Me | OMe | Et | CN | |
| 6-219 | SO₂Me | NO₂ | Et | CN | |
| 6-220 | SO₂Me | SO₂Me | Et | CN | |
| 6-221 | Me | Me | CH₂CH₂OMe | CN | |
| 6-222 | Me | F | CH₂CH₂OMe | CN | |
| 6-223 | Me | Cl | CH₂CH₂OMe | CN | |
| 6-224 | Me | Br | CH₂CH₂OMe | CN | |
| 6-225 | Me | I | CH₂CH₂OMe | CN | |
| 6-226 | Me | CF₃ | CH₂CH₂OMe | CN | |
| 6-227 | Me | CHF₂ | CH₂CH₂OMe | CN | |
| 6-228 | Me | CF₂Cl | CH₂CH₂OMe | CN | |
| 6-229 | Me | OMe | CH₂CH₂OMe | CN | |
| 6-230 | Me | NO₂ | CH₂CH₂OMe | CN | |
| 6-231 | Me | SO2Me | CH₂CH₂OMe | CN | |
| 6-232 | Cl | Me | CH₂CH₂OMe | CN | |
| 6-233 | Cl | F | CH₂CH₂OMe | CN | |
| 6-234 | Cl | Cl | CH₂CH₂OMe | CN | |
| 6-235 | Cl | Br | CH₂CH₂OMe | CN | |
| 6-236 | Cl | I | CH₂CH₂OMe | CN | |
| 6-237 | Cl | CF₃ | CH₂CH₂OMe | CN | |
| 6-238 | Cl | CHF₂ | CH₂CH₂OMe | CN | |
| 6-239 | Cl | CF₂Cl | CH₂CH₂OMe | CN | |
| 6-240 | Cl | OMe | CH₂CH₂OMe | CN | |
| 6-241 | Cl | NO₂ | CH₂CH₂OMe | CN | |
| 6-242 | Cl | SO₂Me | CH₂CH₂OMe | CN | |
| 6-243 | OMe | Me | CH₂CH₂OMe | CN | |
| 6-244 | OMe | F | CH₂CH₂OMe | CN | |
| 6-245 | OMe | Cl | CH₂CH₂OMe | CN | |
| 6-246 | OMe | Br | CH₂CH₂OMe | CN | |
| 6-247 | OMe | I | CH₂CH₂OMe | CN | |
| 6-248 | OMe | CF₃ | CH₂CH₂OMe | CN | |
| 6-249 | OMe | CHF₂ | CH₂CH₂OMe | CN | |
| 6-250 | OMe | CF₂Cl | CH₂CH₂OMe | CN | |
| 6-251 | OMe | OMe | CH₂CH₂OMe | CN | |
| 6-252 | OMe | NO₂ | CH₂CH₂OMe | CN | |
| 6-253 | OMe | SO₂Me | CH₂CH₂OMe | CN | |
| 6-254 | SO₂Me | Me | CH₂CH₂OMe | CN | |
| 6-255 | SO₂Me | F | CH₂CH₂OMe | CN | |
| 6-256 | SO₂Me | Cl | CH₂CH₂OMe | CN | |
| 6-257 | SO₂Me | Br | CH₂CH₂OMe | CN | |
| 6-258 | SO₂Me | I | CH₂CH₂OMe | CN | |
| 6-259 | SO₂Me | CF₃ | CH₂CH₂OMe | CN | |
| 6-260 | SO₂Me | CHF₂ | CH₂CH₂OMe | CN | |
| 6-261 | SO₂Me | CF₂Cl | CH₂CH₂OMe | CN | |
| 6-262 | SO₂Me | OMe | CH₂CH₂OMe | CN | |
| 6-263 | SO₂Me | NO₂ | CH₂CH₂OMe | CN | |
| 6-264 | SO₂Me | SO₂Me | CH₂CH₂OMe | CN | |

**Tabelle 7: Erfindungsgemäße Verbindungen der allgemeinen Formel (I) in Form der Natriumsalze, worin Q für Q3, Rⁱ für Methyl,R^{k} sowie W jeweils für Wasserstoff stehen, t = 1 ist und die anderen Reste die in der Tabelle angegebenen Bedeutungen haben**

| | | | | | |
|---|---|---|---|---|---|
| 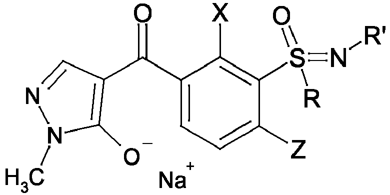 | | | | | |

| Nr. | X | Z | R | R' | Physikalische Daten (1H-NMR) |
|---|---|---|---|---|---|
| 7-1 | Me | Me | Me | H | |
| 7-2 | Me | Cl | Me | H | |
| 7-3 | Me | CF₃ | Me | H | |
| 7-4 | Me | CHF₂ | Me | H | |
| 7-5 | Cl | Me | Me | H | |
| 7-6 | Cl | Cl | Me | H | |
| 7-7 | Cl | CF₃ | Me | H | |
| 7-8 | Cl | CHF₂ | Me | H | |
| 7-9 | OMe | Me | Me | H | |
| 7-10 | OMe | Cl | Me | H | |
| 7-11 | OMe | CF₃ | Me | H | |
| 7-12 | OMe | CHF₂ | Me | H | |
| 7-13 | SO₂Me | Me | Me | H | |
| 7-14 | SO₂Me | Cl | Me | H | |
| 7-15 | SO₂Me | CF₃ | Me | H | |
| 7-16 | SO₂Me | CHF₂ | Me | H | |
| 7-17 | Me | Me | Et | H | |
| 7-18 | Me | Cl | Et | H | |
| 7-19 | Me | CF₃ | Et | H | |
| 7-20 | Me | CHF₂ | Et | H | |
| 7-21 | Cl | Me | Et | H | |
| 7-22 | Cl | Cl | Et | H | |
| 7-23 | Cl | CF₃ | Et | H | |
| 7-24 | Cl | CHF₂ | Et | H | |
| 7-25 | OMe | Me | Et | H | |
| 7-26 | OMe | Cl | Et | H | |
| 7-27 | OMe | CF₃ | Et | H | |
| 7-28 | OMe | CHF₂ | Et | H | |
| 7-29 | SO₂Me | Me | Et | H | |
| 7-30 | SO₂Me | Cl | Et | H | |
| 7-31 | SO₂Me | CF₃ | Et | H | |
| 7-32 | SO₂Me | CHF₂ | Et | H | |
| 7-33 | Me | Me | CH₂CH₂OMe | H | |
| 7-34 | Me | Cl | CH₂CH₂OMe | H | |
| 7-35 | Me | CF₃ | CH₂CH₂OMe | H | |
| 7-36 | Me | CHF₂ | CH₂CH₂OMe | H | |
| 7-37 | Cl | Me | CH₂CH₂OMe | H | |
| 7-38 | Cl | Cl | CH₂CH₂OMe | H | |
| 7-39 | Cl | CF₃ | CH₂CH₂OMe | H | |
| 7-40 | Cl | CHF₂ | CH₂CH₂OMe | H | |
| 7-41 | OMe | Me | CH₂CH₂OMe | H | |
| 7-42 | OMe | Cl | CH₂CH₂OMe | H | |
| 7-43 | OMe | CF₃ | CH₂CH₂OMe | H | |
| 7-44 | OMe | CHF₂ | CH₂CH₂OMe | H | |
| 7-45 | SO₂Me | Me | CH₂CH₂OMe | H | |
| 7-46 | SO₂Me | Cl | CH₂CH₂OMe | H | |
| 7-47 | SO₂Me | CF₃ | CH₂CH₂OMe | H | |
| 7-48 | SO₂Me | CHF₂ | CH₂CH₂OMe | H | |
| 7-49 | Me | Me | Me | CN | |
| 7-50 | Me | Cl | Me | CN | |
| 7-51 | Me | CF₃ | Me | CN | |
| 7-52 | Me | CHF₂ | Me | CN | |
| 7-53 | Cl | Me | Me | CN | |
| 7-54 | Cl | Cl | Me | CN | |
| 7-55 | Cl | CF₃ | Me | CN | |
| 7-56 | Cl | CHF₂ | Me | CN | |
| 7-57 | OMe | Me | Me | CN | |
| 7-58 | OMe | Cl | Me | CN | |
| 7-59 | OMe | CF₃ | Me | CN | |
| 7-60 | OMe | CHF₂ | Me | CN | |
| 7-61 | SO₂Me | Me | Me | CN | |
| 7-62 | SO₂Me | Cl | Me | CN | |
| 7-63 | SO₂Me | CF₃ | Me | CN | |
| 7-64 | SO₂Me | CHF₂ | Me | CN | |
| 7-65 | Me | Me | Et | CN | |
| 7-66 | Me | Cl | Et | CN | |
| 7-67 | Me | CF₃ | Et | CN | |
| 7-68 | Me | CHF₂ | Et | CN | |
| 7-69 | Cl | Me | Et | CN | |
| 7-70 | Cl | Cl | Et | CN | |
| 7-71 | Cl | CF₃ | Et | CN | |
| 7-72 | Cl | CHF₂ | Et | CN | |
| 7-73 | OMe | Me | Et | CN | |
| 7-74 | OMe | Cl | Et | CN | |
| 7-75 | OMe | CF₃ | Et | CN | |
| 7-76 | OMe | CHF₂ | Et | CN | |
| 7-77 | SO₂Me | Me | Et | CN | |
| 7-78 | SO₂Me | Cl | Et | CN | |
| 7-79 | SO₂Me | CF₃ | Et | CN | |
| 7-80 | SO₂Me | CHF₂ | Et | CN | |
| 7-81 | Me | Me | CH₂CH₂OMe | CN | |
| 7-82 | Me | Cl | CH₂CH₂OMe | CN | |
| 7-83 | Me | CF₃ | CH₂CH₂OMe | CN | |
| 7-84 | Me | CHF₂ | CH₂CH₂OMe | CN | |
| 7-85 | Cl | Me | CH₂CH₂OMe | CN | |
| 7-86 | Cl | Cl | CH₂CH₂OMe | CN | |
| 7-87 | Cl | CF₃ | CH₂CH₂OMe | CN | |
| 7-88 | Cl | CHF₂ | CH₂CH₂OMe | CN | |
| 7-89 | OMe | Me | CH₂CH₂OMe | CN | |
| 7-90 | OMe | Cl | CH₂CH₂OMe | CN | |
| 7-91 | OMe | CF₃ | CH₂CH₂OMe | CN | |
| 7-92 | OMe | CHF₂ | CH₂CH₂OMe | CN | |
| 7-93 | SO₂Me | Me | CH₂CH₂OMe | CN | |
| 7-94 | SO₂Me | Cl | CH₂CH₂OMe | CN | |
| 7-95 | SO₂Me | CF₃ | CH₂CH₂OMe | CN | |
| 7-96 | SO₂Me | CHF₂ | CH₂CH₂OMe | CN | |

**Tabelle 8: Erfindungsgemäße Verbindungen der allgemeinen Formel (I), worin Q für Q1, R^{a} für eine Hydroxylgruppe, die Reste R^{b}, R^{c}, R^{d}, R^{e}, R^{f}, R^{g} und W jeweils für Wasserstoff stehen, t = 0 ist und die anderen Reste die in der Tabelle angegebenen Bedeutungen haben**

| | | | | | |
|---|---|---|---|---|---|
| 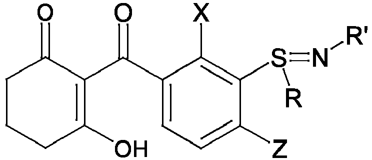 | | | | | |

| Nr. | X | Z | R | R' | Physikalische Daten (¹H-NMR) |
|---|---|---|---|---|---|
| 8-1 | Me | Me | Me | H | |
| 8-2 | Me | F | Me | H | |
| 8-3 | Me | Cl | Me | H | |
| 8-4 | Me | Br | Me | H | |
| 8-5 | Me | I | Me | H | |
| 8-6 | Me | CF₃ | Me | H | |
| 8-7 | Me | CHF₂ | Me | H | |
| 8-8 | Me | CF₂Cl | Me | H | |
| 8-9 | Me | OMe | Me | H | |
| 8-10 | Me | NO₂ | Me | H | |
| 8-11 | Me | SO₂Me | Me | H | |
| 8-12 | Cl | Me | Me | H | |
| 8-13 | Cl | F | Me | H | |
| 8-14 | Cl | Cl | Me | H | |
| 8-15 | Cl | Br | Me | H | |
| 8-16 | Cl | I | Me | H | |
| 8-17 | Cl | CF₃ | Me | H | |
| 8-18 | Cl | CHF₂ | Me | H | |
| 8-19 | Cl | CF₂Cl | Me | H | |
| 8-20 | Cl | OMe | Me | H | |
| 8-21 | Cl | NO₂ | Me | H | |
| 8-22 | Cl | SO₂Me | Me | H | |
| 8-23 | OMe | Me | Me | H | |
| 8-24 | OMe | F | Me | H | |
| 8-25 | OMe | Cl | Me | H | |
| 8-26 | OMe | Br | Me | H | |
| 8-27 | OMe | I | Me | H | |
| 8-28 | OMe | CF₃ | Me | H | |
| 8-29 | OMe | CHF₂ | Me | H | |
| 8-30 | OMe | CF₂Cl | Me | H | |
| 8-31 | OMe | OMe | Me | H | |
| 8-32 | OMe | NO₂ | Me | H | |
| 8-33 | OMe | SO₂Me | Me | H | |
| 8-34 | SO₂Me | Me | Me | H | |
| 8-35 | SO₂Me | F | Me | H | |
| 8-36 | SO₂Me | Cl | Me | H | |
| 8-37 | SO₂Me | Br | Me | H | |
| 8-38 | SO₂Me | I | Me | H | |
| 8-39 | SO₂Me | CF₃ | Me | H | |
| 8-40 | SO₂Me | CHF₂ | Me | H | |
| 8-41 | SO₂Me | CF₂Cl | Me | H | |
| 8-42 | SO₂Me | OMe | Me | H | |
| 8-43 | SO₂Me | NO₂ | Me | H | |
| 8-44 | SO₂Me | SO₂Me | Me | H | |
| 8-45 | Me | Me | Et | H | |
| 8-46 | Me | F | Et | H | |
| 8-47 | Me | Cl | Et | H | |
| 8-48 | Me | Br | Et | H | |
| 8-49 | Me | I | Et | H | |
| 8-50 | Me | CF₃ | Et | H | |
| 8-51 | Me | CHF₂ | Et | H | |
| 8-52 | Me | CF₂Cl | Et | H | |
| 8-53 | Me | OMe | Et | H | |
| 8-54 | Me | NO₂ | Et | H | |
| 8-55 | Me | SO₂Me | Et | H | |
| 8-56 | Cl | Me | Et | H | |
| 8-57 | Cl | F | Et | H | |
| 8-58 | Cl | Cl | Et | H | |
| 8-59 | Cl | Br | Et | H | |
| 8-60 | Cl | I | Et | H | |
| 8-61 | Cl | CF₃ | Et | H | |
| 8-62 | Cl | CHF₂ | Et | H | |
| 8-63 | Cl | CF₂Cl | Et | H | |
| 8-64 | Cl | OMe | Et | H | |
| 8-65 | Cl | NO₂ | Et | H | |
| 8-66 | Cl | SO₂Me | Et | H | |
| 8-67 | OMe | Me | Et | H | |
| 8-68 | OMe | F | Et | H | |
| 8-69 | OMe | Cl | Et | H | |
| 8-70 | OMe | Br | Et | H | |
| 8-71 | OMe | I | Et | H | |
| 8-72 | OMe | CF₃ | Et | H | |
| 8-73 | OMe | CHF₂ | Et | H | |
| 8-74 | OMe | CF₂Cl | Et | H | |
| 8-75 | OMe | OMe | Et | H | |
| 8-76 | OMe | NO₂ | Et | H | |
| 8-77 | OMe | SO₂Me | Et | H | |
| 8-78 | SO₂Me | Me | Et | H | |
| 8-79 | SO₂Me | F | Et | H | |
| 8-80 | SO₂Me | Cl | Et | H | |
| 8-81 | SO₂Me | Br | Et | H | |
| 8-82 | SO₂Me | I | Et | H | |
| 8-83 | SO₂Me | CF₃ | Et | H | |
| 8-84 | SO₂Me | CHF₂ | Et | H | |
| 8-85 | SO₂Me | CF₂Cl | Et | H | |
| 8-86 | SO₂Me | OMe | Et | H | |
| 8-87 | SO₂Me | NO₂ | Et | H | |
| 8-88 | SO₂Me | SO₂Me | Et | H | |
| 8-89 | Me | Me | CH₂CH₂OMe | H | |
| 8-90 | Me | F | CH₂CH₂OMe | H | |
| 8-91 | Me | Cl | CH₂CH₂OMe | H | |
| 8-92 | Me | Br | CH₂CH₂OMe | H | |
| 8-93 | Me | I | CH₂CH₂OMe | H | |
| 8-94 | Me | CF₃ | CH₂CH₂OMe | H | |
| 8-95 | Me | CHF₂ | CH₂CH₂OMe | H | |
| 8-96 | Me | CF₂Cl | CH₂CH₂OMe | H | |
| 8-97 | Me | OMe | CH₂CH₂OMe | H | |
| 8-98 | Me | NO₂ | CH₂CH₂OMe | H | |
| 8-99 | Me | SO₂Me | CH₂CH₂OMe | H | |
| 8-100 | Cl | Me | CH₂CH₂OMe | H | |
| 8-101 | Cl | F | CH₂CH₂OMe | H | |
| 8-102 | Cl | Cl | CH₂CH₂OMe | H | |
| 8-103 | Cl | Br | CH₂CH₂OMe | H | |
| 8-104 | Cl | I | CH₂CH₂OMe | H | |
| 8-105 | Cl | CF₃ | CH₂CH₂OMe | H | |
| 8-106 | Cl | CHF₂ | CH₂CH₂OMe | H | |
| 8-107 | Cl | CF₂Cl | CH₂CH₂OMe | H | |
| 8-108 | Cl | OMe | CH₂CH₂OMe | H | |
| 8-109 | Cl | NO₂ | CH₂CH₂OMe | H | |
| 8-110 | Cl | SO₂Me | CH₂CH₂OMe | H | |
| 8-111 | OMe | Me | CH₂CH₂OMe | H | |
| 8-112 | OMe | F | CH₂CH₂OMe | H | |
| 8-113 | OMe | Cl | CH₂CH₂OMe | H | |
| 8-114 | OMe | Br | CH₂CH₂OMe | H | |
| 8-115 | OMe | I | CH₂CH₂OMe | H | |
| 8-116 | OMe | CF₃ | CH₂CH₂OMe | H | |
| 8-117 | OMe | CHF₂ | CH₂CH₂OMe | H | |
| 8-118 | OMe | CF₂Cl | CH₂CH₂OMe | H | |
| 8-119 | OMe | OMe | CH₂CH₂OMe | H | |
| 8-120 | OMe | NO₂ | CH₂CH₂OMe | H | |
| 8-121 | OMe | SO₂Me | CH₂CH₂OMe | H | |
| 8-122 | SO₂Me | Me | CH₂CH₂OMe | H | |
| 8-123 | SO₂Me | F | CH₂CH₂OMe | H | |
| 8-124 | SO₂Me | Cl | CH₂CH₂OMe | H | |
| 8-125 | SO₂Me | Br | CH₂CH₂OMe | H | |
| 8-126 | SO₂Me | I | CH₂CH₂OMe | H | |
| 8-127 | SO₂Me | CF₃ | CH₂CH₂OMe | H | |
| 8-128 | SO₂Me | CHF₂ | CH₂CH₂OMe | H | |
| 8-129 | SO₂Me | CF₂Cl | CH₂CH₂OMe | H | |
| 8-130 | SO₂Me | OMe | CH₂CH₂OMe | H | |
| 8-131 | SO₂Me | NO₂ | CH₂CH₂OMe | H | |
| 8-132 | SO₂Me | SO₂Me | CH₂CH₂OMe | H | |
| 8-133 | Me | Me | Me | CN | |
| 8-134 | Me | F | Me | CN | |
| 8-135 | Me | Cl | Me | CN | |
| 8-136 | Me | Br | Me | CN | |
| 8-137 | Me | I | Me | CN | |
| 8-138 | Me | CF₃ | Me | CN | |
| 8-139 | Me | CHF₂ | Me | CN | |
| 8-140 | Me | CF₂Cl | Me | CN | |
| 8-141 | Me | OMe | Me | CN | |
| 8-142 | Me | NO₂ | Me | CN | |
| 8-143 | Me | SO₂Me | Me | CN | |
| 8-144 | Cl | Me | Me | CN | |
| 8-145 | Cl | F | Me | CN | |
| 8-146 | Cl | Cl | Me | CN | |
| 8-147 | Cl | Br | Me | CN | |
| 8-148 | Cl | I | Me | CN | |
| 8-149 | Cl | CF₃ | Me | CN | |
| 8-150 | Cl | CHF₂ | Me | CN | |
| 8-151 | Cl | CF₂Cl | Me | CN | |
| 8-152 | Cl | OMe | Me | CN | |
| 8-153 | Cl | NO₂ | Me | CN | |
| 8-154 | Cl | SO₂Me | Me | CN | |
| 8-155 | OMe | Me | Me | CN | |
| 8-156 | OMe | F | Me | CN | |
| 8-157 | OMe | Cl | Me | CN | |
| 8-158 | OMe | Br | Me | CN | |
| 8-159 | OMe | I | Me | CN | |
| 8-160 | OMe | CF₃ | Me | CN | |
| 8-161 | OMe | CHF₂ | Me | CN | |
| 8-162 | OMe | CF₂Cl | Me | CN | |
| 8-163 | OMe | OMe | Me | CN | |
| 8-164 | OMe | NO₂ | Me | CN | |
| 8-165 | OMe | SO₂Me | Me | CN | |
| 8-166 | SO₂Me | Me | Me | CN | |
| 8-167 | SO₂Me | F | Me | CN | |
| 8-168 | SO₂Me | Cl | Me | CN | |
| 8-169 | SO₂Me | Br | Me | CN | |
| 8-170 | SO₂Me | I | Me | CN | |
| 8-171 | SO₂Me | CF₃ | Me | CN | |
| 8-172 | SO₂Me | CHF₂ | Me | CN | |
| 8-173 | SO₂Me | CF₂Cl | Me | CN | |
| 8-174 | SO₂Me | OMe | Me | CN | |
| 8-175 | SO₂Me | NO₂ | Me | CN | |
| 8-176 | SO₂Me | SO₂Me | Me | CN | |
| 8-177 | Me | Me | Et | CN | |
| 8-178 | Me | F | Et | CN | |
| 8-179 | Me | Cl | Et | CN | |
| 8-180 | Me | Br | Et | CN | |
| 8-181 | Me | I | Et | CN | |
| 8-182 | Me | CF₃ | Et | CN | |
| 8-183 | Me | CHF₂ | Et | CN | |
| 8-184 | Me | CF₂Cl | Et | CN | |
| 8-185 | Me | OMe | Et | CN | |
| 8-186 | Me | NO₂ | Et | CN | |
| 8-187 | Me | SO₂Me | Et | CN | |
| 8-188 | Cl | Me | Et | CN | |
| 8-189 | Cl | F | Et | CN | |
| 8-190 | Cl | Cl | Et | CN | |
| 8-191 | Cl | Br | Et | CN | |
| 8-192 | Cl | I | Et | CN | |
| 8-193 | Cl | CF₃ | Et | CN | |
| 8-194 | Cl | CHF₂ | Et | CN | |
| 8-195 | Cl | CF₂Cl | Et | CN | |
| 8-196 | Cl | OMe | Et | CN | |
| 8-197 | Cl | NO₂ | Et | CN | |
| 8-198 | Cl | SO₂Me | Et | CN | |
| 8-199 | OMe | Me | Et | CN | |
| 8-200 | OMe | F | Et | CN | |
| 8-201 | OMe | Cl | Et | CN | |
| 8-202 | OMe | Br | Et | CN | |
| 8-203 | OMe | I | Et | CN | |
| 8-204 | OMe | CF₃ | Et | CN | |
| 8-205 | OMe | CHF₂ | Et | CN | |
| 8-206 | OMe | CF₂Cl | Et | CN | |
| 8-207 | OMe | OMe | Et | CN | |
| 8-208 | OMe | NO₂ | Et | CN | |
| 8-209 | OMe | SO₂Me | Et | CN | |
| 8-210 | SO₂Me | Me | Et | CN | |
| 8-211 | SO₂Me | F | Et | CN | |
| 8-212 | SO₂Me | Cl | Et | CN | |
| 8-213 | SO₂Me | Br | Et | CN | |
| 8-214 | SO₂Me | I | Et | CN | |
| 8-215 | SO₂Me | CF₃ | Et | CN | |
| 8-216 | SO₂Me | CHF₂ | Et | CN | |
| 8-217 | SO₂Me | CF₂Cl | Et | CN | |
| 8-218 | SO₂Me | OMe | Et | CN | |
| 8-219 | SO₂Me | NO₂ | Et | CN | |
| 8-220 | SO₂Me | SO₂Me | Et | CN | |
| 8-221 | Me | Me | CH₂CH₂OMe | CN | |
| 8-222 | Me | F | CH₂CH₂OMe | CN | |
| 8-223 | Me | Cl | CH₂CH₂OMe | CN | |
| 8-224 | Me | Br | CH₂CH₂OMe | CN | |
| 8-225 | Me | I | CH₂CH₂OMe | CN | |
| 8-226 | Me | CF₃ | CH₂CH₂OMe | CN | |
| 8-227 | Me | CHF₂ | CH₂CH₂OMe | CN | |
| 8-228 | Me | CF₂Cl | CH₂CH₂OMe | CN | |
| 8-229 | Me | OMe | CH₂CH₂OMe | CN | |
| 8-230 | Me | NO₂ | CH₂CH₂OMe | CN | |
| 8-231 | Me | SO₂Me | CH₂CH₂OMe | CN | |
| 8-232 | Cl | Me | CH₂CH₂OMe | CN | |
| 8-233 | Cl | F | CH₂CH₂OMe | CN | |
| 8-234 | Cl | Cl | CH₂CH₂OMe | CN | |
| 8-235 | Cl | Br | CH₂CH₂OMe | CN | |
| 8-236 | Cl | I | CH₂CH₂OMe | CN | |
| 8-237 | Cl | CF₃ | CH₂CH₂OMe | CN | |
| 8-238 | Cl | CHF₂ | CH₂CH₂OMe | CN | |
| 8-239 | Cl | CF₂Cl | CH₂CH₂OMe | CN | |
| 8-240 | Cl | OMe | CH₂CH₂OMe | CN | |
| 8-241 | Cl | NO₂ | CH₂CH₂OMe | CN | |
| 8-242 | Cl | SO₂Me | CH₂CH₂OMe | CN | |
| 8-243 | OMe | Me | CH₂CH₂OMe | CN | |
| 8-244 | OMe | F | CH₂CH₂OMe | CN | |
| 8-245 | OMe | Cl | CH₂CH₂OMe | CN | |
| 8-246 | OMe | Br | CH₂CH₂OMe | CN | |
| 8-247 | OMe | I | CH₂CH₂OMe | CN | |
| 8-248 | OMe | CF₃ | CH₂CH₂OMe | CN | |
| 8-249 | OMe | CHF₂ | CH₂CH₂OMe | CN | |
| 8-250 | OMe | CF₂Cl | CH₂CH₂OMe | CN | |
| 8-251 | OMe | OMe | CH₂CH₂OMe | CN | |
| 8-252 | OMe | NO₂ | CH₂CH₂OMe | CN | |
| 8-253 | OMe | SO₂Me | CH₂CH₂OMe | CN | |
| 8-254 | SO₂Me | Me | CH₂CH₂OMe | CN | |
| 8-255 | SO₂Me | F | CH₂CH₂OMe | CN | |
| 8-256 | SO₂Me | Cl | CH₂CH₂OMe | CN | |
| 8-257 | SO₂Me | Br | CH₂CH₂OMe | CN | |
| 8-258 | SO₂Me | I | CH₂CH₂OMe | CN | |
| 8-259 | SO₂Me | CF₃ | CH₂CH₂OMe | CN | |
| 8-260 | SO₂Me | CHF₂ | CH₂CH₂OMe | CN | |
| 8-261 | SO₂Me | CF₂Cl | CH₂CH₂OMe | CN | |
| 8-262 | SO₂Me | OMe | CH₂CH₂OMe | CN | |
| 8-263 | SO₂Me | NO₂ | CH₂CH₂OMe | CN | |
| 8-264 | SO₂Me | SO₂Me | CH₂CH₂OMe | CN | |

**Tabelle 9: Erfindungsgemäße Verbindungen der allgemeinen Formel (I), worin Q für Q2, R^{a} für eine Hydroxylgruppe, R^{b}, R^{f} und W jeweils für Wasserstoff, A für CH₂CH₂, Y für CH₂ stehen, t = 0 ist und die anderen Reste die in der Tabelle angegebenen Bedeutungen haben**

| | | | | | |
|---|---|---|---|---|---|
| 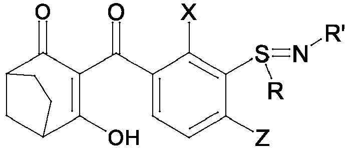 | | | | | |

| Nr. | X | Z | R | R' | Physikalische Daten (¹H-NMR) |
|---|---|---|---|---|---|
| 9-1 | Me | Me | Me | H | |
| 9-2 | Me | F | Me | H | |
| 9-3 | Me | Cl | Me | H | |
| 9-4 | Me | Br | Me | H | |
| 9-5 | Me | I | Me | H | |
| 9-6 | Me | CF₃ | Me | H | |
| 9-7 | Me | CHF₂ | Me | H | |
| 9-8 | Me | CF₂Cl | Me | H | |
| 9-9 | Me | OMe | Me | H | |
| 9-10 | Me | NO₂ | Me | H | |
| 9-11 | Me | SO₂Me | Me | H | |
| 9-12 | Cl | Me | Me | H | |
| 9-13 | Cl | F | Me | H | |
| 9-14 | Cl | Cl | Me | H | |
| 9-15 | Cl | Br | Me | H | |
| 9-16 | Cl | I | Me | H | |
| 9-17 | Cl | CF₃ | Me | H | |
| 9-18 | Cl | CHF₂ | Me | H | |
| 9-19 | Cl | CF₂Cl | Me | H | |
| 9-20 | Cl | OMe | Me | H | |
| 9-21 | Cl | NO₂ | Me | H | |
| 9-22 | Cl | SO₂Me | Me | H | |
| 9-23 | OMe | Me | Me | H | |
| 9-24 | OMe | F | Me | H | |
| 9-25 | OMe | Cl | Me | H | |
| 9-26 | OMe | Br | Me | H | |
| 9-27 | OMe | I | Me | H | |
| 9-28 | OMe | CF₃ | Me | H | |
| 9-29 | OMe | CHF₂ | Me | H | |
| 9-30 | OMe | CF₂Cl | Me | H | |
| 9-31 | OMe | OMe | Me | H | |
| 9-32 | OMe | NO₂ | Me | H | |
| 9-33 | OMe | SO₂Me | Me | H | |
| 9-34 | SO₂Me | Me | Me | H | |
| 9-35 | SO₂Me | F | Me | H | |
| 9-36 | SO₂Me | Cl | Me | H | |
| 9-37 | SO₂Me | Br | Me | H | |
| 9-38 | SO₂Me | I | Me | H | |
| 9-39 | SO₂Me | CF₃ | Me | H | |
| 9-40 | SO₂Me | CHF₂ | Me | H | |
| 9-41 | SO₂Me | CF₂Cl | Me | H | |
| 9-42 | SO₂Me | OMe | Me | H | |
| 9-43 | SO₂Me | NO₂ | Me | H | |
| 9-44 | SO₂Me | SO₂Me | Me | H | |
| 9-45 | Me | Me | Et | H | |
| 9-46 | Me | F | Et | H | |
| 9-47 | Me | Cl | Et | H | |
| 9-48 | Me | Br | Et | H | |
| 9-49 | Me | I | Et | H | |
| 9-50 | Me | CF₃ | Et | H | |
| 9-51 | Me | CHF₂ | Et | H | |
| 9-52 | Me | CF₂Cl | Et | H | |
| 9-53 | Me | OMe | Et | H | |
| 9-54 | Me | NO₂ | Et | H | |
| 9-55 | Me | SO₂Me | Et | H | |
| 9-56 | Cl | Me | Et | H | |
| 9-57 | Cl | F | Et | H | |
| 9-58 | Cl | Cl | Et | H | |
| 9-59 | Cl | Br | Et | H | |
| 9-60 | Cl | I | Et | H | |
| 9-61 | Cl | CF₃ | Et | H | |
| 9-62 | Cl | CHF₂ | Et | H | |
| 9-63 | Cl | CF₂Cl | Et | H | |
| 9-64 | Cl | OMe | Et | H | |
| 9-65 | Cl | NO₂ | Et | H | |
| 9-66 | Cl | SO₂Me | Et | H | |
| 9-67 | OMe | Me | Et | H | |
| 9-68 | OMe | F | Et | H | |
| 9-69 | OMe | Cl | Et | H | |
| 9-70 | OMe | Br | Et | H | |
| 9-71 | OMe | I | Et | H | |
| 9-72 | OMe | CF₃ | Et | H | |
| 9-73 | OMe | CHF₂ | Et | H | |
| 9-74 | OMe | CF₂Cl | Et | H | |
| 9-75 | OMe | OMe | Et | H | |
| 9-76 | OMe | NO₂ | Et | H | |
| 9-77 | OMe | SO₂Me | Et | H | |
| 9-78 | SO₂Me | Me | Et | H | |
| 9-79 | SO₂Me | F | Et | H | |
| 9-80 | SO₂Me | Cl | Et | H | |
| 9-81 | SO₂Me | Br | Et | H | |
| 9-82 | SO₂Me | I | Et | H | |
| 9-83 | SO₂Me | CF₃ | Et | H | |
| 9-84 | SO₂Me | CHF₂ | Et | H | |
| 9-85 | SO₂Me | CF₂Cl | Et | H | |
| 9-86 | SO₂Me | OMe | Et | H | |
| 9-87 | SO₂Me | NO₂ | Et | H | |
| 9-88 | SO₂Me | SO₂Me | Et | H | |
| 9-89 | Me | Me | CH₂CH₂OMe | H | |
| 9-90 | Me | F | CH₂CH₂OMe | H | |
| 9-91 | Me | Cl | CH₂CH₂OMe | H | |
| 9-92 | Me | Br | CH₂CH₂OMe | H | |
| 9-93 | Me | I | CH₂CH₂OMe | H | |
| 9-94 | Me | CF₃ | CH₂CH₂OMe | H | |
| 9-95 | Me | CHF₂ | CH₂CH₂OMe | H | |
| 9-96 | Me | CF₂Cl | CH₂CH₂OMe | H | |
| 9-97 | Me | OMe | CH₂CH₂OMe | H | |
| 9-98 | Me | NO₂ | CH₂CH₂OMe | H | |
| 9-99 | Me | SO₂Me | CH₂CH₂OMe | H | |
| 9-100 | Cl | Me | CH₂CH₂OMe | H | |
| 9-101 | Cl | F | CH₂CH₂OMe | H | |
| 9-102 | Cl | Cl | CH₂CH₂OMe | H | |
| 9-103 | Cl | Br | CH₂CH₂OMe | H | |
| 9-104 | Cl | I | CH₂CH₂OMe | H | |
| 9-105 | Cl | CF₃ | CH₂CH₂OMe | H | |
| 9-106 | Cl | CHF₂ | CH₂CH₂OMe | H | |
| 9-107 | Cl | CF₂Cl | CH₂CH₂OMe | H | |
| 9-108 | Cl | OMe | CH₂CH₂OMe | H | |
| 9-109 | Cl | NO₂ | CH₂CH₂OMe | H | |
| 9-110 | Cl | SO₂Me | CH₂CH₂OMe | H | |
| 9-111 | OMe | Me | CH₂CH₂OMe | H | |
| 9-112 | OMe | F | CH₂CH₂OMe | H | |
| 9-113 | OMe | Cl | CH₂CH₂OMe | H | |
| 9-114 | OMe | Br | CH₂CH₂OMe | H | |
| 9-115 | OMe | I | CH₂CH₂OMe | H | |
| 9-116 | OMe | CF₃ | CH₂CH₂OMe | H | |
| 9-117 | OMe | CHF₂ | CH₂CH₂OMe | H | |
| 9-118 | OMe | CF₂Cl | CH₂CH₂OMe | H | |
| 9-119 | OMe | OMe | CH₂CH₂OMe | H | |
| 9-120 | OMe | NO₂ | CH₂CH₂OMe | H | |
| 9-121 | OMe | SO₂Me | CH₂CH₂OMe | H | |
| 9-122 | SO₂Me | Me | CH₂CH₂OMe | H | |
| 9-123 | SO₂Me | F | CH₂CH₂OMe | H | |
| 9-124 | SO₂Me | Cl | CH₂CH₂OMe | H | |
| 9-125 | SO₂Me | Br | CH₂CH₂OMe | H | |
| 9-126 | SO₂Me | I | CH₂CH₂OMe | H | |
| 9-127 | SO₂Me | CF₃ | CH₂CH₂OMe | H | |
| 9-128 | SO₂Me | CHF₂ | CH₂CH₂OMe | H | |
| 9-129 | SO₂Me | CF₂Cl | CH₂CH₂OMe | H | |
| 9-130 | SO₂Me | OMe | CH₂CH₂OMe | H | |
| 9-131 | SO₂Me | NO₂ | CH₂CH₂OMe | H | |
| 9-132 | SO₂Me | SO₂Me | CH₂CH₂OMe | H | |
| 9-133 | Me | Me | Me | CN | |
| 9-134 | Me | F | Me | CN | |
| 9-135 | Me | Cl | Me | CN | |
| 9-136 | Me | Br | Me | CN | |
| 9-137 | Me | I | Me | CN | |
| 9-138 | Me | CF₃ | Me | CN | |
| 9-139 | Me | CHF₂ | Me | CN | |
| 9-140 | Me | CF₂Cl | Me | CN | |
| 9-141 | Me | OMe | Me | CN | |
| 9-142 | Me | NO₂ | Me | CN | |
| 9-143 | Me | SO₂Me | Me | CN | |
| 9-144 | Cl | Me | Me | CN | |
| 9-145 | Cl | F | Me | CN | |
| 9-146 | Cl | Cl | Me | CN | |
| 9-147 | Cl | Br | Me | CN | |
| 9-148 | Cl | I | Me | CN | |
| 9-149 | Cl | CF₃ | Me | CN | |
| 9-150 | Cl | CHF₂ | Me | CN | |
| 9-151 | Cl | CF₂Cl | Me | CN | |
| 9-152 | Cl | OMe | Me | CN | |
| 9-153 | Cl | NO₂ | Me | CN | |
| 9-154 | Cl | SO₂Me | Me | CN | |
| 9-155 | OMe | Me | Me | CN | |
| 9-156 | OMe | F | Me | CN | |
| 9-157 | OMe | Cl | Me | CN | |
| 9-158 | OMe | Br | Me | CN | |
| 9-159 | OMe | I | Me | CN | |
| 9-160 | OMe | CF₃ | Me | CN | |
| 9-161 | OMe | CHF₂ | Me | CN | |
| 9-162 | OMe | CF₂Cl | Me | CN | |
| 9-163 | OMe | OMe | Me | CN | |
| 9-164 | OMe | NO₂ | Me | CN | |
| 9-165 | OMe | SO₂Me | Me | CN | |
| 9-166 | SO₂Me | Me | Me | CN | |
| 9-167 | SO₂Me | F | Me | CN | |
| 9-168 | SO₂Me | Cl | Me | CN | |
| 9-169 | SO₂Me | Br | Me | CN | |
| 9-170 | SO₂Me | I | Me | CN | |
| 9-171 | SO₂Me | CF₃ | Me | CN | |
| 9-172 | SO₂Me | CHF₂ | Me | CN | |
| 9-173 | SO₂Me | CF₂Cl | Me | CN | |
| 9-174 | SO₂Me | OMe | Me | CN | |
| 9-175 | SO₂Me | NO₂ | Me | CN | |
| 9-176 | SO₂Me | SO₂Me | Me | CN | |
| 9-177 | Me | Me | Et | CN | |
| 9-178 | Me | F | Et | CN | |
| 9-179 | Me | Cl | Et | CN | |
| 9-180 | Me | Br | Et | CN | |
| 9-181 | Me | I | Et | CN | |
| 9-182 | Me | CF₃ | Et | CN | |
| 9-183 | Me | CHF₂ | Et | CN | |
| 9-184 | Me | CF₂Cl | Et | CN | |
| 9-185 | Me | OMe | Et | CN | |
| 9-186 | Me | NO₂ | Et | CN | |
| 9-187 | Me | SO₂Me | Et | CN | |
| 9-188 | Cl | Me | Et | CN | |
| 9-189 | Cl | F | Et | CN | |
| 9-190 | Cl | Cl | Et | CN | |
| 9-191 | Cl | Br | Et | CN | |
| 9-192 | Cl | I | Et | CN | |
| 9-193 | Cl | CF₃ | Et | CN | |
| 9-194 | Cl | CHF₂ | Et | CN | |
| 9-195 | Cl | CF₂Cl | Et | CN | |
| 9-196 | Cl | OMe | Et | CN | |
| 9-197 | Cl | NO₂ | Et | CN | |
| 9-198 | Cl | SO₂Me | Et | CN | |
| 9-199 | OMe | Me | Et | CN | |
| 9-200 | OMe | F | Et | CN | |
| 9-201 | OMe | Cl | Et | CN | |
| 9-202 | OMe | Br | Et | CN | |
| 9-203 | OMe | I | Et | CN | |
| 9-204 | OMe | CF₃ | Et | CN | |
| 9-205 | OMe | CHF₂ | Et | CN | |
| 9-206 | OMe | CF₂Cl | Et | CN | |
| 9-207 | OMe | OMe | Et | CN | |
| 9-208 | OMe | NO₂ | Et | CN | |
| 9-209 | OMe | SO₂Me | Et | CN | |
| 9-210 | SO₂Me | Me | Et | CN | |
| 9-211 | SO₂Me | F | Et | CN | |
| 9-212 | SO₂Me | Cl | Et | CN | |
| 9-213 | SO₂Me | Br | Et | CN | |
| 9-214 | SO₂Me | I | Et | CN | |
| 9-215 | SO₂Me | CF₃ | Et | CN | |
| 9-216 | SO₂Me | CHF₂ | Et | CN | |
| 9-217 | SO₂Me | CF₂Cl | Et | CN | |
| 9-218 | SO₂Me | OMe | Et | CN | |
| 9-219 | SO₂Me | NO₂ | Et | CN | |
| 9-220 | SO₂Me | SO₂Me | Et | CN | |
| 9-221 | Me | Me | CH₂CH₂OMe | CN | |
| 9-222 | Me | F | CH₂CH₂OMe | CN | |
| 9-223 | Me | Cl | CH₂CH₂OMe | CN | |
| 9-224 | Me | Br | CH₂CH₂OMe | CN | |
| 9-225 | Me | I | CH₂CH₂OMe | CN | |
| 9-226 | Me | CF₃ | CH₂CH₂OMe | CN | |
| 9-227 | Me | CHF₂ | CH₂CH₂OMe | CN | |
| 9-228 | Me | CF₂Cl | CH₂CH₂OMe | CN | |
| 9-229 | Me | OMe | CH₂CH₂OMe | CN | |
| 9-230 | Me | NO₂ | CH₂CH₂OMe | CN | |
| 9-231 | Me | SO₂Me | CH₂CH₂OMe | CN | |
| 9-232 | Cl | Me | CH₂CH₂OMe | CN | |
| 9-233 | Cl | F | CH₂CH₂OMe | CN | |
| 9-234 | Cl | Cl | CH₂CH₂OMe | CN | |
| 9-235 | Cl | Br | CH₂CH₂OMe | CN | |
| 9-236 | Cl | I | CH₂CH₂OMe | CN | |
| 9-237 | Cl | CF₃ | CH₂CH₂OMe | CN | |
| 9-238 | Cl | CHF₂ | CH₂CH₂OMe | CN | |
| 9-239 | Cl | CF₂Cl | CH₂CH₂OMe | CN | |
| 9-240 | Cl | OMe | CH₂CH₂OMe | CN | |
| 9-241 | Cl | NO₂ | CH₂CH₂OMe | CN | |
| 9-242 | Cl | SO₂Me | CH₂CH₂OMe | CN | |
| 9-243 | OMe | Me | CH₂CH₂OMe | CN | |
| 9-244 | OMe | F | CH₂CH₂OMe | CN | |
| 9-245 | OMe | Cl | CH₂CH₂OMe | CN | |
| 9-246 | OMe | Br | CH₂CH₂OMe | CN | |
| 9-247 | OMe | I | CH₂CH₂OMe | CN | |
| 9-248 | OMe | CF₃ | CH₂CH₂OMe | CN | |
| 9-249 | OMe | CHF₂ | CH₂CH₂OMe | CN | |
| 9-250 | OMe | CF₂Cl | CH₂CH₂OMe | CN | |
| 9-251 | OMe | OMe | CH₂CH₂OMe | CN | |
| 9-252 | OMe | NO₂ | CH₂CH₂OMe | CN | |
| 9-253 | OMe | SO₂Me | CH₂CH₂OMe | CN | |
| 9-254 | SO₂Me | Me | CH₂CH₂OMe | CN | |
| 9-255 | SO₂Me | F | CH₂CH₂OMe | CN | |
| 9-256 | SO₂Me | Cl | CH₂CH₂OMe | CN | |
| 9-257 | SO₂Me | Br | CH₂CH₂OMe | CN | |
| 9-258 | SO₂Me | I | CH₂CH₂OMe | CN | |
| 9-259 | SO₂Me | CF₃ | CH₂CH₂OMe | CN | |
| 9-260 | SO₂Me | CHF₂ | CH₂CH₂OMe | CN | |
| 9-261 | SO₂Me | CF₂Cl | CH₂CH₂OMe | CN | |
| 9-262 | SO₂Me | OMe | CH₂CH₂OMe | CN | |
| 9-263 | SO₂Me | NO₂ | CH₂CH₂OMe | CN | |
| 9-264 | SO₂Me | SO₂Me | CH₂CH₂OMe | CN | |

**Tabelle 10: Erfindungsgemäße Verbindungen der allgemeinen Formel (I), worin Q für Q3, Rⁱ für Methyl und R^{h} , R^{k} sowie W jeweils für Wasserstoff stehen, t = 0 ist und die anderen Reste die in der Tabelle angegebenen Bedeutungen haben**

| | | | | | |
|---|---|---|---|---|---|
| 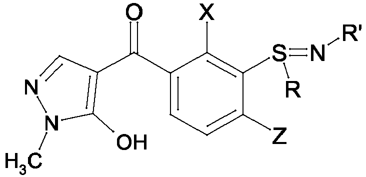 | | | | | |

| Nr. | X | Z | R | R' | Physikalische Daten (¹H-NMR) |
|---|---|---|---|---|---|
| 10-1 | Me | Me | Me | H | |
| 10-2 | Me | F | Me | H | |
| 10-3 | Me | Cl | Me | H | |
| 10-4 | Me | Br | Me | H | |
| 10-5 | Me | I | Me | H | |
| 10-6 | Me | CF₃ | Me | H | |
| 10-7 | Me | CHF₂ | Me | H | |
| 10-8 | Me | CF₂Cl | Me | H | |
| 10-9 | Me | OMe | Me | H | |
| 10-10 | Me | NO₂ | Me | H | |
| 10-11 | Me | SO₂Me | Me | H | |
| 10-12 | Cl | Me | Me | H | |
| 10-13 | Cl | F | Me | H | |
| 10-14 | Cl | Cl | Me | H | |
| 10-15 | Cl | Br | Me | H | |
| 10-16 | Cl | I | Me | H | |
| 10-17 | Cl | CF₃ | Me | H | |
| 10-18 | Cl | CHF₂ | Me | H | |
| 10-19 | Cl | CF₂Cl | Me | H | |
| 10-20 | Cl | OMe | Me | H | |
| 10-21 | Cl | NO₂ | Me | H | |
| 10-22 | Cl | SO₂Me | Me | H | |
| 10-23 | OMe | Me | Me | H | |
| 10-24 | OMe | F | Me | H | |
| 10-25 | OMe | Cl | Me | H | |
| 10-26 | OMe | Br | Me | H | |
| 10-27 | OMe | I | Me | H | |
| 10-28 | OMe | CF₃ | Me | H | |
| 10-29 | OMe | CHF₂ | Me | H | |
| 10-30 | OMe | CF₂Cl | Me | H | |
| 10-31 | OMe | OMe | Me | H | |
| 10-32 | OMe | NO₂ | Me | H | |
| 10-33 | OMe | SO₂Me | Me | H | |
| 10-34 | SO₂Me | Me | Me | H | |
| 10-35 | SO₂Me | F | Me | H | |
| 10-36 | SO₂Me | Cl | Me | H | |
| 10-37 | SO₂Me | Br | Me | H | |
| 10-38 | SO₂Me | I | Me | H | |
| 10-39 | SO₂Me | CF₃ | Me | H | |
| 10-40 | SO₂Me | CHF₂ | Me | H | |
| 10-41 | SO₂Me | CF₂Cl | Me | H | |
| 10-42 | SO₂Me | OMe | Me | H | |
| 10-43 | SO₂Me | NO₂ | Me | H | |
| 10-44 | SO₂Me | SO₂Me | Me | H | |
| 10-45 | Me | Me | Et | H | |
| 10-46 | Me | F | Et | H | |
| 10-47 | Me | Cl | Et | H | |
| 10-48 | Me | Br | Et | H | |
| 10-49 | Me | I | Et | H | |
| 10-50 | Me | CF₃ | Et | H | |
| 10-51 | Me | CHF₂ | Et | H | |
| 10-52 | Me | CF₂Cl | Et | H | |
| 10-53 | Me | OMe | Et | H | |
| 10-54 | Me | NO₂ | Et | H | |
| 10-55 | Me | SO₂Me | Et | H | |
| 10-56 | Cl | Me | Et | H | |
| 10-57 | Cl | F | Et | H | |
| 10-58 | Cl | Cl | Et | H | |
| 10-59 | Cl | Br | Et | H | |
| 10-60 | Cl | I | Et | H | |
| 10-61 | Cl | CF₃ | Et | H | |
| 10-62 | Cl | CHF₂ | Et | H | |
| 10-63 | Cl | CF₂Cl | Et | H | |
| 10-64 | Cl | OMe | Et | H | |
| 10-65 | Cl | NO₂ | Et | H | |
| 10-66 | Cl | SO₂Me | Et | H | |
| 10-67 | OMe | Me | Et | H | |
| 10-68 | OMe | F | Et | H | |
| 10-69 | OMe | Cl | Et | H | |
| 10-70 | OMe | Br | Et | H | |
| 10-71 | OMe | I | Et | H | |
| 10-72 | OMe | CF₃ | Et | H | |
| 10-73 | OMe | CHF₂ | Et | H | |
| 10-74 | OMe | CF₂Cl | Et | H | |
| 10-75 | OMe | OMe | Et | H | |
| 10-76 | OMe | NO₂ | Et | H | |
| 10-77 | OMe | SO₂Me | Et | H | |
| 10-78 | SO₂Me | Me | Et | H | |
| 10-79 | SO₂Me | F | Et | H | |
| 10-80 | SO₂Me | Cl | Et | H | |
| 10-81 | SO₂Me | Br | Et | H | |
| 10-82 | SO₂Me | I | Et | H | |
| 10-83 | SO₂Me | CF₃ | Et | H | |
| 10-84 | SO₂Me | CHF₂ | Et | H | |
| 10-85 | SO₂Me | CF₂Cl | Et | H | |
| 10-86 | SO₂Me | OMe | Et | H | |
| 10-87 | SO₂Me | NO₂ | Et | H | |
| 10-88 | SO₂Me | SO₂Me | Et | H | |
| 10-89 | Me | Me | CH₂CH₂OMe | H | |
| 10-90 | Me | F | CH₂CH₂OMe | H | |
| 10-91 | Me | Cl | CH₂CH₂OMe | H | |
| 10-92 | Me | Br | CH₂CH₂OMe | H | |
| 10-93 | Me | I | CH₂CH₂OMe | H | |
| 10-94 | Me | CF₃ | CH₂CH₂OMe | H | |
| 10-95 | Me | CHF₂ | CH₂CH₂OMe | H | |
| 10-96 | Me | CF₂Cl | CH₂CH₂OMe | H | |
| 10-97 | Me | OMe | CH₂CH₂OMe | H | |
| 10-98 | Me | NO₂ | CH₂CH₂OMe | H | |
| 10-99 | Me | SO₂Me | CH₂CH₂OMe | H | |
| 10-100 | Cl | Me | CH₂CH₂OMe | H | |
| 10-101 | Cl | F | CH₂CH₂OMe | H | |
| 10-102 | Cl | Cl | CH₂CH₂OMe | H | |
| 10-103 | Cl | Br | CH₂CH₂OMe | H | |
| 10-104 | Cl | I | CH₂CH₂OMe | H | |
| 10-105 | Cl | CF₃ | CH₂CH₂OMe | H | |
| 10-106 | Cl | CHF₂ | CH₂CH₂OMe | H | |
| 10-107 | Cl | CF₂Cl | CH₂CH₂OMe | H | |
| 10-108 | Cl | OMe | CH₂CH₂OMe | H | |
| 10-109 | Cl | NO₂ | CH₂CH₂OMe | H | |
| 10-110 | Cl | SO₂Me | CH₂CH₂OMe | H | |
| 10-111 | OMe | Me | CH₂CH₂OMe | H | |
| 10-112 | OMe | F | CH₂CH₂OMe | H | |
| 10-113 | OMe | Cl | CH₂CH₂OMe | H | |
| 10-114 | OMe | Br | CH₂CH₂OMe | H | |
| 10-115 | OMe | I | CH₂CH₂OMe | H | |
| 10-116 | OMe | CF₃ | CH₂CH₂OMe | H | |
| 10-117 | OMe | CHF₂ | CH₂CH₂OMe | H | |
| 10-118 | OMe | CF₂Cl | CH₂CH₂OMe | H | |
| 10-119 | OMe | OMe | CH₂CH₂OMe | H | |
| 10-120 | OMe | NO₂ | CH₂CH₂OMe | H | |
| 10-121 | OMe | SO₂Me | CH₂CH₂OMe | H | |
| 10-122 | SO₂Me | Me | CH₂CH₂OMe | H | |
| 10-123 | SO₂Me | F | CH₂CH₂OMe | H | |
| 10-124 | SO₂Me | Cl | CH₂CH₂OMe | H | |
| 10-125 | SO₂Me | Br | CH₂CH₂OMe | H | |
| 10-126 | SO₂Me | I | CH₂CH₂OMe | H | |
| 10-127 | SO₂Me | CF₃ | CH₂CH₂OMe | H | |
| 10-128 | SO₂Me | CHF₂ | CH₂CH₂OMe | H | |
| 10-129 | SO₂Me | CF₂Cl | CH₂CH₂OMe | H | |
| 10-130 | SO₂Me | OMe | CH₂CH₂OMe | H | |
| 10-131 | SO₂Me | NO₂ | CH₂CH₂OMe | H | |
| 10-132 | SO₂Me | SO₂Me | CH₂CH₂OMe | H | |
| 10-133 | Me | Me | Me | CN | |
| 10-134 | Me | F | Me | CN | |
| 10-135 | Me | Cl | Me | CN | |
| 10-136 | Me | Br | Me | CN | |
| 10-137 | Me | I | Me | CN | |
| 10-138 | Me | CF₃ | Me | CN | |
| 10-139 | Me | CHF₂ | Me | CN | |
| 10-140 | Me | CF₂Cl | Me | CN | |
| 10-141 | Me | OMe | Me | CN | |
| 10-142 | Me | NO₂ | Me | CN | |
| 10-143 | Me | SO₂Me | Me | CN | |
| 10-144 | Cl | Me | Me | CN | |
| 10-145 | Cl | F | Me | CN | |
| 10-146 | Cl | Cl | Me | CN | |
| 10-147 | Cl | Br | Me | CN | |
| 10-148 | Cl | I | Me | CN | |
| 10-149 | Cl | CF₃ | Me | CN | |
| 10-150 | Cl | CHF₂ | Me | CN | |
| 10-151 | Cl | CF₂Cl | Me | CN | |
| 10-152 | Cl | OMe | Me | CN | |
| 10-153 | Cl | NO₂ | Me | CN | |
| 10-154 | Cl | SO₂Me | Me | CN | |
| 10-155 | OMe | Me | Me | CN | |
| 10-156 | OMe | F | Me | CN | |
| 10-157 | OMe | Cl | Me | CN | |
| 10-158 | OMe | Br | Me | CN | |
| 10-159 | OMe | I | Me | CN | |
| 10-160 | OMe | CF₃ | Me | CN | |
| 10-161 | OMe | CHF₂ | Me | CN | |
| 10-162 | OMe | CF₂Cl | Me | CN | |
| 10-163 | OMe | OMe | Me | CN | |
| 10-164 | OMe | NO₂ | Me | CN | |
| 10-165 | OMe | SO₂Me | Me | CN | |
| 10-166 | SO₂Me | Me | Me | CN | |
| 10-167 | SO₂Me | F | Me | CN | |
| 10-168 | SO₂Me | Cl | Me | CN | |
| 10-169 | SO₂Me | Br | Me | CN | |
| 10-170 | SO₂Me | I | Me | CN | |
| 10-171 | SO₂Me | CF₃ | Me | CN | |
| 10-172 | SO₂Me | CHF₂ | Me | CN | |
| 10-173 | SO₂Me | CF₂Cl | Me | CN | |
| 10-174 | SO₂Me | OMe | Me | CN | |
| 10-175 | SO₂Me | NO₂ | Me | CN | |
| 10-176 | SO₂Me | SO₂Me | Me | CN | |
| 10-177 | Me | Me | Et | CN | |
| 10-178 | Me | F | Et | CN | |
| 10-179 | Me | Cl | Et | CN | |
| 10-180 | Me | Br | Et | CN | |
| 10-181 | Me | I | Et | CN | |
| 10-182 | Me | CF₃ | Et | CN | |
| 10-183 | Me | CHF₂ | Et | CN | |
| 10-184 | Me | CF₂Cl | Et | CN | |
| 10-185 | Me | OMe | Et | CN | |
| 10-186 | Me | NO₂ | Et | CN | |
| 10-187 | Me | SO₂Me | Et | CN | |
| 10-188 | Cl | Me | Et | CN | |
| 10-189 | Cl | F | Et | CN | |
| 10-190 | Cl | Cl | Et | CN | |
| 10-191 | Cl | Br | Et | CN | |
| 10-192 | Cl | I | Et | CN | |
| 10-193 | Cl | CF₃ | Et | CN | |
| 10-194 | Cl | CHF₂ | Et | CN | |
| 10-195 | Cl | CF₂Cl | Et | CN | |
| 10-196 | Cl | OMe | Et | CN | |
| 10-197 | Cl | NO₂ | Et | CN | |
| 10-198 | Cl | SO₂Me | Et | CN | |
| 10-199 | OMe | Me | Et | CN | |
| 10-200 | OMe | F | Et | CN | |
| 10-201 | OMe | Cl | Et | CN | |
| 10-202 | OMe | Br | Et | CN | |
| 10-203 | OMe | I | Et | CN | |
| 10-204 | OMe | CF₃ | Et | CN | |
| 10-205 | OMe | CHF₂ | Et | CN | |
| 10-206 | OMe | CF₂Cl | Et | CN | |
| 10-207 | OMe | OMe | Et | CN | |
| 10-208 | OMe | NO₂ | Et | CN | |
| 10-209 | OMe | SO₂Me | Et | CN | |
| 10-210 | SO₂Me | Me | Et | CN | |
| 10-211 | SO₂Me | F | Et | CN | |
| 10-212 | SO₂Me | Cl | Et | CN | |
| 10-213 | SO₂Me | Br | Et | CN | |
| 10-214 | SO₂Me | I | Et | CN | |
| 10-215 | SO₂Me | CF₃ | Et | CN | |
| 10-216 | SO₂Me | CHF₂ | Et | CN | |
| 10-217 | SO₂Me | CF₂Cl | Et | CN | |
| 10-218 | SO₂Me | OMe | Et | CN | |
| 10-219 | SO₂Me | NO₂ | Et | CN | |
| 10-220 | SO₂Me | SO₂Me | Et | CN | |
| 10-221 | Me | Me | CH₂CH₂OMe | CN | |
| 10-222 | Me | F | CH₂CH₂OMe | CN | |
| 10-223 | Me | Cl | CH₂CH₂OMe | CN | |
| 10-224 | Me | Br | CH₂CH₂OMe | CN | |
| 10-225 | Me | I | CH₂CH₂OMe | CN | |
| 10-226 | Me | CF₃ | CH₂CH₂OMe | CN | |
| 10-227 | Me | CHF₂ | CH₂CH₂OMe | CN | |
| 10-228 | Me | CF₂Cl | CH₂CH₂OMe | CN | |
| 10-229 | Me | OMe | CH₂CH₂OMe | CN | |
| 10-230 | Me | NO₂ | CH₂CH₂OMe | CN | |
| 10-231 | Me | SO₂Me | CH₂CH₂OMe | CN | |
| 10-232 | Cl | Me | CH₂CH₂OMe | CN | |
| 10-233 | Cl | F | CH₂CH₂OMe | CN | |
| 10-234 | Cl | Cl | CH₂CH₂OMe | CN | |
| 10-235 | Cl | Br | CH₂CH₂OMe | CN | |
| 10-236 | Cl | I | CH₂CH₂OMe | CN | |
| 10-237 | Cl | CF₃ | CH₂CH₂OMe | CN | |
| 10-238 | Cl | CHF₂ | CH₂CH₂OMe | CN | |
| 10-239 | Cl | CF₂Cl | CH₂CH₂OMe | CN | |
| 10-240 | Cl | OMe | CH₂CH₂OMe | CN | |
| 10-241 | Cl | NO₂ | CH₂CH₂OMe | CN | |
| 10-242 | Cl | SO₂Me | CH₂CH₂OMe | CN | |
| 10-243 | OMe | Me | CH₂CH₂OMe | CN | |
| 10-244 | OMe | F | CH₂CH₂OMe | CN | |
| 10-245 | OMe | Cl | CH₂CH₂OMe | CN | |
| 10-246 | OMe | Br | CH₂CH₂OMe | CN | |
| 10-247 | OMe | I | CH₂CH₂OMe | CN | |
| 10-248 | OMe | CF₃ | CH₂CH₂OMe | CN | |
| 10-249 | OMe | CHF₂ | CH₂CH₂OMe | CN | |
| 10-250 | OMe | CF₂Cl | CH₂CH₂OMe | CN | |
| 10-251 | OMe | OMe | CH₂CH₂OMe | CN | |
| 10-252 | OMe | NO₂ | CH₂CH₂OMe | CN | |
| 10-253 | OMe | SO₂Me | CH₂CH₂OMe | CN | |
| 10-254 | SO₂Me | Me | CH₂CH₂OMe | CN | |
| 10-255 | SO₂Me | F | CH₂CH₂OMe | CN | |
| 10-256 | SO₂Me | Cl | CH₂CH₂OMe | CN | |
| 10-257 | SO₂Me | Br | CH₂CH₂OMe | CN | |
| 10-258 | SO₂Me | I | CH₂CH₂OMe | CN | |
| 10-259 | SO₂Me | CF₃ | CH₂CH₂OMe | CN | |
| 10-260 | SO₂Me | CHF₂ | CH₂CH₂OMe | CN | |
| 10-261 | SO₂Me | CF₂Cl | CH₂CH₂OMe | CN | |
| 10-262 | SO₂Me | OMe | CH₂CH₂OMe | CN | |
| 10-263 | SO₂Me | NO₂ | CH₂CH₂OMe | CN | |
| 10-264 | SO₂Me | SO₂Me | CH₂CH₂OMe | CN | |

**Tabelle 10a: Erfindungsgemäße Verbindungen der allgemeinen Formel (I), worin Q für Q3, Rⁱ für Ethyl und R^{h}, R^{k} sowie W jeweils für Wasserstoff stehen, t = 0 ist und die anderen Reste die in der Tabelle angegebenen Bedeutungen haben**

| | | | | | |
|---|---|---|---|---|---|
| 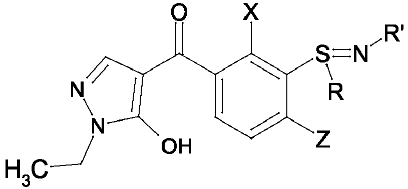 | | | | | |

| Nr. | X | Z | R | R' | Physikalische Daten (¹H-NMR) |
|---|---|---|---|---|---|
| 10a-1 | Me | Me | Me | H | |
| 10a-2 | Me | F | Me | H | |
| 10a-3 | Me | Cl | Me | H | |
| 10a-4 | Me | Br | Me | H | |
| 10a-5 | Me | I | Me | H | |
| 10a-6 | Me | CF₃ | Me | H | |
| 10a-7 | Me | CHF₂ | Me | H | |
| 10a-8 | Me | CF₂Cl | Me | H | |
| 10a-9 | Me | OMe | Me | H | |
| 10a-10 | Me | NO₂ | Me | H | |
| 10a-11 | Me | SO₂Me | Me | H | |
| 10a-12 | Cl | Me | Me | H | |
| 10a-13 | Cl | F | Me | H | |
| 10a-14 | Cl | Cl | Me | H | |
| 10a-15 | Cl | Br | Me | H | |
| 10a-16 | Cl | I | Me | H | |
| 10a-17 | Cl | CF₃ | Me | H | |
| 10a-18 | Cl | CHF₂ | Me | H | |
| 10a-19 | Cl | CF₂Cl | Me | H | |
| 10a-20 | Cl | OMe | Me | H | |
| 10a-21 | Cl | NO₂ | Me | H | |
| 10a-22 | Cl | SO₂Me | Me | H | |
| 10a-23 | OMe | Me | Me | H | |
| 10a-24 | OMe | F | Me | H | |
| 10a-25 | OMe | Cl | Me | H | |
| 10a-26 | OMe | Br | Me | H | |
| 10a-27 | OMe | I | Me | H | |
| 10a-28 | OMe | CF₃ | Me | H | |
| 10a-29 | OMe | CHF₂ | Me | H | |
| 10a-30 | OMe | CF₂Cl | Me | H | |
| 10a-31 | OMe | OMe | Me | H | |
| 10a-32 | OMe | NO₂ | Me | H | |
| 10a-33 | OMe | SO₂Me | Me | H | |
| 10a-34 | SO₂Me | Me | Me | H | |
| 10a-35 | SO₂Me | F | Me | H | |
| 10a-36 | SO₂Me | Cl | Me | H | |
| 10a-37 | SO₂Me | Br | Me | H | |
| 10a-38 | SO₂Me | I | Me | H | |
| 10a-39 | SO₂Me | CF₃ | Me | H | |
| 10a-40 | SO₂Me | CHF₂ | Me | H | |
| 10a-41 | SO₂Me | CF₂Cl | Me | H | |
| 10a-42 | SO₂Me | OMe | Me | H | |
| 10a-43 | SO₂Me | NO₂ | Me | H | |
| 10a-44 | SO₂Me | SO₂Me | Me | H | |
| 10a-45 | Me | Me | Et | H | |
| 10a-46 | Me | F | Et | H | |
| 10a-47 | Me | Cl | Et | H | |
| 10a-48 | Me | Br | Et | H | |
| 10a-49 | Me | I | Et | H | |
| 10a-50 | Me | CF₃ | Et | H | |
| 10a-51 | Me | CHF₂ | Et | H | |
| 10a-52 | Me | CF₂Cl | Et | H | |
| 10a-53 | Me | OMe | Et | H | |
| 10a-54 | Me | NO₂ | Et | H | |
| 10a-55 | Me | SO₂Me | Et | H | |
| 10a-56 | Cl | Me | Et | H | |
| 10a-57 | Cl | F | Et | H | |
| 10a-58 | Cl | Cl | Et | H | |
| 10a-59 | Cl | Br | Et | H | |
| 10a-60 | Cl | I | Et | H | |
| 10a-61 | Cl | CF₃ | Et | H | |
| 10a-62 | Cl | CHF₂ | Et | H | |
| 10a-63 | Cl | CF₂Cl | Et | H | |
| 10a-64 | Cl | OMe | Et | H | |
| 10a-65 | Cl | NO₂ | Et | H | |
| 10a-66 | Cl | SO₂Me | Et | H | |
| 10a-67 | OMe | Me | Et | H | |
| 10a-68 | OMe | F | Et | H | |
| 10a-69 | OMe | Cl | Et | H | |
| 10a-70 | OMe | Br | Et | H | |
| 10a-71 | OMe | I | Et | H | |
| 10a-72 | OMe | CF₃ | Et | H | |
| 10a-73 | OMe | CHF₂ | Et | H | |
| 10a-74 | OMe | CF₂Cl | Et | H | |
| 10a-75 | OMe | OMe | Et | H | |
| 10a-76 | OMe | NO₂ | Et | H | |
| 10a-77 | OMe | SO₂Me | Et | H | |
| 10a-78 | SO₂Me | Me | Et | H | |
| 10a-79 | SO₂Me | F | Et | H | |
| 10a-80 | SO₂Me | Cl | Et | H | |
| 10a-81 | SO₂Me | Br | Et | H | |
| 10a-82 | SO₂Me | I | Et | H | |
| 10a-83 | SO₂Me | CF₃ | Et | H | |
| 10a-84 | SO₂Me | CHF₂ | Et | H | |
| 10a-85 | SO₂Me | CF₂Cl | Et | H | |
| 10a-86 | SO₂Me | OMe | Et | H | |
| 10a-87 | SO₂Me | NO₂ | Et | H | |
| 10a-88 | SO₂Me | SO₂Me | Et | H | |
| 10a-89 | Me | Me | CH₂CH₂OMe | H | |
| 10a-90 | Me | F | CH₂CH₂OMe | H | |
| 10a-91 | Me | Cl | CH₂CH₂OMe | H | |
| 10a-92 | Me | Br | CH₂CH₂OMe | H | |
| 10a-93 | Me | I | CH₂CH₂OMe | H | |
| 10a-94 | Me | CF₃ | CH₂CH₂OMe | H | |
| 10a-95 | Me | CHF₂ | CH₂CH₂OMe | H | |
| 10a-96 | Me | CF₂Cl | CH₂CH₂OMe | H | |
| 10a-97 | Me | OMe | CH₂CH₂OMe | H | |
| 10a-98 | Me | NO₂ | CH₂CH₂OMe | H | |
| 10a-99 | Me | SO₂Me | CH₂CH₂OMe | H | |
| 10a-100 | Cl | Me | CH₂CH₂OMe | H | |
| 10a-101 | Cl | F | CH₂CH₂OMe | H | |
| 10a-102 | Cl | Cl | CH₂CH₂OMe | H | |
| 10a-103 | Cl | Br | CH₂CH₂OMe | H | |
| 10a-104 | Cl | I | CH₂CH₂OMe | H | |
| 10a-105 | Cl | CF₃ | CH₂CH₂OMe | H | |
| 10a-106 | Cl | CHF₂ | CH₂CH₂OMe | H | |
| 10a-107 | Cl | CF₂Cl | CH₂CH₂OMe | H | |
| 10a-108 | Cl | OMe | CH₂CH₂OMe | H | |
| 10a-109 | Cl | NO₂ | CH₂CH₂OMe | H | |
| 10a-110 | Cl | SO₂Me | CH₂CH₂OMe | H | |
| 10a-111 | OMe | Me | CH₂CH₂OMe | H | |
| 10a-112 | OMe | F | CH₂CH₂OMe | H | |
| 10a-113 | OMe | Cl | CH₂CH₂OMe | H | |
| 10a-114 | OMe | Br | CH₂CH₂OMe | H | |
| 10a-115 | OMe | I | CH₂CH₂OMe | H | |
| 10a-116 | OMe | CF₃ | CH₂CH₂OMe | H | |
| 10a-117 | OMe | CHF₂ | CH₂CH₂OMe | H | |
| 10a-118 | OMe | CF₂Cl | CH₂CH₂OMe | H | |
| 10a-119 | OMe | OMe | CH₂CH₂OMe | H | |
| 10a-120 | OMe | NO₂ | CH₂CH₂OMe | H | |
| 10a-121 | OMe | SO₂Me | CH₂CH₂OMe | H | |
| 10a-122 | SO₂Me | Me | CH₂CH₂OMe | H | |
| 10a-123 | SO₂Me | F | CH₂CH₂OMe | H | |
| 10a-124 | SO₂Me | Cl | CH₂CH₂OMe | H | |
| 10a-125 | SO₂Me | Br | CH₂CH₂OMe | H | |
| 10a-126 | SO₂Me | I | CH₂CH₂OMe | H | |
| 10a-127 | SO₂Me | CF₃ | CH₂CH₂OMe | H | |
| 10a-128 | SO₂Me | CHF₂ | CH₂CH₂OMe | H | |
| 10a-129 | SO₂Me | CF₂Cl | CH₂CH₂OMe | H | |
| 10a-130 | SO₂Me | OMe | CH₂CH₂OMe | H | |
| 10a-131 | SO₂Me | NO₂ | CH₂CH₂OMe | H | |
| 10a-132 | SO₂Me | SO₂Me | CH₂CH₂OMe | H | |
| 10a-133 | Me | Me | Me | CN | |
| 10a-134 | Me | F | Me | CN | |
| 10a-135 | Me | Cl | Me | CN | |
| 10a-136 | Me | Br | Me | CN | |
| 10a-137 | Me | I | Me | CN | |
| 10a-138 | Me | CF₃ | Me | CN | |
| 10a-139 | Me | CHF₂ | Me | CN | |
| 10a-140 | Me | CF₂Cl | Me | CN | |
| 10a-141 | Me | OMe | Me | CN | |
| 10a-142 | Me | NO₂ | Me | CN | |
| 10a-143 | Me | SO₂Me | Me | CN | |
| 10a-144 | Cl | Me | Me | CN | |
| 10a-145 | Cl | F | Me | CN | |
| 10a-146 | Cl | Cl | Me | CN | |
| 10a-147 | Cl | Br | Me | CN | |
| 10a-148 | Cl | I | Me | CN | |
| 10a-149 | Cl | CF₃ | Me | CN | |
| 10a-150 | Cl | CHF₂ | Me | CN | |
| 10a-151 | Cl | CF₂Cl | Me | CN | |
| 10a-152 | Cl | OMe | Me | CN | |
| 10a-153 | Cl | NO₂ | Me | CN | |
| 10a-154 | Cl | SO₂Me | Me | CN | |
| 10a-155 | OMe | Me | Me | CN | |
| 10a-156 | OMe | F | Me | CN | |
| 10a-157 | OMe | Cl | Me | CN | |
| 10a-158 | OMe | Br | Me | CN | |
| 10a-159 | OMe | I | Me | CN | |
| 10a-160 | OMe | CF₃ | Me | CN | (400 MHz, CDCl₃ δ, ppm) 7.82 (d,1H), 7.61 (d,1H), 7.48 (s,1H), 4.12 (q,2H), 4.06 (s,3H), 3.36 (s,3H), 1.48 (t,3H) |
| 10a-161 | OMe | CHF₂ | Me | CN | |
| 10a-162 | OMe | CF₂Cl | Me | CN | |
| 10a-163 | OMe | OMe | Me | CN | |
| 10a-164 | OMe | NO₂ | Me | CN | |
| 10a-165 | OMe | SO₂Me | Me | CN | |
| 10a-166 | SO₂Me | Me | Me | CN | |
| 10a-167 | SO₂Me | F | Me | CN | |
| 10a-168 | SO₂Me | Cl | Me | CN | |
| 10a-169 | SO₂Me | Br | Me | CN | |
| 10a-170 | SO₂Me | I | Me | CN | |
| 10a-171 | SO₂Me | CF₃ | Me | CN | |
| 10a-172 | SO₂Me | CHF₂ | Me | CN | |
| 10a-173 | SO₂Me | CF₂Cl | Me | CN | |
| 10a-174 | SO₂Me | OMe | Me | CN | |
| 10a-175 | SO₂Me | NO₂ | Me | CN | |
| 10a-176 | SO₂Me | SO₂Me | Me | CN | |
| 10a-177 | Me | Me | Et | CN | |
| 10a-178 | Me | F | Et | CN | |
| 10a-179 | Me | Cl | Et | CN | |
| 10a-180 | Me | Br | Et | CN | |
| 10a-181 | Me | I | Et | CN | |
| 10a-182 | Me | CF₃ | Et | CN | |
| 10a-183 | Me | CHF₂ | Et | CN | |
| 10a-184 | Me | CF₂Cl | Et | CN | |
| 10a-185 | Me | OMe | Et | CN | |
| 10a-186 | Me | NO₂ | Et | CN | |
| 10a-187 | Me | SO₂Me | Et | CN | |
| 10a-188 | Cl | Me | Et | CN | |
| 10a-189 | Cl | F | Et | CN | |
| 10a-190 | Cl | Cl | Et | CN | |
| 10a-191 | Cl | Br | Et | CN | |
| 10a-192 | Cl | I | Et | CN | |
| 10a-193 | Cl | CF₃ | Et | CN | |
| 10a-194 | Cl | CHF₂ | Et | CN | |
| 10a-195 | Cl | CF₂Cl | Et | CN | |
| 10a-196 | Cl | OMe | Et | CN | |
| 10a-197 | Cl | NO₂ | Et | CN | |
| 10a-198 | Cl | SO₂Me | Et | CN | |
| 10a-199 | OMe | Me | Et | CN | |
| 10a-200 | OMe | F | Et | CN | |
| 10a-201 | OMe | Cl | Et | CN | |
| 10a-202 | OMe | Br | Et | CN | |
| 10a-203 | OMe | I | Et | CN | |
| 10a-204 | OMe | CF₃ | Et | CN | |
| 10a-205 | OMe | CHF₂ | Et | CN | |
| 10a-206 | OMe | CF₂Cl | Et | CN | |
| 10a-207 | OMe | OMe | Et | CN | |
| 10a-208 | OMe | NO₂ | Et | CN | |
| 10a-209 | OMe | SO₂Me | Et | CN | |
| 10a-210 | SO₂Me | Me | Et | CN | |
| 10a-211 | SO₂Me | F | Et | CN | |
| 10a-212 | SO₂Me | Cl | Et | CN | |
| 10a-213 | SO₂Me | Br | Et | CN | |
| 10a-214 | SO₂Me | I | Et | CN | |
| 10a-215 | SO₂Me | CF₃ | Et | CN | |
| 10a-216 | SO₂Me | CHF₂ | Et | CN | |
| 10a-217 | SO₂Me | CF₂Cl | Et | CN | |
| 10a-218 | SO₂Me | OMe | Et | CN | |
| 10a-219 | SO₂Me | NO₂ | Et | CN | |
| 10a-220 | SO₂Me | SO₂Me | Et | CN | |
| 10a-221 | Me | Me | CH₂CH₂OMe | CN | |
| 10a-222 | Me | F | CH₂CH₂OMe | CN | |
| 10a-223 | Me | Cl | CH₂CH₂OMe | CN | |
| 10a-224 | Me | Br | CH₂CH₂OMe | CN | |
| 10a-225 | Me | I | CH₂CH₂OMe | CN | |
| 10a-226 | Me | CF₃ | CH₂CH₂OMe | CN | |
| 10a-227 | Me | CHF₂ | CH₂CH₂OMe | CN | |
| 10a-228 | Me | CF₂Cl | CH₂CH₂OMe | CN | |
| 10a-229 | Me | OMe | CH₂CH₂OMe | CN | |
| 10a-230 | Me | NO₂ | CH₂CH₂OMe | CN | |
| 10a-231 | Me | SO₂Me | CH₂CH₂OMe | CN | |
| 10a-232 | Cl | Me | CH₂CH₂OMe | CN | |
| 10a-233 | Cl | F | CH₂CH₂OMe | CN | |
| 10a-234 | Cl | Cl | CH₂CH₂OMe | CN | |
| 10a-235 | Cl | Br | CH₂CH₂OMe | CN | |
| 10a-236 | Cl | I | CH₂CH₂OMe | CN | |
| 10a-237 | Cl | CF₃ | CH₂CH₂OMe | CN | |
| 10a-238 | Cl | CHF₂ | CH₂CH₂OMe | CN | |
| 10a-239 | Cl | CF₂Cl | CH₂CH₂OMe | CN | |
| 10a-240 | Cl | OMe | CH₂CH₂OMe | CN | |
| 10a-241 | Cl | NO₂ | CH₂CH₂OMe | CN | |
| 10a-242 | Cl | SO₂Me | CH₂CH₂OMe | CN | |
| 10a-243 | OMe | Me | CH₂CH₂OMe | CN | |
| 10a-244 | OMe | F | CH₂CH₂OMe | CN | |
| 10a-245 | OMe | Cl | CH₂CH₂OMe | CN | |
| 10a-246 | OMe | Br | CH₂CH₂OMe | CN | |
| 10a-247 | OMe | I | CH₂CH₂OMe | CN | |
| 10a-248 | OMe | CF₃ | CH₂CH₂OMe | CN | |
| 10a-249 | OMe | CHF₂ | CH₂CH₂OMe | CN | |
| 10a-250 | OMe | CF₂Cl | CH₂CH₂OMe | CN | |
| 10a-251 | OMe | OMe | CH₂CH₂OMe | CN | |
| 10a-252 | OMe | NO₂ | CH₂CH₂OMe | CN | |
| 10a-253 | OMe | SO₂Me | CH₂CH₂OMe | CN | |
| 10a-254 | SO₂Me | Me | CH₂CH₂OMe | CN | |
| 10a-255 | SO₂Me | F | CH₂CH₂OMe | CN | |
| 10a-256 | SO₂Me | Cl | CH₂CH₂OMe | CN | |
| 10a-257 | SO₂Me | Br | CH₂CH₂OMe | CN | |
| 10a-258 | SO₂Me | I | CH₂CH₂OMe | CN | |
| 10a-259 | SO₂Me | CF₃ | CH₂CH₂OMe | CN | |
| 10a-260 | SO₂Me | CHF₂ | CH₂CH₂OMe | CN | |
| 10a-261 | SO₂Me | CF₂Cl | CH₂CH₂OMe | CN | |
| 10a-262 | SO₂Me | OMe | CH₂CH₂OMe | CN | |
| 10a-263 | SO₂Me | NO₂ | CH₂CH₂OMe | CN | |
| 10a-264 | SO₂Me | SO₂Me | CH₂CH₂OMe | CN | |

**Tabelle 11: Erfindungsgemäße Verbindungen der allgemeinen Formel (I), worin Q für Q3, Rⁱ und R^{k} jeweils für Methyl, R^{h} sowie W jeweils für Wasserstoff stehen, t = 0 ist und die anderen Reste die in der Tabelle angegebenen Bedeutungen haben**

| | | | | | |
|---|---|---|---|---|---|
| 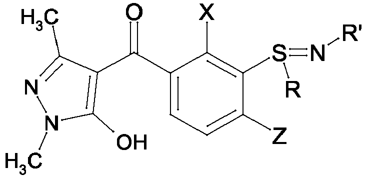 | | | | | |

| Nr. | X | Z | R | R' | Physikalische Daten (1H-NMR) |
|---|---|---|---|---|---|
| 11-1 | Me | Me | Me | H | |
| 11-2 | Me | F | Me | H | |
| 11-3 | Me | Cl | Me | H | |
| 11-4 | Me | Br | Me | H | |
| 11-5 | Me | I | Me | H | |
| 11-6 | Me | CF₃ | Me | H | |
| 11-7 | Me | CHF₂ | Me | H | |
| 11-8 | Me | CF₂Cl | Me | H | |
| 11-9 | Me | OMe | Me | H | |
| 11-10 | Me | NO₂ | Me | H | |
| 11-11 | Me | SO₂Me | Me | H | |
| 11-12 | Cl | Me | Me | H | |
| 11-13 | Cl | F | Me | H | |
| 11-14 | Cl | Cl | Me | H | |
| 11-15 | Cl | Br | Me | H | |
| 11-16 | Cl | I | Me | H | |
| 11-17 | Cl | CF₃ | Me | H | |
| 11-18 | Cl | CHF₂ | Me | H | |
| 11-19 | Cl | CF₂Cl | Me | H | |
| 11-20 | Cl | OMe | Me | H | |
| 11-21 | Cl | NO₂ | Me | H | |
| 11-22 | Cl | SO₂Me | Me | H | |
| 11-23 | OMe | Me | Me | H | |
| 11-24 | OMe | F | Me | H | |
| 11-25 | OMe | Cl | Me | H | |
| 11-26 | OMe | Br | Me | H | |
| 11-27 | OMe | I | Me | H | |
| 11-28 | OMe | CF₃ | Me | H | |
| 11-29 | OMe | CHF₂ | Me | H | |
| 11-30 | OMe | CF₂Cl | Me | H | |
| 11-31 | OMe | OMe | Me | H | |
| 11-32 | OMe | NO₂ | Me | H | |
| 11-33 | OMe | SO₂Me | Me | H | |
| 11-34 | SO₂Me | Me | Me | H | |
| 11-35 | SO₂Me | F | Me | H | |
| 11-36 | SO₂Me | Cl | Me | H | |
| 11-37 | SO₂Me | Br | Me | H | |
| 11-38 | SO₂Me | I | Me | H | |
| 11-39 | SO₂Me | CF₃ | Me | H | |
| 11-40 | SO₂Me | CHF₂ | Me | H | |
| 11-41 | SO₂Me | CF₂Cl | Me | H | |
| 11-42 | SO₂Me | OMe | Me | H | |
| 11-43 | SO₂Me | NO₂ | Me | H | |
| 11-44 | SO₂Me | SO₂Me | Me | H | |
| 11-45 | Me | Me | Et | H | |
| 11-46 | Me | F | Et | H | |
| 11-47 | Me | Cl | Et | H | |
| 11-48 | Me | Br | Et | H | |
| 11-49 | Me | I | Et | H | |
| 11-50 | Me | CF₃ | Et | H | |
| 11-51 | Me | CHF₂ | Et | H | |
| 11-52 | Me | CF₂Cl | Et | H | |
| 11-53 | Me | OMe | Et | H | |
| 11-54 | Me | NO₂ | Et | H | |
| 11-55 | Me | SO₂Me | Et | H | |
| 11-56 | Cl | Me | Et | H | |
| 11-57 | Cl | F | Et | H | |
| 11-58 | Cl | Cl | Et | H | |
| 11-59 | Cl | Br | Et | H | |
| 11-60 | Cl | I | Et | H | |
| 11-61 | Cl | CF₃ | Et | H | |
| 11-62 | Cl | CHF₂ | Et | H | |
| 11-63 | Cl | CF₂Cl | Et | H | |
| 11-64 | Cl | OMe | Et | H | |
| 11-65 | Cl | NO₂ | Et | H | |
| 11-66 | Cl | SO₂Me | Et | H | |
| 11-67 | OMe | Me | Et | H | |
| 11-68 | OMe | F | Et | H | |
| 11-69 | OMe | Cl | Et | H | |
| 11-70 | OMe | Br | Et | H | |
| 11-71 | OMe | I | Et | H | |
| 11-72 | OMe | CF₃ | Et | H | |
| 11-73 | OMe | CHF₂ | Et | H | |
| 11-74 | OMe | CF₂Cl | Et | H | |
| 11-75 | OMe | OMe | Et | H | |
| 11-76 | OMe | NO₂ | Et | H | |
| 11-77 | OMe | SO₂Me | Et | H | |
| 11-78 | SO₂Me | Me | Et | H | |
| 11-79 | SO₂Me | F | Et | H | |
| 11-80 | SO₂Me | Cl | Et | H | |
| 11-81 | SO₂Me | Br | Et | H | |
| 11-82 | SO₂Me | I | Et | H | |
| 11-83 | SO₂Me | CF₃ | Et | H | |
| 11-84 | SO₂Me | CHF₂ | Et | H | |
| 11-85 | SO₂Me | CF₂Cl | Et | H | |
| 11-86 | SO₂Me | OMe | Et | H | |
| 11-87 | SO₂Me | NO₂ | Et | H | |
| 11-88 | SO₂Me | SO₂Me | Et | H | |
| 11-89 | Me | Me | CH₂CH₂OMe | H | |
| 11-90 | Me | F | CH₂CH₂OMe | H | |
| 11-91 | Me | Cl | CH₂CH₂OMe | H | |
| 11-92 | Me | Br | CH₂CH₂OMe | H | |
| 11-93 | Me | I | CH₂CH₂OMe | H | |
| 11-94 | Me | CF₃ | CH₂CH₂OMe | H | |
| 11-95 | Me | CHF₂ | CH₂CH₂OMe | H | |
| 11-96 | Me | CF₂Cl | CH₂CH₂OMe | H | |
| 11-97 | Me | OMe | CH₂CH₂OMe | H | |
| 11-98 | Me | NO₂ | CH₂CH₂OMe | H | |
| 11-99 | Me | SO₂Me | CH₂CH₂OMe | H | |
| 11-100 | Cl | Me | CH₂CH₂OMe | H | |
| 11-101 | Cl | F | CH₂CH₂OMe | H | |
| 11-102 | Cl | Cl | CH₂CH₂OMe | H | |
| 11-103 | Cl | Br | CH₂CH₂OMe | H | |
| 11-104 | Cl | I | CH₂CH₂OMe | H | |
| 11-105 | Cl | CF₃ | CH₂CH₂OMe | H | |
| 11-106 | Cl | CHF₂ | CH₂CH₂OMe | H | |
| 11-107 | Cl | CF₂Cl | CH₂CH₂OMe | H | |
| 11-108 | Cl | OMe | CH₂CH₂OMe | H | |
| 11-109 | Cl | NO₂ | CH₂CH₂OMe | H | |
| 11-110 | Cl | SO₂Me | CH₂CH₂OMe | H | |
| 11-111 | OMe | Me | CH₂CH₂OMe | H | |
| 11-112 | OMe | F | CH₂CH₂OMe | H | |
| 11-113 | OMe | Cl | CH₂CH₂OMe | H | |
| 11-114 | OMe | Br | CH₂CH₂OMe | H | |
| 11-115 | OMe | I | CH₂CH₂OMe | H | |
| 11-116 | OMe | CF₃ | CH₂CH₂OMe | H | |
| 11-117 | OMe | CHF₂ | CH₂CH₂OMe | H | |
| 11-118 | OMe | CF₂Cl | CH₂CH₂OMe | H | |
| 11-119 | OMe | OMe | CH₂CH₂OMe | H | |
| 11-120 | OMe | NO₂ | CH₂CH₂OMe | H | |
| 11-121 | OMe | SO₂Me | CH₂CH₂OMe | H | |
| 11-122 | SO₂Me | Me | CH₂CH₂OMe | H | |
| 11-123 | SO₂Me | F | CH₂CH₂OMe | H | |
| 11-124 | SO₂Me | Cl | CH₂CH₂OMe | H | |
| 11-125 | SO₂Me | Br | CH₂CH₂OMe | H | |
| 11-126 | SO₂Me | I | CH₂CH₂OMe | H | |
| 11-127 | SO₂Me | CF₃ | CH₂CH₂OMe | H | |
| 11-128 | SO₂Me | CHF₂ | CH₂CH₂OMe | H | |
| 11-129 | SO₂Me | CF₂Cl | CH₂CH₂OMe | H | |
| 11-130 | SO₂Me | OMe | CH₂CH₂OMe | H | |
| 11-131 | SO₂Me | NO₂ | CH₂CH₂OMe | H | |
| 11-132 | SO₂Me | SO₂Me | CH₂CH₂OMe | H | |
| 11-133 | Me | Me | Me | CN | |
| 11-134 | Me | F | Me | CN | |
| 11-135 | Me | Cl | Me | CN | |
| 11-136 | Me | Br | Me | CN | |
| 11-137 | Me | I | Me | CN | |
| 11-138 | Me | CF₃ | Me | CN | |
| 11-139 | Me | CHF₂ | Me | CN | |
| 11-140 | Me | CF₂Cl | Me | CN | |
| 11-141 | Me | OMe | Me | CN | |
| 11-142 | Me | NO₂ | Me | CN | |
| 11-143 | Me | SO₂Me | Me | CN | |
| 11-144 | Cl | Me | Me | CN | |
| 11-145 | Cl | F | Me | CN | |
| 11-146 | Cl | Cl | Me | CN | |
| 11-147 | Cl | Br | Me | CN | |
| 11-148 | Cl | I | Me | CN | |
| 11-149 | Cl | CF₃ | Me | CN | |
| 11-150 | Cl | CHF₂ | Me | CN | |
| 11-151 | Cl | CF₂Cl | Me | CN | |
| 11-152 | Cl | OMe | Me | CN | |
| 11-153 | Cl | NO₂ | Me | CN | |
| 11-154 | Cl | SO₂Me | Me | CN | |
| 11-155 | OMe | Me | Me | CN | |
| 11-156 | OMe | F | Me | CN | |
| 11-157 | OMe | Cl | Me | CN | |
| 11-158 | OMe | Br | Me | CN | |
| 11-159 | OMe | I | Me | CN | |
| 11-160 | OMe | CF₃ | Me | CN | |
| 11-161 | OMe | CHF₂ | Me | CN | |
| 11-162 | OMe | CF₂Cl | Me | CN | |
| 11-163 | OMe | OMe | Me | CN | |
| 11-164 | OMe | NO₂ | Me | CN | |
| 11-165 | OMe | SO₂Me | Me | CN | |
| 11-166 | SO₂Me | Me | Me | CN | |
| 11-167 | SO₂Me | F | Me | CN | |
| 11-168 | SO₂Me | Cl | Me | CN | |
| 11-169 | SO₂Me | Br | Me | CN | |
| 11-170 | SO₂Me | I | Me | CN | |
| 11-171 | SO₂Me | CF₃ | Me | CN | |
| 11-172 | SO₂Me | CHF₂ | Me | CN | |
| 11-173 | SO₂Me | CF₂Cl | Me | CN | |
| 11-174 | SO₂Me | OMe | Me | CN | |
| 11-175 | SO₂Me | NO₂ | Me | CN | |
| 11-176 | SO₂Me | SO₂Me | Me | CN | |
| 11-177 | Me | Me | Et | CN | |
| 11-178 | Me | F | Et | CN | |
| 11-179 | Me | Cl | Et | CN | |
| 11-180 | Me | Br | Et | CN | |
| 11-181 | Me | I | Et | CN | |
| 11-182 | Me | CF₃ | Et | CN | |
| 11-183 | Me | CHF₂ | Et | CN | |
| 11-184 | Me | CF₂Cl | Et | CN | |
| 11-185 | Me | OMe | Et | CN | |
| 11-186 | Me | NO₂ | Et | CN | |
| 11-187 | Me | SO₂Me | Et | CN | |
| 11-188 | Cl | Me | Et | CN | |
| 11-189 | Cl | F | Et | CN | |
| 11-190 | Cl | Cl | Et | CN | |
| 11-191 | Cl | Br | Et | CN | |
| 11-192 | Cl | I | Et | CN | |
| 11-193 | Cl | CF₃ | Et | CN | |
| 11-194 | Cl | CHF₂ | Et | CN | |
| 11-195 | Cl | CF₂Cl | Et | CN | |
| 11-196 | Cl | OMe | Et | CN | |
| 11-197 | Cl | NO₂ | Et | CN | |
| 11-198 | Cl | SO₂Me | Et | CN | |
| 11-199 | OMe | Me | Et | CN | |
| 11-200 | OMe | F | Et | CN | |
| 11-201 | OMe | Cl | Et | CN | |
| 11-202 | OMe | Br | Et | CN | |
| 11-203 | OMe | I | Et | CN | |
| 11-204 | OMe | CF₃ | Et | CN | |
| 11-205 | OMe | CHF₂ | Et | CN | |
| 11-206 | OMe | CF₂Cl | Et | CN | |
| 11-207 | OMe | OMe | Et | CN | |
| 11-208 | OMe | NO₂ | Et | CN | |
| 11-209 | OMe | SO₂Me | Et | CN | |
| 11-210 | SO₂Me | Me | Et | CN | |
| 11-211 | SO₂Me | F | Et | CN | |
| 11-212 | SO₂Me | Cl | Et | CN | |
| 11-213 | SO₂Me | Br | Et | CN | |
| 11-214 | SO₂Me | I | Et | CN | |
| 11-215 | SO₂Me | CF₃ | Et | CN | |
| 11-216 | SO₂Me | CHF₂ | Et | CN | |
| 11-217 | SO₂Me | CF₂Cl | Et | CN | |
| 11-218 | SO₂Me | OMe | Et | CN | |
| 11-219 | SO₂Me | NO₂ | Et | CN | |
| 11-220 | SO₂Me | SO₂Me | Et | CN | |
| 11-221 | Me | Me | CH₂CH₂OMe | CN | |
| 11-222 | Me | F | CH₂CH₂OMe | CN | |
| 11-223 | Me | Cl | CH₂CH₂OMe | CN | |
| 11-224 | Me | Br | CH₂CH₂OMe | CN | |
| 11-225 | Me | I | CH₂CH₂OMe | CN | |
| 11-226 | Me | CF₃ | CH₂CH₂OMe | CN | |
| 11-227 | Me | CHF₂ | CH₂CH₂OMe | CN | |
| 11-228 | Me | CF₂Cl | CH₂CH₂OMe | CN | |
| 11-229 | Me | OMe | CH₂CH₂OMe | CN | |
| 11-230 | Me | NO₂ | CH₂CH₂OMe | CN | |
| 11-231 | Me | SO₂Me | CH₂CH₂OMe | CN | |
| 11-232 | Cl | Me | CH₂CH₂OMe | CN | |
| 11-233 | Cl | F | CH₂CH₂OMe | CN | |
| 11-234 | Cl | Cl | CH₂CH₂OMe | CN | |
| 11-235 | Cl | Br | CH₂CH₂OMe | CN | |
| 11-236 | Cl | I | CH₂CH₂OMe | CN | |
| 11-237 | Cl | CF₃ | CH₂CH₂OMe | CN | |
| 11-238 | Cl | CHF₂ | CH₂CH₂OMe | CN | |
| 11-239 | Cl | CF₂Cl | CH₂CH₂OMe | CN | |
| 11-240 | Cl | OMe | CH₂CH₂OMe | CN | |
| 11-241 | Cl | NO₂ | CH₂CH₂OMe | CN | |
| 11-242 | Cl | SO₂Me | CH₂CH₂OMe | CN | |
| 11-243 | OMe | Me | CH₂CH₂OMe | CN | |
| 11-244 | OMe | F | CH₂CH₂OMe | CN | |
| 11-245 | OMe | Cl | CH₂CH₂OMe | CN | |
| 11-246 | OMe | Br | CH₂CH₂OMe | CN | |
| 11-247 | OMe | I | CH₂CH₂OMe | CN | |
| 11-248 | OMe | CF₃ | CH₂CH₂OMe | CN | |
| 11-249 | OMe | CHF₂ | CH₂CH₂OMe | CN | |
| 11-250 | OMe | CF₂Cl | CH₂CH₂OMe | CN | |
| 11-251 | OMe | OMe | CH₂CH₂OMe | CN | |
| 11-252 | OMe | NO₂ | CH₂CH₂OMe | CN | |
| 11-253 | OMe | SO₂Me | CH₂CH₂OMe | CN | |
| 11-254 | SO₂Me | Me | CH₂CH₂OMe | CN | |
| 11-255 | SO₂Me | F | CH₂CH₂OMe | CN | |
| 11-256 | SO₂Me | Cl | CH₂CH₂OMe | CN | |
| 11-257 | SO₂Me | Br | CH₂CH₂OMe | CN | |
| 11-258 | SO₂Me | I | CH₂CH₂OMe | CN | |
| 11-259 | SO₂Me | CF₃ | CH₂CH₂OMe | CN | |
| 11-260 | SO₂Me | CHF₂ | CH₂CH₂OMe | CN | |
| 11-261 | SO₂Me | CF₂Cl | CH₂CH₂OMe | CN | |
| 11-262 | SO₂Me | OMe | CH₂CH₂OMe | CN | |
| 11-263 | SO₂Me | NO₂ | CH₂CH₂OMe | CN | |
| 11-264 | SO₂Me | SO₂Me | CH₂CH₂OMe | CN | |

**Tabelle 12: Erfindungsgemäße Verbindungen der allgemeinen Formel (I), worin Q für Q4 steht, W Wasserstoff bedeutet, t = 0 ist und die anderen Reste die in der Tabelle angegebenen Bedeutungen haben**

| | | | | | |
|---|---|---|---|---|---|
| 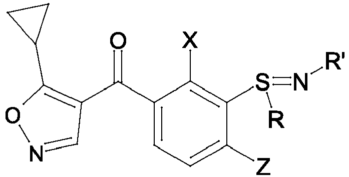 | | | | | |

| Nr. | X | Z | R | R' | Physikalische Daten (1H-NMR) |
|---|---|---|---|---|---|
| 12-1 | Me | Me | Me | H | |
| 12-2 | Me | F | Me | H | |
| 12-3 | Me | Cl | Me | H | |
| 12-4 | Me | Br | Me | H | |
| 12-5 | Me | I | Me | H | |
| 12-6 | Me | CF₃ | Me | H | |
| 12-7 | Me | CHF₂ | Me | H | |
| 12-8 | Me | CF₂Cl | Me | H | |
| 12-9 | Me | OMe | Me | H | |
| 12-10 | Me | NO₂ | Me | H | |
| 12-11 | Me | SO₂Me | Me | H | |
| 12-12 | Cl | Me | Me | H | |
| 12-13 | Cl | F | Me | H | |
| 12-14 | Cl | Cl | Me | H | |
| 12-15 | Cl | Br | Me | H | |
| 12-16 | Cl | I | Me | H | |
| 12-17 | Cl | CF₃ | Me | H | |
| 12-18 | Cl | CHF₂ | Me | H | |
| 12-19 | Cl | CF₂Cl | Me | H | |
| 12-20 | Cl | OMe | Me | H | |
| 12-21 | Cl | NO₂ | Me | H | |
| 12-22 | Cl | SO₂Me | Me | H | |
| 12-23 | OMe | Me | Me | H | |
| 12-24 | OMe | F | Me | H | |
| 12-25 | OMe | Cl | Me | H | |
| 12-26 | OMe | Br | Me | H | |
| 12-27 | OMe | I | Me | H | |
| 12-28 | OMe | CF₃ | Me | H | |
| 12-29 | OMe | CHF₂ | Me | H | |
| 12-30 | OMe | CF₂Cl | Me | H | |
| 12-31 | OMe | OMe | Me | H | |
| 12-32 | OMe | NO₂ | Me | H | |
| 12-33 | OMe | SO₂Me | Me | H | |
| 12-34 | SO₂Me | Me | Me | H | |
| 12-35 | SO₂Me | F | Me | H | |
| 12-36 | SO₂Me | Cl | Me | H | |
| 12-37 | SO₂Me | Br | Me | H | |
| 12-38 | SO₂Me | I | Me | H | |
| 12-39 | SO₂Me | CF₃ | Me | H | |
| 12-40 | SO₂Me | CHF₂ | Me | H | |
| 12-41 | SO₂Me | CF₂Cl | Me | H | |
| 12-42 | SO₂Me | OMe | Me | H | |
| 12-43 | SO₂Me | NO₂ | Me | H | |
| 12-44 | SO₂Me | SO₂Me | Me | H | |
| 12-45 | Me | Me | Et | H | |
| 12-46 | Me | F | Et | H | |
| 12-47 | Me | Cl | Et | H | |
| 12-48 | Me | Br | Et | H | |
| 12-49 | Me | I | Et | H | |
| 12-50 | Me | CF₃ | Et | H | |
| 12-51 | Me | CHF₂ | Et | H | |
| 12-52 | Me | CF₂Cl | Et | H | |
| 12-53 | Me | OMe | Et | H | |
| 12-54 | Me | NO₂ | Et | H | |
| 12-55 | Me | SO₂Me | Et | H | |
| 12-56 | Cl | Me | Et | H | |
| 12-57 | Cl | F | Et | H | |
| 12-58 | Cl | Cl | Et | H | |
| 12-59 | Cl | Br | Et | H | |
| 12-60 | Cl | I | Et | H | |
| 12-61 | Cl | CF₃ | Et | H | |
| 12-62 | Cl | CHF₂ | Et | H | |
| 12-63 | Cl | CF₂Cl | Et | H | |
| 12-64 | Cl | OMe | Et | H | |
| 12-65 | Cl | NO₂ | Et | H | |
| 12-66 | Cl | SO₂Me | Et | H | |
| 12-67 | OMe | Me | Et | H | |
| 12-68 | OMe | F | Et | H | |
| 12-69 | OMe | Cl | Et | H | |
| 12-70 | OMe | Br | Et | H | |
| 12-71 | OMe | I | Et | H | |
| 12-72 | OMe | CF₃ | Et | H | |
| 12-73 | OMe | CHF₂ | Et | H | |
| 12-74 | OMe | CF₂Cl | Et | H | |
| 12-75 | OMe | OMe | Et | H | |
| 12-76 | OMe | NO₂ | Et | H | |
| 12-77 | OMe | SO₂Me | Et | H | |
| 12-78 | SO₂Me | Me | Et | H | |
| 12-79 | SO₂Me | F | Et | H | |
| 12-80 | SO₂Me | Cl | Et | H | |
| 12-81 | SO₂Me | Br | Et | H | |
| 12-82 | SO₂Me | I | Et | H | |
| 12-83 | SO₂Me | CF₃ | Et | H | |
| 12-84 | SO₂Me | CHF₂ | Et | H | |
| 12-85 | SO₂Me | CF₂Cl | Et | H | |
| 12-86 | SO₂Me | OMe | Et | H | |
| 12-87 | SO₂Me | NO₂ | Et | H | |
| 12-88 | SO₂Me | SO₂Me | Et | H | |
| 12-89 | Me | Me | CH₂CH₂OMe | H | |
| 12-90 | Me | F | CH₂CH₂OMe | H | |
| 12-91 | Me | Cl | CH₂CH₂OMe | H | |
| 12-92 | Me | Br | CH₂CH₂OMe | H | |
| 12-93 | Me | I | CH₂CH₂OMe | H | |
| 12-94 | Me | CF₃ | CH₂CH₂OMe | H | |
| 12-95 | Me | CHF₂ | CH₂CH₂OMe | H | |
| 12-96 | Me | CF₂Cl | CH₂CH₂OMe | H | |
| 12-97 | Me | OMe | CH₂CH₂OMe | H | |
| 12-98 | Me | NO₂ | CH₂CH₂OMe | H | |
| 12-99 | Me | SO₂Me | CH₂CH₂OMe | H | |
| 12-100 | Cl | Me | CH₂CH₂OMe | H | |
| 12-101 | Cl | F | CH₂CH₂OMe | H | |
| 12-102 | Cl | Cl | CH₂CH₂OMe | H | |
| 12-103 | Cl | Br | CH₂CH₂OMe | H | |
| 12-104 | Cl | I | CH₂CH₂OMe | H | |
| 12-105 | Cl | CF₃ | CH₂CH₂OMe | H | |
| 12-106 | Cl | CHF₂ | CH₂CH₂OMe | H | |
| 12-107 | Cl | CF₂Cl | CH₂CH₂OMe | H | |
| 12-108 | Cl | OMe | CH₂CH₂OMe | H | |
| 12-109 | Cl | NO₂ | CH₂CH₂OMe | H | |
| 12-110 | Cl | SO₂Me | CH₂CH₂OMe | H | |
| 12-111 | OMe | Me | CH₂CH₂OMe | H | |
| 12-112 | OMe | F | CH₂CH₂OMe | H | |
| 12-113 | OMe | Cl | CH₂CH₂OMe | H | |
| 12-114 | OMe | Br | CH₂CH₂OMe | H | |
| 12-115 | OMe | I | CH₂CH₂OMe | H | |
| 12-116 | OMe | CF₃ | CH₂CH₂OMe | H | |
| 12-117 | OMe | CHF₂ | CH₂CH₂OMe | H | |
| 12-118 | OMe | CF₂Cl | CH₂CH₂OMe | H | |
| 12-119 | OMe | OMe | CH₂CH₂OMe | H | |
| 12-120 | OMe | NO₂ | CH₂CH₂OMe | H | |
| 12-121 | OMe | SO₂Me | CH₂CH₂OMe | H | |
| 12-122 | SO₂Me | Me | CH₂CH₂OMe | H | |
| 12-123 | SO₂Me | F | CH₂CH₂OMe | H | |
| 12-124 | SO₂Me | Cl | CH₂CH₂OMe | H | |
| 12-125 | SO₂Me | Br | CH₂CH₂OMe | H | |
| 12-126 | SO₂Me | I | CH₂CH₂OMe | H | |
| 12-127 | SO₂Me | CF₃ | CH₂CH₂OMe | H | |
| 12-128 | SO₂Me | CHF₂ | CH₂CH₂OMe | H | |
| 12-129 | SO₂Me | CF₂Cl | CH₂CH₂OMe | H | |
| 12-130 | SO₂Me | OMe | CH₂CH₂OMe | H | |
| 12-131 | SO₂Me | NO₂ | CH₂CH₂OMe | H | |
| 12-132 | SO₂Me | SO₂Me | CH₂CH₂OMe | H | |
| 12-133 | Me | Me | Me | CN | |
| 12-134 | Me | F | Me | CN | |
| 12-135 | Me | Cl | Me | CN | |
| 12-136 | Me | Br | Me | CN | |
| 12-137 | Me | I | Me | CN | |
| 12-138 | Me | CF₃ | Me | CN | |
| 12-139 | Me | CHF₂ | Me | CN | |
| 12-140 | Me | CF₂Cl | Me | CN | |
| 12-141 | Me | OMe | Me | CN | |
| 12-142 | Me | NO₂ | Me | CN | |
| 12-143 | Me | SO₂Me | Me | CN | |
| 12-144 | Cl | Me | Me | CN | |
| 12-145 | Cl | F | Me | CN | |
| 12-146 | Cl | Cl | Me | CN | |
| 12-147 | Cl | Br | Me | CN | |
| 12-148 | Cl | I | Me | CN | |
| 12-149 | Cl | CF₃ | Me | CN | |
| 12-150 | Cl | CHF₂ | Me | CN | |
| 12-151 | Cl | CF₂Cl | Me | CN | |
| 12-152 | Cl | OMe | Me | CN | |
| 12-153 | Cl | NO₂ | Me | CN | |
| 12-154 | Cl | SO₂Me | Me | CN | |
| 12-155 | OMe | Me | Me | CN | |
| 12-156 | OMe | F | Me | CN | |
| 12-157 | OMe | Cl | Me | CN | |
| 12-158 | OMe | Br | Me | CN | |
| 12-159 | OMe | I | Me | CN | |
| 12-160 | OMe | CF₃ | Me | CN | |
| 12-161 | OMe | CHF₂ | Me | CN | |
| 12-162 | OMe | CF₂Cl | Me | CN | |
| 12-163 | OMe | OMe | Me | CN | |
| 12-164 | OMe | NO₂ | Me | CN | |
| 12-165 | OMe | SO₂Me | Me | CN | |
| 12-166 | SO₂Me | Me | Me | CN | |
| 12-167 | SO₂Me | F | Me | CN | |
| 12-168 | SO₂Me | Cl | Me | CN | |
| 12-169 | SO₂Me | Br | Me | CN | |
| 12-170 | SO₂Me | I | Me | CN | |
| 12-171 | SO₂Me | CF₃ | Me | CN | |
| 12-172 | SO₂Me | CHF₂ | Me | CN | |
| 12-173 | SO₂Me | CF₂Cl | Me | CN | |
| 12-174 | SO₂Me | OMe | Me | CN | |
| 12-175 | SO₂Me | NO₂ | Me | CN | |
| 12-176 | SO₂Me | SO₂Me | Me | CN | |
| 12-177 | Me | Me | Et | CN | |
| 12-178 | Me | F | Et | CN | |
| 12-179 | Me | Cl | Et | CN | |
| 12-180 | Me | Br | Et | CN | |
| 12-181 | Me | I | Et | CN | |
| 12-182 | Me | CF₃ | Et | CN | |
| 12-183 | Me | CHF₂ | Et | CN | |
| 12-184 | Me | CF₂Cl | Et | CN | |
| 12-185 | Me | OMe | Et | CN | |
| 12-186 | Me | NO₂ | Et | CN | |
| 12-187 | Me | SO₂Me | Et | CN | |
| 12-188 | Cl | Me | Et | CN | |
| 12-189 | Cl | F | Et | CN | |
| 12-190 | Cl | Cl | Et | CN | |
| 12-191 | Cl | Br | Et | CN | |
| 12-192 | Cl | I | Et | CN | |
| 12-193 | Cl | CF₃ | Et | CN | |
| 12-194 | Cl | CHF₂ | Et | CN | |
| 12-195 | Cl | CF₂Cl | Et | CN | |
| 12-196 | Cl | OMe | Et | CN | |
| 12-197 | Cl | NO₂ | Et | CN | |
| 12-198 | Cl | SO₂Me | Et | CN | |
| 12-199 | OMe | Me | Et | CN | |
| 12-200 | OMe | F | Et | CN | |
| 12-201 | OMe | Cl | Et | CN | |
| 12-202 | OMe | Br | Et | CN | |
| 12-203 | OMe | I | Et | CN | |
| 12-204 | OMe | CF₃ | Et | CN | |
| 12-205 | OMe | CHF₂ | Et | CN | |
| 12-206 | OMe | CF₂Cl | Et | CN | |
| 12-207 | OMe | OMe | Et | CN | |
| 12-208 | OMe | NO₂ | Et | CN | |
| 12-209 | OMe | SO₂Me | Et | CN | |
| 12-210 | SO₂Me | Me | Et | CN | |
| 12-211 | SO₂Me | F | Et | CN | |
| 12-212 | SO₂Me | Cl | Et | CN | |
| 12-213 | SO₂Me | Br | Et | CN | |
| 12-214 | SO₂Me | I | Et | CN | |
| 12-215 | SO₂Me | CF₃ | Et | CN | |
| 12-216 | SO₂Me | CHF₂ | Et | CN | |
| 12-217 | SO₂Me | CF₂Cl | Et | CN | |
| 12-218 | SO₂Me | OMe | Et | CN | |
| 12-219 | SO₂Me | NO₂ | Et | CN | |
| 12-220 | SO₂Me | SO₂Me | Et | CN | |
| 12-221 | Me | Me | CH₂CH₂OMe | CN | |
| 12-222 | Me | F | CH₂CH₂OMe | CN | |
| 12-223 | Me | Cl | CH₂CH₂OMe | CN | |
| 12-224 | Me | Br | CH₂CH₂OMe | CN | |
| 12-225 | Me | I | CH₂CH₂OMe | CN | |
| 12-226 | Me | CF₃ | CH₂CH₂OMe | CN | |
| 12-227 | Me | CHF₂ | CH₂CH₂OMe | CN | |
| 12-228 | Me | CF₂Cl | CH₂CH₂OMe | CN | |
| 12-229 | Me | OMe | CH₂CH₂OMe | CN | |
| 12-230 | Me | NO₂ | CH₂CH₂OMe | CN | |
| 12-231 | Me | SO₂Me | CH₂CH₂OMe | CN | |
| 12-232 | Cl | Me | CH₂CH₂OMe | CN | |
| 12-233 | Cl | F | CH₂CH₂OMe | CN | |
| 12-234 | Cl | Cl | CH₂CH₂OMe | CN | |
| 12-235 | Cl | Br | CH₂CH₂OMe | CN | |
| 12-236 | Cl | I | CH₂CH₂OMe | CN | |
| 12-237 | Cl | CF₃ | CH₂CH₂OMe | CN | |
| 12-238 | Cl | CHF₂ | CH₂CH₂OMe | CN | |
| 12-239 | Cl | CF₂Cl | CH₂CH₂OMe | CN | |
| 12-240 | Cl | OMe | CH₂CH₂OMe | CN | |
| 12-241 | Cl | NO₂ | CH₂CH₂OMe | CN | |
| 12-242 | Cl | SO₂Me | CH₂CH₂OMe | CN | |
| 12-243 | OMe | Me | CH₂CH₂OMe | CN | |
| 12-244 | OMe | F | CH₂CH₂OMe | CN | |
| 12-245 | OMe | Cl | CH₂CH₂OMe | CN | |
| 12-246 | OMe | Br | CH₂CH₂OMe | CN | |
| 12-247 | OMe | I | CH₂CH₂OMe | CN | |
| 12-248 | OMe | CF₃ | CH₂CH₂OMe | CN | |
| 12-249 | OMe | CHF₂ | CH₂CH₂OMe | CN | |
| 12-250 | OMe | CF₂Cl | CH₂CH₂OMe | CN | |
| 12-251 | OMe | OMe | CH₂CH₂OMe | CN | |
| 12-252 | OMe | NO₂ | CH₂CH₂OMe | CN | |
| 12-253 | OMe | SO₂Me | CH₂CH₂OMe | CN | |
| 12-254 | SO₂Me | Me | CH₂CH₂OMe | CN | |
| 12-255 | SO₂Me | F | CH₂CH₂OMe | CN | |
| 12-256 | SO₂Me | Cl | CH₂CH₂OMe | CN | |
| 12-257 | SO₂Me | Br | CH₂CH₂OMe | CN | |
| 12-258 | SO₂Me | I | CH₂CH₂OMe | CN | |
| 12-259 | SO₂Me | CF₃ | CH₂CH₂OMe | CN | |
| 12-260 | SO₂Me | CHF₂ | CH₂CH₂OMe | CN | |
| 12-261 | SO₂Me | CF₂Cl | CH₂CH₂OMe | CN | |
| 12-262 | SO₂Me | OMe | CH₂CH₂OMe | CN | |
| 12-263 | SO₂Me | NO₂ | CH₂CH₂OMe | CN | |
| 12-264 | SO₂Me | SO₂Me | CH₂CH₂OMe | CN | |

**Tabelle 13: Erfindungsgemäße Verbindungen der allgemeinen Formel (I), worin Q für Q5 steht, W Wasserstoff bedeutet, t = 0 ist und die anderen Reste die in der Tabelle angegebenen Bedeutungen haben**

| | | | | | |
|---|---|---|---|---|---|
| 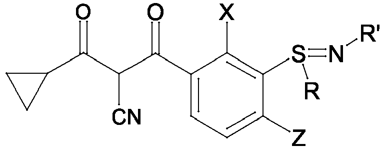 | | | | | |

| Nr. | X | Z | R | R' | Physikalische Daten (1 H-NMR) |
|---|---|---|---|---|---|
| 13-1 | Me | Me | Me | H | |
| 13-2 | Me | F | Me | H | |
| 13-3 | Me | Cl | Me | H | |
| 13-4 | Me | Br | Me | H | |
| 13-5 | Me | I | Me | H | |
| 13-6 | Me | CF₃ | Me | H | |
| 13-7 | Me | CHF₂ | Me | H | |
| 13-8 | Me | CF₂Cl | Me | H | |
| 13-9 | Me | OMe | Me | H | |
| 13-10 | Me | NO₂ | Me | H | |
| 13-11 | Me | SO₂Me | Me | H | |
| 13-12 | Cl | Me | Me | H | |
| 13-13 | Cl | F | Me | H | |
| 13-14 | Cl | Cl | Me | H | |
| 13-15 | Cl | Br | Me | H | |
| 13-16 | Cl | I | Me | H | |
| 13-17 | Cl | CF₃ | Me | H | |
| 13-18 | Cl | CHF₂ | Me | H | |
| 13-19 | Cl | CF₂Cl | Me | H | |
| 13-20 | Cl | OMe | Me | H | |
| 13-21 | Cl | NO₂ | Me | H | |
| 13-22 | Cl | SO₂Me | Me | H | |
| 13-23 | OMe | Me | Me | H | |
| 13-24 | OMe | F | Me | H | |
| 13-25 | OMe | Cl | Me | H | |
| 13-26 | OMe | Br | Me | H | |
| 13-27 | OMe | I | Me | H | |
| 13-28 | OMe | CF₃ | Me | H | |
| 13-29 | OMe | CHF₂ | Me | H | |
| 13-30 | OMe | CF₂Cl | Me | H | |
| 13-31 | OMe | OMe | Me | H | |
| 13-32 | OMe | NO₂ | Me | H | |
| 13-33 | OMe | SO₂Me | Me | H | |
| 13-34 | SO₂Me | Me | Me | H | |
| 13-35 | SO₂Me | F | Me | H | |
| 13-36 | SO₂Me | Cl | Me | H | |
| 13-37 | SO₂Me | Br | Me | H | |
| 13-38 | SO₂Me | I | Me | H | |
| 13-39 | SO₂Me | CF₃ | Me | H | |
| 13-40 | SO₂Me | CHF₂ | Me | H | |
| 13-41 | SO₂Me | CF₂Cl | Me | H | |
| 13-42 | SO₂Me | OMe | Me | H | |
| 13-43 | SO₂Me | NO₂ | Me | H | |
| 13-44 | SO₂Me | SO₂Me | Me | H | |
| 13-45 | Me | Me | Et | H | |
| 13-46 | Me | F | Et | H | |
| 13-47 | Me | Cl | Et | H | |
| 13-48 | Me | Br | Et | H | |
| 13-49 | Me | I | Et | H | |
| 13-50 | Me | CF₃ | Et | H | |
| 13-51 | Me | CHF₂ | Et | H | |
| 13-52 | Me | CF₂Cl | Et | H | |
| 13-53 | Me | OMe | Et | H | |
| 13-54 | Me | NO₂ | Et | H | |
| 13-55 | Me | SO₂Me | Et | H | |
| 13-56 | Cl | Me | Et | H | |
| 13-57 | Cl | F | Et | H | |
| 13-58 | Cl | Cl | Et | H | |
| 13-59 | Cl | Br | Et | H | |
| 13-60 | Cl | I | Et | H | |
| 13-61 | Cl | CF₃ | Et | H | |
| 13-62 | Cl | CHF₂ | Et | H | |
| 13-63 | Cl | CF₂Cl | Et | H | |
| 13-64 | Cl | OMe | Et | H | |
| 13-65 | Cl | NO₂ | Et | H | |
| 13-66 | Cl | SO₂Me | Et | H | |
| 13-67 | OMe | Me | Et | H | |
| 13-68 | OMe | F | Et | H | |
| 13-69 | OMe | Cl | Et | H | |
| 13-70 | OMe | Br | Et | H | |
| 13-71 | OMe | I | Et | H | |
| 13-72 | OMe | CF₃ | Et | H | |
| 13-73 | OMe | CHF₂ | Et | H | |
| 13-74 | OMe | CF₂Cl | Et | H | |
| 13-75 | OMe | OMe | Et | H | |
| 13-76 | OMe | NO₂ | Et | H | |
| 13-77 | OMe | SO₂Me | Et | H | |
| 13-78 | SO₂Me | Me | Et | H | |
| 13-79 | SO₂Me | F | Et | H | |
| 13-80 | SO₂Me | Cl | Et | H | |
| 13-81 | SO₂Me | Br | Et | H | |
| 13-82 | SO₂Me | I | Et | H | |
| 13-83 | SO₂Me | CF₃ | Et | H | |
| 13-84 | SO₂Me | CHF₂ | Et | H | |
| 13-85 | SO₂Me | CF₂Cl | Et | H | |
| 13-86 | SO₂Me | OMe | Et | H | |
| 13-87 | SO₂Me | NO₂ | Et | H | |
| 13-88 | SO₂Me | SO₂Me | Et | H | |
| 13-89 | Me | Me | CH₂CH₂OMe | H | |
| 13-90 | Me | F | CH₂CH₂OMe | H | |
| 13-91 | Me | Cl | CH₂CH₂OMe | H | |
| 13-92 | Me | Br | CH₂CH₂OMe | H | |
| 13-93 | Me | I | CH₂CH₂OMe | H | |
| 13-94 | Me | CF₃ | CH₂CH₂OMe | H | |
| 13-95 | Me | CHF₂ | CH₂CH₂OMe | H | |
| 13-96 | Me | CF₂Cl | CH₂CH₂OMe | H | |
| 13-97 | Me | OMe | CH₂CH₂OMe | H | |
| 13-98 | Me | NO₂ | CH₂CH₂OMe | H | |
| 13-99 | Me | SO₂Me | CH₂CH₂OMe | H | |
| 13-100 | Cl | Me | CH₂CH₂OMe | H | |
| 13-101 | Cl | F | CH₂CH₂OMe | H | |
| 13-102 | Cl | Cl | CH₂CH₂OMe | H | |
| 13-103 | Cl | Br | CH₂CH₂OMe | H | |
| 13-104 | Cl | I | CH₂CH₂OMe | H | |
| 13-105 | Cl | CF₃ | CH₂CH₂OMe | H | |
| 13-106 | Cl | CHF₂ | CH₂CH₂OMe | H | |
| 13-107 | Cl | CF₂Cl | CH₂CH₂OMe | H | |
| 13-108 | Cl | OMe | CH₂CH₂OMe | H | |
| 13-109 | Cl | NO₂ | CH₂CH₂OMe | H | |
| 13-110 | Cl | SO₂Me | CH₂CH₂OMe | H | |
| 13-111 | OMe | Me | CH₂CH₂OMe | H | |
| 13-112 | OMe | F | CH₂CH₂OMe | H | |
| 13-113 | OMe | Cl | CH₂CH₂OMe | H | |
| 13-114 | OMe | Br | CH₂CH₂OMe | H | |
| 13-115 | OMe | I | CH₂CH₂OMe | H | |
| 13-116 | OMe | CF₃ | CH₂CH₂OMe | H | |
| 13-117 | OMe | CHF₂ | CH₂CH₂OMe | H | |
| 13-118 | OMe | CF₂Cl | CH₂CH₂OMe | H | |
| 13-119 | OMe | OMe | CH₂CH₂OMe | H | |
| 13-120 | OMe | NO₂ | CH₂CH₂OMe | H | |
| 13-121 | OMe | SO₂Me | CH₂CH₂OMe | H | |
| 13-122 | SO₂Me | Me | CH₂CH₂OMe | H | |
| 13-123 | SO₂Me | F | CH₂CH₂OMe | H | |
| 13-124 | SO₂Me | Cl | CH₂CH₂OMe | H | |
| 13-125 | SO₂Me | Br | CH₂CH₂OMe | H | |
| 13-126 | SO₂Me | I | CH₂CH₂OMe | H | |
| 13-127 | SO₂Me | CF₃ | CH₂CH₂OMe | H | |
| 13-128 | SO₂Me | CHF₂ | CH₂CH₂OMe | H | |
| 13-129 | SO₂Me | CF₂Cl | CH₂CH₂OMe | H | |
| 13-130 | SO₂Me | OMe | CH₂CH₂OMe | H | |
| 13-131 | SO₂Me | NO₂ | CH₂CH₂OMe | H | |
| 13-132 | SO₂Me | SO₂Me | CH₂CH₂OMe | H | |
| 13-133 | Me | Me | Me | CN | |
| 13-134 | Me | F | Me | CN | |
| 13-135 | Me | Cl | Me | CN | |
| 13-136 | Me | Br | Me | CN | |
| 13-137 | Me | I | Me | CN | |
| 13-138 | Me | CF₃ | Me | CN | |
| 13-139 | Me | CHF₂ | Me | CN | |
| 13-140 | Me | CF₂Cl | Me | CN | |
| 13-141 | Me | OMe | Me | CN | |
| 13-142 | Me | NO₂ | Me | CN | |
| 13-143 | Me | SO₂Me | Me | CN | |
| 13-144 | Cl | Me | Me | CN | |
| 13-145 | Cl | F | Me | CN | |
| 13-146 | Cl | Cl | Me | CN | |
| 13-147 | Cl | Br | Me | CN | |
| 13-148 | Cl | I | Me | CN | |
| 13-149 | Cl | CF₃ | Me | CN | |
| 13-150 | Cl | CHF₂ | Me | CN | |
| 13-151 | Cl | CF₂Cl | Me | CN | |
| 13-152 | Cl | OMe | Me | CN | |
| 13-153 | Cl | NO₂ | Me | CN | |
| 13-154 | Cl | SO₂Me | Me | CN | |
| 13-155 | OMe | Me | Me | CN | |
| 13-156 | OMe | F | Me | CN | |
| 13-157 | OMe | Cl | Me | CN | |
| 13-158 | OMe | Br | Me | CN | |
| 13-159 | OMe | I | Me | CN | |
| 13-160 | OMe | CF₃ | Me | CN | |
| 13-161 | OMe | CHF₂ | Me | CN | |
| 13-162 | OMe | CF₂Cl | Me | CN | |
| 13-163 | OMe | OMe | Me | CN | |
| 13-164 | OMe | NO₂ | Me | CN | |
| 13-165 | OMe | SO₂Me | Me | CN | |
| 13-166 | SO₂Me | Me | Me | CN | |
| 13-167 | SO₂Me | F | Me | CN | |
| 13-168 | SO₂Me | Cl | Me | CN | |
| 13-169 | SO₂Me | Br | Me | CN | |
| 13-170 | SO₂Me | I | Me | CN | |
| 13-171 | SO₂Me | CF₃ | Me | CN | |
| 13-172 | SO₂Me | CHF₂ | Me | CN | |
| 13-173 | SO₂Me | CF₂Cl | Me | CN | |
| 13-174 | SO₂Me | OMe | Me | CN | |
| 13-175 | SO₂Me | NO₂ | Me | CN | |
| 13-176 | SO₂Me | SO₂Me | Me | CN | |
| 13-177 | Me | Me | Et | CN | |
| 13-178 | Me | F | Et | CN | |
| 13-179 | Me | Cl | Et | CN | |
| 13-180 | Me | Br | Et | CN | |
| 13-181 | Me | I | Et | CN | |
| 13-182 | Me | CF₃ | Et | CN | |
| 13-183 | Me | CHF₂ | Et | CN | |
| 13-184 | Me | CF₂Cl | Et | CN | |
| 13-185 | Me | OMe | Et | CN | |
| 13-186 | Me | NO₂ | Et | CN | |
| 13-187 | Me | SO₂Me | Et | CN | |
| 13-188 | Cl | Me | Et | CN | |
| 13-189 | Cl | F | Et | CN | |
| 13-190 | Cl | Cl | Et | CN | |
| 13-191 | Cl | Br | Et | CN | |
| 13-192 | Cl | I | Et | CN | |
| 13-193 | Cl | CF₃ | Et | CN | |
| 13-194 | Cl | CHF₂ | Et | CN | |
| 13-195 | Cl | CF₂Cl | Et | CN | |
| 13-196 | Cl | OMe | Et | CN | |
| 13-197 | Cl | NO₂ | Et | CN | |
| 13-198 | Cl | SO₂Me | Et | CN | |
| 13-199 | OMe | Me | Et | CN | |
| 13-200 | OMe | F | Et | CN | |
| 13-201 | OMe | Cl | Et | CN | |
| 13-202 | OMe | Br | Et | CN | |
| 13-203 | OMe | I | Et | CN | |
| 13-204 | OMe | CF₃ | Et | CN | |
| 13-205 | OMe | CHF₂ | Et | CN | |
| 13-206 | OMe | CF₂Cl | Et | CN | |
| 13-207 | OMe | OMe | Et | CN | |
| 13-208 | OMe | NO₂ | Et | CN | |
| 13-209 | OMe | SO₂Me | Et | CN | |
| 13-210 | SO₂Me | Me | Et | CN | |
| 13-211 | SO₂Me | F | Et | CN | |
| 13-212 | SO₂Me | Cl | Et | CN | |
| 13-213 | SO₂Me | Br | Et | CN | |
| 13-214 | SO₂Me | I | Et | CN | |
| 13-215 | SO₂Me | CF₃ | Et | CN | |
| 13-216 | SO₂Me | CHF₂ | Et | CN | |
| 13-217 | SO₂Me | CF₂Cl | Et | CN | |
| 13-218 | SO₂Me | OMe | Et | CN | |
| 13-219 | SO₂Me | NO₂ | Et | CN | |
| 13-220 | SO₂Me | SO₂Me | Et | CN | |
| 13-221 | Me | Me | CH₂CH₂OMe | CN | |
| 13-222 | Me | F | CH₂CH₂OMe | CN | |
| 13-223 | Me | Cl | CH₂CH₂OMe | CN | |
| 13-224 | Me | Br | CH₂CH₂OMe | CN | |
| 13-225 | Me | I | CH₂CH₂OMe | CN | |
| 13-226 | Me | CF₃ | CH₂CH₂OMe | CN | |
| 13-227 | Me | CHF₂ | CH₂CH₂OMe | CN | |
| 13-228 | Me | CF₂Cl | CH₂CH₂OMe | CN | |
| 13-229 | Me | OMe | CH₂CH₂OMe | CN | |
| 13-230 | Me | NO₂ | CH₂CH₂OMe | CN | |
| 13-231 | Me | SO₂Me | CH₂CH₂OMe | CN | |
| 13-232 | Cl | Me | CH₂CH₂OMe | CN | |
| 13-233 | Cl | F | CH₂CH₂OMe | CN | |
| 13-234 | Cl | Cl | CH₂CH₂OMe | CN | |
| 13-235 | Cl | Br | CH₂CH₂OMe | CN | |
| 13-236 | Cl | I | CH₂CH₂OMe | CN | |
| 13-237 | Cl | CF₃ | CH₂CH₂OMe | CN | |
| 13-238 | Cl | CHF₂ | CH₂CH₂OMe | CN | |
| 13-239 | Cl | CF₂Cl | CH₂CH₂OMe | CN | |
| 13-240 | Cl | OMe | CH₂CH₂OMe | CN | |
| 13-241 | Cl | NO₂ | CH₂CH₂OMe | CN | |
| 13-242 | Cl | SO₂Me | CH₂CH₂OMe | CN | |
| 13-243 | OMe | Me | CH₂CH₂OMe | CN | |
| 13-244 | OMe | F | CH₂CH₂OMe | CN | |
| 13-245 | OMe | Cl | CH₂CH₂OMe | CN | |
| 13-246 | OMe | Br | CH₂CH₂OMe | CN | |
| 13-247 | OMe | I | CH₂CH₂OMe | CN | |
| 13-248 | OMe | CF₃ | CH₂CH₂OMe | CN | |
| 13-249 | OMe | CHF₂ | CH₂CH₂OMe | CN | |
| 13-250 | OMe | CF₂Cl | CH₂CH₂OMe | CN | |
| 13-251 | OMe | OMe | CH₂CH₂OMe | CN | |
| 13-252 | OMe | NO₂ | CH₂CH₂OMe | CN | |
| 13-253 | OMe | SO₂Me | CH₂CH₂OMe | CN | |
| 13-254 | SO₂Me | Me | CH₂CH₂OMe | CN | |
| 13-255 | SO₂Me | F | CH₂CH₂OMe | CN | |
| 13-256 | SO₂Me | Cl | CH₂CH₂OMe | CN | |
| 13-257 | SO₂Me | Br | CH₂CH₂OMe | CN | |
| 13-258 | SO₂Me | I | CH₂CH₂OMe | CN | |
| 13-259 | SO₂Me | CF₃ | CH₂CH₂OMe | CN | |
| 13-260 | SO₂Me | CHF₂ | CH₂CH₂OMe | CN | |
| 13-261 | SO₂Me | CF₂Cl | CH₂CH₂OMe | CN | |
| 13-262 | SO₂Me | OMe | CH₂CH₂OMe | CN | |
| 13-263 | SO₂Me | NO₂ | CH₂CH₂OMe | CN | |
| 13-264 | SO₂Me | SO₂Me | CH₂CH₂OMe | CN | |

**Tabelle 14: Erfindungsgemäße Verbindungen der allgemeinen Formel (I) in Form der Natriumsalze, worin Q für Q3, Rⁱ für Methyl,R^{k} sowie W jeweils für Wasserstoff stehen, t = 0 ist und die anderen Reste die in der Tabelle angegebenen Bedeutungen haben**

| | | | | | |
|---|---|---|---|---|---|
| 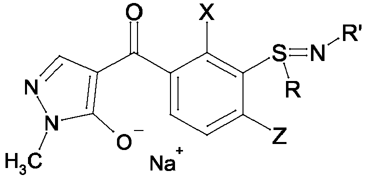 | | | | | |

| Nr. | X | Z | R | R' | Physikalische Daten (1 H-NMR) |
|---|---|---|---|---|---|
| 14-1 | Me | Me | Me | H | |
| 14-2 | Me | Cl | Me | H | |
| 14-3 | Me | CF₃ | Me | H | |
| 14-4 | Me | CHF₂ | Me | H | |
| 14-5 | Cl | Me | Me | H | |
| 14-6 | Cl | Cl | Me | H | |
| 14-7 | Cl | CF₃ | Me | H | |
| 14-8 | Cl | CHF₂ | Me | H | |
| 14-9 | OMe | Me | Me | H | |
| 14-10 | OMe | Cl | Me | H | |
| 14-11 | OMe | CF₃ | Me | H | |
| 14-12 | OMe | CHF₂ | Me | H | |
| 14-13 | SO₂Me | Me | Me | H | |
| 14-14 | SO₂Me | Cl | Me | H | |
| 14-15 | SO₂Me | CF₃ | Me | H | |
| 14-16 | SO₂Me | CHF₂ | Me | H | |
| 14-17 | Me | Me | Et | H | |
| 14-18 | Me | Cl | Et | H | |
| 14-19 | Me | CF₃ | Et | H | |
| 14-20 | Me | CHF₂ | Et | H | |
| 14-21 | Cl | Me | Et | H | |
| 14-22 | Cl | Cl | Et | H | |
| 14-23 | Cl | CF₃ | Et | H | |
| 14-24 | Cl | CHF₂ | Et | H | |
| 14-25 | OMe | Me | Et | H | |
| 14-26 | OMe | Cl | Et | H | |
| 14-27 | OMe | CF₃ | Et | H | |
| 14-28 | OMe | CHF₂ | Et | H | |
| 14-29 | SO₂Me | Me | Et | H | |
| 14-30 | SO₂Me | Cl | Et | H | |
| 14-31 | SO₂Me | CF₃ | Et | H | |
| 14-32 | SO₂Me | CHF₂ | Et | H | |
| 14-33 | Me | Me | CH₂CH₂OMe | H | |
| 14-34 | Me | Cl | CH₂CH₂OMe | H | |
| 14-35 | Me | CF₃ | CH₂CH₂OMe | H | |
| 14-36 | Me | CHF₂ | CH₂CH₂OMe | H | |
| 14-37 | Cl | Me | CH₂CH₂OMe | H | |
| 14-38 | Cl | Cl | CH₂CH₂OMe | H | |
| 14-39 | Cl | CF₃ | CH₂CH₂OMe | H | |
| 14-40 | Cl | CHF₂ | CH₂CH₂OMe | H | |
| 14-41 | OMe | Me | CH₂CH₂OMe | H | |
| 14-42 | OMe | Cl | CH₂CH₂OMe | H | |
| 14-43 | OMe | CF₃ | CH₂CH₂OMe | H | |
| 14-44 | OMe | CHF₂ | CH₂CH₂OMe | H | |
| 14-45 | SO₂Me | Me | CH₂CH₂OMe | H | |
| 14-46 | SO₂Me | Cl | CH₂CH₂OMe | H | |
| 14-47 | SO₂Me | CF₃ | CH₂CH₂OMe | H | |
| 14-48 | SO₂Me | CHF₂ | CH₂CH₂OMe | H | |
| 14-49 | Me | Me | Me | CN | |
| 14-50 | Me | Cl | Me | CN | |
| 14-51 | Me | CF₃ | Me | CN | |
| 14-52 | Me | CHF₂ | Me | CN | |
| 14-53 | Cl | Me | Me | CN | |
| 14-54 | Cl | Cl | Me | CN | |
| 14-55 | Cl | CF₃ | Me | CN | |
| 14-56 | Cl | CHF₂ | Me | CN | |
| 14-57 | OMe | Me | Me | CN | |
| 14-58 | OMe | Cl | Me | CN | |
| 14-59 | OMe | CF₃ | Me | CN | |
| 14-60 | OMe | CHF₂ | Me | CN | |
| 14-61 | SO₂Me | Me | Me | CN | |
| 14-62 | SO₂Me | Cl | Me | CN | |
| 14-63 | SO₂Me | CF₃ | Me | CN | |
| 14-64 | SO₂Me | CHF₂ | Me | CN | |
| 14-65 | Me | Me | Et | CN | |
| 14-66 | Me | Cl | Et | CN | |
| 14-67 | Me | CF₃ | Et | CN | |
| 14-68 | Me | CHF₂ | Et | CN | |
| 14-69 | Cl | Me | Et | CN | |
| 14-70 | Cl | Cl | Et | CN | |
| 14-71 | Cl | CF₃ | Et | CN | |
| 14-72 | Cl | CHF₂ | Et | CN | |
| 14-73 | OMe | Me | Et | CN | |
| 14-74 | OMe | Cl | Et | CN | |
| 14-75 | OMe | CF₃ | Et | CN | |
| 14-76 | OMe | CHF₂ | Et | CN | |
| 14-77 | SO₂Me | Me | Et | CN | |
| 14-78 | SO₂Me | Cl | Et | CN | |
| 14-79 | SO₂Me | CF₃ | Et | CN | |
| 14-80 | SO₂Me | CHF₂ | Et | CN | |
| 14-81 | Me | Me | CH₂CH₂OMe | CN | |
| 14-82 | Me | Cl | CH₂CH₂OMe | CN | |
| 14-83 | Me | CF₃ | CH₂CH₂OMe | CN | |
| 14-84 | Me | CHF₂ | CH₂CH₂OMe | CN | |
| 14-85 | Cl | Me | CH₂CH₂OMe | CN | |
| 14-86 | Cl | Cl | CH₂CH₂OMe | CN | |
| 14-87 | Cl | CF₃ | CH₂CH₂OMe | CN | |
| 14-88 | Cl | CHF₂ | CH₂CH₂OMe | CN | |
| 14-89 | OMe | Me | CH₂CH₂OMe | CN | |
| 14-90 | OMe | Cl | CH₂CH₂OMe | CN | |
| 14-91 | OMe | CF₃ | CH₂CH₂OMe | CN | |
| 14-92 | OMe | CHF₂ | CH₂CH₂OMe | CN | |
| 14-93 | SO₂Me | Me | CH₂CH₂OMe | CN | |
| 14-94 | SO₂Me | Cl | CH₂CH₂OMe | CN | |
| 14-95 | SO₂Me | CF₃ | CH₂CH₂OMe | CN | |
| 14-96 | SO₂Me | CHF₂ | CH₂CH₂OMe | CN | |

**Tabelle 15: Erfindungsgemäße Verbindungen der allgemeinen Formel (II), worin Q* für Hydroxy und R' für Cyano stehen, t für 1 steht, und die anderen Reste die in der Tabelle angegebenen Bedeutungen haben**

| | | | | |
|---|---|---|---|---|
| 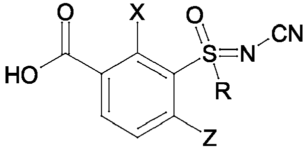 | | | | |

| Nr. | X | Z | R | Physikalische Daten (¹H-NMR) |
|---|---|---|---|---|
| 15-1 | Me | Me | Me | |
| 15-2 | Me | F | Me | |
| 15-3 | Me | Cl | Me | |
| 15-4 | Me | Br | Me | |
| 15-5 | Me | I | Me | |
| 15-6 | Me | CF₃ | Me | |
| 15-7 | Me | CHF₂ | Me | |
| 15-8 | Me | CF₂Cl | Me | |
| 15-9 | Me | OMe | Me | |
| 15-10 | Me | NO₂ | Me | |
| 15-11 | Me | SO₂Me | Me | |
| 15-12 | Cl | Me | Me | |
| 15-13 | Cl | F | Me | |
| 15-14 | Cl | Cl | Me | |
| 15-15 | Cl | Br | Me | |
| 15-16 | Cl | I | Me | |
| 15-17 | Cl | CF₃ | Me | |
| 15-18 | Cl | CHF₂ | Me | |
| 15-19 | Cl | CF₂Cl | Me | |
| 15-20 | Cl | OMe | Me | |
| 15-21 | Cl | NO₂ | Me | |
| 15-22 | Cl | SO₂Me | Me | |
| 15-23 | OMe | Me | Me | |
| 15-24 | OMe | F | Me | |
| 15-25 | OMe | Cl | Me | |
| 15-26 | OMe | Br | Me | |
| 15-27 | OMe | I | Me | |
| 15-28 | OMe | CF₃ | Me | (400 MHz, CDCl₃ δ, ppm) 8.34 (d,1 H), 7.84 (d,1H), 4.16 (s,3H), 3.68 (s,3H) |
| 15-29 | OMe | CHF₂ | Me | |
| 15-30 | OMe | CF₂Cl | Me | |
| 15-31 | OMe | OMe | Me | |
| 15-32 | OMe | NO₂ | Me | |
| 15-33 | OMe | SO₂Me | Me | |
| 15-34 | SO₂Me | Me | Me | |
| 15-35 | SO₂Me | F | Me | |
| 15-36 | SO₂Me | Cl | Me | |
| 15-37 | SO₂Me | Br | Me | |
| 15-38 | SO₂Me | I | Me | |
| 15-39 | SO₂Me | CF₃ | Me | |
| 15-40 | SO₂Me | CHF₂ | Me | |
| 15-41 | SO₂Me | CF₂Cl | Me | |
| 15-42 | SO₂Me | OMe | Me | |
| 15-43 | SO₂Me | NO₂ | Me | |
| 15-44 | SO₂Me | SO₂Me | Me | |
| 15-45 | Me | Me | Et | |
| 15-46 | Me | F | Et | |
| 15-47 | Me | Cl | Et | |
| 15-48 | Me | Br | Et | |
| 15-49 | Me | I | Et | |
| 15-50 | Me | CF₃ | Et | |
| 15-51 | Me | CHF₂ | Et | |
| 15-52 | Me | CF₂Cl | Et | |
| 15-53 | Me | OMe | Et | |
| 15-54 | Me | NO₂ | Et | |
| 15-55 | Me | SO₂Me | Et | |
| 15-56 | Cl | Me | Et | |
| 15-57 | Cl | F | Et | |
| 15-58 | Cl | Cl | Et | |
| 15-59 | Cl | Br | Et | |
| 15-60 | Cl | I | Et | |
| 15-61 | Cl | CF₃ | Et | |
| 15-62 | Cl | CHF₂ | Et | |
| 15-63 | Cl | CF₂Cl | Et | |
| 15-64 | Cl | OMe | Et | |
| 15-65 | Cl | NO₂ | Et | |
| 15-66 | Cl | SO₂Me | Et | |
| 15-67 | OMe | Me | Et | |
| 15-68 | OMe | F | Et | |
| 15-69 | OMe | Cl | Et | |
| 15-70 | OMe | Br | Et | |
| 15-71 | OMe | I | Et | |
| 15-72 | OMe | CF₃ | Et | |
| 15-73 | OMe | CHF₂ | Et | |
| 15-74 | OMe | CF₂Cl | Et | |
| 15-75 | OMe | OMe | Et | |
| 15-76 | OMe | NO₂ | Et | |
| 15-77 | OMe | SO₂Me | Et | |
| 15-78 | SO₂Me | Me | Et | |
| 15-79 | SO₂Me | F | Et | |
| 15-80 | SO₂Me | Cl | Et | |
| 15-81 | SO₂Me | Br | Et | |
| 15-82 | SO₂Me | I | Et | |
| 15-83 | SO₂Me | CF₃ | Et | |
| 15-84 | SO₂Me | CHF₂ | Et | |
| 15-85 | SO₂Me | CF₂Cl | Et | |
| 15-86 | SO₂Me | OMe | Et | |
| 15-87 | SO₂Me | NO₂ | Et | |
| 15-88 | SO₂Me | SO₂Me | Et | |
| 15-89 | Me | Me | CH₂CH₂OMe | |
| 15-90 | Me | F | CH₂CH₂OMe | |
| 15-91 | Me | Cl | CH₂CH₂OMe | |
| 15-92 | Me | Br | CH₂CH₂OMe | |
| 15-93 | Me | I | CH₂CH₂OMe | |
| 15-94 | Me | CF₃ | CH₂CH₂OMe | |
| 15-95 | Me | CHF₂ | CH₂CH₂OMe | |
| 15-96 | Me | CF₂Cl | CH₂CH₂OMe | |
| 15-97 | Me | OMe | CH₂CH₂OMe | |
| 15-98 | Me | NO₂ | CH₂CH₂OMe | |
| 15-99 | Me | SO₂Me | CH₂CH₂OMe | |
| 15-100 | Cl | Me | CH₂CH₂OMe | |
| 15-101 | Cl | F | CH₂CH₂OMe | |
| 15-102 | Cl | Cl | CH₂CH₂OMe | |
| 15-103 | Cl | Br | CH₂CH₂OMe | |
| 15-104 | Cl | I | CH₂CH₂OMe | |
| 15-105 | Cl | CF₃ | CH₂CH₂OMe | |
| 15-106 | Cl | CHF₂ | CH₂CH₂OMe | |
| 15-107 | Cl | CF₂Cl | CH₂CH₂OMe | |
| 15-108 | Cl | OMe | CH₂CH₂OMe | |
| 15-109 | Cl | NO₂ | CH₂CH₂OMe | |
| 15-110 | Cl | SO₂Me | CH₂CH₂OMe | |
| 15-111 | OMe | Me | CH₂CH₂OMe | |
| 15-112 | OMe | F | CH₂CH₂OMe | |
| 15-113 | OMe | Cl | CH₂CH₂OMe | |
| 15-114 | OMe | Br | CH₂CH₂OMe | |
| 15-115 | OMe | I | CH₂CH₂OMe | |
| 15-116 | OMe | CF₃ | CH₂CH₂OMe | |
| 15-117 | OMe | CHF₂ | CH₂CH₂OMe | |
| 15-118 | OMe | CF₂Cl | CH₂CH₂OMe | |
| 15-119 | OMe | OMe | CH₂CH₂OMe | |
| 15-120 | OMe | NO₂ | CH₂CH₂OMe | |
| 15-121 | OMe | SO₂Me | CH₂CH₂OMe | |
| 15-122 | SO₂Me | Me | CH₂CH₂OMe | |
| 15-123 | SO₂Me | F | CH₂CH₂OMe | |
| 15-124 | SO₂Me | Cl | CH₂CH₂OMe | |
| 15-125 | SO₂Me | Br | CH₂CH₂OMe | |
| 15-126 | SO₂Me | I | CH₂CH₂OMe | |
| 15-127 | SO₂Me | CF₃ | CH₂CH₂OMe | |
| 15-128 | SO₂Me | CHF₂ | CH₂CH₂OMe | |
| 15-129 | SO₂Me | CF₂Cl | CH₂CH₂OMe | |
| 15-130 | SO₂Me | OMe | CH₂CH₂OMe | |
| 15-131 | SO₂Me | NO₂ | CH₂CH₂OMe | |
| 15-132 | SO₂Me | SO₂Me | CH₂CH₂OMe | |

**Tabelle 16: Erfindungsgemäße Verbindungen der allgemeinen Formel (II), worin Q* für Hydroxy und R' für Cyano stehen, t für 0 steht, und die anderen Reste die in der Tabelle angegebenen Bedeutungen haben**

| | | | | |
|---|---|---|---|---|
| 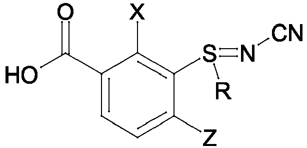 | | | | |

| Nr. | X | Z | R | Physikalische Daten (¹H-NMR) |
|---|---|---|---|---|
| 16-1 | Me | Me | Me | |
| 16-2 | Me | F | Me | |
| 16-3 | Me | Cl | Me | |
| 16-4 | Me | Br | Me | |
| 16-5 | Me | I | Me | |
| 16-6 | Me | CF₃ | Me | |
| 16-7 | Me | CHF₂ | Me | |
| 16-8 | Me | CF₂Cl | Me | |
| 16-9 | Me | OMe | Me | |
| 16-10 | Me | NO₂ | Me | |
| 16-11 | Me | SO₂Me | Me | |
| 16-12 | Cl | Me | Me | |
| 16-13 | Cl | F | Me | |
| 16-14 | Cl | Cl | Me | |
| 16-15 | Cl | Br | Me | |
| 16-16 | Cl | I | Me | |
| 16-17 | Cl | CF₃ | Me | |
| 16-18 | Cl | CHF₂ | Me | |
| 16-19 | Cl | CF₂Cl | Me | |
| 16-20 | Cl | OMe | Me | |
| 16-21 | Cl | NO₂ | Me | |
| 16-22 | Cl | SO₂Me | Me | |
| 16-23 | OMe | Me | Me | |
| 16-24 | OMe | F | Me | |
| 16-25 | OMe | Cl | Me | |
| 16-26 | OMe | Br | Me | |
| 16-27 | OMe | I | Me | |
| 16-28 | OMe | CF₃ | Me | |
| 16-29 | OMe | CHF₂ | Me | |
| 16-30 | OMe | CF₂Cl | Me | |
| 16-31 | OMe | OMe | Me | |
| 16-32 | OMe | NO₂ | Me | |
| 16-33 | OMe | SO₂Me | Me | |
| 16-34 | SO₂Me | Me | Me | |
| 16-35 | SO₂Me | F | Me | |
| 16-36 | SO₂Me | Cl | Me | |
| 16-37 | SO₂Me | Br | Me | |
| 16-38 | SO₂Me | I | Me | |
| 16-39 | SO₂Me | CF₃ | Me | |
| 16-40 | SO₂Me | CHF₂ | Me | |
| 16-41 | SO₂Me | CF₂Cl | Me | |
| 16-42 | SO₂Me | OMe | Me | |
| 16-43 | SO₂Me | NO₂ | Me | |
| 16-44 | SO₂Me | SO₂Me | Me | |
| 16-45 | Me | Me | Et | |
| 16-46 | Me | F | Et | |
| 16-47 | Me | Cl | Et | |
| 16-48 | Me | Br | Et | |
| 16-49 | Me | I | Et | |
| 16-50 | Me | CF₃ | Et | |
| 16-51 | Me | CHF₂ | Et | |
| 16-52 | Me | CF₂Cl | Et | |
| 16-53 | Me | OMe | Et | |
| 16-54 | Me | NO₂ | Et | |
| 16-55 | Me | SO₂Me | Et | |
| 16-56 | Cl | Me | Et | |
| 16-57 | Cl | F | Et | |
| 16-58 | Cl | Cl | Et | |
| 16-59 | Cl | Br | Et | |
| 16-60 | Cl | I | Et | |
| 16-61 | Cl | CF₃ | Et | |
| 16-62 | Cl | CHF₂ | Et | |
| 16-63 | Cl | CF₂Cl | Et | |
| 16-64 | Cl | OMe | Et | |
| 16-65 | Cl | NO₂ | Et | |
| 16-66 | Cl | SO₂Me | Et | |
| 16-67 | OMe | Me | Et | |
| 16-68 | OMe | F | Et | |
| 16-69 | OMe | Cl | Et | |
| 16-70 | OMe | Br | Et | |
| 16-71 | OMe | I | Et | |
| 16-72 | OMe | CF₃ | Et | |
| 16-73 | OMe | CHF₂ | Et | |
| 16-74 | OMe | CF₂Cl | Et | |
| 16-75 | OMe | OMe | Et | |
| 16-76 | OMe | NO₂ | Et | |
| 16-77 | OMe | SO₂Me | Et | |
| 16-78 | SO₂Me | Me | Et | |
| 16-79 | SO₂Me | F | Et | |
| 16-80 | SO₂Me | Cl | Et | |
| 16-81 | SO₂Me | Br | Et | |
| 16-82 | SO₂Me | I | Et | |
| 16-83 | SO₂Me | CF₃ | Et | |
| 16-84 | SO₂Me | CHF₂ | Et | |
| 16-85 | SO₂Me | CF₂Cl | Et | |
| 16-86 | SO₂Me | OMe | Et | |
| 16-87 | SO₂Me | NO₂ | Et | |
| 16-88 | SO₂Me | SO₂Me | Et | |
| 16-89 | Me | Me | CH₂CH₂OMe | |
| 16-90 | Me | F | CH₂CH₂OMe | |
| 16-91 | Me | Cl | CH₂CH₂OMe | |
| 16-92 | Me | Br | CH₂CH₂OMe | |
| 16-93 | Me | I | CH₂CH₂OMe | |
| 16-94 | Me | CF₃ | CH₂CH₂OMe | |
| 16-95 | Me | CHF₂ | CH₂CH₂OMe | |
| 16-96 | Me | CF₂Cl | CH₂CH₂OMe | |
| 16-97 | Me | OMe | CH₂CH₂OMe | |
| 16-98 | Me | NO₂ | CH₂CH₂OMe | |
| 16-99 | Me | SO₂Me | CH₂CH₂OMe | |
| 16-100 | Cl | Me | CH₂CH₂OMe | |
| 16-101 | Cl | F | CH₂CH₂OMe | |
| 16-102 | Cl | Cl | CH₂CH₂OMe | |
| 16-103 | Cl | Br | CH₂CH₂OMe | |
| 16-104 | Cl | I | CH₂CH₂OMe | |
| 16-105 | Cl | CF₃ | CH₂CH₂OMe | |
| 16-106 | Cl | CHF₂ | CH₂CH₂OMe | |
| 16-107 | Cl | CF₂Cl | CH₂CH₂OMe | |
| 16-108 | Cl | OMe | CH₂CH₂OMe | |
| 16-109 | Cl | NO₂ | CH₂CH₂OMe | |
| 16-110 | Cl | SO₂Me | CH₂CH₂OMe | |
| 16-111 | OMe | Me | CH₂CH₂OMe | |
| 16-112 | OMe | F | CH₂CH₂OMe | |
| 16-113 | OMe | Cl | CH₂CH₂OMe | |
| 16-114 | OMe | Br | CH₂CH₂OMe | |
| 16-115 | OMe | I | CH₂CH₂OMe | |
| 16-116 | OMe | CF₃ | CH₂CH₂OMe | |
| 16-117 | OMe | CHF₂ | CH₂CH₂OMe | |
| 16-118 | OMe | CF₂Cl | CH₂CH₂OMe | |
| 16-119 | OMe | OMe | CH₂CH₂OMe | |
| 16-120 | OMe | NO₂ | CH₂CH₂OMe | |
| 16-121 | OMe | SO₂Me | CH₂CH₂OMe | |
| 16-122 | SO₂Me | Me | CH₂CH₂OMe | |
| 16-123 | SO₂Me | F | CH₂CH₂OMe | |
| 16-124 | SO₂Me | Cl | CH₂CH₂OMe | |
| 16-125 | SO₂Me | Br | CH₂CH₂OMe | |
| 16-126 | SO₂Me | I | CH₂CH₂OMe | |
| 16-127 | SO₂Me | CF₃ | CH₂CH₂OMe | |
| 16-128 | SO₂Me | CHF₂ | CH₂CH₂OMe | |
| 16-129 | SO₂Me | CF₂Cl | CH₂CH₂OMe | |
| 16-130 | SO₂Me | OMe | CH₂CH₂OMe | |
| 16-131 | SO₂Me | NO₂ | CH₂CH₂OMe | |
| 16-132 | SO₂Me | SO₂Me | CH₂CH₂OMe | |

**Tabelle 17: Erfindungsgemäße Verbindungen der allgemeinen Formel (II), worin Q* für Chlor und R' für Cyano stehen, t für 1 steht, und die anderen Reste die in der Tabelle angegebenen Bedeutungen haben**

| | | | | |
|---|---|---|---|---|
| 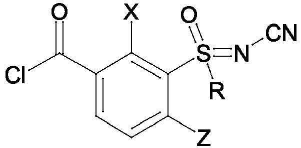 | | | | |

| Nr. | X | Z | R | Physikalische Daten (¹H-NMR) |
|---|---|---|---|---|
| 17-1 | Me | Me | Me | |
| 17-2 | Me | F | Me | |
| 17-3 | Me | Cl | Me | |
| 17-4 | Me | Br | Me | |
| 17-5 | Me | I | Me | |
| 17-6 | Me | CF₃ | Me | |
| 17-7 | Me | CHF₂ | Me | |
| 17-8 | Me | CF₂Cl | Me | |
| 17-9 | Me | OMe | Me | |
| 17-10 | Me | NO₂ | Me | |
| 17-11 | Me | SO₂Me | Me | |
| 17-12 | Cl | Me | Me | |
| 17-13 | Cl | F | Me | |
| 17-14 | Cl | Cl | Me | |
| 17-15 | Cl | Br | Me | |
| 17-16 | Cl | I | Me | |
| 17-17 | Cl | CF₃ | Me | |
| 17-18 | Cl | CHF₂ | Me | |
| 17-19 | Cl | CF₂Cl | Me | |
| 17-20 | Cl | OMe | Me | |
| 17-21 | Cl | NO₂ | Me | |
| 17-22 | Cl | SO₂Me | Me | |
| 17-23 | OMe | Me | Me | |
| 17-24 | OMe | F | Me | |
| 17-25 | OMe | Cl | Me | |
| 17-26 | OMe | Br | Me | |
| 17-27 | OMe | I | Me | |
| 17-28 | OMe | CF₃ | Me | |
| 17-29 | OMe | CHF₂ | Me | |
| 17-30 | OMe | CF₂Cl | Me | |
| 17-31 | OMe | OMe | Me | |
| 17-32 | OMe | NO₂ | Me | |
| 17-33 | OMe | SO₂Me | Me | |
| 17-34 | SO₂Me | Me | Me | |
| 17-35 | SO₂Me | F | Me | |
| 17-36 | SO₂Me | Cl | Me | |
| 17-37 | SO₂Me | Br | Me | |
| 17-38 | SO₂Me | I | Me | |
| 17-39 | SO₂Me | CF₃ | Me | |
| 17-40 | SO₂Me | CHF₂ | Me | |
| 17-41 | SO₂Me | CF₂Cl | Me | |
| 17-42 | SO₂Me | OMe | Me | |
| 17-43 | SO₂Me | NO₂ | Me | |
| 17-44 | SO₂Me | SO₂Me | Me | |
| 17-45 | Me | Me | Et | |
| 17-46 | Me | F | Et | |
| 17-47 | Me | Cl | Et | |
| 17-48 | Me | Br | Et | |
| 17-49 | Me | I | Et | |
| 17-50 | Me | CF₃ | Et | |
| 17-51 | Me | CHF₂ | Et | |
| 17-52 | Me | CF₂Cl | Et | |
| 17-53 | Me | OMe | Et | |
| 17-54 | Me | NO₂ | Et | |
| 17-55 | Me | SO₂Me | Et | |
| 17-56 | Cl | Me | Et | |
| 17-57 | Cl | F | Et | |
| 17-58 | Cl | Cl | Et | |
| 17-59 | Cl | Br | Et | |
| 17-60 | Cl | I | Et | |
| 17-61 | Cl | CF₃ | Et | |
| 17-62 | Cl | CHF₂ | Et | |
| 17-63 | Cl | CF₂Cl | Et | |
| 17-64 | Cl | OMe | Et | |
| 17-65 | Cl | NO₂ | Et | |
| 17-66 | Cl | SO₂Me | Et | |
| 17-67 | OMe | Me | Et | |
| 17-68 | OMe | F | Et | |
| 17-69 | OMe | Cl | Et | |
| 17-70 | OMe | Br | Et | |
| 17-71 | OMe | I | Et | |
| 17-72 | OMe | CF₃ | Et | |
| 17-73 | OMe | CHF₂ | Et | |
| 17-74 | OMe | CF₂Cl | Et | |
| 17-75 | OMe | OMe | Et | |
| 17-76 | OMe | NO₂ | Et | |
| 17-77 | OMe | SO₂Me | Et | |
| 17-78 | SO₂Me | Me | Et | |
| 17-79 | SO₂Me | F | Et | |
| 17-80 | SO₂Me | Cl | Et | |
| 17-81 | SO₂Me | Br | Et | |
| 17-82 | SO₂Me | I | Et | |
| 17-83 | SO₂Me | CF₃ | Et | |
| 17-84 | SO₂Me | CHF₂ | Et | |
| 17-85 | SO₂Me | CF₂Cl | Et | |
| 17-86 | SO₂Me | OMe | Et | |
| 17-87 | SO₂Me | NO₂ | Et | |
| 17-88 | SO₂Me | SO₂Me | Et | |
| 17-89 | Me | Me | CH₂CH₂OMe | |
| 17-90 | Me | F | CH₂CH₂OMe | |
| 17-91 | Me | Cl | CH₂CH₂OMe | |
| 17-92 | Me | Br | CH₂CH₂OMe | |
| 17-93 | Me | I | CH₂CH₂OMe | |
| 17-94 | Me | CF₃ | CH₂CH₂OMe | |
| 17-95 | Me | CHF₂ | CH₂CH₂OMe | |
| 17-96 | Me | CF₂Cl | CH₂CH₂OMe | |
| 17-97 | Me | OMe | CH₂CH₂OMe | |
| 17-98 | Me | NO₂ | CH₂CH₂OMe | |
| 17-99 | Me | SO₂Me | CH₂CH₂OMe | |
| 17-100 | Cl | Me | CH₂CH₂OMe | |
| 17-101 | Cl | F | CH₂CH₂OMe | |
| 17-102 | Cl | Cl | CH₂CH₂OMe | |
| 17-103 | Cl | Br | CH₂CH₂OMe | |
| 17-104 | Cl | I | CH₂CH₂OMe | |
| 17-105 | Cl | CF₃ | CH₂CH₂OMe | |
| 17-106 | Cl | CHF₂ | CH₂CH₂OMe | |
| 17-107 | Cl | CF₂Cl | CH₂CH₂OMe | |
| 17-108 | Cl | OMe | CH₂CH₂OMe | |
| 17-109 | Cl | NO₂ | CH₂CH₂OMe | |
| 17-110 | Cl | SO₂Me | CH₂CH₂OMe | |
| 17-111 | OMe | Me | CH₂CH₂OMe | |
| 17-112 | OMe | F | CH₂CH₂OMe | |
| 17-113 | OMe | Cl | CH₂CH₂OMe | |
| 17-114 | OMe | Br | CH₂CH₂OMe | |
| 17-115 | OMe | I | CH₂CH₂OMe | |
| 17-116 | OMe | CF₃ | CH₂CH₂OMe | |
| 17-117 | OMe | CHF₂ | CH₂CH₂OMe | |
| 17-118 | OMe | CF₂Cl | CH₂CH₂OMe | |
| 17-119 | OMe | OMe | CH₂CH₂OMe | |
| 17-120 | OMe | NO₂ | CH₂CH₂OMe | |
| 17-121 | OMe | SO₂Me | CH₂CH₂OMe | |
| 17-122 | SO₂Me | Me | CH₂CH₂OMe | |
| 17-123 | SO₂Me | F | CH₂CH₂OMe | |
| 17-124 | SO₂Me | Cl | CH₂CH₂OMe | |
| 17-125 | SO₂Me | Br | CH₂CH₂OMe | |
| 17-126 | SO₂Me | I | CH₂CH₂OMe | |
| 17-127 | SO₂Me | CF₃ | CH₂CH₂OMe | |
| 17-128 | SO₂Me | CHF₂ | CH₂CH₂OMe | |
| 17-129 | SO₂Me | CF₂Cl | CH₂CH₂OMe | |
| 17-130 | SO₂Me | OMe | CH₂CH₂OMe | |
| 17-131 | SO₂Me | NO₂ | CH₂CH₂OMe | |
| 17-132 | SO₂Me | SO₂Me | CH₂CH₂OMe | |

**Tabelle 18: Erfindungsgemäße Verbindungen der allgemeinen Formel (II), worin Q* für Chlor und R' für Cyano stehen, t für 0 steht, und die anderen Reste die in der Tabelle angegebenen Bedeutungen haben**

| | | | | |
|---|---|---|---|---|
| 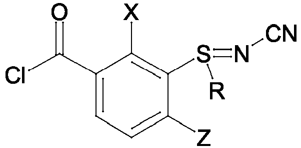 | | | | |

| Nr. | X | Z | R | Physikalische Daten (¹H-NMR) |
|---|---|---|---|---|
| 18-1 | Me | Me | Me | |
| 18-2 | Me | F | Me | |
| 18-3 | Me | Cl | Me | |
| 18-4 | Me | Br | Me | |
| 18-5 | Me | I | Me | |
| 18-6 | Me | CF₃ | Me | |
| 18-7 | Me | CHF₂ | Me | |
| 18-8 | Me | CF₂Cl | Me | |
| 18-9 | Me | OMe | Me | |
| 18-10 | Me | NO₂ | Me | |
| 18-11 | Me | SO₂Me | Me | |
| 18-12 | Cl | Me | Me | |
| 18-13 | Cl | F | Me | |
| 18-14 | Cl | Cl | Me | |
| 18-15 | Cl | Br | Me | |
| 18-16 | Cl | I | Me | |
| 18-17 | Cl | CF₃ | Me | |
| 18-18 | Cl | CHF₂ | Me | |
| 18-19 | Cl | CF₂Cl | Me | |
| 18-20 | Cl | OMe | Me | |
| 18-21 | Cl | NO₂ | Me | |
| 18-22 | Cl | SO₂Me | Me | |
| 18-23 | OMe | Me | Me | |
| 18-24 | OMe | F | Me | |
| 18-25 | OMe | Cl | Me | |
| 18-26 | OMe | Br | Me | |
| 18-27 | OMe | I | Me | |
| 18-28 | OMe | CF₃ | Me | |
| 18-29 | OMe | CHF₂ | Me | |
| 18-30 | OMe | CF₂Cl | Me | |
| 18-31 | OMe | OMe | Me | |
| 18-32 | OMe | NO₂ | Me | |
| 18-33 | OMe | SO₂Me | Me | |
| 18-34 | SO₂Me | Me | Me | |
| 18-35 | SO₂Me | F | Me | |
| 18-36 | SO₂Me | Cl | Me | |
| 18-37 | SO₂Me | Br | Me | |
| 18-38 | SO₂Me | I | Me | |
| 18-39 | SO₂Me | CF₃ | Me | |
| 18-40 | SO₂Me | CHF₂ | Me | |
| 18-41 | SO₂Me | CF₂Cl | Me | |
| 18-42 | SO₂Me | OMe | Me | |
| 18-43 | SO₂Me | NO₂ | Me | |
| 18-44 | SO₂Me | SO₂Me | Me | |
| 18-45 | Me | Me | Et | |
| 18-46 | Me | F | Et | |
| 18-47 | Me | Cl | Et | |
| 18-48 | Me | Br | Et | |
| 18-49 | Me | I | Et | |
| 18-50 | Me | CF₃ | Et | |
| 18-51 | Me | CHF₂ | Et | |
| 18-52 | Me | CF₂Cl | Et | |
| 18-53 | Me | OMe | Et | |
| 18-54 | Me | NO₂ | Et | |
| 18-55 | Me | SO₂Me | Et | |
| 18-56 | Cl | Me | Et | |
| 18-57 | Cl | F | Et | |
| 18-58 | Cl | Cl | Et | |
| 18-59 | Cl | Br | Et | |
| 18-60 | Cl | I | Et | |
| 18-61 | Cl | CF₃ | Et | |
| 18-62 | Cl | CHF₂ | Et | |
| 18-63 | Cl | CF₂Cl | Et | |
| 18-64 | Cl | OMe | Et | |
| 18-65 | Cl | NO₂ | Et | |
| 18-66 | Cl | SO₂Me | Et | |
| 18-67 | OMe | Me | Et | |
| 18-68 | OMe | F | Et | |
| 18-69 | OMe | Cl | Et | |
| 18-70 | OMe | Br | Et | |
| 18-71 | OMe | I | Et | |
| 18-72 | OMe | CF₃ | Et | |
| 18-73 | OMe | CHF₂ | Et | |
| 18-74 | OMe | CF₂Cl | Et | |
| 18-75 | OMe | OMe | Et | |
| 18-76 | OMe | NO₂ | Et | |
| 18-77 | OMe | SO₂Me | Et | |
| 18-78 | SO₂Me | Me | Et | |
| 18-79 | SO₂Me | F | Et | |
| 18-80 | SO₂Me | Cl | Et | |
| 18-81 | SO₂Me | Br | Et | |
| 18-82 | SO₂Me | I | Et | |
| 18-83 | SO₂Me | CF₃ | Et | |
| 18-84 | SO₂Me | CHF₂ | Et | |
| 18-85 | SO₂Me | CF₂Cl | Et | |
| 18-86 | SO₂Me | OMe | Et | |
| 18-87 | SO₂Me | NO₂ | Et | |
| 18-88 | SO₂Me | SO₂Me | Et | |
| 18-89 | Me | Me | CH₂CH₂OMe | |
| 18-90 | Me | F | CH₂CH₂OMe | |
| 18-91 | Me | Cl | CH₂CH₂OMe | |
| 18-92 | Me | Br | CH₂CH₂OMe | |
| 18-93 | Me | I | CH₂CH₂OMe | |
| 18-94 | Me | CF₃ | CH₂CH₂OMe | |
| 18-95 | Me | CHF₂ | CH₂CH₂OMe | |
| 18-96 | Me | CF₂Cl | CH₂CH₂OMe | |
| 18-97 | Me | OMe | CH₂CH₂OMe | |
| 18-98 | Me | NO₂ | CH₂CH₂OMe | |
| 18-99 | Me | SO₂Me | CH₂CH₂OMe | |
| 18-100 | Cl | Me | CH₂CH₂OMe | |
| 18-101 | Cl | F | CH₂CH₂OMe | |
| 18-102 | Cl | Cl | CH₂CH₂OMe | |
| 18-103 | Cl | Br | CH₂CH₂OMe | |
| 18-104 | Cl | I | CH₂CH₂OMe | |
| 18-105 | Cl | CF₃ | CH₂CH₂OMe | |
| 18-106 | Cl | CHF₂ | CH₂CH₂OMe | |
| 18-107 | Cl | CF₂Cl | CH₂CH₂OMe | |
| 18-108 | Cl | OMe | CH₂CH₂OMe | |
| 18-109 | Cl | NO₂ | CH₂CH₂OMe | |
| 18-110 | Cl | SO₂Me | CH₂CH₂OMe | |
| 18-111 | OMe | Me | CH₂CH₂OMe | |
| 18-112 | OMe | F | CH₂CH₂OMe | |
| 18-113 | OMe | Cl | CH₂CH₂OMe | |
| 18-114 | OMe | Br | CH₂CH₂OMe | |
| 18-115 | OMe | I | CH₂CH₂OMe | |
| 18-116 | OMe | CF₃ | CH₂CH₂OMe | |
| 18-117 | OMe | CHF₂ | CH₂CH₂OMe | |
| 18-118 | OMe | CF₂Cl | CH₂CH₂OMe | |
| 18-119 | OMe | OMe | CH₂CH₂OMe | |
| 18-120 | OMe | NO₂ | CH₂CH₂OMe | |
| 18-121 | OMe | SO₂Me | CH₂CH₂OMe | |
| 18-122 | SO₂Me | Me | CH₂CH₂OMe | |
| 18-123 | SO₂Me | F | CH₂CH₂OMe | |
| 18-124 | SO₂Me | Cl | CH₂CH₂OMe | |
| 18-125 | SO₂Me | Br | CH₂CH₂OMe | |
| 18-126 | SO₂Me | I | CH₂CH₂OMe | |
| 18-127 | SO₂Me | CF₃ | CH₂CH₂OMe | |
| 18-128 | SO₂Me | CHF₂ | CH₂CH₂OMe | |
| 18-129 | SO₂Me | CF₂Cl | CH₂CH₂OMe | |
| 18-130 | SO₂Me | OMe | CH₂CH₂OMe | |
| 18-131 | SO₂Me | NO₂ | CH₂CH₂OMe | |
| 18-132 | SO₂Me | SO₂Me | CH₂CH₂OMe | |

**Tabelle 19: Erfindungsgemäße Verbindungen der allgemeinen Formel (II), worin Q* für Methoxy und R' für Cyano stehen, t für 1 steht, und die anderen Reste die in der Tabelle angegebenen Bedeutungen haben**

| | | | | |
|---|---|---|---|---|
| 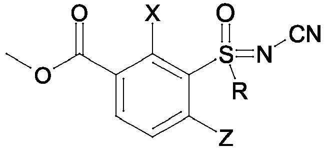 | | | | |

| Nr. | X | Z | R | Physikalische Daten (¹H-NMR) |
|---|---|---|---|---|
| 19-1 | Me | Me | Me | |
| 19-2 | Me | F | Me | |
| 19-3 | Me | Cl | Me | |
| 19-4 | Me | Br | Me | |
| 19-5 | Me | I | Me | |
| 19-6 | Me | CF₃ | Me | |
| 19-7 | Me | CHF₂ | Me | |
| 19-8 | Me | CF₂Cl | Me | |
| 19-9 | Me | OMe | Me | |
| 19-10 | Me | NO₂ | Me | |
| 19-11 | Me | SO₂Me | Me | |
| 19-12 | Cl | Me | Me | |
| 19-13 | Cl | F | Me | |
| 19-14 | Cl | Cl | Me | |
| 19-15 | Cl | Br | Me | |
| 19-16 | Cl | I | Me | |
| 19-17 | Cl | CF₃ | Me | |
| 19-18 | Cl | CHF₂ | Me | |
| 19-19 | Cl | CF₂Cl | Me | |
| 19-20 | Cl | OMe | Me | |
| 19-21 | Cl | NO₂ | Me | |
| 19-22 | Cl | SO₂Me | Me | |
| 19-23 | OMe | Me | Me | |
| 19-24 | OMe | F | Me | |
| 19-25 | OMe | Cl | Me | |
| 19-26 | OMe | Br | Me | |
| 19-27 | OMe | I | Me | |
| 19-28 | OMe | CF₃ | Me | |
| 19-29 | OMe | CHF₂ | Me | |
| 19-30 | OMe | CF₂Cl | Me | |
| 19-31 | OMe | OMe | Me | |
| 19-32 | OMe | NO₂ | Me | |
| 19-33 | OMe | SO₂Me | Me | |
| 19-34 | SO₂Me | Me | Me | |
| 19-35 | SO₂Me | F | Me | |
| 19-36 | SO₂Me | Cl | Me | |
| 19-37 | SO₂Me | Br | Me | |
| 19-38 | SO₂Me | I | Me | |
| 19-39 | SO₂Me | CF₃ | Me | |
| 19-40 | SO₂Me | CHF₂ | Me | |
| 19-41 | SO₂Me | CF₂Cl | Me | |
| 19-42 | SO₂Me | OMe | Me | |
| 19-43 | SO₂Me | NO₂ | Me | |
| 19-44 | SO₂Me | SO₂Me | Me | |
| 19-45 | Me | Me | Et | |
| 19-46 | Me | F | Et | |
| 19-47 | Me | Cl | Et | |
| 19-48 | Me | Br | Et | |
| 19-49 | Me | I | Et | |
| 19-50 | Me | CF₃ | Et | |
| 19-51 | Me | CHF₂ | Et | |
| 19-52 | Me | CF₂Cl | Et | |
| 19-53 | Me | OMe | Et | |
| 19-54 | Me | NO₂ | Et | |
| 19-55 | Me | SO₂Me | Et | |
| 19-56 | Cl | Me | Et | |
| 19-57 | Cl | F | Et | |
| 19-58 | Cl | Cl | Et | |
| 19-59 | Cl | Br | Et | |
| 19-60 | Cl | I | Et | |
| 19-61 | Cl | CF₃ | Et | |
| 19-62 | Cl | CHF₂ | Et | |
| 19-63 | Cl | CF₂Cl | Et | |
| 19-64 | Cl | OMe | Et | |
| 19-65 | Cl | NO₂ | Et | |
| 19-66 | Cl | SO₂Me | Et | |
| 19-67 | OMe | Me | Et | |
| 19-68 | OMe | F | Et | |
| 19-69 | OMe | Cl | Et | |
| 19-70 | OMe | Br | Et | |
| 19-71 | OMe | I | Et | |
| 19-72 | OMe | CF₃ | Et | |
| 19-73 | OMe | CHF₂ | Et | |
| 19-74 | OMe | CF₂Cl | Et | |
| 19-75 | OMe | OMe | Et | |
| 19-76 | OMe | NO₂ | Et | |
| 19-77 | OMe | SO₂Me | Et | |
| 19-78 | SO₂Me | Me | Et | |
| 19-79 | SO₂Me | F | Et | |
| 19-80 | SO₂Me | Cl | Et | |
| 19-81 | SO₂Me | Br | Et | |
| 19-82 | SO₂Me | I | Et | |
| 19-83 | SO₂Me | CF₃ | Et | |
| 19-84 | SO₂Me | CHF₂ | Et | |
| 19-85 | SO₂Me | CF₂Cl | Et | |
| 19-86 | SO₂Me | OMe | Et | |
| 19-87 | SO₂Me | NO₂ | Et | |
| 19-88 | SO₂Me | SO₂Me | Et | |
| 19-89 | Me | Me | CH₂CH₂OMe | |
| 19-90 | Me | F | CH₂CH₂OMe | |
| 19-91 | Me | Cl | CH₂CH₂OMe | |
| 19-92 | Me | Br | CH₂CH₂OMe | |
| 19-93 | Me | I | CH₂CH₂OMe | |
| 19-94 | Me | CF₃ | CH₂CH₂OMe | |
| 19-95 | Me | CHF₂ | CH₂CH₂OMe | |
| 19-96 | Me | CF₂Cl | CH₂CH₂OMe | |
| 19-97 | Me | OMe | CH₂CH₂OMe | |
| 19-98 | Me | NO₂ | CH₂CH₂OMe | |
| 19-99 | Me | SO₂Me | CH₂CH₂OMe | |
| 19-100 | Cl | Me | CH₂CH₂OMe | |
| 19-101 | Cl | F | CH₂CH₂OMe | |
| 19-102 | Cl | Cl | CH₂CH₂OMe | |
| 19-103 | Cl | Br | CH₂CH₂OMe | |
| 19-104 | Cl | I | CH₂CH₂OMe | |
| 19-105 | Cl | CF₃ | CH₂CH₂OMe | |
| 19-106 | Cl | CHF₂ | CH₂CH₂OMe | |
| 19-107 | Cl | CF₂Cl | CH₂CH₂OMe | |
| 19-108 | Cl | OMe | CH₂CH₂OMe | |
| 19-109 | Cl | NO₂ | CH₂CH₂OMe | |
| 19-110 | Cl | SO₂Me | CH₂CH₂OMe | |
| 19-111 | OMe | Me | CH₂CH₂OMe | |
| 19-112 | OMe | F | CH₂CH₂OMe | |
| 19-113 | OMe | Cl | CH₂CH₂OMe | |
| 19-114 | OMe | Br | CH₂CH₂OMe | |
| 19-115 | OMe | I | CH₂CH₂OMe | |
| 19-116 | OMe | CF₃ | CH₂CH₂OMe | |
| 19-117 | OMe | CHF₂ | CH₂CH₂OMe | |
| 19-118 | OMe | CF₂Cl | CH₂CH₂OMe | |
| 19-119 | OMe | OMe | CH₂CH₂OMe | |
| 19-120 | OMe | NO₂ | CH₂CH₂OMe | |
| 19-121 | OMe | SO₂Me | CH₂CH₂OMe | |
| 19-122 | SO₂Me | Me | CH₂CH₂OMe | |
| 19-123 | SO₂Me | F | CH₂CH₂OMe | |
| 19-124 | SO₂Me | Cl | CH₂CH₂OMe | |
| 19-125 | SO₂Me | Br | CH₂CH₂OMe | |
| 19-126 | SO₂Me | I | CH₂CH₂OMe | |
| 19-127 | SO₂Me | CF₃ | CH₂CH₂OMe | |
| 19-128 | SO₂Me | CHF₂ | CH₂CH₂OMe | |
| 19-129 | SO₂Me | CF₂Cl | CH₂CH₂OMe | |
| 19-130 | SO₂Me | OMe | CH₂CH₂OMe | |
| 19-131 | SO₂Me | NO₂ | CH₂CH₂OMe | |
| 19-132 | SO₂Me | SO₂Me | CH₂CH₂OMe | |

**Tabelle 20: Erfindungsgemäße Verbindungen der allgemeinen Formel (II), worin Q* für Methoxy und R' für Cyano stehen, t für 0 steht, und die anderen Reste die in der Tabelle angegebenen Bedeutungen haben**

| | | | | |
|---|---|---|---|---|
| 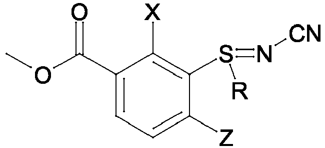 | | | | |

| Nr. | X | Z | R | Physikalische Daten (¹H-NMR) |
|---|---|---|---|---|
| 20-1 | Me | Me | Me | |
| 20-2 | Me | F | Me | |
| 20-3 | Me | Cl | Me | |
| 20-4 | Me | Br | Me | |
| 20-5 | Me | I | Me | |
| 20-6 | Me | CF₃ | Me | (400 MHz, CDCl₃ δ, ppm) 8.01 (d,1 H), 7.75 (d,1H), 3.98 (s,3H), 3.18 (s,3H), 3.08 (s,3H) |
| 20-7 | Me | CHF₂ | Me | |
| 20-8 | Me | CF₂Cl | Me | |
| 20-9 | Me | OMe | Me | |
| 20-10 | Me | NO₂ | Me | |
| 20-11 | Me | SO₂Me | Me | |
| 20-12 | Cl | Me | Me | |
| 20-13 | Cl | F | Me | |
| 20-14 | Cl | Cl | Me | |
| 20-15 | Cl | Br | Me | |
| 20-16 | Cl | I | Me | |
| 20-17 | Cl | CF₃ | Me | |
| 20-18 | Cl | CHF₂ | Me | |
| 20-19 | Cl | CF₂Cl | Me | |
| 20-20 | Cl | OMe | Me | |
| 20-21 | Cl | NO₂ | Me | |
| 20-22 | Cl | SO₂Me | Me | |
| 20-23 | OMe | Me | Me | |
| 20-24 | OMe | F | Me | |
| 20-25 | OMe | Cl | Me | |
| 20-26 | OMe | Br | Me | |
| 20-27 | OMe | I | Me | |
| 20-28 | OMe | CF₃ | Me | |
| 20-29 | OMe | CHF₂ | Me | |
| 20-30 | OMe | CF₂Cl | Me | |
| 20-31 | OMe | OMe | Me | |
| 20-32 | OMe | NO₂ | Me | |
| 20-33 | OMe | SO₂Me | Me | |
| 20-34 | SO₂Me | Me | Me | |
| 20-35 | SO₂Me | F | Me | |
| 20-36 | SO₂Me | Cl | Me | |
| 20-37 | SO₂Me | Br | Me | |
| 20-38 | SO₂Me | I | Me | |
| 20-39 | SO₂Me | CF₃ | Me | |
| 20-40 | SO₂Me | CHF₂ | Me | |
| 20-41 | SO₂Me | CF₂Cl | Me | |
| 20-42 | SO₂Me | OMe | Me | |
| 20-43 | SO₂Me | NO₂ | Me | |
| 20-44 | SO₂Me | SO₂Me | Me | |
| 20-45 | Me | Me | Et | |
| 20-46 | Me | F | Et | |
| 20-47 | Me | Cl | Et | |
| 20-48 | Me | Br | Et | |
| 20-49 | Me | I | Et | |
| 20-50 | Me | CF₃ | Et | |
| 20-51 | Me | CHF₂ | Et | |
| 20-52 | Me | CF₂Cl | Et | |
| 20-53 | Me | OMe | Et | |
| 20-54 | Me | NO₂ | Et | |
| 20-55 | Me | SO₂Me | Et | |
| 20-56 | Cl | Me | Et | |
| 20-57 | Cl | F | Et | |
| 20-58 | Cl | Cl | Et | |
| 20-59 | Cl | Br | Et | |
| 20-60 | Cl | I | Et | |
| 20-61 | Cl | CF₃ | Et | |
| 20-62 | Cl | CHF₂ | Et | |
| 20-63 | Cl | CF₂Cl | Et | |
| 20-64 | Cl | OMe | Et | |
| 20-65 | Cl | NO₂ | Et | |
| 20-66 | Cl | SO₂Me | Et | |
| 20-67 | OMe | Me | Et | |
| 20-68 | OMe | F | Et | |
| 20-69 | OMe | Cl | Et | |
| 20-70 | OMe | Br | Et | |
| 20-71 | OMe | I | Et | |
| 20-72 | OMe | CF₃ | Et | |
| 20-73 | OMe | CHF₂ | Et | |
| 20-74 | OMe | CF₂Cl | Et | |
| 20-75 | OMe | OMe | Et | |
| 20-76 | OMe | NO₂ | Et | |
| 20-77 | OMe | SO₂Me | Et | |
| 20-78 | SO₂Me | Me | Et | |
| 20-79 | SO₂Me | F | Et | |
| 20-80 | SO₂Me | Cl | Et | |
| 20-81 | SO₂Me | Br | Et | |
| 20-82 | SO₂Me | I | Et | |
| 20-83 | SO₂Me | CF₃ | Et | |
| 20-84 | SO₂Me | CHF₂ | Et | |
| 20-85 | SO₂Me | CF₂Cl | Et | |
| 20-86 | SO₂Me | OMe | Et | |
| 20-87 | SO₂Me | NO₂ | Et | |
| 20-88 | SO₂Me | SO₂Me | Et | |
| 20-89 | Me | Me | CH₂CH₂OMe | |
| 20-90 | Me | F | CH₂CH₂OMe | |
| 20-91 | Me | Cl | CH₂CH₂OMe | |
| 20-92 | Me | Br | CH₂CH₂OMe | |
| 20-93 | Me | I | CH₂CH₂OMe | |
| 20-94 | Me | CF₃ | CH₂CH₂OMe | |
| 20-95 | Me | CHF₂ | CH₂CH₂OMe | |
| 20-96 | Me | CF₂Cl | CH₂CH₂OMe | |
| 20-97 | Me | OMe | CH₂CH₂OMe | |
| 20-98 | Me | NO₂ | CH₂CH₂OMe | |
| 20-99 | Me | SO₂Me | CH₂CH₂OMe | |
| 20-100 | Cl | Me | CH₂CH₂OMe | |
| 20-101 | Cl | F | CH₂CH₂OMe | |
| 20-102 | Cl | Cl | CH₂CH₂OMe | |
| 20-103 | Cl | Br | CH₂CH₂OMe | |
| 20-104 | Cl | I | CH₂CH₂OMe | |
| 20-105 | Cl | CF₃ | CH₂CH₂OMe | |
| 20-106 | Cl | CHF₂ | CH₂CH₂OMe | |
| 20-107 | Cl | CF₂Cl | CH₂CH₂OMe | |
| 20-108 | Cl | OMe | CH₂CH₂OMe | |
| 20-109 | Cl | NO**₂** | CH₂CH₂OMe | |
| 20-110 | Cl | SO₂Me | CH₂CH₂OMe | |
| 20-111 | OMe | Me | CH₂CH₂OMe | |
| 20-112 | OMe | F | CH₂CH₂OMe | |
| 20-113 | OMe | Cl | CH₂CH₂OMe | |
| 20-114 | OMe | Br | CH₂CH₂OMe | |
| 20-115 | OMe | I | CH₂CH₂OMe | |
| 20-116 | OMe | CF₃ | CH₂CH₂OMe | |
| 20-117 | OMe | CHF₂ | CH₂CH₂OMe | |
| 20-118 | OMe | CF₂Cl | CH₂CH₂0Me | |
| 20-119 | OMe | OMe | CH₂CH₂OMe | |
| 20-120 | OMe | NO₂ | CH₂CH₂OMe | |
| 20-121 | OMe | SO₂Me | CH₂CH₂OMe | |
| 20-122 | SO₂Me | Me | CH₂CH₂OMe | |
| 20-123 | SO₂Me | F | CH₂CH₂OMe | |
| 20-124 | SO₂Me | Cl | CH₂CH₂OMe | |
| 20-125 | SO₂Me | Br | CH₂CH₂OMe | |
| 20-126 | SO₂Me | I | CH₂CH₂OMe | |
| 20-127 | SO₂Me | CF₃ | CH₂CH₂OMe | |
| 20-128 | SO₂Me | CHF₂ | CH₂CH₂OMe | |
| 20-129 | SO₂Me | CF₂Cl | CH₂CH₂OMe | |
| 20-130 | SO₂Me | OMe | CH₂CH₂OMe | |
| 20-131 | SO₂Me | NO₂ | CH₂CH₂OMe | |
| 20-132 | SO₂Me | SO₂Me | CH₂CH₂OMe | |

### B. Formulierungsbeispiele

a) Ein Stäubemittel wird erhalten, indem man 10 Gew.-Teile einer Verbindung der Formel (I) und/oder deren Salze und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I) und/oder deren Salze, 64 Gew.-Teile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gew.-Teile einer Verbindung der Formel (I) und/oder deren Salze mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z.B. ca. 255 bis über 277 C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I) und/oder deren Salze, 75 Gew.-Teilen Cyclohexanon als Lösungsmittel und 10 Gew.-Teilen oxethyliertes Nonylphenol als Emulgator.
e) Ein in Wasser dispergierbares Granulat wird erhalten indem man
   75 Gew.-Teile einer Verbindung der Formel (I) und/oder deren Salze,
   10 Gew.-Teile ligninsulfonsaures Calcium,
   5 Gew.-Teile Natriumlaurylsulfat,
   3 Gew.-Teile Polyvinylalkohol und
   7 Gew.-Teile Kaolin
   mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man 25 Gew.-Teile einer Verbindung der Formel (I) und/oder deren Salze, 5 Gew.-Teile 2,2'-dinaphthylmethan-6,6'-disulfonsaures Natrium
   2 Gew.-Teile oleoylmethyltaurinsaures Natrium,
   1 Gew.-Teil Polyvinylalkohol,
   17 Gew.-Teile Calciumcarbonat und
   50 Gew.-Teile Wasser
   auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung gegen Schadpflanzen im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) oder als Emulsionskonzentrate (EC) formulierten erfindungsgemäßen Verbindungen werden dann als wäßrige Suspension bzw. Emulsion mit einer Wasseraufwandmenge von umgerechnet 600 bis 800 I/ha unter Zusatz von 0,2% Netzmittel auf die Oberfläche der Abdeckerde appliziert. Nach der Behandlung werden die Töpfe im Gewächshaus aufgestellt und unter guten Wachstumsbedingungen für die Testpflanzen gehalten. Die visuelle Bonitur der Schäden an den Versuchspflanzen erfolgt nach einer Versuchszeit von 3 Wochen im Vergleich zu unbehandelten Kontrollen (herbizide Wirkung in Prozent (%): 100% Wirkung = Pflanzen sind abgestorben, 0 % Wirkung = wie Kontrollpflanzen). Dabei zeigen beispielsweise die Verbindungen Nr. 3-160 und 4-160 bei einer Aufwandmenge von 320 g/ha jeweils eine mindestens 80%ige Wirkung gegen Abutilon theophrasti und Amaranthus retroflexus. Die Verbindungen Nr. 2-160 und 10a-160 zeigen bei einer Aufwandmenge von 320 g/ha jeweils eine mindestens 80%ige Wirkung gegen Polygonum convolvulus und Stellaria media.

### 2. Herbizide Wirkung gegen Schadpflanzen im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) oder als Emulsionskonzentrate (EC) formulierten erfindungsgemäßen Verbindungen werden dann als wäßrige Suspension bzw.

Emulsion mit einer Wasseraufwandmenge von umgerechnet 600 bis 800 I/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen). Dabei zeigen beispielsweise die Verbindungen Nr. 2-160, 3-160, 4-160 und 10a-160 bei einer Aufwandmenge von 80 g/ha jeweils eine mindestens 80%ige Wirkung gegen Abutilon theophrasti und Echinochloa crus galli.

## Patentansprüche

1. Sulfinimidoyl- und Sulfonimidoylbenzoylderivate der Formel (I) oder deren Salze 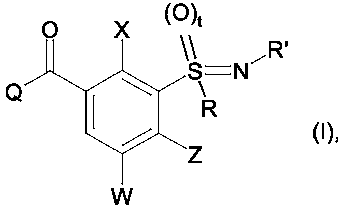 worin
Q bedeutet einen Rest Q1, Q2, Q3, Q4 oder Q5, 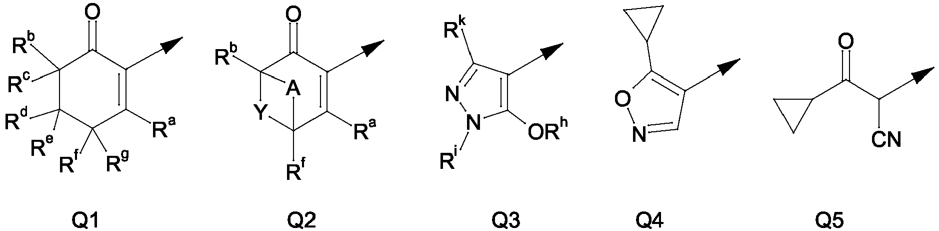
R^{a} bedeutet Hydroxy, R⁶S, R⁷(R⁸)N,
R^{b}, R^{c}, R^{f} und R^{g} bedeuten unabhängig voneinander jeweils Wasserstoff oder (C₁-C₄)-Alkyl,
R^{d}, R^{e} bedeuten unabhängig voneinander jeweils Wasserstoff oder (C₁-C₄)-Alkyl oder bilden gemeinsam mit dem Kohlenstoffatom, an das sie gebunden sind, eine Carbonylgruppe,
R^{h} bedeutet Wasserstoff, (C₁-C₆)-Alkylsulfonyl, (C₁-C₄)-Alkoxy-(C₁-C₆)-alkylsulfonyl, Phenylsulfonyl, Thiophenyl-2-sulfonyl, Benzoyl, Benzoyl-(C₁-C₆)-alkyl, Benzyl, wobei die fünf letztgenannten Reste gegebenenfalls einfach oder mehrfach durch Halogen, (C₁-C₄)-Alkyl oder (C₁-C₄)-Alkoxy substituiert sein können,
Rⁱ bedeutet (C₁-C₄)-Alkyl,
R^{k} bedeutet Wasserstoff, (C₁-C₄)-Alkyl, (C₃-C₇)-Cycloalkyl,
A und Y bedeuten unabhängig voneinander jeweils Sauerstoff, S(O)ₙ, N(R³), Carbonyl oder durch n Reste R⁹ substituiertes (C₁-C₄)-Alkylen, das durch n Elemente aus der Gruppe bestehend aus Sauerstoff, S(O)ₙ, N(R³) und Carbonyl unterbrochen ist,
X bedeutet Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkenyl, Halogen-(C₃-C₆)-cycloalkenyl, (C₃-C₆)-Cycloalkyl-(C₁-C₆)-alkyl, Halogen-(C₃-C₆)-cycloalkyl-(C₁-C₆)-alkyl, (C₃-C₆)-Cycloalkenyl-(C₁-C₆)-alkyl, Halogen-(C₃-C₆)-cycloalkenyl-(C₁-C₆)-alkyl, R¹(O)C, R¹(R¹ON=)C, R¹O(O)C, (R¹)₂N(O)C, R¹(R¹O)N(O)C, (R¹)₂N(R¹)N(O)C, R¹(O)C(R¹)N(O)C, R²O(O)C(R¹)N(O)C, (R¹)₂N(O)C(R¹)N(O)C, R²(O)₂S(R¹)N(O)C, R¹O(O)₂S(R¹)N(O)C, (R¹)₂N(O)₂S(R¹)N(O)C, R¹O, R¹(O)CO, R²(O)₂SO, R²O(O)CO, (R¹)₂N(O)CO, (R¹)₂N, R¹(O)C(R¹)N, R²(O)₂S(R¹)N, R²O(O)C(R¹)N, (R¹)₂N(O)C(R¹)N, R¹O(O)₂S(R¹)N, (R¹)₂N(O)₂S(R¹)N, R²(O)ₙS, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S, (R⁵O)₂(O)P, R¹(O)C-(C₁-C₆)-Alkyl, R¹O(O)C-(C₁-C₆)-Alkyl, (R¹)₂N(O)C-(C₁-C₆)-Alkyl, (R¹O)(R¹)N(O)C-(C₁-C₆)-Alkyl, (R¹)₂N(R¹)N(O)C-(C₁-C₆)-Alkyl, R¹(O)C(R¹)N(O)C-(C₁-C₆)-Alkyl, R²O(O)C(R¹)N(O)C-(C₁-C₆)-Alkyl, (R¹)₂N(O)C(R¹)N(O)C-(C₁-C₆)-Alkyl, R²(O)₂S(R¹)N(O)C-(C₁-C₆)-Alkyl, R¹O(O)₂S(R¹)N(O)C-(C₁-C₆)-Alkyl, (R¹)₂N(O)₂S(R¹)N(O)C-(C₁-C₆)-Alkyl, NC-(C₁-C₆)-Alkyl, R¹O-(C₁-C₆)-Alkyl, R¹(O)CO-(C₁-C₆)-Alkyl, R²(O)₂SO-(C₁-C₆)-Alkyl, R²O(O)CO-(C₁-C₆)-Alkyl, (R¹)₂N(O)CO-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R²O(O)C(R¹)N-(C₁-C₆)-Alkyl, (R¹)₂N(O)C(R¹)N-(C₁-C₆)-Alkyl, R¹O(O)₂S(R¹)N-(C₁-C₆)-Alkyl, (R¹)₂N(O)₂S(R¹)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, R¹(O)C(R¹)N(O)₂S-(C₁-C₆)-Alkyl, R²O(O)C(R¹)N(O)₂S-(C₁-C₆)-Alkyl, (R¹)₂N(O)C(R¹)N(O)₂S-(C₁-C₆)-Alkyl, (R⁵O)₂(O)P-(C₁-C₆)-Alkyl, Phenyl, Heteroaryl, Heterocyclyl, Phenyl-(C₁-C₆)-alkyl, Heteroaryl-(C₁-C₆)-alkyl, Heterocyclyl-(C₁-C₆)-alkyl, wobei die sechs letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹O(O)C, (R¹)₂N(O)C, R¹O, (R¹)₂N, R₂(O)ₙS, R¹O(O)₂S, (R¹)₂N(O)₂S und R¹O-(C₁-C₆)-Alkyl substituiert sind, und wobei Heterocyclyl n Oxogruppen trägt,
Z bedeutet Wasserstoff, Nitro, Halogen, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkenyl, Halogen-(C₃-C₆)-cycloalkenyl, (C₃-C₆)-Cycloalkyl-(C₁-C₆)-alkyl, Halogen-(C₃-C₆)-cycloalkyl-(C₁-C₆)-alkyl, (C₃-C₆)-Cycloalkenyl-(C₁-C₆)-alkyl, Halogen-(C₃-C₆)-cycloalkenyl-(C₁-C₆)-alkyl, R¹(O)C, R¹(R¹ON=)C, R¹O(O)C, (R¹)₂N(O)C, R¹(R¹O)N(O)C, (R¹)₂N(R¹)N(O)C, R¹(O)C(R¹)N(O)C, R²O(O)C(R¹)N(O)C, (R¹)₂N(O)C(R¹)N(O)C, R²(O)₂S(R¹)N(O)C, R¹O(O)₂S(R¹)N(O)C, (R¹)₂N(O)₂S(R¹)N(O)C, R¹O, R¹(O)CO, R²(O)₂SO, R²O(O)CO, (R¹)₂N(O)CO, (R¹)₂N, R¹(O)C(R¹)N, R²(O)₂S(R¹)N, R²O(O)C(R¹)N, (R¹)₂N(O)C(R¹)N, R¹O(O)₂S(R¹)N, (R¹)₂N(O)₂S(R¹)N, R²(O)ₙS, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S, (R⁵O)₂(O)P, R¹(O)C-(C₁-C₆)-Alkyl, R¹O(O)C-(C₁-C₆)-Alkyl, (R¹)₂N(O)C-(C₁-C₆)-Alkyl, (R¹O)(R¹)N(O)C-(C₁-C₆)-Alkyl, (R¹)₂N(R¹)N(O)C-(C₁-C₆)-Alkyl, R¹(O)C(R¹)N(O)C-(C₁-C₆)-Alkyl, R²O(O)C(R¹)N(O)C-(C₁-C₆)-Alkyl, (R¹)₂N(O)C(R¹)N(O)C-(C₁-C₆)-Alkyl, R²(O)₂S(R¹)N(O)C-(C₁-C₆)-Alkyl, R¹O(O)₂S(R¹)N(O)C-(C₁-C₆)-Alkyl, (R¹)₂N(O)₂S(R¹)N(O)C-(C₁-C₆)-Alkyl, NC-(C₁-C₆)-Alkyl, R¹O-(C₁-C₆)-Alkyl, R¹(O)CO-(C₁-C₆)-Alkyl, R²(O)₂SO-(C₁-C₆)-Alkyl, R²O(O)CO-(C₁-C₆)-Alkyl, (R¹)₂N(O)CO-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R²O(O)C(R¹)N-(C₁-C₆)-Alkyl, (R¹)₂N(O)C(R¹)N-(C₁-C₆)-Alkyl, R¹O(O)₂S(R¹)N-(C₁-C₆)-Alkyl, (R¹)₂N(O)₂S(R¹)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, R¹(O)C(R¹)N(O)₂S-(C₁-C₆)-Alkyl, R²O(O)C(R¹)N(O)₂S-(C₁-C₆)-Alkyl, (R¹)₂N(O)C(R¹)N(O)₂S-(C₁-C₆)-Alkyl, (R⁵O)₂(O)P-(C₁-C₆)-Alkyl, Phenyl, Heteroaryl, Heterocyclyl, Phenyl-(C₁-C₆)-alkyl, Heteroaryl-(C₁-C₆)-alkyl, Heterocyclyl-(C₁-C₆)-alkyl, wobei die sechs letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl substituiert sind, und wobei Heterocyclyl n Oxogruppen trägt,
W bedeutet Wasserstoff, Halogen, Nitro, Cyano, Rhodano, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₇)-Cycloalkyl, Halogen-(C₃-C₇)-cycloalkyl, (C₁-C₆)-Alkoxy, Halogen-(C₁-C₆)-alkoxy, (C₁-C₆)-Alkyl-(O)ₙS-, (C₁-C₆)-Halogenalkyl-(O)ₙS-, (C₁-C₆)-Alkoxy-(C₁-C₄)-alkyl, (C₁-C₆)-Alkoxy-(C₁-C₄)-halogenalkyl, R¹(O)C, R¹(R¹ON=)C, R¹O(O)C, (R¹)₂N, R¹(O)C(R¹)N oder R²(O)₂S(R¹)N,
R bedeutet jeweils durch s Reste aus der Gruppe bestehend aus Nitro, Halogen, Cyano, Rhodano, (C₃-C₆)-Cycloalkyl, R¹(O)C, R¹(R¹ON=)C, R¹O(O)C, (R¹)₂N(O)C, R¹(R¹O)N(O)C, R²(O)₂S(R¹)N(O)C, R¹O(O)₂S(R¹)N(O)C, (R¹)₂N(O)₂S(R¹)N(O)C, R¹S(O)C, R¹O, R¹(O)CO, R²(O)₂SO, R²O(O)CO, (R¹)₂N(O)CO, (R¹)₂N, R¹O(R¹)N, R¹(O)C(R¹)N, R²(O)₂S(R¹)N, R²O(O)C(R¹)N, (R¹)₂N(O)C(R¹)N, R¹O(O)₂S(R¹)N, (R¹)₂N(O)₂S(R¹)N, R²(O)ₙS, R¹C(O)S, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S und (R⁵O)₂(O)P substituiertes (C₁-C₆)-Alkyl, (C₂-C₆)-Alkenyl oder (C₂-C₆)-Alkinyl, oder
jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹(O)C, R¹(R¹ON=)C, R¹O(O)C, (R¹)₂N(O)C, R¹(R¹O)N(O)C, R²(O)₂S(R¹)N(O)C, R¹O(O)₂S(R¹)N(O)C, (R¹)₂N(O)₂S(R¹)N(O)C, R¹S(O)C, R¹O, R¹(O)CO, R²(O)₂SO, R²O(O)CO, (R¹)₂N(O)CO, (R¹)₂N, R¹O(R¹)N, R¹(O)C(R¹)N, R²(O)₂S(R¹)N, R²O(O)C(R¹)N, (R¹)₂N(O)C(R¹)N, R¹O(O)₂S(R¹)N, (R¹)₂N(O)₂S(R¹)N, R²(O)ₙS, R¹C(O)S, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S, (R⁵O)₂(O)P und R¹O-(C₁-C₆)-Alkyl im cyclischen Teil substituiertes (C₃-C₆)-Cycloalkyl, (C₃-C₆)-Cycloalkenyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, Phenyl-N(R¹)-(C₁-C₆)-alkyl, Heteroaryl- N(R¹)-(C₁-C₆)-alkyl, Heterocyclyl- N(R¹)-(C₁-C₆)-alkyl, Phenyl-S(O)ₙ-(C₁-C₆)-alkyl, Heteroaryl-S(O)ₙ-(C₁-C₆)-alkyl oder Heterocyclyl-S(O)ₙ-(C₁-C₆)-alkyl, und wobei Heterocyclyl n Oxogruppen trägt,
R' bedeutet Wasserstoff, Nitro, Halogen, Cyano, (C₁-C₆)-Alkyl, Halogen-(C₁-C₆)-alkyl, (C₃-C₆)-Alkenyl, Halogen-(C₃-C₆)-alkenyl, (C₂-C₆)-Alkinyl, Halogen-(C₃-C₆)-alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C, R²O(O)C, (R¹)₂N(O)C, R²S(O)C, (R¹)₂N(S)C, R¹(R¹O)N(O)C, R²(O)₂S(R¹)N(O)C, (R¹)₂N(O)₂S(R¹)N(O)C, R¹O, (R¹)₂N, R²(O)ₙS, (R²)₃Si-(C₁-C₆)-Alkyl-(O)ₙS, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S, R²(O)₂S(R¹)N(O)₂S, (R⁵O)₂(O)P, (R²)₃Si, R¹(O)C-(C₁-C₆)-Alkyl, R¹O(O)C-(C₁-C₆)-Alkyl, (R¹)₂N(O)C-(C₁-C₆)-Alkyl, (R¹O)(R¹)N(O)C-(C₁-C₆)-Alkyl, R²(O)₂S(R¹)N(O)C-(C₁-C₆)-Alkyl, R¹O(O)₂S(R¹)N(O)C-(C₁-C₆)-Alkyl, (R¹)₂N(O)₂S(R¹)N(O)C-(C₁-C₆)-Alkyl, R¹O-(C₁-C₆)-Alkyl, R¹(O)CO-(C₁-C₆)-Alkyl, R²(O)₂SO-(C₁-C₆)-Alkyl, R²O(O)CO-(C₁-C₆)-Alkyl, (R¹)₂N(O)CO-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R²O(O)C(R1)N-(C₁-C₆)-Alkyl, (R¹)₂N(O)C(R¹)N-(C₁-C₆)-Alkyl, R¹O(O)₂S(R¹)N-(C₁-C₆)-Alkyl, (R¹)₂N(O)₂S(R¹)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, R¹(O)C(R¹)N(O)₂S-(C₁-C₆)-Alkyl, R²O(O)C(R¹)N(O)₂S-(C₁-C₆)-Alkyl, (R¹)₂N(O)C(R¹)N(O)₂S-(C₁-C₆)-Alkyl, (R⁵O)₂(O)P-(C₁-C₆)-Alkyl, (R²)₃Si-(C₁-C₆)-Alkyl, Phenyl, Heteroaryl, Heterocyclyl, Phenyl-(C₁-C₆)-alkyl, Heteroaryl-(C₁-C₆)-alkyl, Heterocyclyl-(C₁-C₆)-alkyl, wobei die sechs vorstehend genannten Phenyl-, Heteroaryl- und Heterocyclylreste im cyclischen Teil jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R¹O(O)C, (R¹)₂N(O)C, R¹O, (R¹)₂N, R²(O)ₙS, R¹O(O)₂S, (R¹)₂N(O)₂S und R¹O-(C₁-C₆)-Alkyl substituiert sind, und wobei Heterocyclyl n Oxogruppen trägt,
R¹ bedeutet Wasserstoff, (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, Phenyl-N(R³)-(C₁-C₆)-alkyl, Heteroaryl-N(R³)-(C₁-C₆)-alkyl, Heterocyclyl-N(R³)-(C₁-C₆)-alkyl, Phenyl-S(O)ₙ-(C₁-C₆)-alkyl, Heteroaryl-S(O)ₙ-(C₁-C₆)-alkyl, Heterocyclyl-S(O)ₙ-(C₁-C₆)-alkyl, wobei die fünfzehn letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R³O(O)C, (R³)₂N(O)C, R³O, (R³)₂N, R⁴(O)ₙS, R³O(O)₂S, (R³)₂N(O)₂S und R³O-(C₁-C₆)-Alkyl substituiert sind, und wobei Heterocyclyl n Oxogruppen trägt,
R² bedeutet (C₁-C₆)-Alkyl, Halogen-(C₁-C₆)-alkyl, (C₂-C₆)-Alkenyl, Halogen-(C₂-C₆)-alkenyl, (C₂-C₆)-Alkinyl, Halogen-(C₃-C₆)-alkinyl, (C₃-C₆)-Cycloalkyl, (C₃-C₆)-Cycloalkenyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, Phenyl-N(R³)-(C₁-C₆)-alkyl, Heteroaryl-N(R³)-(C₁-C₆)-alkyl, Heterocyclyl-N(R³)-(C₁-C₆)-alkyl, Phenyl-S(O)ₙ-(C₁-C₆)-alkyl, Heteroaryl-S(O)ₙ-(C₁-C₆)-alkyl, Heterocyclyl-S(O)ₙ-(C₁-C₆)-alkyl, wobei die fünfzehn letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, (C₃-C₆)-Cycloalkyl, R³O(O)C, (R³)₂N(O)C, R³O, (R³)₂N, R⁴(O)ₙS, R³O(O)₂S, (R³)₂N(O)₂S und R³O-(C₁-C₆)-Alkyl substituiert sind, und wobei Heterocyclyl n Oxogruppen trägt,
R³ bedeutet Wasserstoff, (C₁-C₆)-Alkyl, Halogen-(C₁-C₆)-alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl,
R⁴ bedeutet (C₁-C₆)-Alkyl, Halogen-(C₁-C₆)-alkyl, (C₂-C₆)-Alkenyl, (C₂-C₆)-Alkinyl, (C₃-C₆)-Cycloalkyl, (C₃-C₆)-Cycloalkyl-(C₁-C₆)-alkyl oder Phenyl,
R⁵ bedeutet Wasserstoff oder (C₁-C₄)-Alkyl,
R⁶ bedeutet (C₁-C₄)-Alkyl oder durch m Reste aus der Gruppe bestehend aus Halogen, Nitro, Cyano, (C₁-C₄)-Alkyl, (C₁-C₄)-Halogenalkyl, (C₁-C₄)-Alkoxy und (C₁-C₄)-Halogenalkoxy substituiertes Phenyl,
R⁷ bedeutet Wasserstoff, (C₁-C₄)-Alkyl oder (C₁-C₄)-Alkoxy,
R⁸ bedeutet Wasserstoff oder (C₁-C₄)-Alkyl,
oder
R⁷ und R⁸ bilden gemeinsam mit dem Stickstoffatom, an das sie gebunden sind, einen 5- oder 6-gliedrigen gesättigten, partiell gesättigten oder ungesättigten Ring, der zusätzlich n Heteroatome aus der Gruppe bestehend aus Stickstoff, Sauerstoff und Schwefel enthält und der durch m Reste aus der Gruppe bestehend aus Halogen, Cyano, (C₁-C₄)-Alkyl, (C₁-C₄)-Halogenalkyl, (C₁-C₄)-Alkoxy und (C₁-C₄)-Halogenalkoxy substituiert ist,
R⁹ bedeutet Halogen, (C₁-C₄)-Alkyl, (C₁-C₄)-Halogenalkyl, (C₁-C₄)-Alkoxy, (C₁-C₄)-Halogenalkoxy oder (C₁-C₄)-Alkoxy-(C₁-C₄)-alkyl,
m bedeutet 0, 1, 2, 3, 4 oder 5,
n bedeutet 0, 1 oder 2,
s bedeutet 0, 1, 2 oder 3,
t bedeutet 0 oder 1.

2. Sulfinimidoyl- und Sulfonimidoylbenzoylderivate nach Anspruch 1, worin
Q bedeutet einen Rest Q1, Q2, Q3, Q4 oder Q5, 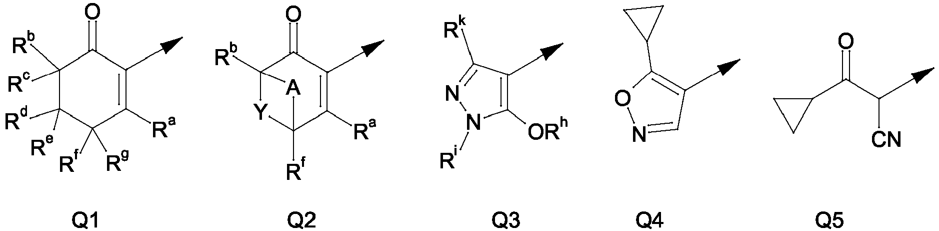
R^{a} bedeutet Hydroxy,
R^{b}, R^{c}, R^{f} und R^{g} bedeuten unabhängig voneinander jeweils Wasserstoff oder (C₁-C₄)-Alkyl,
R^{d}, R^{e} bedeuten unabhängig voneinander jeweils Wasserstoff oder (C₁-C₄)-Alkyl oder bilden gemeinsam mit dem Kohlenstoffatom, an das sie gebunden sind, eine Carbonylgruppe,
R^{h} bedeutet Wasserstoff,
Rⁱ bedeutet (C₁-C₄)-Alkyl,
R^{k} bedeutet Wasserstoff, (C₁-C₄)-Alkyl, (C₃-C₇)-Cycloalkyl,
A und Y bedeuten unabhängig voneinander jeweils Sauerstoff oder durch n Reste R⁹ substituiertes (C₁-C₄)-Alkylen,
X bedeutet Nitro, Halogen, Cyano, (C₁-C₆)-Alkyl, Halogen-(C₁-C₆)-alkyl, (C₂-C₆)-Alkenyl, (C₂-C₆)-Alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C, R¹(R¹ON=)C, R¹O(O)C, (R¹)₂N(O)C, R¹O, (R¹)₂N, R¹(O)C(R¹)N, R²(O)₂S(R¹)N, R²O(O)C(R¹)N, (R¹)₂N(O)C(R¹)N, R²(O)ₙS, R¹O(O)₂S, (R¹)₂N(O)₂S, (R⁵O)₂(O)P, R¹(O)C-(C₁-C₆)-Alkyl, R¹O(O)C-(C₁-C₆)-Alkyl, (R¹)₂N(O)C-(C₁-C₆)-Alkyl, NC-(C₁-C₆)-Alkyl, R¹O-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R²O(O)C(R¹)N-(C₁-C₆)-Alkyl, (R¹)₂N(O)C(R¹)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, (R⁵O)₂(O)P-(C₁-C₆)-Alkyl, Phenyl, Heteroaryl, Heterocyclyl, Phenyl-(C₁-C₆)-alkyl, Heteroaryl-(C₁-C₆)-alkyl, Heterocyclyl-(C₁-C₆)-alkyl, wobei die sechs letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, R¹O, (R¹)₂N, R²(O)ₙS, R¹O(O)₂S, (R¹)₂N(O)₂S und R¹O-(C₁-C₆)-Alkyl substituiert sind und wobei Heterocyclyl n Oxogruppen trägt,
Z bedeutet Wasserstoff, Nitro, Halogen, Cyano, (C₁-C₆)-Alkyl, Halogen-(C₁-C₆)-alkyl, (C₂-C₆)-Alkenyl, (C₂-C₆)-Alkinyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C, R¹(R¹ON=)C, R¹O(O)C, (R¹)₂N(O)C, R¹O, (R¹)₂N, R¹(O)C(R¹)N, R²(O)₂S(R¹)N, R²O(O)C(R¹)N, (R¹)₂N(O)C(R¹)N, R²(O)ₙS, R¹O(O)₂S, (R¹)₂N(O)₂S, (R⁵O)₂(O)P, R¹(O)C-(C₁-C₆)-Alkyl, R¹O(O)C-(C₁-C₆)-Alkyl, (R¹)₂N(O)C-(C₁-C₆)-Alkyl, NC-(C₁-C₆)-Alkyl, R¹O-(C₁-C₆)-Alkyl, (R¹)₂N-(C₁-C₆)-Alkyl, R¹(O)C(R¹)N-(C₁-C₆)-Alkyl, R²(O)₂S(R¹)N-(C₁-C₆)-Alkyl, R²O(O)C(R¹)N-(C₁-C₆)-Alkyl, (R¹)₂N(O)C(R¹)N-(C₁-C₆)-Alkyl, R²(O)ₙS-(C₁-C₆)-Alkyl, R¹O(O)₂S-(C₁-C₆)-Alkyl, (R¹)₂N(O)₂S-(C₁-C₆)-Alkyl, (R⁵O)₂(O)P-(C₁-C₆)-Alkyl, Phenyl, Heteroaryl, Heterocyclyl, Phenyl-(C₁-C₆)-alkyl, Heteroaryl-(C₁-C₆)-alkyl, Heterocyclyl-(C₁-C₆)-alkyl, wobei die sechs letztgenannten Reste jeweils durch s Reste aus der Gruppe bestehend aus Nitro, Halogen, Cyano, Rhodano, (C₁-C₆)-Alkyl, Halogen-(C₁-C₆)-alkyl, R¹O, (R¹)₂N, R²(O)ₙS, R¹O(O)₂S, (R¹)₂N(O)₂S und R¹O-(C₁-C₆)-Alkyl substituiert sind und wobei Heterocyclyl n Oxogruppen trägt,
W bedeutet Wasserstoff, Halogen, Nitro, Cyano, (C₁-C₆)-Alkyl, Halogen-(C₁-C₆)-alkyl, (C₃-C₇)-Cycloalkyl, (C₁-C₆)-Alkoxy, (C₁-C₆)-Alkyl-(O)ₙS-, R¹O(O)C, (R¹)₂N, R¹(O)C(R¹)N oder R²(O)₂S(R¹)N,
R bedeutet jeweils durch s Reste aus der Gruppe bestehend aus Halogen, Cyano, (C₃-C₆)-Cycloalkyl, R¹(O)C, R¹(R¹ON=)C, R¹O(O)C, (R¹)₂N(O)C, R²(O)₂S(R¹)N(O)C, R¹O, (R¹)₂N, R¹(O)C(R¹)N, R²(O)₂S(R¹)N, R²O(O)C(R¹)N, (R¹)₂N(O)C(R¹)N, R²(O)ₙS, R¹O(O)₂S, (R¹)₂N(O)₂S, R¹(O)C(R¹)N(O)₂S, R²O(O)C(R¹)N(O)₂S und (R¹)₂N(O)C(R¹)N(O)₂S substituiertes (C₁-C₆)-Alkyl oder
jeweils durch s Reste aus der Gruppe bestehend aus Halogen, (C₁-C₆)-Alkyl, Halogen-(C₁-C₆)-alkyl, (C₃-C₆)-Cycloalkyl, R¹O(O)C und (R¹)₂N(O)C substituiertes (C₃-C₆)-Cycloalkyl,
R' bedeutet Wasserstoff, Nitro, Cyano, (C₁-C₆)-Alkyl, Halogen-(C₁-C₆)-alkyl, (C₃-C₆)-Cycloalkyl, Halogen-(C₃-C₆)-cycloalkyl, (C₃-C₆)-Cycloalkyl-(C₁-C₆)-alkyl, Halogen-(C₃-C₆)-cycloalkyl-(C₁-C₆)-alkyl, R¹(O)C, R²O(O)C, (R¹)₂N(O)C, R²(O)₂S, R¹(O)C-(C₁-C₆)-Alkyl, R¹O(O)C-(C₁-C₆)-Alkyl, (R¹)₂N(O)C-(C₁-C₆)-Alkyl, R¹O-(C₁-C₆)-Alkyl, (R¹)₂N-(C₁-C₆)-Alkyl, R²(O)ₙS-(C₁-C₆)-Alkyl,
R¹ bedeutet Wasserstoff, (C₁-C₆)-Alkyl, Halogen-(C₁-C₆)-alkyl, (C₃-C₆)-Cycloalkyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, wobei die neun letztgenannten Reste jeweils durch s Reste aus der Gruppe bestehend aus Nitro, Halogen, (C₁-C₆)-Alkyl, Halogen-(C₁-C₆)-alkyl, R³O(O)C, (R³)₂N(O)C, R³O, (R³)₂N, R⁴(O)ₙS und R³O-(C₁-C₆)-Alkyl substituiert sind, und wobei Heterocyclyl n Oxogruppen trägt,
R² bedeutet (C₁-C₆)-Alkyl, Halogen-(C₁-C₆)-alkyl, (C₃-C₆)-Cycloalkyl, Halogen-(C₃-C₆)-cycloalkyl, (C₃-C₆)-Cycloalkyl-(C₁-C₆)-alkyl, (C₁-C₆)-Alkyl-O-(C₁-C₆)-alkyl, Cycloalkyl-(C₁-C₆)-alkyl-O-(C₁-C₆)-alkyl, Phenyl, Phenyl-(C₁-C₆)-alkyl, Heteroaryl, Heteroaryl-(C₁-C₆)-alkyl, Heterocyclyl, Heterocyclyl-(C₁-C₆)-alkyl, Phenyl-O-(C₁-C₆)-alkyl, Heteroaryl-O-(C₁-C₆)-alkyl, Heterocyclyl-O-(C₁-C₆)-alkyl, wobei die neun letztgenannten Reste jeweils durch s Reste aus der Gruppe bestehend aus Nitro, Halogen, (C₁-C₆)-Alkyl, Halogen-(C₁-C₆)-alkyl, R³O(O)C, (R³)₂N(O)C, R³O (R³)₂N, R⁴(O)ₙS und R³O-(C₁-C₆)-Alkyl substituiert sind, und wobei Heterocyclyl n Oxogruppen trägt,
R³ bedeutet Wasserstoff oder (C₁-C₆)-Alkyl,
R⁴ bedeutet (C₁-C₆)-Alkyl,
R⁵ bedeutet Wasserstoff oder (C₁-C₄)-Alkyl,
R⁹ bedeutet Halogen, (C₁-C₄)-Alkyl, (C₁-C₄)-Halogenalkyl, (C₁-C₄)-Alkoxy, (C₁-C₄)-Halogenalkoxy oder (C₁-C₄)-Alkoxy-(C₁-C₄)-alkyl,
n bedeutet 0, 1 oder 2,
s bedeutet 0, 1, 2 oder 3,
t bedeutet 0 oder 1.

3. Sulfinimidoyl- und Sulfonimidoylbenzoylderivate nach Anspruch 1 oder 2, worin
Q bedeutet einen Rest Q1, Q2, Q3, Q4 oder Q5, 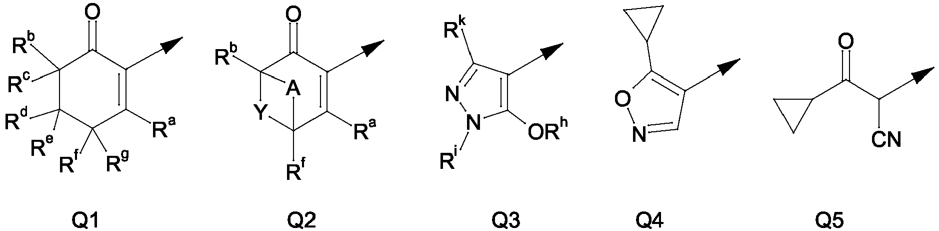
R^{a} bedeutet Hydroxy,
R^{b}, R^{c}, R^{f} und R^{g} bedeuten unabhängig voneinander jeweils Wasserstoff oder Methyl,
R^{d}, R^{e} bedeuten Wasserstoff oder bilden gemeinsam mit dem Kohlenstoffatom, an das sie gebunden sind, eine Carbonylgruppe,
R^{h} bedeutet Wasserstoff,
Rⁱ bedeutet Methyl oder Ethyl,
R^{k} bedeutet Wasserstoff, Methyl oder Cyclopropyl,
A und Y bedeuten unabhängig voneinander jeweils CH₂ oder CH₂CH₂,
X bedeutet Nitro, Halogen, Methyl, Ethyl, n-Propyl, iso-Propyl, Trifluormethyl, Difluormethyl, Chlordifluormethyl, Dichlorfluormethyl, Trichlormethyl, Pentafluorethyl, Heptafluorisopropyl, Cyclopropyl, Methoxy, Ethoxy, Methylsulfanyl, Methylsulfinyl, Methylsulfonyl, Methoxymethyl, Ethoxymethyl, Methoxyethyl, Methoxyethoxymethyl, Methylthiomethyl, Methylsulfinylmethyl oder Methylsulfonylmethyl,
Z bedeutet Wasserstoff, Nitro, Cyano, Halogen, Methyl, Ethyl, n-Propyl, iso-Propyl, Trifluormethyl, Difluormethyl, Chlordifluormethyl, Dichlorfluormethyl, Trichlormethyl, Pentafluorethyl, Heptafluorisopropyl, Cyclopropyl, Methoxy, Ethoxy, Methylsulfanyl, Methylsulfinyl oder Methylsulfonyl,
W bedeutet Wasserstoff, Chlor oder Methyl,
R bedeutet Methyl, Ethyl oder n-Propyl,
R' bedeutet Wasserstoff oder Cyano,
t bedeutet 0 oder 1.

4. Herbizide Mittel, **gekennzeichnet durch** einen herbizid wirksamen Gehalt an mindestens einer Verbindung der Formel (I) gemäß einem der Ansprüche 1 bis 3.

5. Herbizide Mittel nach Anspruch 4 in Mischung mit Formulierungshilfsmitteln.

6. Herbizide Mittel nach Anspruch 4 oder 5 enthaltend mindestens einen weiteren pestizid wirksamen Stoff aus der Gruppe Insektizide, Akarizide, Herbizide, Fungizide, Safener und Wachstumsregulatoren.

7. Herbizide Mittel nach Anspruch 6 enthaltend einen Safener.

8. Herbizide Mittel nach Anspruch 7 enthaltend cyprosulfamid, cloquintocet-mexyl, mefenpyr-diethyl oder isoxadifen-ethyl.

9. Herbizide Mittel nach einem der Ansprüche 6 bis 8 enthaltend ein weiteres Herbizid.

10. Verfahren zur Bekämpfung unerwünschter Pflanzen, **dadurch gekennzeichnet, daß** man eine wirksame Menge mindestens einer Verbindung der Formel (I) gemäß einem der Ansprüche 1 bis 3 oder eines herbiziden Mittels nach einem der Ansprüche 4 bis 9 auf die Pflanzen oder auf den Ort des unerwünschten Pflanzenwachstums appliziert.

11. Verwendung von Verbindungen der Formel (I) gemäß einem der Ansprüche 1 bis 3 oder von herbiziden Mitteln nach einem der Ansprüche 4 bis 9 zur Bekämpfung unerwünschter Pflanzen.

12. Verwendung nach Anspruch 11, **dadurch gekennzeichnet, daß** die Verbindungen der Formel (I) zur Bekämpfung unerwünschter Pflanzen in Kulturen von Nutzpflanzen eingesetzt werden.

13. Verwendung nach Anspruch 12, **dadurch gekennzeichnet, daß** die Nutzpflanzen transgene Nutzpflanzen sind.

14. Verbindungen der Formel (II) 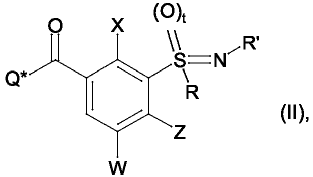
worin Q* Hydroxy, Ethoxy, Methoxy oder Chlor bedeutet,
und R, R', X, W, Z und t wie in einem der Ansprüche 1 bis 3 definiert sind.

15. Verwendung der Verbindungen der Formel (II) nach Anspruch 14 zur Herstellung von Verbindungen der Formel (I) nach einem de Ansprüche 1 bis 3.

## Claims

1. Sulfinimidoyl- and sulfonimidoylbenzoyl derivatives of the formula (I) or salts thereof 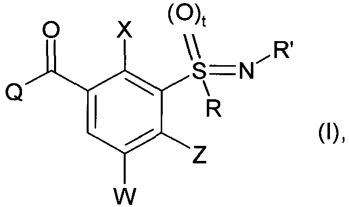 in which
Q is a radical Q1, Q2, Q3, Q4 or Q5, 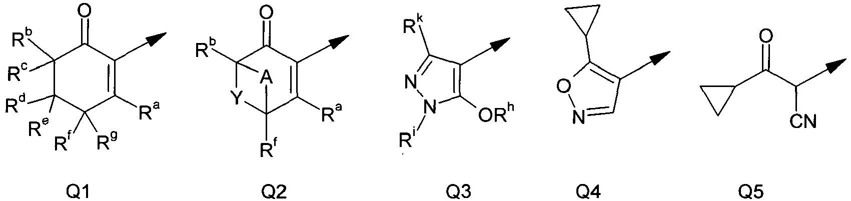
R^{a} is hydroxyl, R⁶S, R⁷ (R⁸) N,
R^{b}, R^{c}, R^{f} and R^{g} independently of one another are each hydrogen or (C₁-C₄) -alkyl,
R^{d}, R^{e} independently of one another are each hydrogen or (C₁-C₄)-alkyl or together with the carbon atom to which they are attached form a carbonyl group, R^{h} is hydrogen, (C₁-C₆) -alkylsulfonyl, (C₁-C₄)-alkoxy-(C₁-C₆)-alkylsulfonyl, phenylsulfonyl, phiophenyl-2-sulfonyl, benzoyl, benzoyl-(C₁-C₆)-alkyl, benzyl, where the five last-mentioned radicals may optionally be mono- or polysubstituted by halogen, (C₁-C₉)-alkyl or (C₁-C₄) -alkoxy,
Rⁱ is (C₁-C₄) -alkyl,
R^{k} is hydrogen, (C₁-C₄) -alkyl, (C₃-C₇) -cycloalkyl,
A and Y independently of one another are each oxygen, S (O)ₙ, N (R³), carbonyl or (C₁-C₄)-alkylene which is substituted by n radicals R⁹ and interrupted by n elements from the group consisting of oxygen, S(O)ₙ, N (R³) and carbonyl,
X is nitro, halogen, cyano, thiocyanato, (C₁-C₆)-alkyl, halo-(C₁-C₆)-alkyl, (C₂-C₆)-alkenyl, halo-(C₂-C₆)-alkenyl, (C₂-C₆)-alkynyl, halo- (C₃-C₆) -alkynyl, (C₃-C₆)-cycloalkyl, halo- (C₃-C₆)-cycloalkyl, (C₃-C₆)-cycloalkenyl, halo-(C₃-C₆) -cycloalkenyl, (C₃-C₆)-cycloalkyl-(C₁-C₆)-alkyl, halo-(C₃-C₆)-cycloalkyl-(C₁-C₆) -alkyl, (C₃-C₆)-cycloalkenyl-(C₁-C₆)-alkyl, halo- (C₃-C₆)-cycloalkenyl-(C₁-C₆)-alkyl, R¹(O)C, R¹(R¹ON=)C, R¹O(O)C,(R¹)₂N(O)C,R¹(R¹O)N(O)C,(R¹)_{z}N(R¹)N(0)C, R¹(O)C(R¹)N(O)C,R²O(O)C(R¹)N(O)C, (R¹)₂N(O)C(R¹)N(O)C, R²(O)₂S(R¹)N(O)C, R¹O(O)₂S(R¹)N(O)C, (R¹)₂N(O)₂S(R¹)N(O)C, R¹O, R¹(O)CO, R²(O)₂SO, R²O(O)CO, (R¹)₂N(O)CO, (R¹)₂N, R¹(O)C(R¹)N, R²(O)₂S(R¹)N, R²O(O)C(R¹)N, (R¹)₂N(O)C(R¹)N, R¹O(O)₂S(R¹)N, (R¹)₂N(O)₂S(R¹)N, R²(O)ₙS, R¹O(O)₂S, (R¹)₂N(O)₂S, R¹(O)C(R¹)N(O)₂S, R²O (O) C (R¹) N (0) ₂S, (R¹)₂N(O)C(R¹)N(O)₂S, (R⁵O)₂(O)P, R¹ (O) C-(C₁-C₆)-alkyl, R¹O(O)C-(C₁-C₆)-alkyl, (R¹)₂N(O)C-(C₁-C₆)-alkyl, (R¹O) (R¹)N(O)C-(C₁-C₆)-alkyl, (R¹) ₂N (R¹) N (0) C- (C₁-C₆) alkyl, R¹(O)C(R¹)N(O)C-(C₁-C₆)-alkyl, R²O(O)C(R¹)N(O)C-(C₁-C₆)-alkyl, (R¹)₂N(O)C(R¹)N(O)C- (C₁-C₆) -alkyl, R²(O)₂S(R¹)N(O)C-(C₁-C₆)-alkyl,R¹O(O)₂S(R¹)N(O)C-(C₁-C₆)-alkyl, (R¹)₂N(O)₂S(R¹)N(O)C-(C₁-C₆)-alkyl, NC-(C₁-C₆)-alkyl, R¹O-(C₁-C₆)-alkyl, R¹(O)CO-(C₁-C₆)-alkyl, R²(O)₂SO-(C₁-C₆)-alkyl, R²O(O)CO- (C₁-C₆)-alkyl, (R¹)₂N(O)CO-(C₁-C₆)-alkyl, (R¹)₂N-(C₁-C₆)-alkyl, R¹(O)C(R¹)N-(C₁-C₆)-alkyl, R²(O)₂S(R¹)N-(C₁-C₆)-alkyl, R²O(O)C(R¹)N-(C₁-C₆)-alkyl, (R¹)₂N(O)C(R¹)N-(C₁-C₆)-alkyl, R¹O(O)₂S(R¹)N-(C₁-C₆)-alkyl, (R¹)₂N(O)₂S(R¹)N-(C₁-C₆)-alkyl, R²(O)ₙS-(C₁-C₆)-alkyl, R¹O(O)₂S-(C₁-C₆)-alkyl, (R¹)₂N(O)₂S-(C₁-C₆)-alkyl, R¹(O)C(R¹)N(O)₂S-(C₁-C₆)-alkyl,R²O(O)C(R¹)N(O)₂S-(C₁-C₆)-alkyl, (R¹)₂N(O)C(R¹)N(O)₂S-(C₁-C6)-alkyl, (R⁵O)₂(O)P-(C₁-C₆)-alkyl, phenyl, heteroaryl, heterocyclyl, phenyl-(C₁-C₆)-alkyl, heteroaryl-(C₁-C₆)-alkyl, heterocyclyl-(C₁-C₆)-alkyl, where the six last-mentioned radicals are each substituted by s radicals from the group consisting of nitro, halogen, cyano, thiocyanato, (C₁-C₆)-alkyl, halo-(C₁-C₆)-alkyl, (C₃-C₆)-cycloalkyl, R¹O(O)C, (R¹)₂N(O)C, R¹O, (R¹)₂N, R²(O)ₙS, R¹O(O)₂S, (R¹)₂N(O)₂S and R¹O-(C₁-C₆)-alkyl, and where heterocyclyl carries n oxo groups,
Z is hydrogen, nitro, halogen, cyano, thiocyanato, (C₁-C₆)-alkyl, halo-(C₁-C₆)-alkyl, (C₂-C₆)-alkenyl, halo-(C₂-C₆)-alkenyl, (C₂-C₆)-alkynyl, halo- (C₃-C₆)-alkynyl, (C₃-C₆)-cycloalkyl, halo- (C₃-C₆)-cycloalkyl, (C₃-C₆)-cycloalkenyl, halo- (C₃-C₆)-cycloalkenyl, (C₃-C₆)-cycloalkyl-(C₁-C₆)-alkyl, halo-(C₃-C₆)-cycloalkyl-(C₁-C₆) -alkyl, (C₃-C₆)-cycloalkenyl-(C₁-C₆)-alkyl, halo- (C₃-C₆)-cycloalkenyl-(C₁-C₆)-alkyl, R¹(O)C, R¹(R¹ON=)C, R¹O(O)C, (R¹))₂N(O)C, R¹(R¹O)N(O)C, (R¹)₂N(R¹)N(O)C, R¹(O)C(R¹)N(O)C, R²O(O)C(R¹)N(O)C, (R¹)₂N(O)C(R¹)N(O)C, R² (O)₂S(R¹)N(O)C, R¹O(O)₂S(R¹)N(O)C, (R¹)₂N(O)₂S(R¹)N(O)C, R¹O, R¹(O)CO, R²(O)₂SO, R²O(O)CO, (R¹)₂N(O)CO, (R¹)₂N, R¹(O)C(R¹)N, R² (O) ₂S (R¹) N, R²O(O)C(R¹) N, (R¹)₂N (O) C (R¹) N, R¹O(O)₂S(R¹)N, (R¹))₂N(O)₂S(R¹)N, R²(O)ₙS, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S, (R⁵O)2(O)P, R¹(O)C-(C₁-C₆)-alkyl, R¹O(O)C-(C₁-C₆)-alkyl, (R¹)₂N(O)C-(C₁-C₆)-alkyl, (R¹O)(R¹)N(O)C-(C₁-C₆) -alkyl, (R¹)₂N(R¹)N(O)C-(C₁-C₆)-alkyl, R¹(O)C(R¹)N(O)C-(C₁-C₆)-alkyl, R²O(O)C(R¹)N(O)C-(C₁-C₆)-alkyl, (R¹)₂N(O)C(R¹)N(O)C-(C₁-C₆)-alkyl, R²(O)₂S(R¹)N(O)C-(C₁-C₆)-alkyl, R¹O(O)₂S(R¹)N(O)C-(C₁-C₆)-alkyl, (R¹)₂N(O)₂S(R¹)N(O)C-(C₁-C₆)-alkyl, NC-(C₁-C₆)-alkyl, R¹O-(C₁-C₆)-alkyl, R¹(O)CO-(C₁-C₆)-alkyl, R²(O)₂SO-(C₁-C₆)-alkyl, R²O(O)CO- (C₁-C₆)-alkyl, (R¹)₂N(O)CO-(C₁-C₆)-alkyl, (R¹)₂N-(C₁-C₆)-alkyl, R¹(O)C(R¹)N-(C₁-C₆)-alkyl, R²(O)₂S(R¹)N-(C₁-C₆)-alkyl, R²O(O)C(R¹)N-(C₁-C₆)-alkyl, (R¹)₂N(O)C(R¹)N-(C₁-C₆)-alkyl, R¹O(O)₂S(R¹)N-(C₁-C₆)-alkyl, (R¹)₂N(O)₂S(R¹)N-(C₁-C₆)-alkyl, R²(O)ₙS- (C₁-C₆) alkyl, R¹O(O)₂S-(C₁-C₆)-alkyl, (R¹)₂N(O)₂S-(C₁-C₆)-alkyl, R¹(O)C(R¹)N(O)₂S-(C₁-C₆)-alkyl, R²O(O)C(R¹)N(O)₂S-(C₁-C₆)-alkyl, (R¹)₂N(O)C(R¹)N(O)₂S-(C₁-C₆)-alkyl, (R⁵O)₂(O)P-(C₁-C₆)-alkyl, phenyl, heteroaryl, heterocyclyl, phenyl-(C₁-C₆)-alkyl, heteroaryl-(C₁-C₆)-alkyl, heterocyclyl-(C₁-C₆)-alkyl, where the six last-mentioned radicals are each substituted by s radicals from the group consisting of nitro, halogen, cyano, thiocyanato, (C₁-C₆)-alkyl, halo-(C₁-C₆)-alkyl, (C₃-C₆-cycloalkyl, R¹O(O)C, (R¹)₂N(O)C, R¹O, (R¹)₂N, R²(O)ₙS, R¹O(O)₂S, (R¹)₂N(O)₂S and R¹O-(C₁-C₆)-alkyl, and where heterocyclyl carries n oxo groups,
W is hydrogen, halogen, nitro, cyano, thiocyanato, (C₁-C₆)-alkyl, halo-(C₁-C₆)-alkyl, (C₂-C₆)-alkenyl, halo-(C₂-C₆)-alkenyl, (C₂-C₆)-alkynyl, halo- (C₃-C₆) -alkynyl, (C₃-C₇)-cycloalkyl, halo- (C₃-C₇) -cycloalkyl, (C₁-C₆)-alkoxy, halo- (C₁-C₆)-alkoxy, (C₁-C₆)-alkyl-(O)ₙS-, (C₁-C₆)-haloalkyl-(O)ₙS-, (C₁-C₆)-alkoxy-(C₁-C₄)-alkyl, (C₁-C₆)-alkoxy-(C₁-C₄)-haloalkyl, R¹(O)C, R¹(R¹ON=)C, R¹O(O)C, (R¹)₂N, R¹(O)C(R¹)N or R²(O)₂S(R¹)N,
R is (C₁-C₆)-alkyl, (C₂-C₆) -alkenyl or (C₂-C₆)-alkynyl, each of which is substituted by s radicals from the group consisting of nitro, halogen, cyano, thiocyanato, (C₃-C₆)-cycloalkyl, R¹(O)C, R¹(R¹ON=)C, R¹O(O)C, (R¹)₂N(O)C, R¹(R¹O)N(O)C, R²(O)₂S(R¹) N (0) C, R¹O (O) ₂S (R¹) N (O) C, (R¹) ₂N (O) ₂S (R¹) N (O) C, R¹S(O)C, R¹O, R¹(O)CO, R²(O)₂SO, R²O(O)CO, (R¹)₂N(O)CO, (R¹)₂N, R¹O(R¹)N, R¹(O)C(R¹)N, R²(O)₂S(R¹)N, R²O(O)C(R¹)N, (R¹)₂N(O)C(R¹)N, R¹O(O)₂S(R¹)N, (R¹)₂N(O)₂S(R¹)N, R²(O)ₙS, R¹C(O)S, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S and (R⁵O)₂(O)P, or
is (C₃-C₆) -cycloalkyl, (C₃-C₆) -cycloalkenyl, phenyl, phenyl-(C₁-C₆)-alkyl, heteroaryl, heteroaryl-(C₁-C₆)-alkyl, heterocyclyl, heterocyclyl-(C₁-C₆)-alkyl, phenyl-O- (C₁-C₆) -alkyl, heteroaryl-O- (C₁-C₆) -alkyl, heterocyclyl-O- (C₁-C₆) -alkyl, phenyl-N (R¹) - (C₁-C₆) -alkyl, heteroaryl- N(R¹)-(C₁-C₆)-alkyl, heterocyclyl- N(R¹)-(C₁-C₆)-alkyl, phenyl-S(O)ₙ-(C₁-C₆)-alkyl, heteroaryl-S(O)ₙ-(C₁-C₆)-alkyl or heterocyclyl-S(O)ₙ-(C₁-C₆)-alkyl, each of which is substituted in the cyclic moiety by s radicals from the group consisting of nitro, halogen, cyano, thiocyanato, (C₁-C₆) -alkyl, halo- (C₁-C₆) -alkyl, (C₃-C₆)-cycloalkyl, R¹(O)C, R¹(R¹ON=)C, R¹O(O)C, (R¹)₂N(O)C, R¹(R¹O)N(O)C, R²(O)₂S(R¹)N(O)C,
R¹O (O) ₂S (R¹) N (O) C, (R¹) ₂N (O) ₂S (R¹) N (0) C, R¹S(O)C, R¹O, R¹(O)CO, R²(O)₂SO, R²O(O)CO, (R¹)₂N(O)CO, (R¹)₂N, R¹O(R¹)N, R¹ (0) C (R¹) N, R² (O) ₂S (R¹) N, R²O (0) C (R¹) N, (R¹) ₂N (O) C (R¹) N, R¹O(O)₂S(R¹)N, (R¹)₂N(O)₂S(R¹)N, R²(O)ₙS, R¹C(O)S, R¹O(O)₂S, (R¹)₂N(O)₂S, R¹(O)C(R¹)N(O)₂S, R²O(O)C(R¹))N(O)₂S, (R¹) ₂N (O) C (R¹) N (0) ₂S, (R⁵O)₂(O)P and R¹O- (C₁-C₆) -alkyl, and where heterocyclyl carries n oxo groups,
R' is hydrogen, nitro, halogen, cyano, (C₁-C₆)-alkyl, halo- (C₁-C₆) -alkyl, (C₃-C₆)-alkenyl, halo-(C₃-C₆)-alkenyl, (C₂-C₆)-alkynyl, halo- (C₃-C₆) -alkynyl, (C₃-C₆)-cycloalkyl, halo- (C₃-C₆) -cycloalkyl, (C₃-C₆)-cycloalkyl-(C₁-C₆)-alkyl, halo-(C₃-C₆) -cycloalkyl- (C₁-C₆) -alkyl, R¹(O)C, R²O(O)C, (R¹)₂N(O)C, R²S(O)C, (R¹)₂N(S)C, R¹(R¹O)N(O)C, R²(O)₂S(R¹)N(O)C, (R¹)₂N(O)₂S(R¹)N(O)C, R¹O, (R¹)₂N, R²(O)ₙS, (R²) ₃Si-(C₁-C₆)-alkyl-(O)ₙS, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S, R²(O)₂S(R¹)N(O)₂S, (R⁵O)2(O)P, (R²) ₃Si, R¹(O)C-(C₁-C₆)-alkyl, R¹O(O)C-(C₁-C₆)-alkyl, (R¹) ₂N (0) C- (C₁-C₆) -alkyl, (R¹O) (R¹) N (0) C- (C₁-C₆) -alkyl, R² (O)₂S(R¹)N(O)C-(C₁-C₆)-alkyl, R¹(O)₂S(R¹)N(O)C-(C₁-C₆)-alkyl, (R¹)₂N(O)₂S(R¹)N(O)C-(C₁-C₆)-alkyl, R¹O-(C₁-C₆)-alkyl, R¹(O)CO-(C₁-C₆)-alkyl, R²(O)₂SO-(C₁-C₆)-alkyl, R²O (O) CO- (C₁-C₆)-alkyl, (R¹)₂N(O)CO-(C₁-C₆)-alkyl, (R¹)₂N-(C₁-C₆)-alkyl, R¹(O)C(R¹)N-(C₁-C₆)-alkyl, R² (0) ₂S (R¹) N-(C₁-C₆)-alkyl, R²O(O)C(R¹)N-(C₁-C₆)-alkyl, (R¹)₂N(O)C(R¹)N-(C₁-C₆)-alkyl, R¹O(O)₂S (R¹)N-(C₁-C₆)-alkyl, (R¹)₂N(O)₂S(R¹)N-(C₁-C₆)-alkyl, R²(O)ₙS-(C₁-C₆) alkyl, R¹O(O)₂S-(C₁-C₆)-alkyl, (R¹)₂N(O)₂S-(C₁-C₆)-alkyl, R¹(O)C(R¹)N(O)₂S-(C₁-C₆)-alkyl, R²O(O)C(R¹)N(O)₂S-(C₁-C₆)-alkyl, (R¹)₂N(O)C(R¹)N(O)₂S-(C₁-C₆)-alkyl, (R⁵O)₂(O)P-(C₁-C₆)-alkyl, (R²) ₃Si-(C₁-C₆)-alkyl, phenyl, heteroaryl, heterocyclyl, phenyl-(C₁-C₆)-alkyl, heteroaryl-(C₁-C₆-alkyl, heterocyclyl-(C₁-C₆)-alkyl, where the six above-mentioned phenyl, heteroaryl and heterocyclyl radicals are substituted in the cyclic moiety by s radicals from the group consisting of nitro, halogen, cyano, thiocyanato, (C₁-C₆) -alkyl, halo- (C₁-C₆) -alkyl, (C₃-C₆)-cycloalkyl, R¹O(O)C, (R¹)₂N(O)C, R¹O, (R¹)₂N, R²(O)ₙS, R¹O(O)₂S, (R¹)₂N(O)₂S and R¹O-(C₁-C₆)-alkyl, and where heterocyclyl carries n oxo groups,
R¹ is hydrogen, (C₁-C₆)-alkyl, halo- (C₁-C₆) -alkyl, (C₂-C₆)-alkenyl, halo- (C₂-C₆) -alkenyl, (C₂-C₆)-alkynyl, halo-(C₃-C₆)-alkynyl, (C₃-C₆) -cycloalkyl, (C₃-C₆)-cycloalkenyl, halo- (C₃-C₆) -cycloalkyl, (C₃-C₆)-cycloalkyl- (C₁-C₆) -alkyl, (C₁-C₆) -alkyl-O-(C₁-C₆) -alkyl, cycloalkyl-(C₁-C₆)-alkyl-O-(C₁-C₆)-alkyl, phenyl, phenyl-(C₁-C₆)-alkyl, heteroaryl, heteroaryl-(C₁-C₆)-alkyl, heterocyclyl, heterocyclyl-(C₁-C₆)-alkyl, phenyl-O-(C₁-C₆)-alkyl, heteroaryl-O-(C₁-C₆)-alkyl, heterocyclyl-O-(C₁-C₆)-alkyl, phenyl-N(R³)- (C₁-C₆) -alkyl, heteroaryl-N(R³)-(C₁-C₆)-alkyl, heterocyclyl-N(R³)-(C₁-C₆)-alkyl, phenyl-S(O)ₙ-(C₁-C₆)-alkyl, heteroaryl-S(O)ₙ-(C₁-C₆)-alkyl, heterocyclyl-S(O)ₙ-(C₁-C₆)-alkyl, where the fifteen last-mentioned radicals are each substituted by s radicals from the group consisting of nitro, halogen, cyano, thiocyanato, (C₁-C₆)-alkyl, halo-(C₁-C₆) -alkyl, (C₃-C₆)-cycloalkyl, R³O(O)C, (R³))₂N(O)C, R³O, (R³))₂N, R⁴(O)ₙS, R³O(O)₂S, (R³)₂N(O)₂S and R³O-(C₁-C₆)-alkyl, and where heterocyclyl carries n oxo groups,
R² is (C₁-C₆)-alkyl, halo- (C₁-C₆) -alkyl, (C₂-C₆)-alkenyl, halo- (C₂-C₆) -alkenyl, (C₂-C₆) -alkynyl, halo- (C₃-C₆)-alkynyl, (C₃-C₆) -cycloalkyl, (C₃-C₆) -cycloalkenyl, halo-(C₃-C₆) -cycloalkyl, (C₃-C₆) -cycloalkyl- (C₁-C₆)-alkyl, (C₁-C₆)-alkyl-O-(C₁-C₆)-alkyl, cycloalkyl- (C₁-C₆)-alkyl-O-(C₁-C₆)-alkyl, phenyl, phenyl-(C₁-C₆)-alkyl, heteroaryl, heteroaryl-(C₁-C₆)-alkyl, heterocyclyl, heterocyclyl-(C₁-C₆)-alkyl, phenyl-O-(C₁-C₆)-alkyl, heteroaryl-O-(C₁-C₆)-alkyl, heterocyclyl-O-(C₁-C₆)-alkyl, phenyl-N(R³)-(C₁-C₆)-alkyl, heteroaryl-N(R³) -(C₁-C₆)-alkyl, heterocyclyl-N(R³)-(C₁-C₆)-alkyl, phenyl-S(O)ₙ-(C₁-C₆)-alkyl, heteroaryl-S(O)ₙ-(C₁-C₆)-alkyl, heterocyclyl-S(O)ₙ-(C₁-C₆)-alkyl, where the fifteen last-mentioned radicals are each substituted by s radicals from the group consisting of nitro, halogen, cyano, thiocyanato, (C₁-C₆)-alkyl, halo- (C₁-C₆)-alkyl, (C₃-C₆)-cycloalkyl, R³O(O)C, (R³)₂N(O)C, R³O, (R³)₂N, R⁴(O)ₙS, R³O(O)₂S, (R³)₂N(O)₂S and R³O- (C₁-C₆) -alkyl, and where heterocyclyl carries n oxo groups,
R³ is hydrogen, (C₁-C₆)-alkyl, halo- (C₁-C₆) -alkyl, (C₂-C₆)-alkenyl, (C₂-C₆) -alkynyl, (C₃-C₆) -cycloalkyl, (C₃-C₆)-cycloalkyl-(C₁-C₆)-alkyl or phenyl,
R⁴ is (C₁-C₆)-alkyl, halo-(C₁-C₆)-alkyl, (C₂-C₆)-alkenyl, (C₂-C₆)-alkynyl, (C₃-C₆) -cycloalkyl, (C₃-C₆)-cycloalkyl-(C₁-C₆)-alkyl or phenyl,
R⁵ is hydrogen or (C₁-C₄)-alkyl,
R⁶ is (C₁-C₄)-alkyl or is phenyl which is substituted by m radicals from the group consisting of halogen, nitro, cyano, (C₁-C₄)-alkyl, (C₁-C₄) -haloalkyl, (C₁-C₄)-alkoxy and (C₁-C₄)-haloalkoxy,
R⁷ is hydrogen, (C₁-C₄)-alkyl or (C₁-C₄)-alkoxy,
R⁸ is hydrogen or (C₁-C₄)-alkyl,
or
R⁷ and R⁸ together with the nitrogen atom to which they are attached form a 5- or 6-membered saturated, partially saturated or unsaturated ring which additionally contained n heteroatoms from the group consisting of nitrogen, oxygen and sulfur and which is substituted by m radicals from the group consisting of halogen, cyano, (C₁-C₄)-alkyl, (C₁-C₄)-haloalkyl, (C₁-C₄)-alkoxy and (C₁-C₄)-haloalkoxy,
R⁹ is halogen, (C₁-C₄)-alkyl, (C₁-C₄)-haloalkyl, (C₁-C₄)-alkoxy,
(C₁-C₄)-haloalkoxy or (C₁-C₄)-alkoxy-(C₁-C₄)-alkyl,
m is 0, 1, 2, 3, 4 or 5,
n is 0, 1 or 2,
s is 0, 1, 2 or 3,
t is 0 or 1.

2. Sulfinimidoyl- or sulfonimidoylbenzoyl derivatives according to Claim 1 in which
Q is a radical Q1, Q2, Q3, Q4 or Q5, 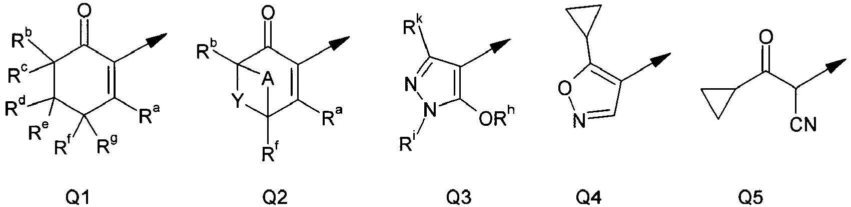
R^{a} is hydroxyl,
R^{b}, R^{c}, R^{f} and R^{g} independently of one another are each hydrogen or (C₁-C₄)-alkyl,
R^{d}, R^{e} independently of one another are each hydrogen or (C₁-C₄)-alkyl or together with the carbon atom to which they are attached form a carbonyl group,
R^{h} is hydrogen,
Rⁱ is (C₁-C₄) -alkyl,
R^{k} is hydrogen, (C₁-C₄) -alkyl, (C₃**-**C₇) -cycloalkyl,
A and Y independently of one another are each oxygen or (C₁-C₄)-alkylene which is substituted by n radicals R⁹,
X is nitro, halogen, cyano, (C₁-C₆)-alkyl, halo-(C₁-C₆-alkyl, (C₂-C₆) -alkenyl, (C₂-C₆) -alkynyl, (C₃-C₆)-cycloalkyl, halo- (C₃-C₆)-cycloalkyl, (C₃-C₆)-cycloalkyl-(C₁-C₆)-alkyl, halo-(C₃-C₆)-cycloalkyl-(C₁-C₆)-alkyl, R¹(O)C, R¹ (R¹ON=) C, R¹O(O)C, (R¹)₂N(O)C, R¹O, (R¹)₂N, R¹(O)C(R¹)N, R² (O) ₂S (R¹) N, R²O (O) C (R¹) N, (R¹) ₂N (0) C (R¹) N, R²(O)ₙS, R¹O(O)₂S, (R¹)₂N(O)₂S, (R⁵O) ₂(O)P, R¹(O)C-(C₁-C₅)-alkyl, R¹O(O)C-(C₁-C₆)-alkyl, (R¹)₂N(O)C-(C₁-C₆)-alkyl, NC-(C₁-C₆)-alkyl, R¹O-(C₁-C₆)-alkyl, (R¹)₂N-(C₁-C₆)-alkyl, R¹(O)C(R¹)N-(C₁-C₆) -alkyl, R²(O)₂S(R¹)N-(C₁-C₆) -alkyl, R²O(O)C(R¹)N-(C₁-C₆)-alkyl, (R¹)₂N(O)C(R¹)N-(C₁-C₆)-alkyl, R²(O)ₙS-(C₁-C₆)-alkyl, R¹O(O)₂S-(C₁-C₆)-alkyl, (R¹)₂N(O)₂S-(C₁-C₆)-alkyl, (R⁵O)₂(O)P-(C₁-C₆)-alkyl, phenyl, heteroaryl, heterocyclyl, phenyl-(C₁-C₆)-alkyl, heteroaryl-(C₁-C₆)-alkyl, heterocyclyl-(C₁-C₆)-alkyl, where the six last-mentioned radicals are each substituted by s radicals from the group consisting of nitro, halogen, cyano, thiocyanato, (C₁-C₆)-alkyl, halo-(C₁-C₆) -alkyl, R¹O, (R¹)₂N, R²(O)ₙS, R¹O(O)₂S, (R¹)₂N(O)₂S and R¹O-(C₁-C₆)-alkyl and where heterocyclyl carries n oxo groups,
Z is hydrogen, nitro, halogen, cyano, (C₁-C₆)-alkyl, halo- (C₁-C₆) -alkyl, (C₂-C₆)-alkenyl, (C₂-C₆)-alkynyl, (C₃-C₆)-cycloalkyl, halo- (C₃-C₆) -cycloalkyl, (C₃-C₆)-cycloalkyl-(C₁-C₆)-alkyl, halo-(C₃-C₆)-cycloalkyl-(C₁-C₆)-alkyl, R¹(O)C, R¹(R¹ON=)C, R¹O(O)C, (R¹)₂N(O)C, R¹O, (R¹) ₂N, R¹(O)C(R¹)N, R²(O)₂S(R¹)N, R²O(O)C(R¹)N, (R¹)₂N(O)C(R¹)N, R²(O)ₙS, R¹O(O)₂S, (R¹)₂N(O)₂S, (R⁵O)₂(O)P, R¹(O)C-(C₁-C₆)-alkyl, R¹O(O)C-(C₁-C₆)-alkyl, (R¹)₂N(O)C-(C₁-C₆)-alkyl, NC-(C₁-C₆)-alkyl, R¹O-(C₁-C₆)-alkyl, (R¹)₂N-(C₁-C₆)-alkyl, R¹(O)C(R¹)N-(C₁-C₆)-alkyl, R² (O)₂S(R¹)N-(C₁-C₆)-alkyl, R²O(O)C(R¹)N-(C₁-C₆)-alkyl, (R¹)₂N(O)C(R¹)N-(C₁-C₆)-alkyl, R²(O)ₙS-(C₁-C₆) -alkyl, R¹O(O)₂S-(C₁-C₆)-alkyl, (R¹)₂N(O)₂S-(C₁-C6)-alkyl, (R⁵O)₂(O)P-(C₁-C₆)-alkyl, phenyl, heteroaryl, heterocyclyl, phenyl-(C₁-C₆)-alkyl, heteroaryl-(C₁-C₆)-alkyl, heterocyclyl-(C₁-C₆)-alkyl, where the six last-mentioned radicals are each substituted by s radicals from the group consisting of nitro, halogen, cyano, thiocyanato, (C₁-C₆)-alkyl, halo- (C₁-C₆) -alkyl, R¹O, (R¹)₂N, R²(O)ₙS, R¹O(O)₂S, (R¹)₂N(O)₂S and R¹O-(C₁-C₆)-alkyl and where heterocyclyl carries n oxo groups,
W is hydrogen, halogen, nitro, cyano, (C₁-C₆)-alkyl, halo-(C₁-C₆)-alkyl, (C₃-C₇) -cycloalkyl, (C₁-C₆) -alkoxy, (C₁-C₆)-alkyl-(O)ₙS-, R¹O(O)C, (R¹)₂N, R¹(O)C(R¹)N or R²(O)₂S(R¹)N,
R is (C₁-C₆)-alkyl which is in each case substituted by s radicals from the group consisting of halogen, cyano, (C₃-C₆)-cycloalkyl, R¹(O)C, R¹(R¹ON=)C, R¹O(O)C, (R¹)₂N(O)C, R²(O)₂S(R¹)N(O)C, R¹O, (R¹)₂N, R¹(O)C(R¹)N, R² (O)₂S(R¹)N, R²O(O)C(R¹)N, (R¹)₂N(O)C(R¹)N, R²(O)ₙS, R¹O(O)₂S, (R¹)₂N(O)₂S, R¹(O)C(R¹)N(O)₂S, R²O(O)C(R¹)N(O)₂S and (R¹)₂N(O)C(R¹)N(O)₂S or
is (C₃-C₆)-cycloalkyl which is in each case substituted by s radicals from the group consisting of halogen, (C₁-C₆)-alkyl, halo-(C₁-C₆)-alkyl, (C₃-C₆)-cycloalkyl, R¹O(O)C and (R¹)₂N(O)C,
R' is hydrogen, nitro, cyano, (C₁-C₆)-alkyl, halo-(C₁-C₆)-alkyl, (C₃-C₆)-cycloalkyl, halo- (C₃-C₆) -cycloalkyl, (C₃-C₆)-cycloalkyl-(C₁-C₆)-alkyl, halo-(C₃-C₆)-cycloalkyl-(C₁-C₆)-alkyl, R¹(O)C, R²O(O)C, (R¹)₂N(O)C, R²(O)₂S, R¹(O)C-(C₁-C₆)-alkyl, R¹O(O)C-(C₁-C₆)-alkyl, (R¹)₂N(O)C-(C₁-C₆)-alkyl, R¹O-(C₁-C₆)-alkyl, (R¹)₂N-(C₁-C₆)-alkyl, R²(O)ₙS-(C₁-C₆)-alkyl,
R¹ is hydrogen, (C₁-C₆) -alkyl, halo- (C₁-C₆) -alkyl, (C₃-C₆) -cycloalkyl, halo- (C₃-C₆) -cycloalkyl, (C₃-C₆)-cycloalkyl-(C₁-C₆)-alkyl, (C₁-C₆)-alkyl-O-(C₁-C₆)-alkyl, cycloalkyl-(C₁-C₆)-alkyl-O-(C₁-C₆)-alkyl, phenyl, phenyl-(C₁-C₆)-alkyl, heteroaryl, heteroaryl-(C₁-C₆)-alkyl, heterocyclyl, heterocyclyl-(C₁-C₆)-alkyl, phenyl-O-(C₁-C₆)-alkyl, heteroaryl-O-(C₁-C₆)-alkyl, heterocyclyl-O-(C₁-C₆)-alkyl, where the nine last-mentioned radicals are in each case substituted by s radicals from the group consisting of nitro, halogen, (C₁-C₆)-alkyl, halo-(C₁-C₆)-alkyl, R³O(O)C, (R³)₂N(O)C, R³O, (R³)₂N, R⁴(O)ₙS and R³O- (C₁-C₆)-alkyl, and where heterocyclyl carries n oxo groups,
R² is (C₁-C₆)-alkyl, halo-(C₁-C₆)-alkyl, (C₃-C₆)-cycloalkyl, halo-(C₃-C₆)-cycloalkyl, (C₃-C₆)-cycloalkyl-(C₁-C₆)-alkyl, (C₁-C₆)-alkyl-O-(C₁-C₆)-alkyl, cycloalkyl-(C₁-C₆)-alkyl-O-(C₁-C₆)-alkyl, phenyl, phenyl-(C₁-C₆)-alkyl, heteroaryl, heteroaryl-(C₁-C₆)-alkyl, heterocyclyl, heterocyclyl-(C₁-C₆)-alkyl, phenyl-O-(C₁-C₆)-alkyl, heteroaryl-O-(C₁-C₆)-alkyl, heterocyclyl-O-(C₁-C₆)-alkyl, where the nine last-mentioned radicals are in each case substituted by s radicals from the group consisting of nitro, halogen, (C₁-C₆)-alkyl, halo-(C₁-C₆)-alkyl, R³O(O)C, (R³)₂N(O)C, R³O, (R³)₂N, R⁴(O)ₙS and R³O-(C₁-C₆)-alkyl, and where heterocyclyl carries n oxo groups,
R³ is hydrogen or (C₁-C₆)-alkyl,
R⁴ is (C₁-C₆)-alkyl,
R⁵ is hydrogen or (C₁-C₄)-alkyl,
R⁹ is halogen, (C₁-C₄)-alkyl, (C₁-C₄)-haloalkyl, (C₁-C₄)-alkoxy,
(C₁-C₄)-haloalkoxy or (C₁-C₄)-alkoxy-(C₁-C₄)-alkyl,
n is 0, 1 or 2,
s is 0, 1, 2 or 3,
t is 0 or 1.

3. Sulfinimidoyl- or sulfonimidoylbenzoyl derivatives according to Claim 1 or 2 in which
Q is a radical Q1, Q2, Q3, Q4 or Q5, 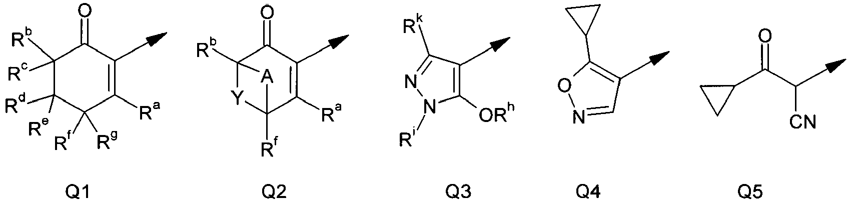
R^{a} is hydroxyl,
R^{b}, R^{c}, R^{f} and R^{g} independently of one another are each hydrogen or methyl,
R^{d}, R^{e} are hydrogen or together with the carbon atom to which they are attached form a carbonyl group,
R^{h} is hydrogen,
Rⁱ is methyl or ethyl,
R^{k} is hydrogen, methyl or cyclopropyl,
A and Y independently of one another are each CH₂ or CH₂CH₂,
X is nitro, halogen, methyl, ethyl, n-propyl, isopropyl, trifluoromethyl, difluoromethyl, chlorodifluoromethyl, dichlorofluoromethyl, trichloromethyl, pentafluoroethyl, heptafluoroisopropyl, cyclopropyl, methoxy, ethoxy, methylsulfanyl, methylsulfinyl, methylsulfonyl, methoxymethyl, ethoxymethyl, methoxyethyl, methoxyethoxymethyl, methylthiomethyl, methylsulfinylmethyl or methylsulfonylmethyl,
Z is hydrogen, nitro, cyano, halogen, methyl, ethyl, n-propyl, isopropyl, trifluoromethyl, difluoromethyl, chlorodifluoromethyl, dichlorofluoromethyl, trichloromethyl, pentafluoroethyl, heptafluoroisopropyl, cyclopropyl, methoxy, ethoxy, methylsulfanyl, methylsulfinyl or methylsulfonyl,
W is hydrogen, chlorine or methyl,
R is methyl, ethyl or n-propyl,
R' is hydrogen or cyano,
t is 0 or 1.

4. Herbicidal compositions **characterized by** a herbicidally active amount of at least one compound of the formula (I) according to any of Claims 1 to 3.

5. Herbicidal compositions according to Claim 4 in a mixture with formulation auxiliaries.

6. Herbicidal compositions according to Claim 4 or 5 which comprise at least one further pesticidally active compound from the group of the insecticides, acaricides, herbicides, fungicides, safeners and growth regulators.

7. Herbicidal compositions according to Claim 6 which comprise a safener.

8. Herbicidal compositions according to Claim 7 which comprise cyprosulfamide, cloquintocet-mexyl, mefenpyr-diethyl or isoxadifen-ethyl.

9. Herbicidal compositions according to any of Claims 6 to 8 which comprise a further herbicide.

10. Method of controlling unwanted plants, **characterized in that** an effective amount of at least one compound of the formula (I) according to any of Claims 1 to 3 or of a herbicidal composition according to any of Claims 4 to 9 is applied to the plants or to the location of the unwanted plant growth.

11. Use of compounds of the formula (I) according to any of Claims 1 to 3 or of herbicidal compositions according to any of Claims 4 to 9 for controlling unwanted plants.

12. Use according to Claim 11, **characterized in that** the compounds of the formula (I) are employed for controlling unwanted plants in crops of useful plants.

13. Use according to Claim 12, **characterized in that** the useful plants are transgenic useful plants.

14. Compounds of the formula (II) 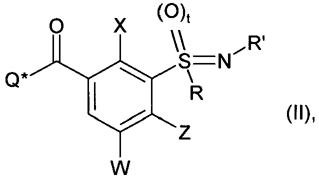 in which Q* is hydroxyl, ethoxy, methoxy or chlorine, and R, R', X, W, Z and t are as defined in any of Claims 1 to 3.

15. Use of the compounds of the formula (II) according to Claim 14 for preparing compounds of the formula (I) according to any of Claims 1 to 3.

## Revendications

1. Dérivés de sulfinimidoyle et de sulfonimidoylbenzoyle de formule (I) ou leurs sels 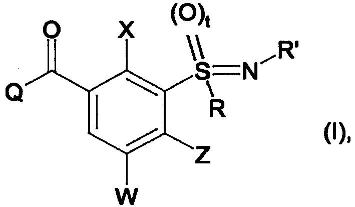 dans laquelle
Q signifie un radical Q1, Q2, Q3, Q4 ou Q5, 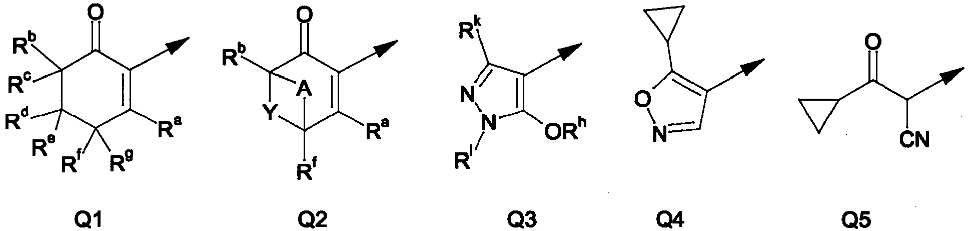
R^{a} signifie hydroxy, R⁶S, R⁷(R⁸)N,
R^{b}, R^{c}, R^{f} et R^{g} signifient, indépendamment les uns des autres, à chaque fois hydrogène ou (C₁-C₄)-alkyle,
R^{d}, R^{e} signifient, indépendamment l'un de l'autre, à chaque fois hydrogène ou (C₁-C₄)-alkyle ou forment ensemble avec l'atome de carbone auquel ils sont liés un groupe carbonyle,
R^{h} signifie hydrogène, (C₁-C₆) -alkylsulfonyle, (C₁-C₄)-alcoxy-(C₁-C₆)-alkylsulfonyle, phénylsulfonyle, thiophényl-2-sulfonyle, benzoyle, benzoyl-(C₁-C₆)-alkyle, benzyle, les cinq derniers radicaux mentionnés pouvant le cas échéant être monosubstitués ou polysubstitués par halogéno, (C₁-C₄)-alkyle ou (C₁-C₄)-alcoxy,
Rⁱ signifie (C₁-C₄)-alkyle,
R^{k} signifie hydrogène, (C₁-C₄)-alkyle, (C₃-C₇)-cycloalkyle,
A et Y signifient, indépendamment l'un de l'autre, à chaque fois oxygène, S(O)ₙ, N(R³), carbonyle ; ou (C₁-C₄)-alkylène qui est substitué par n radicaux R⁹ et qui est interrompu par n éléments du groupe constitué par oxygène, S(O)ₙ, N(R³) et carbonyle,
X signifie nitro, halogéno, cyano, thiocyano, (C₁-C₆)-alkyle, halogéno-(C₁-C₆)-alkyle, (C₂-C₆)-alcényle, halogéno-(C₂-C₆)-alcényle, (C₂-C₆)-alcynyle, halogéno-(C₃-C₆)-alcynyle, (C₃-C₆)-cycloalkyle, halogéno-(C₃-C₆)-cycloalkyle, (C₃-C₆)-cycloalcényle, halogéno-(C₃-C₆)-cycloalcényle, (C₃-C₆)-cycloalkyl-(C₁-C₆)-alkyle, halogéno-(C₃-C₆)-cycloalkyl- (C₁-C₆) -alkyle, (C₃-C₆) -cycloalcényl- (C₁-C₆)-alkyle, halogéno-(C₃-C₆)-cycloalcényl-(C₁-C₆)-alkyle, R¹(O)C, R^{l} (R¹ON=)C, R¹O(O)C, (R¹)₂N(O)C, R¹(R¹O)N(O)C, (R¹)₂N(R¹)N(O)C, R¹(O)C(R¹)N(O)C, R²O (0) C (R¹) N (0) C, (R¹) ₂N (0) C (R¹) N (0) C, R² (0) ₂S (R¹) N (0) C, R¹O (O)₂S(R¹)N(O)C, (R¹)₂N(O)₂S(R¹)N(O)C, R¹O, R¹(O)CO, R²(O)₂SO, R²O (0) CO, (R¹)₂N(O)CO, (R¹)₂N, R¹(O)C(R¹)N, R²(O)₂S(R¹) N, R²O(O)C(R¹)N, (R¹) ₂N (O) C (R¹) N, R¹O(O)₂S(R¹)N, (R¹)₂N(O)₂S(R¹)N, R²(O)ₙS, R¹O(O)₂S, (R¹)₂N(O)₂S, R¹ (0) C (R¹) N (O) ₂S, R²O(O)C(R¹)N(O)₂S, (R¹)₂N(O)C(R¹)N(O)₂S, (R⁵O)₂(O)P, R¹(O)C-(C₁-C₆)-alkyle, R¹O(O)C- (C₁-C₆) -alkyle, (R¹)₂N(O)C-(C₁-C₆)-alkyle, (R¹O) (R¹) N (0) C- (C₁-C₆) -alkyle, (R¹)₂N(R¹)N(O)C-(C₁-C₆)-alkyle, R¹ (0) C (R¹) N(O)C-(C₁-C₆)-alkyle, R²O(O)C(R¹)N(O)C-(C₁-C₆)-alkyle, (R¹)₂N(O)C(R¹)N(O)C-(C₁-C₆)-alkyle, R² (O) ₂S (R¹) N (0) C-(C₁-C₆)-alkyle, R¹O(O)₂S (R¹)N(O)C-(C₁-C₆)-alkyle, (R¹)₂N(O)₂S (R¹)N(O)C-(C₁-C₆)-alkyle, NC-(C₁-C₆)-alkyle, R¹O-(C₁-C₆)-alkyle, R¹(O)CO-(C₁-C₆)-alkyle, R²(O)₂SO-(C₁-C₆)-alkyle, R²O(O)CO-(C₁-C₆)-alkyle, (R¹)₂N(O)CO-(C₁-C₆)-alkyle, (R¹)₂N-(C₁-C₆)-alkyle, R¹(O)C(R¹)N-(C₁-C₆) -alkyle, R²(O)₂S(R¹)N-(C₁-C₆)-alkyle, R²O(O)C(R¹)N-(C₁-C₆)-alkyle, (R¹)₂N(O)C(R¹)N-(C₁-C₆)-alkyle, R¹O(O)₂S (R¹)N-(C₁-C₆)-alkyle, (R¹)₂N(O)₂S(R¹)N-(C₁-C₆)-alkyle, R²(O)ₙS-(C₁-C₆)-alkyle, R¹O(O)₂S-(C₁-C₆)-alkyle, (R¹)₂N(O)₂S-(C₁-C₆)-alkyle, R¹(O)C(R¹)N(O)₂S-(C₁-C₆)-alkyle, R²O(O)C(R¹)N(O)₂S-(C₁-C₆)-alkyle, (R¹)₂N(O)C(R¹)N(O)₂S-(C₁-C₆)-alkyle, (R⁵O)₂(O)P-(C₁-C₆)-alkyle, phényle, hétéroaryle, hétérocyclyle, phényl-(C₁-C₆)-alkyle, hétéroaryl-(C₁-C₆)-alkyle, hétérocyclyl-(C₁-C₆)-alkyle, les six derniers radicaux mentionnés étant à chaque fois substitués par s radicaux du groupe constitué par nitro, halogéno, cyano, thiocyano, (C₁-C₆)-alkyle, halogéno- (C₁-C₆) -alkyle, (C₃-C₆) -cycloalkyle, R¹O(O)C, (R¹)₂N(O)C, R¹O, (R¹)₂N, R²(O)ₙS, R¹O(O)₂S, (R¹)₂N(O)₂S et R¹O-(C₁-C₆) -alkyle et hétérocyclyle portant n groupes oxo,
Z signifie hydrogène, nitro, halogéno, cyano, thiocyano, (C₁-C₆)-alkyle, halogéno- (C₁-C₆) -alkyle, (C₂-C₆) -alcényle, halogéno- (C₂-C₆) -alcényle, (C₂-C₆) -alcynyle, halogéno-(C₃-C₆)-alcynyle, (C₃-C₆)-cycloalkyle, halogéno- (C₃-C₆) -cycloalkyle, (C₃-C₆)-cycloalcényle, halogéno-(C₃-C₆)-cycloalcényle, (C₃-C₆)-cycloalkyl-(C₁-C₆)-alkyle, halogéno-(C₃-C₆)-cycloalkyl-(C₁-C₆)-alkyle, (C₃-C₆) -cycloalcényl- (C₁-C₆)-alkyle, halogéno-(C₃-C₆)-cycloalcényl-(C₁-C₆)-alkyle, R¹(O)C, R¹(R¹ON=)C, R¹O(O)C, (R¹)₂N(O)C, R¹(R¹O)N(O)C, (R¹)₂N(R¹)N (O)C, R¹(O)(R¹)N(O)C, R²O(O)C(R¹)N(O)C, (R¹)₂N(O)C(R¹)N(O)C, R² (0) ₂S (R¹) N (0) C, R¹O(O)₂S(R¹)N(O)C, (R¹)₂N(O)₂S(R¹)N(O)C, R¹O, R¹(O)CO, R²(O)₂SO, R²O(O)CO, (R¹)₂N(O)CO, (R¹)₂N, R¹(O)C(R¹)N, R² (0) ₂S (R¹) N, R²O(O)C(R¹)N, (R¹) ₂N (0) C (R¹) N, R¹O(O)₂S(R¹)N, (R¹)₂N(O)₂S(R¹)N, R₂(O)ₙS, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S, (R⁵O)₂(O)P, R¹ (O)C-(C₁-C₆)-alkyle, R¹O(O)C-(C₁-C₆)-alkyle, (R¹)₂N(O)C-(C₁-C₆)-alkyle, (R¹O)(R¹)N(O)C-(C₁-C₆)-alkyle, (R¹)₂N(R¹)N(O)C-(C₁-C₆) -alkyle, R¹(O)C(R¹)N(O)C-(C₁-C₆)-alkyle, R²O(O)C(R¹)N(O)C-(C₁-C₆)-alkyle, (R¹)₂N(O)C(R¹)N(O)C-(C₁-C₆)-alkyle, R²(O)₂S(R¹)N (0) C-(C₁-C₆)-alkyle, R¹O(O)₂S (R¹)N(O)C-(C₁-C₆)-alkyle, (R¹)₂N(O)₂S (R¹)N(O)C-(C₁-C₆)-alkyle, NC-(C₁-C₆)-alkyle, R¹O-(C₁-C₆)-alkyle, R¹(O)CO-(C₁-C₆)-alkyle, R²(O)₂SO-(C₁-C₆)-alkyle, R²O(O)CO-(C₁-C₆)-alkyle, (R¹)₂N(O)CO-(C₁-C₆)-alkyle, (R¹)₂N-(C₁-C₆)-alkyle, R¹(O)C(R¹)N-(C₁-C₆)-alkyle, R² (0) ₂S (R¹) N-(C₁-C₆)-alkyle, R²O(O)C(R¹)N-(C₁-C₆)-alkyle, (R¹)₂N(O)C(R¹)N-(C₁-C₆)-alkyle, R¹O(O)₂S(R¹)N-(C₁-C₆)-alkyle, (R¹)₂N(O)₂S(R¹)N-(C₁-C₆)-alkyle, R²(O)ₙS-(C₁-C₆)-alkyle, R¹O(O)₂S-(C₁-C₆)-alkyle, (R¹)₂N(O)₂S-(C₁-C₆)-alkyle, R¹(O)C(R¹)N(O)₂S-(C₁-C₆)-alkyle, R²O(O)C(R¹)N(O)₂S-(C₁-C₆)-alkyle, (R¹)₂N(O)C(R¹)N(O)₂S-(C₁-C₆)-alkyle, (R⁵O)₂(O)P-(C₁-C₆)-alkyle, phényle, hétéroaryle, hétérocyclyle, phényl-(C₁-C₆)-alkyle, hétéroaryl-(C₁-C₆)-alkyle, hétérocyclyl-(C₁-C₆)-alkyle, les six derniers radicaux mentionnés étant à chaque fois substitués par s radicaux du groupe constitué par nitro, halogéno, cyano, thiocyano, (C₁-C₆)-alkyle, halogéno-(C₁-C₆)-alkyle, (C₃-C₆) -cycloalkyle, R¹O(O)C, (R¹)₂N(O)C, R¹O, (R¹)₂N, R²(O)ₙS, R¹O(O)₂S, (R¹)₂N(O)₂S et R¹O-(C₁-C₆)-alkyle et hétérocyclyle portant n groupes oxo,
W signifie hydrogène, halogéno, nitro, cyano, thiocyano, (C₁-C₆)-alkyle, halogéno-(C₁-C₆) -alkyle, (C₂-C₆)-alcényle, halogéno-(C₂-C₆)-alcényle, (C₂-C₆)-alcynyle, halogéno-(C₃-C₆)-alcynyle, (C₃-C₇)-cycloalkyle, halogéno-(C₃-C₇)-cycloalkyle, (C₁-C₆)-alcoxy, halogéno-(C₁-C₆)-alcoxy, (C₁-C₆)-alkyl-(O)ₙS-, (C₁-C₆)-halogénoalkyl-(O)ₙS-, (C₁-C₆)-alcoxy- (C₁-C₄) -alkyle, (C₁-C₆)-alcoxy-(C₁-C₄)-halogénoalkyle, R¹(O)C, R¹(R¹ON=)C, R¹O(O)C, (R¹)₂N, R¹(O)C(R¹)N ou R²(O)₂S(R¹)N,
R signifie (C₁-C₆)-alkyle, (C₂-C₆)-alcényle ou (C₂-C₆)-alcynyle à chaque fois substitué par s radicaux du groupe constitué par nitro, halogéno, cyano, thiocyano, (C₃-C₆)-cycloalkyle, R¹(O)C, R¹(R¹ON=)C, R¹O(O)C, (R¹)₂N(O)C, R¹(R¹O)N(O)C, R²(O)₂S(R¹)N(O)C, R¹O(O)₂S(R¹)N(O)C, (R¹)₂N(O)₂S(R¹)N(O)C, R¹S(O)C, R¹O, R¹(O)CO, R²(O)₂SO, R²O(O)CO, (R¹)₂N(O)CO, (R¹)₂N, R¹O(R¹)N, R¹(O)C(R¹)N, R²(O)₂S(R¹)N.
R²O(O)C(R¹)N, (R¹)₂N(O)C(R¹)N, R¹O(O)₂S(R¹) N, (R¹)₂N(O)₂S(R¹)N, R²(O)ₙS, R¹C(O)S, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S et (R⁵O)₂(O)P ; ou (C₃-C₆)-cycloalkyle, (C₃-C₆)-cycloalcényle, phényle, phényl-(C₁-C₆)-alkyle, hétéroaryle, hétéroaryl-(C₁-C₆)-alkyle, hétérocyclyle, hétérocyclyl-(C₁-C₆)-alkyle, phényl-O-(C₁-C₆)-alkyle, hétéroaryl-O-(C₁-C₆)-alkyle, hétérocyclyl-O-(C₁-C₆)-alkyle, phényl-N(R¹)-(C₁-C₆)-alkyle, hétéroaryl-N(R¹)-(C₁-C₆)-alkyle, hétérocyclyl-N(R¹)-(C₁-C₆)-alkyle, phényl-S(O)ₙ-(C₁-C₆)-alkyle, hétéroaryl-S(O)ₙ-(C₁-C₆)-alkyle ou hétérocyclyl-S(O)ₙ-(C₁-C₆)-alkyle à chaque fois substitué dans la partie cyclique par s radicaux du groupe constitué par nitro, halogéno, cyano, thiocyano, (C₁-C₆)-alkyle, halogéno- (C₁-C₆)-alkyle, (C₃-C₆)-cycloalkyle, R¹(O)C, R¹(R¹ON=)C, R¹O(O)C, (R¹)₂N(O)C,
R¹(R¹O)N(O)C, R²(O)₂S(R¹)N(O)C, R¹O(O)₂S(R¹)N (0) C, (R¹)₂N(O)₂S(R¹)N(O)C, R¹S(O)C, R¹O, R¹(O)CO, R²(O)₂SO, R²O(O)CO, (R¹)₂N(O)CO, (R¹)₂N, R¹O (R¹) N, R¹(O)C(R¹)N, R²(O)₂S(R¹) N, R²O(O)C(R¹)N, (R¹)₂N(O)C(R¹)N, R¹O(O)₂S(R¹)N, (R¹)₂N(O)₂S(R¹)N, R²(O)ₙS, R¹C(O)S, R¹O(O)₂S, (R¹)₂N(O)₂S,
R¹(O)C(R¹)N(O)₂S, R²O(O)C(R¹)N(O)₂S, (R¹)₂N(O)C(R¹)N(O)₂S, (R⁵O)₂(O)P et R¹O-(C₁-C₆)-alkyle, et hétérocyclyle portant n groupes oxo,
R' signifie hydrogène, nitro, halogéno, cyano, (C₁-C₆)-alkyle, halogéno-(C₁-C₆) -alkyle, (C₃-C₆)-alcényle, halogéno-(C₃-C₆)-alcényle, (C₂-C₆)-alcynyle, halogéno-(C₃-C₆)-alcynyle, (C₃-C₆)-cycloalkyle, halogéno-(C₃-C₆)-cycloalkyle, (C₃-C₆)-cycloalkyl-(C₁-C₆)-alkyle, halogéno-(C₃-C₆)-cycloalkyl-(C₁-C₆)-alkyle, R¹(O)C, R²O(O)C, (R¹)₂N(O)C, R²S(O)C, (R¹)₂N(S)C, R¹(R¹O)N(O)C, R²(O)₂S(R¹)N(O)C, (R¹)₂N(O)₂S(R¹)N(O)C, R¹O, (R¹)₂N, R²(O)ₙS, (R²)₃Si-(C₁-C₆)-alkyl-(O)ₙS, R¹O(O)₂S, (R¹)₂N(O)₂S, R¹(O)C(R¹)N(O)₂S, R²O(O)C(R¹)N(O)₂S, (R¹)₂N(O)C(R¹)N(O)₂S, R²(O)₂S(R¹)N(O)₂S, (R⁵O)₂(O)P, (R²) ₃Si, R¹(O)C-(C₁-C₆)-alkyle, R¹O(O)C-(C₁-C₆)-alkyle, (R¹)₂N(O)C-(C₁-C₆)-alkyle, (R¹O)(R¹) N (O) C-(C₁-C₆)-alkyle, R²(O)₂S(R¹)N(O)C-(C₁-C₆)-alkyle, R¹O(O)₂S(R¹)N(O)C-(C₁-C₆)-alkyle,
(R¹)₂N(O)₂S(R¹)N(O)C-(C₁-C₆) -alkyle, R¹O-(C₁-C₆)-alkyle, R¹(O)CO-(C₁-C₆)-alkyle, R² (O)₂SO-(C₁-C₆)-alkyle, R²O(O)CO-(C₁-C₆)-alkyle, (R¹)₂N(O)CO-(C₁-C₆)-alkyle, (R¹)₂N-(C₁-C₆)-alkyle, R¹(O)C(R¹)N-(C₁-C₆)-alkyle, R²(O)₂S(R¹)N-(C₁-C₆) -alkyle, R²O(O)C(R¹)N-(C₁-C₆)-alkyle, (R¹)₂N(O)C(R¹)N-(C₁-C₆)-alkyle, R¹O(O)₂S(R¹)N-(C₁-C₆)-alkyle, (R¹)₂N(O)₂S(R¹)N-(C₁-C₆)-alkyle, R²(O)ₙS-(C₁-C₆)-alkyle, R¹O(O)₂S-(C₁-C₆)-alkyle, (R¹)₂N(O)₂S-(C₁-C₆)-alkyle, R¹(O)C(R¹)N(O)₂S-(C₁-C₆)-alkyle, R²O(O)C(R¹)N(O)₂S-(C₁-C₆) -alkyle, (R¹)₂N(O)C(R¹)N(O)₂S-(C₁-C₆) -alkyle, (R⁵O)₂(O)P-(C₁-C₆)-alkyle, (R²)₃Si-(C₁-C₆) -alkyle, phényle, hétéroaryle, hétérocyclyle, phényl-(C₁-C₆)-alkyle, hétéroaryl-(C₁-C₆)-alkyle, hétérocyclyl-(C₁-C₆)-alkyle, les six derniers radicaux phényle, hétéroaryle et hétérocyclyle susmentionnés étant à chaque fois substitués dans la partie cyclique par s radicaux du groupe constitué par nitro, halogéno, cyano, thiocyano, (C₁-C₆)-alkyle, halogéno-(C₁-C₆)-alkyle, (C₃-C₆) -cycloalkyle, R¹O(O)C, (R¹)₂N(O)C, R¹O, (R¹)₂N, R²(O)ₙS, R¹O(O)₂S, (R¹)₂N(O)₂S et R¹O-(C₁-C₆)-alkyle, et hétérocyclyle portant n groupes oxo,
R¹ signifie hydrogène, (C₁-C₆)-alkyle, halogéno-(C₁-C₆)-alkyle, (C₂-C₆)-alcényle, halogéno-(C₂-C₆)-alcényle, (C₂-C₆)-alcynyle, halogéno-(C₃-C₆)-alcynyle, (C₃-C₆)-cycloalkyle, (C₃-C₆)-cycloalcényle, halogéno-(C₃-C₆)-cycloalkyle, (C₃-C₆) -cycloalkyl-(C₁-C₆)-alkyle, (C₁-C₆)-alkyl-O-(C₁-C₆)-alkyle, cycloalkyl-(C₁-C₆)-alkyl-O-(C₁-C₆) alkyle, phényle, phényl-(C₁-C₆)-alkyle, hétéroaryle, hétéroaryl-(C₁-C₆)-alkyle, hétérocyclyle, hétérocyclyl-(C₁-C₆)-alkyle, phényl-O-(C₁-C₆)-alkyle, hétéroaryl-O-(C₁-C₆)-alkyle, hétérocyclyl-O-(C₁-C₆)-alkyle, phényl-N(R³)-(C₁-C₆) alkyle, hétéroaryl-N(R³)-(C₁-C₆)-alkyle, hétérocyclyl-N(R³)-(C₁-C₆)-alkyle, phényl-S(O)ₙ-(C₁-C₆)-alkyle, hétéroaryl-S(O)ₙ-(C₁-C₆)-alkyle, hétérocyclyl-S(O)ₙ-(C₁-C₆)-alkyle, les quinze derniers radicaux mentionnés étant à chaque fois substitués par s radicaux du groupe constitué par nitro, halogéno, cyano, thiocyano, (C₁-C₆)-alkyle, halogéno- (C₁-C₆) -alkyle, (C₃-C₆) -cycloalkyle, R³O(O)C, (R³)₂N(O)C, R³O, (R³)₂N, R⁴(O)ₙS, R³O(O)₂S, (R³)₂N(O)₂S et R³O-(C₁-C₆)-alkyle ; et hétérocyclyle portant n groupes oxo,
R² signifie (C₁-C₆)-alkyle, halogéno- (C₁-C₆) -alkyle, (C₂-C₆)-alcényle, halogéno-(C₂-C₆)-alcényle, (C₂-C₆)-alcynyle, halogéno-(C₃-C₆)-alcynyle, (C₃-C₆)-cycloalkyle, (C₃-C₆)-cycloalcényle, halogéno-(C₃-C₆)-cycloalkyle, (C₃-C₆) -cycloalkyl- (C₁-C₆) -alkyle, (C₁-C₆) -alkyl-0- (C₁-C₆) -alkyle, cycloalkyl- (C₁-C₆)-alkyl-O-(C₁-C₆)-alkyle, phényle, phényl-(C₁-C₆)-alkyle, hétéroaryle, hétéroaryl-(C₁-C₆)-alkyle, hétérocyclyle, hétérocyclyl-(C₁-C₆)-alkyle, phényl-O-(C₁-C₆)-alkyle, hétéroaryl-O-(C₁-C₆)-alkyle, hétérocyclyl-O-(C₁-C₆)-alkyle, phényl-N(R³)-(C₁-C₆)-alkyle, hétéroaryl-N(R³)-(C₁-C₆)-alkyle, hétérocyclyl-N(R³)-(C₁-C₆)-alkyle, phényl-S(O)ₙ-(C₁-C₆)-alkyle, hétéroaryl-S(O)ₙ-(C₁-C₆)-alkyle, hétérocyclyl-S(O)ₙ-(C₁-C₆)-alkyle, les quinze derniers radicaux mentionnés étant à chaque fois substitués par s radicaux du groupe constitué par nitro, halogéno, cyano, thiocyano, (C₁-C₆)-alkyle, halogéno-(C₁-C₆)-alkyle, (C₃-C₆)-cycloalkyle, R³O(O)C, (R³)₂N(O)C, R³O, (R³)₂N, R⁴(O)ₙS, R³O(O)₂S, (R³)₂N(O)₂S et R³O-(C₁-C₆)-alkyle ; et hétérocyclyle portant n groupes oxo,
R³ signifie hydrogène, (C₁-C₆)-alkyle, halogéno-(C₁-C₆)-alkyle, (C₂-C₆) -alcényle, (C₂-C₆) -alcynyle, (C₃-C₆)-cycloalkyle, (C₃-C₆) -cycloalkyl-(C₁-C₆)-alkyle ou phényle,
R⁴ signifie (C₁-C₆)-alkyle, halogéno-(C₁-C₆)-alkyle, (C₂-C₆) -alcényle, (C₂-C₆) -alcynyle, (C₃-C₆)-cycloalkyle, (C₃-C₆)-cycloalkyl-(C₁-C₆)-alkyle ou phényle,
R⁵ signifie hydrogène ou (C₁-C₄)-alkyle,
R⁶ signifie (C₁-C₄)-alkyle ; ou phényle substitué par m radicaux du groupe constitué par halogéno, nitro, cyano, (C₁-C₄)-alkyle, (C₁-C₄)-halogénoalkyle, (C₁-C₄)-alcoxy et (C₁-C₄)-halogénoalcoxy,
R⁷ signifie hydrogène, (C₁-C₄)-alkyle ou (C₁-C₄)-alcoxy,
R⁸ signifie hydrogène ou (C₁-C₄)-alkyle, ou
R⁷ et R⁸ forment ensemble avec l'atome d'azote auquel ils sont liés, un cycle de 5 ou 6 chaînons, saturé, partiellement saturé ou insaturé, qui contient en outre n hétéroatomes du groupe constitué par azote, oxygène et soufre et qui est substitué par m radicaux du groupe constitué par halogéno, cyano, (C₁-C₄)-alkyle, (C₁-C₄)-halogénoalkyle, (C₁-C₄)-alcoxy et (C₁-C₄)-halogénoalcoxy,
R⁹ signifie halogéno, (C₁-C₄)-alkyle, (C₁-C₄)-halogénoalkyle, (C₁-C₄)-alcoxy, (C₁-C₄)-halogénoalcoxy ou (C₁-C₄)-alcoxy-(C₁-C₄)-alkyle,
m signifie 0, 1, 2, 3, 4 ou 5,
n signifie 0, 1 ou 2,
s signifie 0, 1, 2 ou 3,
t signifie 0 ou 1.

2. Dérivés de sulfinimidoyle et de sulfonimidoylbenzoyle selon la revendication 1, dans lesquels
Q signifie un radical Q1, Q2, Q3, Q4 ou Q5, 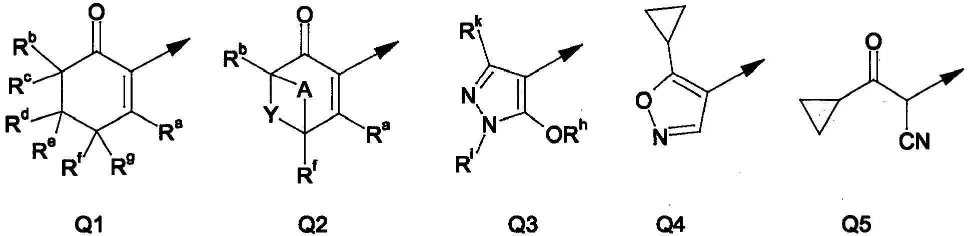
R^{a} signifie hydroxy,
R^{b}, R^{c}, R^{f} et R^{g} signifient, indépendamment les uns des autres, à chaque fois hydrogène ou (C₁-C₄)-alkyle,
R^{d}, R^{e} signifient, indépendamment l'un de l'autre, à chaque fois hydrogène ou (C₁-C₄)-alkyle ou forment ensemble avec l'atome de carbone auquel ils sont liés un groupe carbonyle,
R^{h} signifie hydrogène,
Rⁱ signifie (C₁-C₄)-alkyle,
R^{k} signifie hydrogène, (C₁-C₄)-alkyle, (C₃-C₇)-cycloalkyle,
A et Y signifient, indépendamment l'un de l'autre, à chaque fois oxygène ou (C₁-C₄)-alkylène substitué par n radicaux R⁹,
X signifie nitro, halogéno, cyano, (C₁-C₆)-alkyle, halogéno-(C₁-C₆)-alkyle, (C₂-C₆)-alcényle, (C₂-C₆)-alcynyle, (C₃-C₆)-cycloalkyle, halogéno-(C₃-C₆)-cycloalkyle, (C₃-C₆) -cycloalkyl- (C₁-C₆)-alkyle, halogéno-(C₃-C₆)-cycloalkyl-(C₁-C₆)-alkyle, R¹(O)C, R¹(R¹ON=)C, R¹O(O)C, (R¹)₂N(O)C, R¹O, (R¹)₂N, R¹(O)C(R¹)N, R²(O)₂S(R¹)N, R²O(O)C(R¹)N, (R¹)₂N(O)C(R¹)N, R²(O)ₙS, R¹O(O)₂S, (R¹)₂N(O)₂S, (R⁵O)₂(O)P, R¹(O)C-(C₁-C₆)-alkyle, R¹O(O)C-(C₁-C₆)-alkyle, (R¹)₂N(O)C-(C₁-C₆) -alkyle, NC-(C₁-C₆)-alkyle, R¹O-(C₁-C₆)-alkyle, (R¹)₂N-(C₁-C₆)-alkyle, R¹(O)C(R¹)N- (C₁-C₆) -alkyle, R²(O)₂S(R¹)N-(C₁-C₆)-alkyle, R²O(O)C(R¹)N-(C₁-C₆)-alkyle, (R¹)₂N(O)C(R¹)N-(C₁-C₆)-alkyle, R²(O)ₙS-(C₁-C₆) -alkyle, R¹O(O)₂S-(C₁-C₆)-alkyle, (R¹)₂N(O)₂S- (C₁-C₆) -alkyle, (R⁵O)₂(O)P-(C₁-C₆)-alkyle, phényle, hétéroaryle, hétérocyclyle, phényl-(C₁-C₆)-alkyle, hétéroaryl-(C₁-C₆)-alkyle, hétérocyclyl-(C₁-C₆)-alkyle, les six derniers radicaux mentionnés étant à chaque fois substitués par s radicaux du groupe constitué par nitro, halogéno, cyano, thiocyano, (C₁-C₆)-alkyle, halogéno-(C₁-C₆)-alkyle, R¹O, (R¹)₂N, R²(O)ₙS, R¹O(O)₂S, (R¹)₂N(O)₂S et R¹O-(C₁-C₆)-alkyle ; et hétérocyclyle portant n groupes oxo,
Z signifie hydrogène, nitro, halogéno, cyano, (C₁-C₆) -alkyle, halogéno-(C₁-C₆)-alkyle, (C₂-C₆)-alcényle, (C₂-C₆) -alcynyle, (C₃-C₆) -cycloalkyle, halogéno- (C₃-C₆) -cycloalkyle, (C₃-C₆) -cycloalkyl-(C₁-C₆)-alkyle, halogéno-(C₃-C₆)-cycloalkyl-(C₁-C₆) alkyle, R¹(O)C, R¹(R¹ON=)C, R¹O(O)C, (R¹)₂N(O)C, R¹O, (R¹)₂N, R¹(O)C(R¹)N, R²(O)₂S(R¹)N, R²O(O)C(R¹)N, (R¹)₂N(O)C(R¹)N, R²(O)ₙS, R¹O(O)₂S, (R¹)₂N(O)₂S, (R⁵O)₂(O)P, R¹(O)C-(C₁-C₆)-alkyle, R¹O(O)C-(C₁-C₆)-alkyle, (R¹)₂N(O)C-(C₁-C₆)-alkyle, NC-(C₁-C₆)-alkyle, R¹O-(C₁-C₆)-alkyle, (R¹)₂N-(C₁-C₆)-alkyle, R¹ (O)C(R¹)N-(C₁-C₆) -alkyle, R²(O)₂S(R¹)N-(C₁-C₆)-alkyle, R²O(O)C(R¹)N-(C₁-C₆)-alkyle, (R¹)₂N(O)C(R¹)N-(C₁-C₆) -alkyle, R²(O)ₙS-(C₁-C₆)-alkyle, R¹O(O)₂S-(C₁-C₆)-alkyle, (R¹)₂N(O)₂S-(C₁-C₆)-alkyle, (R⁵O)₂(O)P-(C₁-C₆)-alkyle, phényle, hétéroaryle, hétérocyclyle, phényl-(C₁-C₆)-alkyle, hétéroaryl-(C₁-C₆)-alkyle, hétérocyclyl-(C₁-C₆)-alkyle, les six derniers radicaux mentionnés étant à chaque fois substitués par s radicaux du groupe constitué par nitro, halogéno, cyano, thiocyano, (C₁-C₆)-alkyle, halogéno-(C₁-C₆)-alkyle, R¹O, (R¹)₂N, R²(O)ₙS, R¹O(O)₂S, (R¹)₂N(O)₂S et R¹O-(C₁-C₆)-alkyle ; et hétérocyclyle portant n groupes oxo,
W signifie hydrogène, halogéno, nitro, cyano, (C₁-C₆) -alkyle, halogéno-(C₁-C₆)-alkyle, (C₃-C₇)-cycloalkyle, (C₁-C₆)-alcoxy, (C₁-C₆)-alkyl-(O)ₙS-, R¹O(O)C, (R¹)₂N, R¹(O)C(R¹)N ou R²(O)₂S(R¹)N,
R signifie (C₁-C₆)-alkyle à chaque fois substitué par s radicaux du groupe halogéno, cyano, (C₃-C₆)-cycloalkyle, R¹(O)C, R¹(R¹ON=)C, R¹O(O)C, (R¹)₂N(O)C, R²(O)₂S(R¹)N(O)C, R¹O, (R¹)₂N, R¹(O)C(R¹)N, R²(O)₂S(R¹)N, R²O(O)C(R¹)N, (R¹)₂N(O)C(R¹)N, R²(O)ₙS, R¹O(O)₂S, (R¹)₂N(O)₂S, R¹(O)C(R¹)N(O)₂S, R²O(O)C(R¹)N(O)₂S et (R¹)₂N(O)C(R¹)N(O)₂S ; ou (C₃-C₆)-cycloalkyle à chaque fois substitué par s radicaux du groupe constitué par halogéno, (C₁-C₆)-alkyle, halogéno-(C₁-C₆)-alkyle, (C₃-C₆)-cycloalkyle, R¹O(O)C et (R¹)₂N(O)C,
R' signifie hydrogène, nitro, cyano, (C₁-C₆)-alkyle, halogéno- (C₁-C₆) -alkyle, (C₃-C₆) -cycloalkyle, halogéno- (C₃-C₆) -cycloalkyle, (C₃-C₆) -cycloalkyl-(C₁-C₆)-alkyle, halogéno- (C₃-C₆) -cycloalkyl- (C₁-C₆)-alkyle, R¹(O)C, R²O(O)C, (R¹)₂N(O)C, R²(O)₂S, R¹(O)C-(C₁-C₆)-alkyle, R¹O(O)C-(C₁-C₆)-alkyle, (R¹)₂N(O)C-(C₁-C₆)-alkyle, R¹O-(C₁-C₆)-alkyle, (R¹)₂N-(C₁-C₆)-alkyle ou R²(O)ₙS-(C₁-C₆)-alkyle,
R¹ signifie hydrogène, (C₁-C₆)-alkyle, halogéno-(C₁-C₆)-alkyle, (C₃-C₆)-cycloalkyle, halogéno-(C₃-C₆)-cycloalkyle, (C₃-C₆) -cycloalkyl- (C₁-C₆) -alkyle, (C₁-C₆) -alkyl-0- (C₁-C₆) -alkyle, cycloalkyl- (C₁-C₆)-alkyl-O-(C₁-C₆)-alkyle, phényle, phényl-(C₁-C₆)-alkyle, hétéroaryle, hétéroaryl-(C₁-C₆)-alkyle, hétérocyclyle, hétérocyclyl-(C₁-C₆)-alkyle, phényl-0- (C₁-C₆) -alkyle, hétéroaryl-O- (C₁-C₆) -alkyle, hétérocyclyl-O-(C₁-C₆)-alkyle, les neuf derniers radicaux mentionnés étant à chaque fois substitués par s radicaux du groupe constitué par nitro, halogéno, (C₁-C₆)-alkyle, halogéno- (C₁-C₆) -alkyle, R³O(O)C, (R³)₂N(O)C, R³O, (R³)₂N, R⁴(O)ₙS et R³O-(C₁-C₆)-alkyle ; et hétérocyclyle portant n groupes oxo,
R² signifie (C₁-C₆)-alkyle, halogéno-(C₁-C₆)-alkyle, (C₃-C₆) -cycloalkyle, halogéno- (C₃-C₆) -cycloalkyle, (C₃-C₆) -cycloalkyl- (C₁-C₆) -alkyle, (C₁-C₆) -alkyl-0-(C₁-C₆)-alkyle, cycloalkyl-(C₁-C₆)-alkyl-O-(C₁-C₆)-alkyle, phényle, phényl-(C₁-C₆)-alkyle, hétéroaryle, hétéroaryl-(C₁-C₆)-alkyle, hétérocyclyle, hétérocyclyl-(C₁-C₆)-alkyle, phényl-0- (C₁-C₆) -alkyle, hétéroaryl-O- (C₁-C₆) -alkyle, hétérocyclyl-O-(C₁-C₆)-alkyle, les neuf derniers radicaux mentionnés étant à chaque fois substitués par s radicaux du groupe constitué par nitro, halogéno, (C₁-C₆)-alkyle, halogéno-(C₁-C₆)-alkyle, R³O(O)C, (R³)₂N(O)C, R³O, (R³)₂N, R⁴(O)ₙS et R³O-(C₁-C₆)-alkyle ; et hétérocyclyle portant n groupes oxo,
R³ signifie hydrogène ou (C₁-C₆)-alkyle,
R⁴ signifie (C₁-C₆)-alkyle,
R⁵ signifie hydrogène ou (C₁-C₄)-alkyle,
R⁹ signifie halogéno, (C₁-C₄)-alkyle, (C₁-C₄)-halogénoalkyle, (C₁-C₄)-alcoxy, (C₁-C₄)-halogénoalcoxy ou (C₁-C₉)-alcoxy-(C₁-C₄)-alkyle,
n signifie 0, 1 ou 2,
s signifie 0, 1, 2 ou 3,
t signifie 0 ou 1.

3. Dérivés de sulfinimidoyle et de sulfonimidoylbenzoyle selon la revendication 1 ou 2, dans lesquels
Q signifie un radical Q1, Q2, Q3, Q4 ou Q5, 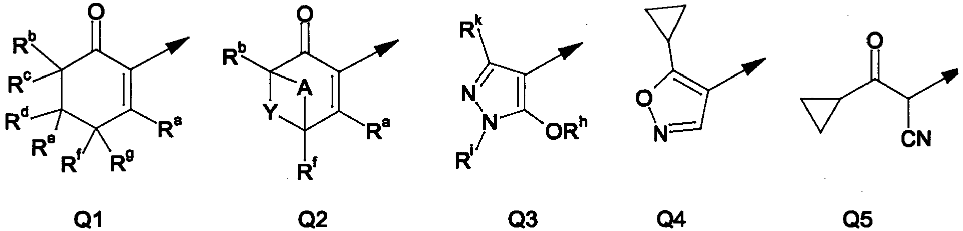
R^{a} signifie hydroxy,
R^{b}, R^{c}, R^{f} et R^{g} signifient, indépendamment l'un de l'autre, à chaque fois hydrogène ou méthyle ;
R^{d}, R^{e} signifient hydrogène ou forment ensemble avec l'atome de carbone auquel ils sont liés un groupe carbonyle,
R^{h} signifie hydrogène,
Rⁱ signifie méthyle ou éthyle,
R^{k} signifie hydrogène, méthyle, éthyle ou cyclopropyle,
A et Y signifient, indépendamment l'un de l'autre, à chaque fois CH₂ ou CH₂CH₂ ;
X signifie nitro, halogéno, méthyle, éthyle, n-propyle, iso-propyle, trifluorométhyle, difluorométhyle, chlorodifluorométhyle, dichlorofluorométhyle, trichlorométhyle, pentafluoroéthyle, heptafluoro-isopropyle, cyclopropyle, méthoxy, éthoxy, méthylsulfanyle, méthylsulfinyle, méthylsulfonyle, méthoxyméthyle, éthoxyméthyle, méthoxyéthyle, méthoxyéthoxyméthyle, méthylthiométhyle, méthylsulfinylméthyle ou méthylsulfonylméthyle,
Z signifie hydrogène, nitro, cyano, halogéno, méthyle, éthyle, n-propyle, iso-propyle, trifluorométhyle, difluorométhyle, chlorodifluorométhyle, dichlorofluorométhyle, trichlorométhyle, pentafluoroéthyle, heptafluoro-isopropyle, cyclopropyle, méthoxy, éthoxy, méthylsulfanyle, méthylsulfinyle ou méthylsulfonyle,
W signifie hydrogène, chlore ou méthyle,
R signifie méthyle, éthyle ou n-propyle,
R' signifie hydrogène ou cyano,
t signifie 0 ou 1.

4. Agents herbicides, **caractérisés par** une teneur active en tant qu'herbicide en au moins un composé de formule (I) selon l'une quelconque des revendications 1 à 3.

5. Agents herbicides selon la revendication 4 en mélange avec des adjuvants de formulation.

6. Agents herbicides selon la revendication 4 ou 5 contenant au moins une autre substance active en tant que pesticide du groupe formé par les insecticides, les acaricides, les herbicides, les fongicides, les antidotes et les régulateurs de croissance.

7. Agents herbicides selon la revendication 6 contenant un antidote.

8. Agents herbicides selon la revendication 7, contenant du cyprosulfamide, du cloquintocet-mexyl, du méfenpyr-diéthyl ou de l'isoxadifen-éthyl.

9. Agents herbicides selon l'une quelconque des revendications 6 à 8 contenant un autre herbicide.

10. Procédé pour lutter contre des plantes non souhaitées, **caractérisé en ce qu'**on applique une quantité active d'au moins un composé de formule (I) selon l'une quelconque des revendications 1 à 3 ou d'un agent herbicide selon l'une quelconque des revendications 4 à 9 sur les plantes ou sur le lieu de la croissance des plantes non souhaitées.

11. Utilisation de composés de formule (I) selon l'une quelconque des revendications 1 à 3 ou d'agents herbicides selon l'une quelconque des revendications 4 à 9 pour lutter contre des plantes non souhaitées.

12. Utilisation selon la revendication 11, **caractérisée en ce que** les composés de formule (I) sont utilisés pour lutter contre des plantes non souhaitées dans les cultures de plantes utiles.

13. Utilisation selon la revendication 12, **caractérisée en ce que** les plantes utiles sont des plantes utiles transgéniques.

14. Composés de formule (II) 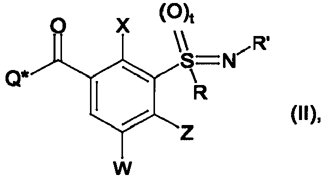 dans laquelle Q* signifie hydroxy, éthoxy, méthoxy ou chlore et R, R', X, W, Z et t sont définis comme dans l'une quelconque des revendications 1 à 3.

15. Utilisation des composés de formule (II) selon la revendication 14 pour la préparation de composés de formule (I) selon l'une quelconque des revendications 1 à 3.
